(19) Europäisches Patentamt
European Patent Office
Office européen des brevets

(11) EP 3 349 777 B1

(12) **EUROPEAN PATENT SPECIFICATION**

(45) Date of publication and mention of the grant of the patent:
**07.05.2025 Bulletin 2025/19**

(21) Application number: **16770462.6**

(22) Date of filing: **15.09.2016**

(51) International Patent Classification (IPC):
***A61K 38/05*** (2006.01) ***A61K 38/07*** (2006.01)
***A61K 38/08*** (2019.01) ***A61K 38/10*** (2006.01)
***A61K 38/12*** (2006.01) ***A61K 38/18*** (2006.01)
***A61P 35/00*** (2006.01) ***A61P 43/00*** (2006.01)
***G16B 15/30*** (2019.01) ***A61K 47/00*** (2006.01)
***C12N 5/00*** (2006.01)

(52) Cooperative Patent Classification (CPC):
**A61K 38/18; A61K 38/05; A61K 38/07; A61K 38/08; A61K 38/10; A61K 38/12; A61K 47/59; A61K 47/62; A61K 47/6903; A61K 47/6923; A61P 35/00; A61P 43/00; C07K 14/4738; C07K 14/475; C12N 5/0693;**
(Cont.)

(86) International application number:
**PCT/EP2016/071785**

(87) International publication number:
**WO 2017/046219 (23.03.2017 Gazette 2017/12)**

(54) **PHARMACEUTICAL ASSOCIATION COMPRISING A GROWTH FACTOR RECEPTOR AGONIST CONJUGATED TO A BIOACTIVE CARRIERFOR CONVERTING A NEOPLASTIC CELL INTO A NON-NEOPLASTIC CELL AND USES THEREOF**

PHARMAZEUTISCHE ASSOZIATION MIT EINEM AN EINEN BIOAKTIVEN TRÄGER KONJUGIERTEN WACHSTUMSFAKTORREZEPTORAGONISTEN ZUR UMWANDLUNG EINER NEOPLASTISCHEN ZELLE IN EINE NICHT-NEOPLASTISCHE ZELLE UND DEREN VERWENDUNGEN

ASSOCIATION PHARMACEUTIQUE POUR CONVERTIR UNE CELLULE NÉOPLASIQUE EN CELLULE NON-NÉOPLASIQUE ET SES UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.09.2015 US 201562219716 P**

(43) Date of publication of application:
**25.07.2018 Bulletin 2018/30**

(60) Divisional application:
**24217572.7 / 4 524 235**

(73) Proprietor: **Histide AG**
**8834 Schindellegi (CH)**

(72) Inventors:
- **ZOUANI, Omar F.**
**8834 Schindellegi (CH)**
- **GOCHEVA, Veronika**
**8834 Schindellegi (CH)**

(74) Representative: **RVDB Paris**
**27, avenue de l'Opéra**
**75001 Paris (FR)**

(56) References cited:
**WO-A1-91/18098**
**WO-A2-2007/010394**
**US-A1- 2003 185 792**
**US-A1- 2004 023 322**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **KIRSCH T ET AL: "BMP-2 ANTAGONISTS EMERGE FROM ALTERATIONS IN THE LOW-AFFINITY BINDING EPITOPE FOR RECEPTOR BMPR-II", THE EMBO JOURNAL, EUROPEAN MOLECULAR BIOLOGY ORGANIZATION, vol. 19, no. 13, 1 July 2000 (2000-07-01), pages 3314 - 3324, XP001022147, ISSN: 0261-4189, DOI: 10.1093/EMBOJ/19.13.3314**
- **ZOUANI O F ET AL: "Differentiation of pre-osteoblast cells on poly(ethylene terephthalate) grafted with RGD and/or BMPs mimetic peptides", BIOMATERIALS, ELSEVIER SCIENCE PUBLISHERS BV., BARKING, GB, vol. 31, no. 32, 1 November 2010 (2010-11-01), pages 8245 - 8253, XP027259529, ISSN: 0142-9612, [retrieved on 20100725]**
- **GEIGER M ET AL: "Collagen sponges for bone regeneration with rhBMP-2", ADVANCED DRUG DELIVERY REVIEWS, ELSEVIER, AMSTERDAM, NL, vol. 55, no. 12, 28 November 2003 (2003-11-28), pages 1613 - 1629, XP002764796, ISSN: 0169-409X**
- **IDE HISAMITSU ET AL: "Growth regulation of human prostate cancer cells by bone morphogenetic protein-2", CANCER RESEARCH, AMERICAN ASSOCIATION FOR CANCER RESEARCH, US, vol. 57, no. 22, 15 November 1997 (1997-11-15), pages 5022 - 5027, XP002441978, ISSN: 0008-5472**
- **LIAO ANYAN ET AL: "Bone morphogenetic protein 2 mediates epithelial-mesenchymal transition via AKT and ERK signaling pathways in gastric cancer", TUMOR BIOLOGY, KARGER, BASEL, CH, vol. 36, no. 4, 3 December 2014 (2014-12-03), pages 2773 - 2778, XP036092591, ISSN: 1010-4283, [retrieved on 20141203], DOI: 10.1007/S13277-014-2901-1**
- **W. J. PETTY ET AL: "Epidermal Growth Factor Receptor Tyrosine Kinase Inhibition Represses Cyclin D1 in Aerodigestive Tract Cancers", CLINICAL CANCER RESEARCH, vol. 10, no. 22, 15 November 2004 (2004-11-15), pages 7547 - 7554, XP055188457, ISSN: 1078-0432, DOI: 10.1158/1078-0432.CCR-04-1169**
- **PARK HYUNJIN ET AL: "Preventing MEK1 activation influences the responses of human osteosarcoma cells to bone morphogenetic proteins 2 and 9", ANTI-CANCER DRUGS, vol. 24, no. 3, March 2013 (2013-03-01), pages 278 - 290, XP009192664**
- **ULRIKE MAEGDEFRAU ET AL: "BMP activated Smad signaling strongly promotes migration and invasion of hepatocellular carcinoma cells", EXPERIMENTAL AND MOLECULAR PATHOLOGY, vol. 92, no. 1, 15 October 2011 (2011-10-15), pages 74 - 81, XP028464119, ISSN: 0014-4800, [retrieved on 20111015], DOI: 10.1016/J.YEXMP.2011.10.004**


Remarks:

•The complete document including Reference Table(s) and the Sequence Listing(s) can be downloaded from the EPO website

•The file contains technical information submitted after the application was filed and not included in this specification

(52) Cooperative Patent Classification (CPC): (Cont.)
**G16B 15/00; G16B 15/30**

**Description**

## FIELD OF THE INVENTION

The invention relates to associations, combinations, compositions, kits, methods and processes for the design, preparation, manufacture and/or formulation thereof, and methods and uses thereof for converting or recoding a neoplastic cell into a non-neoplastic cell and treating and/or preventing a neoplastic disease.

## BACKGROUND

Neoplastic cells, such as cancer cells, are generally characterized by abnormal and/or uncontrolled proliferation leading, in most cases, to the development of a neoplastic disease, such as cancer. Conventional methods for treating neoplastic diseases include surgical treatments, radiotherapy and chemotherapy.

Surgery is usually practised in order to extract localised (non-circulating) neoplastic cells from a patient's body and is most generally combined with radiotherapy treatments. As well as being limited to the treatment of very early stage, non-metastatic tumors, surgery is an invasive medical procedure which remains traumatic for the treated patient, involves the permanent removal of tissues or organs (which is sometimes not possible as some organs or organ parts are not accessible or cannot be removed due to life threatening consequences), in some cases has been shown to "unblock" dormant tumors, while only offering a "visual" selectivity to distinguish between healthy and tumor cells. Radiotherapy also presents some significant drawbacks for the treated patients including the high cost of the radiation therapy equipment, the high cost of the treatment itself, side effects associated with the damage or destruction of healthy cells, limited effectiveness against metastasized neoplastic diseases, skin rashes caused by external beam radiation, the potential deleterious impact on the functioning of tissues, glands or organs located near the site of treatment, and the possible development of secondary cancers as a result of exposure to the radiations.

Conventional chemotherapy methods generally involve the administration of small synthetic regulatory molecules which inhibit specific intracellular target proteins thought to be responsible for the neoplastic phenotype of the cell. One example is the inhibition of tyrosine kinase signal transduction by small molecule inhibitors to regulate uncontrolled cell proliferation. Typical chemotherapy methods also include treatments wherein DNA is covalently altered by *e.g.* DNA strands crosslinking, or treatments wherein the polymerisation and depolymerisation of microtubules is enhanced prevented thus provoking apoptosis of the damaged cell.

Another method for treating neoplastic diseases includes gene therapy wherein a missing or defective gene is replaced with a functional, healthy copy, which is delivered to the target dysfunctional cells using a "vector." Gene transfer therapy can be done outside the body (ex vivo) by extracting bone marrow or blood from the patient and growing the cells in a laboratory. The corrected copy of the gene is then introduced and allowed to penetrate the cells' DNA before being injected back into the body. Gene transfers can also be done directly inside the patient's body (in vivo). Gene therapy has been applied to a few specific cases of blood cancers (chronic lymphocytic leukemia (CLL), acute lymphocytic leukemia (ALL) and multiple myeloma) through a particular form thereof in which the genetically modified cells were not the neoplastic cells themselves but instead the immune T-cells. Modified T-cells could then target and destroy specific blood cells (neoplastic as well as healthy). The body of the patient is then able to produce healthy blood cells and eventually provide a treatment to certain blood cancer types. However, gene therapy is generally best suited for the treatment of diseases caused by a single defective gene, not neoplastic diseases, which often involve multiple defective genes. Overall, issues such as the correct integration of therapeutic DNA into the genome; the immune system response to the introduction of foreign DNA into the cell; the toxicity, immune and inflammatory responses; gene control and targeting issues of the vectors (usually viral) required to transport the DNA inside the cell; the difficulties associated with the treatment of multigene-associated neoplastic cells; the possibilities of inducing tumors if the DNA is integrated in the wrong place in the genome; and/or the significant cost usually involved with such a therapy, have greatly undermined the development of gene therapy.

Other intracellular therapies have been contemplated for the treatment of neoplastic diseases using, for instance, micro-ribonucleic acids (miRNAs) or small interfering ribonucleic acids (siRNA). Under these conditions, the neoplastic cell is generally forced to down-regulate or repress the expression of one or more specific target genes (*e.g.* oncogenes) thus inhibiting the expression of defective and/or defecting proteins (*e.g.* oncoproteins). One example is the repression of genes encoding key proteins in the proliferation of cancer cells such as vascular endothelial growth factor (VEGF) and kinesin spindle protein (KSP), thus controlling cancer proliferation.

Neoplastic diseases may also be treated using immunotherapy such as antibody therapies wherein the antibodies bind to a target antigen typically on the surface of the neoplastic cell. Cell surface receptors are common targets for antibody therapies and include the epidermal growth factor receptor, HER2, CD52, the vascular endothelial growth factor-A and CD30. Once bound to an antigen (*e.g.* a cancer antigen), antibodies can induce antibody-dependent cell-mediated cytotoxicity, activate the complement system, prevent a receptor interacting with its ligand or deliver a

payload of chemotherapy or radiation, which may all lead to the induction of neoplastic cell death. For instance, Cetuximab (Erbitux) is a chimeric IgG1 monoclonal antibody that targets the extracellular domain of the epidermal growth factor receptor (EGFR). Once a ligand binds to the EGFR on the surface of the cell, signalling pathways are activated inside the cell that are associated with malignant characteristics such as cancer cell proliferation and invasion. Cetuximab competitively inhibits ligand binding, thereby preventing EGFR activation and subsequent cellular signalling. It also activates programmed cell death (apoptosis).

Other intracellular treatments such as messenger ribonucleic acids (mRNAs) -based therapies have also been used in the treatment of neoplastic disease wherein administration of mRNA material into a neoplastic cell causes the neoplastic cell *e.g.* to express specifically encoded antigens and causing the neoplastic cell to be eliminated by the host immune system.

Differentiation therapy is another technique which was developed on the concept that the acquisition of the malignant phenotype (such as neoplasia) in a cell is considered as a progressive de-differentiation or a defective differentiation of that cell. Thus, as e.g. tumor cell populations evolve to greater degrees of malignancy, they usually lose more and more differentiation markers. This led to the suggestion that it may be possible to treat cancer by inducing differentiation of cancer cells. However, some scientific reports have shown that the differentiation therapy does not in fact induce cancer cells differentiation but instead restrains their growth thus allowing the application of more conventional therapies (such as chemotherapy) to eradicate the malignant cells. Examples of differentiation therapy involve the forced (re-)expression of some specific micro-RNAs and thus rely on an intracellular action generally presenting the same drawbacks as in any other intracellular therapies such the siRNA and gene therapies.

A shortcoming of the medical therapies relying on previously reported methods of treatment are numerous and mainly resides in the incapacity of providing a sustainable therapeutic effect *i.e.* the treated cells are not healed but instead are either destroyed (*e.g.* through induced apoptosis) or their proliferation reduced or temporarily halted using a sustained administration of therapeutic molecules until, in most case scenarios, neoplastic cells are able to adapt themselves and render the therapy ineffective. Interrupting a known therapy will usually lead to resumption of the neoplastic cell state.

Other drawbacks associated with previously reported therapies are numerous. For example, they may be very invasive and traumatic for the patient; they may necessitate permanently removing neoplastic tissues or organs which may be in some cases not practicable or feasible (organs or organ parts not accessible) or not possible due to life-threatening consequences; they may not be applied to or have limited effectiveness against metastatic neoplastic cells; they may not possess the ability to remove or treat all neoplastic cell types (*i.e.* neoplastic cells having different invasiveness levels and/or of different lineage origins); they may potentially "unblock" dormant tumors; they are often expensive; they may damage or destroy healthy cells alongside neoplastic cells thereby causing adverse treatment side effects; they may cause skin rashes and skin sensitivity; they may not target cancer stem cells as these are not proliferating; they may have a mutagenic action even towards healthy cells; they may require sustained administration to maintain treatment therapeutic effects; they may display very high cytotoxicity; they may cause multidrug resistance whereby a drug having an intracellular action is no longer imported inside the cancer cell or is systematically exported outside of the cell.

One may also cite, as scientific literature which may be considered as background art of the present invention: WO 2007/010394 A2; US 2004/023322 A1; US 2003/185792 A1; Zouani et al. Biomaterials, vol. 31, no.32, 8245-8253; Geiger et al. Advanced Drug Delivery Reviews, vol. 55, no.12, 1613-1629; Ide et al. Cancer Research, vol. 57, no. 22, 5022-5027; Liao et al., Tumor Biology, vol. 36, no. 4, 2773-2778; Petty et al., vol. 10, no. 22, 7547-7554; Park et al., Anti-Cancer Drugs, vol. 24, no. 3, 278-290; Maegdefrau et al., vol. 92, no. 1, 74-81; and WO 91/18098 A1.

None of these previously known methods allows for the effective recoding of a neoplastic cell thus permitting conversion of a neoplastic cell into a non-neoplastic cell.

The present invention thus provides associations, combinations, compositions, kits, methods and processes for the design, preparation, manufacture and/or formulation thereof, and methods and uses thereof for converting a neoplastic cell into a non-neoplastic cell including converting or recoding the neoplastic cell to induce, provide and/or reintroduce self-recovery or self-healing capabilities thereto.

## BRIEF DESCRIPTION OF THE DRAWINGS

FIG. 1 is a representation of a fluorescence intensity of non-covalent pharmaceutical associations comprising GFR-binding compounds and type-I collagen or apatite ceramics bioactive carriers. The images represent surfaces non-covalently coated with GFR-binding compounds labeled with FITC.

FIG. 2 is (a) a representation of a fluorescence intensity of pharmaceutical associations comprising various GFR-binding compounds and type-I collagen immediately after association or 3, 7 and 10 days after association. The images represent surfaces non-covalently coated with GFR-binding compounds labeled with FITC, (b) a total fluorescence intensity quantification of GFR-binding compounds labeled with FITC associated with apatite ceramics

after incubation in cell culture medium for the given indicated times (up to 10 days).

FIG. 3 is (a) a representation of a Quantitative Real Time PCR analysis of the expression of DMP-1, Sclerostin and RANK-L in neoplastic cells (osteosarcoma cells) cultured on a native bioactive carrier (control) and on a bioactive carrier covalently associated with various GFR-binding compounds after 24 hours of culture, ($P<0.001$), (b) a 3D representation of a neoplastic cell cultured on a native bioactive carrier (left-hand side) and on a bioactive carrier covalently associated with a GFR-binding compound (right-hand side).

FIG. 4 is a representation of Quantitative Real Time PCR analysis of the expression of MLC-1, GATA-4 and α-Sarcomeric actin in neoplastic cells (Rabdomiosarcoma cells) cultured on a native bioactive carrier (control) and on a bioactive carrier covalently associated with various GFR-binding compounds after 24 hours of culture, ($P<0.005$).

FIG. 5 is a representation of Quantitative Real Time PCR analysis of the expression of Sox-9, IBSP and Collagen-IV in neoplastic cells (chondrosarcoma cells) cultured on a native bioactive carrier (control) and on a bioactive carrier covalently associated with various GFR-binding compounds after 24 hours of culture, ($P<0.001$).

FIG. 6 is a representation of Quantitative Real Time PCR analysis of the expression of MMP-9, Vimentin and α-SMA in neoplastic cells (adenocarcinoma cells) cultured on a native bioactive carrier (control) and on a bioactive carrier covalently associated with various GFR-binding compounds after 24 hours of culture, ($P<0.005$).

FIG. 7 is (a) a diagram representing a relative quantification from western blot of the amount of p53 protein present in neoplastic cells (osteosarcoma cells) cultured on a native bioactive carrier (control) and on a bioactive carrier covalently associated with various GFR-binding compounds after 24 hours of culture, (b) a diagram representing a relative quantification from western blot of the extent of phosphorylation of the pRB protein present in neoplastic cells cultured on a native bioactive carrier (control) and on a bioactive carrier covalently associated with various GFR-binding compounds after 24 hours of culture.

FIG. 8 is diagrams representing a relative quantification from western blot of the extent of phosphorylation of the ERK protein (a) and of the Src kinase (b) present in neoplastic cells (osteosarcoma cells) cultured on a native bioactive carrier (control) and on a bioactive carrier covalently associated with various GFR-binding compounds after 24 hours of culture, (c) a diagram representing a relative quantification from western blot of the amount of PDK1 protein present in neoplastic cells cultured on a native bioactive carrier (control) and on a bioactive carrier covalently associated with various GFR-binding compounds after 24 hours of culture.

FIG. 9 is a representation of a Quantitative Real Time PCR analysis of the expression of Paxillin (a) and Vinculin (b) in neoplastic cells (osteosarcoma cells) cultured on a native bioactive carrier (control) and on a bioactive carrier covalently associated with various GFR-binding compounds after 24 hours of culture, ($P<0.001$).

FIG. 10 is a representation of a Quantitative Real Time PCR analysis of the expression of Cyclin D (average gene expression of Cyclin D1, D2 and D3) at different time intervals during 24 hours in neoplastic cells (osteosarcoma cells) cultured on a native bioactive carrier (control) and on a bioactive carrier covalently associated with various GFR-binding compounds.

FIG. 11 is a diagram representing the grafting or association density of various radiolabeled GFR-binding compounds covalently associated with a bioactive carrier using radioactivity quantification.

FIG. 12 is (a) a representation of a Quantitative Real Time PCR analysis of the expression of DMP-1, SCT and RANK-L in neoplastic cells (osteosarcoma cells) cultured on a native polymer scaffold (control) and on a polymer scaffold grafted with a mixture comprising a GFR-binding compound as defined in the present disclosure and a RGD peptide, after 24 hours of culture, (b) immunofluorescence staining of a representative neoplastic cell (osteosarcoma cells) cultured on a polymer scaffold grafted with a mixture comprising a GFR-binding compound as defined in the present disclosure (ID SEQ NO: 2) and a RGD peptide, after 24 hours of culture. The actin filaments were immunostained by using Alexa 488-phalloidin.

The focal adhesions were represented by immunostaining with anti-vinculin. The nucleus was stained with DAPI and is represented by a circle in the center of the cell. Scale bar: 5 μm. (c) is a diagram representing the grafting density of a radiolabeled GFR-binding compound as defined in the present disclosure covalently grafted on a polymer scaffold and of a radiolabeled GFR-binding compound and a non-labeled RGD peptide both grafted on a polymer scaffold. The measurements were performed by using radioactivity quantification (no significant difference was observed). (d) Western Blot analysis of the expression of integrin subunits (α and β) in neoplastic cells cultured on a non-modified polymer scaffold,

after 24h of culture. (e) Immunofluorescence staining of a representative neoplastic cell (osteosarcoma cells) cultured in presence of different integrin subunits (α and β) couples (anti-α3β1, anti-αvβ3 and anti-α5β3).

FIG. 13 is (a) Western blot analysis of the expression integrin α3 and β1 before and after transduction with two independent integrin ∟3 and β1 shRNAs, which efficiently reduced endogenous integrin α3β1 protein levels. (b) Immunofluorescence visualization of a neoplastic cell (osteosarcoma cells) after silencing integrins α3β1 by using shRNAs, after 24 hours of culture on a polymer scaffold covalently grafted with RGD peptides. The silencing of these specific integrin proteins were shown to significantly reduce or suppress neoplastic cell adhesion on the RGD grafted polymers. The actin filaments were immunostained by using Alexa 488-phalloidin. The focal adhesions were

represented by immunostaining with anti-vinculin. The nucleus was stained with DAPI and is represented by a circle in the center of the cell. Scale bar: 5 μm. (c) Immunofluorescence visualization of neoplastic cells cultured on a polymer scaffold covalently modified with a mixture comprising a GFR-binding compound as defined herein (SEQ ID NO: 2) and a RGD peptide, after 24 hours of culture. Scale bar: 500 μm. A magnification of one region (right, Scale bar: 50 μm) indicates that Spheroid-like structures of neoplastic cells become predominant in this case as a result of the RGD induced integrin engagement. The actin filaments were immunostained by using Alexa 488-phalloidin. The focal adhesions were represented by immunostaining with anti-vinculin. The nucleus was stained with DAPI and is represented by a circle in the center of the cell.

FIG. 14 is a screen shot of the Standard Protein Blast online software used in the RMSD calculation procedure.

## DETAILED DESCRIPTION

Neoplastic diseases start when a cell (or neoplastic cell) is somehow altered so that it multiplies out of control. Tumors and cancers are some examples of neoplastic diseases. A tumor is a mass composed of a cluster of such abnormal cells. Most cancers form tumors, but not all tumors are cancerous. Benign, or non-cancerous, tumors - such as freckles and moles - stop growing, do not spread to other parts of the body, and do not create new tumors. Malignant, or cancerous, tumors crowd out healthy cells, interfere with body functions, and draw nutrients from body tissues. Cancers continue to grow and spread by direct extension or through a process called metastasis, whereby the malignant cells travel through the lymphatic or blood vessels, eventually forming new tumors in other parts of the body.

The term "cancer" generally encompasses more than one hundred diseases affecting nearly every part of the body, and all are potentially life threatening. The major types of cancer are carcinoma, sarcoma, melanoma, lymphoma, and leukemia.

Carcinoma is a type of cancer that develops from epithelial cells. Specifically, a carcinoma is a cancer that begins in a tissue that lines the inner or outer surfaces of the body, and that generally arises from cells originating in the endodermal or ectodermal germ layer during embryogenesis.

Sarcoma is a cancer that arises from transformed cells of mesenchymal origin. Thus, malignant tumors made of cancerous bone, cartilage, fat, muscle, vascular or hematopoietic tissues are, by definition, considered sarcomas. This is in contrast to a malignant tumor originating from epithelial cells, which are termed carcinoma. Human sarcomas are quite rare. Common malignancies, such as breast, colon, and lung cancer, are almost always carcinoma.

Melanoma is a type of skin cancer, which forms from melanocytes (pigment-containing cells in the skin).

Lymphoma is a group of blood cell tumors that develop from lymphocytes. It is sometimes used to refer to just the cancerous ones rather than all tumors. There are two main types: Hodgkin lymphoma (HL) and non-Hodgkin lymphoma (NHL), with two others, multiple myeloma and immunoproliferative diseases, also included by the World Health Organization within the category. Non-Hodgkin lymphoma makes up about 90% of cases and includes a large number of sub-types. Lymphomas are part of the broader group of neoplasms called tumors of the hematopoietic and lymphoid tissues.

Leukemia is a group of cancers that usually begins in the bone marrow and results in high numbers of abnormal white blood cells. These white blood cells are not fully developed and are called blasts or leukemia cells. Symptoms may include bleeding and bruising problems, feeling very tired, and an increased risk of infections. These symptoms occur due to a lack of normal blood cells. Diagnosis is typically by blood tests or bone marrow biopsy.

Cancers include, but are not limited to, Adrenal Cancer, Anal Cancer, Bile Duct Cancer, Bladder Cancer, Bone Cancer, Brain/CNS Tumors In Adults, Brain/CNS Tumors In Children, Breast Cancer, Breast Cancer In Men, Cancer in Adolescents, Cancer in Children, Cancer in Young Adults, Cancer of Unknown Primary, Castleman Disease, Cervical Cancer, Colon/Rectum Cancer, Endometrial Cancer, Esophagus Cancer, Ewing Family Of Tumors, Eye Cancer, Gallbladder Cancer, Gastrointestinal Carcinoid Tumors, Gastrointestinal Stromal Tumor (GIST), Gestational Trophoblastic Disease, Hodgkin Disease, Kaposi Sarcoma, Kidney Cancer, Laryngeal and Hypopharyngeal Cancer, Leukemia, Leukemia - Acute Lymphocytic (ALL) in Adults, Leukemia - Acute Myeloid (AML), Leukemia - Chronic Lymphocytic (CLL), Leukemia - Chronic Myeloid (CML), Leukemia - Chronic Myelomonocytic (CMML), Leukemia in Children, Liver Cancer, Lung Cancer, Lung Cancer - Non-Small Cell, Lung Cancer - Small Cell, Lung, Carcinoid Tumor, Lymphoma, Lymphoma of the Skin, Malignant Mesothelioma, Multiple Myeloma, Myelodysplastic Syndrome, Nasal Cavity and Paranasal Sinus Cancer, Nasopharyngeal Cancer, Neuroblastoma, Non-Hodgkin Lymphoma, Non-Hodgkin Lymphoma In Children, Oral Cavity and Oropharyngeal Cancer, Osteosarcoma, Ovarian Cancer, Pancreatic Cancer, Penile Cancer, Pituitary Tumors, Prostate Cancer, Retinoblastoma, Rhabdomyosarcoma, Salivary Gland Cancer, Sarcoma - Adult Soft Tissue Cancer, Skin Cancer, Skin Cancer - Basal and Squamous Cell, Skin Cancer - Melanoma, Skin Cancer - Merkel Cell, Small Intestine Cancer, Stomach Cancer, Testicular Cancer, Thymus Cancer, Thyroid Cancer, Uterine Sarcoma, Vaginal Cancer, Vulvar Cancer, Waldenstrom Macroglobulinemia, Wilms Tumor.

The activation of growth factor receptors (GFR) is commonly known to promote neoplastic cell proliferation. For instance, many cancer treatments rely on the inhibition of growth factors, growth factors receptors and/or downstream

signalling proteins such as protein kinases. Such inhibitors include, non-exhaustively, anti-Met (*e.g.* ARQ-197), anti-VEGF (*e.g.* Bevacizumab), anti-VEGFR (*e.g.* Sunitinib or Semaxinib), anti-Her2 (*e.g.* Trastuzumab), anti-EGFR (*e.g.* Cetuximab, Gefitinib or Erlotinib), anti-PDGFR (*e.g.* Imatinib), anti-IGF-1 (*e.g.* IMC-A12), anti-Ras (*e.g.* Tipifarnib), anti-Raf (*e.g.* Sorafenib), anti-src (*e.g.* Dastinib or Saracatinib), anti-Mek (*e.g.* C1040 or PD-0325901), anti-PI3K (*e.g.* LY294002), anti-PDK (*e.g.* UNC01), anti-HSP90 (*e.g.* 17-AGG or IPI-504), anti-CDK (*e.g.* Flavopiridol) and anti-mTOR (*e.g.* Everolimus).

Unlike previously reported treatments, it has been surprisingly demonstrated that the pharmaceutical associations, combinations and compositions disclosed herein can be used to treat, prevent and/or diagnose neoplasms (*e.g.* tumors or cancers) by activating growth factor receptors of vertebrate cells (such as mammalian cells, especially human cells).

The present invention also aims at providing mechanisms for solving and/or avoiding at least one of, preferably a plurality of, the problems, issues and/or shortcomings associated with previously reported neoplasm treatment therapies.

The present disclosure thus provides embodiments for:

- Converting or recoding a neoplastic cell to induce and/or promote and/or improve self-healing and/or self-recovery thereof, particularly within a shorter period of time
- Converting or recoding a neoplastic cell to induce and/or promote and/or improve self-healing and/or self-recovery thereof, particularly with a higher conversion yield;
- Restoring a neoplastic cell's ability to undergo conversion into a physiologically functional and/or healthy cell, particularly within a shorter period of time;
- Restoring a neoplastic cell's ability to undergo differentiation, particularly within a shorter period of time;
- Converting and/or recoding a circulating or non-circulating neoplastic cell such as a cancer cell, into a non-neoplastic cell, particularly within a shorter period of time;
- Protecting a subject from a neoplastic disease, disorder, condition, or pathology such as cancer, or any symptoms thereof, particularly within a shorter period of time;
- Providing and/or producing a physiologically functional and/or healthy cell including an osteocyte and/or a chondrocyte and/or an adipocyte from a neoplastic cell, particularly within a shorter period of time;
- Re-establishing, restoring and/or reactivating a cell adhesion checkpoint of a neoplastic cell;
- Inducing and/or promoting and/or enhancing neoplastic cell differentiation, particularly within a shorter period of time;
- Inducing and/or promoting and/or enhancing a decrease in Cyclin D gene and protein expression;
- Inducing and/or promoting and/or enhancing a G1 to G0 cell cycle phase transition;
- Inducing and/or promoting and/or enhancing the inactivation of a protein complex between a Cyclin D and at least one of Cyclin-dependent kinase (CDK) 4 or 6;
- Preventing or reducing the phosphorylation of the retinoblastoma (Rb) protein thus activating its tumour suppressor functions;
- Increasing the phosphorylation of the p53 protein thus reducing its degradation and activating its tumor suppressor functions;
- Preventing or reducing the activation of the Ras/MAP kinase signalling cascade;
- Identifying and/or analysing the features and/or markers of a neoplastic cell;
- Regulating and/or activating growth factor receptors of a neoplastic cell;
- Modulating and/or regulating the adhesion between a cell (in particular a neoplastic cell) and its micro-environment *i.e.* the surrounding extracellular matrix;
- Treating, recoding or converting a neoplastic cell without temporarily or permanently damaging or killing said neoplastic cell and/or without permanently inducing a quiescent state thereof;
- Dampening and/or reducing or suppressing cell division and/or cell proliferation of a neoplastic cell;
- Activating and/or promoting and/or regulating anti-mitogen activity and/or tumor suppressor pathways in a neoplastic cell;
- Inhibiting and/or reducing anti-oncogenic activity in a neoplastic cell;
- Converting or recoding a neoplastic cell to induce and/or promote and/or improve self-healing and/or self-recovery thereof, and/or protecting a subject from a neoplastic disease, disorder, condition or pathology such as cancer using extracellular means;
- Converting or recoding a neoplastic cell to induce and/or promote and/or improve self-healing and/or self-recovery thereof, protecting a subject from a neoplastic disease, disorder, condition or pathology such as cancer without modifying the genome of said cell;
- Providing diagnostic tools for diagnosing neoplastic diseases in a subject.

## I. Definitions

Those skilled in the art will recognize, or be able to ascertain using no more than routine experimentation, many

equivalents to the specific embodiments in accordance with the invention described herein. The scope of the present invention is not intended to be limited to the present description, but rather is as set forth in the appended claims.

In the claims, articles such as "a", "an", and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

It is also noted that the term "comprising" is intended to be open and permits but does not require the inclusion of additional elements or steps. When the term "comprising" is used herein, the terms "consisting of", "consisting essentially of", "consisting substantially of" and "consisting exclusively of" are thus also encompassed and disclosed.

As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise indicated, self-evident or contradictory in context (*e.g.* except where such number would exceed 100% of a possible value).

As used herein and unless otherwise indicated or contradictory in context, the term "with" followed by a specific number of amino acids, when used to define a particular peptide, variant or analog thereof, such as in "a peptide with three amino acids", means that such peptide, variant or analog thereof, contains exclusively the specific number of amino acids specified after this term.

As used herein and unless otherwise indicated or contradictory in context, the term "Ci-alkyl" is intended to specifically and individually disclose any branched or unbranched radical, moiety or functional group having "i" carbon atom(s).

The carbon atom content of the various hydrocarbon-containing moieties herein may be indicated by a prefix designating the minimum and maximum number of carbon atoms in the moiety. For example, in certain embodiments, (Ca-Cb)alkyl indicates an alkyl moiety of the integer "a" to the integer "b" carbon atoms, inclusive.

At various places in the present specification, substituents of compounds of the present disclosure may be disclosed in groups or in ranges. It is specifically intended that the present disclosure include each and every individual sub-combination of the members of such groups and ranges. For example, in certain embodiments, the term "C1-C5 alkyl" is an abbreviation for (and thus is specifically intended to individually disclose) C1-alkyl (*i.e.* methyl), C2-alkyl (*i.e.* ethyl), C3-alkyl (*i.e.* 1-propyl and 2-propyl), C4-alkyl (*i.e.* 1-butyl, *sec*-butyl, *iso*-butyl and *tert*-butyl), and C5-alkyl (*i.e.* 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl and 1,1-dimethyl-1-propyl).

As used herein, unless indicated otherwise or contradictory in context, the terms "alkyl" and "(Ca-Cb)alkyl" refer to monovalent hydrocarbon radicals containing the requisite number of carbon atoms as described above, having straight or branched moieties or combinations thereof. As used herein, alkyl groups may be optionally substituted with between one to four substitutes. Non-limiting examples of alkyl groups include, *e.g.* methyl, ethyl, n-propyl, isopropyl, n-butyl, isobutyl, sec-butyl, t-butyl, etc. Of course, other alkyl groups will be readily apparent to those of skilled in the art given the benefit of the present disclosure.

Where ranges are given, endpoints are included. Furthermore, it is to be understood that unless otherwise indicated or otherwise evident from the context and understanding of one of ordinary skill in the art, values that are expressed as ranges can assume any specific value or sub-range within the stated ranges in different embodiments of the invention, to the tenth of the unit of the lower limit of the range, unless the context clearly dictates otherwise. For example, in certain embodiments, a disclosed 0-10 range would, for example, in certain embodiments, also specifically and individually disclose the following values and ranges: 0, 1, 2, 3, 4, 5, 6, 7, 8, 9, 10, 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 0-1, 0-2, 0-3, 0-4, 0-5, 0-6, 0-7, 0-8, 0-9, 1-2, 1-3, 1-4, 1-5, 1-6, 1-7, 1-8, 1-9, 2-3, 2-4, 2-5, 2-6, 2-7, 2-8, 2-9, 2-10, 3-4, 3-5, 3-6, 3-7, 3-8, 3-9, 3-10, 4-5, 4-6, 4-7, 4-8, 4-9, 4-10, 5-6, 5-7, 5-8, 5-9, 5-10, 6-7, 6-8, 6-9, 6-10, 7-8, 7-9, 7-10, 8-9, 8-10, 9-10, 0-0.1, 0-0.2, 0-0.3, 0-0.4, 0-0.5, 0-0.6, 0-0.7, 0-0.8, 0-0.9, 0-1.1, 0-1.2, etc.

As used herein and unless otherwise indicated or contradictory in context, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

In addition, it is to be understood that any particular embodiment of the present invention that falls within the prior

art may be explicitly excluded from any one or more of the claims using the appropriate disclaimer(s) or proviso(s). Since such embodiments are deemed to be known to one of ordinary skill in the art, they may be excluded even if the exclusion is not set forth explicitly herein. Any particular embodiment of the compositions of the invention (e.g., any peptide or peptidomimetic; any method of production; any method of use; etc.) can be excluded from any one or more claims, for any reason, whether or not related to the existence of prior art. In case of conflicting statements of a cited source and the instant application, the statement in the instant application shall control.

As the case may be, and unless otherwise indicated or contradictory in context, macromolecules molecular weights should be understood in the present description as being number averaged molecular weights.

The peptides mentioned in the present description may not follow the usual representation conventions.

For instance, the N-terminal amino acid of a peptide sequence may be the first amino acid in the sequence or the last amino acid. Likewise, the C-terminal amino acid of a peptide sequence may be the first amino acid in the sequence or the last amino acid. For example, in the peptide sequence NAIS, "N" may be N-terminal or C-terminal, and "S" may be N-terminal or C-terminal. Consequently, for the purpose of the present disclosure, e.g. NAIS also covers SIAN, SAIS also covers SIAS, SPIN also covers NIPS, etc.

In the present application, when reference is made to a certain peptide *(e.g.* a GFR-binding compound as provided herein) comprising one or more other peptide(s), said one or more other peptide(s) is(are) understood to be stably (in most cases, covalently) attached/bound to at least one part of said peptide. The attachment/binding may be located anywhere on the peptide unless indicated otherwise, contradictory in context or contradictory to general scientific rules. No specific attachment/binding location of said one or more other peptide(s) to said peptide shall be assumed unless specifically mentioned.

**Peptide or polypeptide:** As used herein, the term "peptide" or "polypeptide" are used interchangeably and refers to a polymer of less than or equal to 100 amino acids long, e.g., about 2, 3, 4, 5, 10, 15, 20, 25, 30, 35, 40, 45, 50, 55, 60, 65, 70, 75, 80, 85, 90, 95 or 100 amino acids long. The terms apply to amino acid polymers in which one or more amino acid residue is an artificial chemical mimetic of a corresponding naturally occurring amino acid, as well as to naturally occurring amino acid polymers, non-naturally occurring amino acid polymers, peptide analogs, peptide variants and peptide mimetics. Conventional techniques for synthesising peptides involve the activation of the carboxylic acid function of an amino acid or of a peptide, using a coupling agent. This activated acid is then contacted with an amino acid or a peptide in which the N-terminal amino acid is not protected, thus forming an amide bond also called peptide bond. Coupling reaction conditions together with coupling agents are well known in the art and described, for instance, in Greene, "Protective Groups in Organic Synthesis", Wiley, New York, 2007 4th edition. In addition, suitable peptide synthesis routes are described, for instance, in Hojo H., Recent progress in the chemical synthesis of proteins, Curr Opin Struct Biol. 2014; 26C:16-23 and Saranya Chandrudu, et al., Chemical Methods for Peptide and Protein Production, Molecules, 2013, 18, 4373-4388, There are two main strategies for peptide synthesis i.e. liquid-phase peptide synthesis and solid-phase peptide synthesis (SPPS) which is now most commonly used for peptide synthesis. Instead of C-terminal protection with a chemical group, the C-terminus of the first amino acid is coupled to an activated solid support, such as polystyrene or polyacrylamide. This type of approach has a two-fold function: the resin acts as the C-terminal protecting group and provides a rapid method to separate the growing peptide product from the different reaction mixtures during synthesis. As with many different biological manufacturing processes, peptide synthesizers have been developed for automation and high-throughput peptide production. SPPS allows the synthesis of natural peptides which are difficult to express in bacteria, the incorporation of unnatural amino acids, peptide/protein backbone modification, and the synthesis of D-proteins, which consist of D-amino acids. Very long peptide can be accessed by using native chemical ligation to couple two peptides together with quantitative yields.

**Peptide analogs:** As used herein, unless indicated otherwise or contradictory in context, the term "peptide analogs" refers to polypeptide variants which differ by one or more amino acid alterations, e.g., substitutions, additions or deletions of amino acid residues that still maintain one or more of the properties of the parent or starting peptide.

**Peptide variants:** As used herein, unless indicated otherwise or contradictory in context, the term "peptide variants" refers to a peptide which has a certain identity with a native or reference compound sequence. In one example, the peptide variant refers to any post- administration, application, injection modified peptide. Such post- administration, application, injection modifications include, but are not limited to, phosphorylation, acetylation, glutamylation, tyrosination, palmitoylation, glycosylation, myristoylation, palmitoylation, isoprenylation, glypiation, lipoylation, phosphopantetheinylation, acylation, alkylation, amidation, arginylation, polyglutamylation, polyglycylation, butyrylation, gamma-carboxylation, glycosylation, polysialylation, malonylation, hydroxylation, iodination, nucleotide addition, oxidation, adenylylation, propionylation, pyroglutamate formation, S-glutathionylation, S-nitrosylation, succinylation, sulfation, glycation, biotinylation, pegylation, ISGylation, SUMOylation, ubiquitination, Neddylation, Pupylation, citrullination, deamidation, eliminylation, carbamylation, and racemization.

**Peptido-mimetic:** As used herein, unless indicated otherwise or contradictory in context, the term "peptido-mimetic" or "peptidomimetic" refers to a synthetic chemical compound which comprises amino acids but not only and that is able to mimic the biological action of a peptide, often because the mimetic has a basic structure that mimics the basic

structure of the peptide and/or has the salient biological properties of that peptide. In one particular example, a peptidomimetic is a hybrid molecule containing both, at least one peptide, and at least one of a polysaccharide, a polynucleotide or a linear or branched, saturated or unsaturated, hydrocarbon chain.

**Linear peptide:** As used herein, unless indicated otherwise or contradictory in context, the term "linear peptide" means a peptide in which the C-terminal and the N-terminal amino acid residues do not covalently interact with each other and none of the C-terminal or the N-terminal amino acid residues covalently interacts with another amino acid residue of the peptide chain.

**Cyclic peptide:** As used herein, unless indicated otherwise or contradictory in context, the term "cyclic peptide" means peptide in which the C-terminal and N-terminal amino acid residues do covalently interact with each other or the C-terminal and/or the N-terminal amino acid residues covalently interact with at least one other amino acid residue of the peptide chain so as to form a ring-like structure.

**Amino acid:** As used herein, unless indicated otherwise or contradictory in context, the term "amino acid" refers to naturally occurring and non-naturally occurring amino acids including amino acid analogs. Naturally occurring amino acids are those encoded by the genetic code, as well as those amino acids that are later modified, e.g., hydroxyproline, [gamma]-carboxyglutamate, and O-phosphoserine. Naturally encoded amino acids are the 20 common amino acids glycine (Gly, G), alanine (Ala, A), valine (Val, V), leucine (Leu, L), isoleucine (Ile, I), serine (Ser, S), threonine (Thr, T), phenylalanine (Phe, F), tyrosine (Tyr, Y), tryptophane (Trp, W), cysteine (Cys, C), methionine (Met, M), proline (Pro, P), aspartic acid (Asp, D), asparagine (Asn, N), glutamine (Gln, Q), glutamic acid (Glu, E), histidine (His, H), arginine (Arg, R) et lysine (Lys, K) and pyrrolysine and selenocysteine. Non-naturally occurring amino acids include, but are not limited to, the dextrogyre (D) isomers of the above-cited naturally-occurring amino acids. Amino acid analogs refers to compounds that have the same basic chemical structure as a naturally occurring amino acid *i.e.,* an [alpha] carbon that is bound to a hydrogen, a carboxyl group, an amino group, and an R group (*i.e.* side chain), and which may be used in replacement thereof without substantially affecting the overall function of the peptide to which it belongs. Amino acid analogs (or non-naturally occurring amino acids) that may be suitable for implementing embodiments of the present invention include, but are not limited to, amino acids comprising a photoactivatable cross-linker, spin-labeled amino acids, fluorescent amino acids, metal binding amino acids, metal-containing amino acids, radioactive amino acids, amino acids with novel functional groups, amino acids that covalently or noncovalently interact with other molecules, photocaged and/or photoisomerizable amino acids, amino acids comprising biotin or a biotin analogue, glycosylated amino acids such as a sugar substituted serine, other carbohydrate modified amino acids, keto-containing amino acids, amino acids comprising polyethylene glycol or polyether, heavy atom substituted amino acids, chemically cleavable and/or photocleavable amino acids, amino acids with an elongated side chains as compared to natural amino acids, including but not limited to, polyethers or long chain hydrocarbons, including but not limited to, greater than about 5 or greater than about 10 carbons, carbon-linked sugar-containing amino acids, redox-active amino acids, amino thioacid containing amino acids, and amino acids comprising one or more toxic moiety. The term "**$AA^{I}$**" (AA roman numeral one) may be used in the description and refers to an amino acid which may be any amino acid as defined above in particular any naturally occurring and non-naturally occurring amino acids.

**Amino acid side chain:** As used herein, unless indicated otherwise or contradictory in context, the term "amino acid side chain" means the functional group of an amino acid that differentiates it from other amino acids. All amino acid structures have a carboxyl group, an amine group and a specific side chain.

**$AA^{II}$** (AA roman numeral two): As used herein, unless indicated otherwise or contradictory in context, the terms "polar amino acid" or "$AA^{II}$" means amino acids having a polar, non-charged group-containing side chain. Polar amino acids are protonated at physiological pH (about 7). Examples of polar amino acids include, but are not limited to, Cys (C), Asn (N), Gln (Q), Ser (S), Thr (T), or Tyr (Y).

**$AA^{III}$** (AA roman numeral three): As used herein, unless indicated otherwise or contradictory in context, the terms "acidic amino acid" or "$AA^{III}$" means amino acids having an acidic group-containing side chain. Acidic amino acid deprotonated forms predominate at physiological pH (about 7). Examples of acidic amino acids include, but are not limited to, Asn (N) and Glu (E).

**$AA^{IV}$** (AA roman numeral four): As used herein, unless indicated otherwise or contradictory in context, the terms "aliphatic amino acid" or "$AA^{IV}$" means amino acids having an aliphatic side chain. Examples of aliphatic amino acids include, but are not limited to, Ala (A), Leu (L), Ile (I), Gly (G), Val (V) and any analogs and derivatives thereof.

**$AA^{V}$** (AA roman numeral five): As used herein, unless indicated otherwise or contradictory in context, the terms "apolar amino acid" or "$AA^{V}$" means amino acids having an apolar side chain. Examples of apolar amino acids include, but are not limited to, Ala (A), Phe (F), Gly (G), Ile (I), Leu (L), Met (M), Pro (P), Val (V) or Trp (W).

**$AA^{VI}$** (AA roman numeral six): As used herein, unless indicated otherwise or contradictory in context, the term "aromatic amino acid" or "$AA^{VI}$" means amino acids having an aromatic group-containing side chain. Examples of aromatic amino acids include, but are not limited to, Trp (W), Tyr (Y) or Phe (F).

**$AA^{VII}$** (AA roman numeral seven): As used herein, unless indicated otherwise or contradictory in context, the term "basic amino acid" or "$AA^{VII}$" means amino acids having a basic group-containing side chain. Basic amino acid protonated

forms predominate at physiological pH (about 7). Examples of basic amino acids include, but are not limited to, Arg (R), His (H), or Lys (K).

**$AA^{VIII}$** (AA roman numeral eight): As used herein, unless indicated otherwise or contradictory in context, the term "$AA^{VIII}$" means Leu (L) or Ile (I) and any analogs and derivatives thereof.

**$AA^{IX}$** (AA roman numeral nine): As used herein, unless indicated otherwise or contradictory in context, the term "charged amino acid" or "$AA^{IX}$" means amino acids having either an acidic group-containing side chain or an basic group-containing side chain. Charged amino acid charged forms predominate at physiological pH (about 7). Examples of charged amino acids include, but are not limited to, Asn (N), Glu (E), His (H), Lys (K) or Arg (R).

**$AA^n$:** As used herein, unless indicated otherwise or contradictory in context, the term "$AA^n$", in which n is a positive integer arbitrarily chosen to identify a specific position within the primary sequence of a peptide.

For instance, $AA^{13}$ means the amino acid of position 13. The terms "amino acid" and "AA" are interchangeably used in the present description.

**N-terminal:** As used herein, unless indicated otherwise or contradictory in context, the term "N-terminal" means the amine ($-NH_2$) function/group/moiety located at one (terminal) end of a protein or polypeptide. This functional group is the only amine group which is not engage in n amide peptide bond.

**C-terminal:** As used herein, unless indicated otherwise or contradictory in context, the term "C-terminal" means the carboxylate ($-CO_2H$) function/group/moiety located at one (terminal) end of a protein or polypeptide. This functional group is the only carboxylic acid group which is not engage in n amide peptide bond.

**Naturally-occurring peptide:** As used herein, unless indicated otherwise or contradictory in context, the terms "naturally-occurring peptide" or "natural peptide" means a peptide which may be found in nature without human direct intervention (except for its extraction and/or isolation).

**Synthetic peptide:** As used herein, unless indicated otherwise or contradictory in context, the terms "synthetic peptide" or "non-natural peptide" means a peptide which may not be found in nature without human direct intervention (except for its extraction and/or isolation). For example, in certain embodiments, a synthetic peptide may have the amino acid sequence of a natural peptide except for at least one amino acid deletion or substitution relative to the natural sequence. In the case of a substitution, an amino acid from the natural sequence is replaced by another, different, naturally-occurring or non-naturally occurring amino acid. For example, in certain embodiments, a synthetic peptide may not possess a post-translational modification of the natural peptide such as the attachment of an acetate group, a phosphate group, a lipid, a carbohydrate, or the formation of a disulfide bridge.

**Covalent interaction:** As used herein, unless indicated otherwise or contradictory in context, the term "interact covalently", "covalent interaction" or "covalent bond" are interchangeably used and means a chemical bond or interaction that involves the sharing of electron pairs between atoms. Examples of such interactions are $\sigma$-bonding and $\pi$-bonding.

**Non-covalent interaction:** As used herein, unless indicated otherwise or contradictory in context, the term "interact non-covalently", "non-covalent interaction" or "non-covalent bond" are interchangeably used and means a chemical bond or interaction that does not involve the sharing of electron pairs between atoms but rather involves more dispersed variations of electromagnetic interactions between molecules or within a molecule. Non-covalent interactions can be generally classified into four categories, electrostatic interactions, $\pi$-interactions, van der Waals forces, and hydrophobic interactions.

**Electrophile:** As used herein, unless indicated otherwise or contradictory in context, the term "electrophile" means an organic molecule attracted to electrons that participates in a chemical reaction by accepting an electron pair in order to bond to a nucleophile. Most electrophiles are positively charged, have an atom that carries a partial positive charge, or have an atom that does not have an octet of electrons.

**Nucleophile:** As used herein, unless indicated otherwise or contradictory in context, the term "nucleophile" means an organic molecule that donates an electron pair to an electrophile to form a chemical bond in relation to a reaction. All molecules or ions with a free pair of electrons or at least one pi bond can act as nucleophiles.

**Polysaccharide:** As used herein, unless indicated otherwise or contradictory in context, the term "polysaccharide" means polymeric carbohydrate molecules composed of long chains of monosaccharide units bound together by glycosidic linkages and which upon hydrolysis provide monosaccharides or oligosaccharides. They range in structure from linear to highly branched polymers.

**Polynucleotide:** As used herein, the term "polynucleotide" or "nucleic acid", which are used interchangeably, refers to the phosphate ester polymeric form of ribonucleosides ("RNA molecules") or deoxyribonucleosides ("DNA molecules"), or any phosphoester analogs thereof, such as phosphorothioates and thioesters, in either single stranded form, or a double-stranded helix. The term "nucleic acid" includes double-stranded DNA round, inter alia, in linear (*e.g.,* restriction fragments) or circular DNA molecules. In particular, nucleic acids as used herein refer to nucleic acids such as RNAs encoding for agonist of growth factor receptors as defined herein.

**Nucleoside:** As used herein, the term "nucleoside" refers to a compound containing a sugar molecule (e.g., a pentose or ribose) or derivative thereof in combination with an organic base (e.g., a purine or pyrimidine) or a derivative thereof (also referred to herein as "nucleobase").

**Nucleotide:** As used herein, the term "nucleotide" refers to a nucleoside including a phosphate group.

**Dendrimer:** As used herein, unless indicated otherwise or contradictory in context, the term "dendrimer" means any repetitively branched molecules. Examples of dendrimers are phosphorous dendrimers, polylysine dendrimers, polypropylenimine dendrimers and PAMAM dendrimers, such as the ones described, for instance, in Scientific World Journal. 2013; 2013:732340; Curr Opin Chem Biol. 1998; 2(6):733-42; J Pept Sci. 1999; 5(5):203-20; and J Pept Sci. 2008; 14(1):2-43, which may be used for implementing embodiments of the present invention,

**Synthetic molecule:** As used herein, unless indicated otherwise or contradictory in context, the term "synthetic molecule" means a molecule which may not be found in nature without human direct intervention (except for its extraction and/or isolation).

**Synthetic polymers:** As used herein, unless indicated otherwise or contradictory in context, the term "synthetic polymer" refers to a macromolecule or polymer which may not be found in nature without human direct intervention (except for its extraction and/or isolation).

**Biocompatible:** As used herein, unless indicated otherwise or contradictory in context, the term "biocompatible" means compatible with living cells, tissues, organs or systems posing little to no risk of injury, toxicity or rejection by the immune system.

**Biologically active:** As used herein, unless indicated otherwise or contradictory in context, the term "biologically active" refers to a characteristic of any substance that has activity in a biological system and/or organism. For instance, a substance that, when administered to an organism, has a biological effect on that organism, is considered to be biologically active. In particular examples, a compound, substance or pharmaceutical composition of the present disclosure may be considered biologically active even if a portion of the compound, substance or pharmaceutical composition is biologically active or mimics an activity considered biologically relevant.

**Stem cells:** As used herein, unless indicated otherwise or contradictory in context, the term "stem cell" refers to the term as it is generally understood in the art. For example, in certain embodiments, stem cells, regardless of their source, are cells that are capable of dividing and renewing themselves for long periods, are at least to a degree unspecialized (undifferentiated), and can give rise to (differentiate into) specialized cell types (i.e., they are progenitor or precursor cells for a variety of different, specialized cell types).

**Mesenchymal stem cells:** As used herein, unless indicated otherwise or contradictory in context, the term "mesenchymal stem cells" generally means multipotent adult stromal cells that can differentiate into a variety of cell types, such as osteoblasts, chondrocytes, and adipocytes.

**Stem cell-like:** As used herein, unless indicated otherwise or contradictory in context, the term "Stem cell-like" refers to a cell which is not a stem cell by its origin but functions as a stem cell and presents similar characteristics such as, for example, the expression of stemness markers like Stro-1 and/or is multipotent thus has the ability to differentiate into various cell types.

**Progenitor cells:** As used herein, unless indicated otherwise or contradictory in context, the term "progenitor cells" generally means a biological cell that, like any stem cell, has a tendency to differentiate into a specific type of cell, but is already more specific than a stem cell and is pushed to differentiate into its "target" cell. Stem cells can generally replicate indefinitely, whereas progenitor cells can divide only a limited number of times.

**Adult stem cells:** As used herein, unless indicated otherwise or contradictory in context, the term "adult stem cells" means undifferentiated cells, found throughout the body after development, that multiply by cell division to replenish dying cells and regenerate damaged tissues. Also known as somatic stem cells, they can be found in juvenile as well as adult animals and human bodies.

**Differentiation:** As used herein, unless indicated otherwise or contradictory in context, the term "differentiation" refers to the process by which a less specialized cell becomes a more specialized cell type and involves a switch from one gene expression pattern to another.

**Differentiated cells:** As used herein, unless indicated otherwise or contradictory in context, the term "differentiated cells" generally means any cell of a specific lineage at the exception of cells containing stem cell specific markers.

**Non-terminally differentiated:** As used herein, unless indicated otherwise or contradictory in context, the term "non-terminally differentiated", when used in relation to a cell, refers to a differentiated cell as defined herein which has not reached its final state of differentiation. For example, in certain embodiments, in the Osteoblast cell lineage, a non-terminally differentiated cell is any differentiated cell of the lineage at the exception of an osteocyte.

**Terminally differentiated:** As used herein, unless indicated otherwise or contradictory in context, the term "terminally differentiated", when used in relation to a cell, refers to a differentiated cell as defined herein which has reached its final state of differentiation. For example, in certain embodiments, in the Osteoblast cell lineage, a terminally differentiated cell is an osteocyte.

**Methods for obtaining stem cells:** Methods for obtaining such stem cells and providing initial culture conditions, such as a liquid culture or semi-solid culture medium, are known in the art. The cells are initially expanded in vivo or in vitro, by contacting the source of the stem cells with a suitable reagent that expands or enriches such cells in the tissue source or in culture. Preferably, adult stem cells are isolated from a tissue source and then expanded or enriched in

vitro by exposure to a suitable agent. Cells are obtained from an individual by any suitable method for obtaining a cell sample from an animal, including, but not limited, to, collection of bone marrow collection of a bodily fluid (e.g., blood), collection of umbilical cord blood, tissue punch, and tissue dissection, including particularly, but not limited to, any biopsies of skin, intestine, cornea, spinal cord, brain tissue, scalp, stomach, breast, lung (*e.g.,* including lavage and bronchioschopy), fine needle aspirates of the bone marrow, amniotic fluid, placenta and yolk sac.

**Cell lineage:** As used herein, unless indicated otherwise or contradictory in context, the term "cell lineage" refers to the developmental history of a particular cell from its primary state in the fertilized egg or embryo through to its fully differentiated state. The different steps and phases involved in the development of a cell produces many intermediate cells which may be referred to as progenitor or precursor cells in the present application and form an integral part of the cell lineage.

**Bone:** It is conventionally known that mature osteoblasts are the cells responsible for bone formation and are derived from osteoblast precursors. Differentiation of human bone marrow mesenchymal stem cells and osteoblast precursors is one of the important processes for bone regeneration. Osteoblasts differentiate from mesenchymal stem cells. Mature osteoblasts differentiate from osteoblast precursors and into osteocytes which are non-dividing cells. Bone-related neoplastic diseases include, but are not limited to, bone primary tumors (benign tumors or cancers) such as osteoma, osteoid osteoma, osteochondroma, osteoblastoma, enchondroma, giant cell tumor of bone, aneurysmal bone cyst, fibrous dysplasia of bone, osteosarcoma, chondrosarcoma, Ewing's sarcoma, fibrosarcoma; and secondary tumors (*i.e.* metastasize) such as, for example, in certain embodiments, carcinomas of the prostate, breasts, lungs, thyroid, and kidneys.

**Osteoblast cell lineage:** As used herein, unless indicated otherwise or contradictory in context, the term "osteoblast cell lineage" refers to bone cells at any stage of their development and thus include, but are not limited to, mesenchymal stem cells, osteoblasts, osteocytes or any precursors thereof.

**Cartilage:** Native chondrocytes are responsible for the synthesis and turnover of the cartilage extracellular matrix (ECM), which provides an environment of nutrition diffusion for chondrocytes and provides the joint surface with biomechanical competence. Chondrogenic cells arise from pluripotential adult mesenchymal stem cells (MSCs) through a series of differentiation pathways. Chondrogenic differentiation of MSCs is induced by various intrinsic and extrinsic factors. Growth factors play an important role in this process. For instance, in the hyaline cartilage, growth factors regulate homeostasis and integrity, as well as development. Cartilage-related neoplastic diseases include, but are not limited to, Chondroma/ecchondroma/enchondroma (Enchondromatosis, Extraskeletal chondroma), Chondrosarcoma (Mesenchymal chondrosarcoma, Myxoid chondrosarcoma), Osteochondroma (Osteochondromatosis), Chondromyxoid fibroma, and Chondroblastoma,

**Chondrocytic cell lineage:** As used herein, unless indicated otherwise or contradictory in context, the term "chondrocytic cell lineage" refers to cartilage cells at any stage of their development and thus include, but are not limited to, mesenchymal stem cells, chondroblasts, chondrocytes or any precursors thereof.

**Muscles:** Skeletal muscle is a highly complex and heterogeneous tissue serving a multitude of functions in the organism. The process of generating muscle - myogenesis - can be divided into several distinct phases. During embryonic myogenesis, mesoderm-derived structures generate the first muscle fibers of the body proper, and in subsequent waves additional fibers are generated along these template fibers. In the perinatal phase, muscle resident myogenic progenitors initially proliferate extensively but, later on, decrease as the number of myonuclei reaches a steady state and myofibrillar protein synthesis peaks. Once the muscle has matured, these progenitors will enter quiescence and henceforth reside within it as satellite cells. Adult skeletal muscle, like all renewing organs, relies on a mechanism that compensates for the turnover of terminally differentiated cells to maintain tissue homeostasis. This type of myogenesis depends on the activation of satellite cells that have the potential to differentiate into new fibers. The most comprehensively studied form of myogenesis takes place when mature muscle is damaged and large cohorts of satellite cells expand mitotically and differentiate to repair the tissue and reestablish homeostasis. It is now generally accepted that satellite cells are closely related to progenitors of somitic origin. The activation of the network of transcription factors that controls skeletal muscle development depends on paracrine factors that are released by adjacent tissues, such as the neural tube, notochord, surface ectoderm and lateral mesoderm. Several secreted factors have been identified that determine the spatial and temporal onset of myogenesis. Signalling molecules, such as Noggin and bone morphogenetic proteins (BMPs) - which inactivate and activate receptors of the transforming growth factor-$\beta$ (TGF$\beta$) superfamily, respectively - are reported to participate in the orchestration of the activation of myogenesis. Muscle-related neoplastic diseases include, but are not limited to, Rhabdomyosarcoma, and Leiomyosarcoma.

**Muscle cell lineage:** As used herein, unless indicated otherwise or contradictory in context, the term "muscle cell lineage" refers to muscle cells at any stage of their development and thus include, but are not limited to, mesenchymal stem cells, myoblasts, myocytes or any precursors thereof.

**Vascular:** The vasculature in the human body forms through two distinct processes: vasculogenesis and angiogenesis. Vasculogenesis is defined as the process of de novo blood vessel formation occurring when endothelial precursor cells (angioblasts) migrate and differentiate into endothelial cells which form the new vessel. These vascular

trees are then extended through angiogenesis which is defined as the new vessel formation secondary to proliferation of endothelial cells from pre-existing vessels. Vasculogenesis as well as angiogenesis occur during the embryologic development of the circulatory system but also in the adult organism from circulating endothelial progenitor cells (derivatives of stem cells) able to contribute, albeit to varying degrees, to neovascularization. Vascular-related neoplastic diseases include, but are not limited to, Hemangiosarcoma, Kaposi's sarcoma, Lymphangiosarcoma, and Infantile hemangio-pericytoma.

**Vascular cell lineage:** As used herein, unless indicated otherwise or contradictory in context, the term "vascular cell lineage" refers to vascular cells at any stage of their development and thus include, but are not limited to, mesenchymal stem cells, angioblast, pericytes and endothelial cells or any precursors thereof.

**Neurons:** It was recently reported that neural cells can be regenerated from neural stem cells (NSCs). These are self-renewing, multipotent adult stem cells that generate the main phenotype of the nervous system. They undergo asymmetric cell division into two daughter cells, one non-specialized and one specialized. NSCs primarily differentiate into neurons, astrocytes, and oligodendrocytes. NSCs are generated throughout an adult's life via the process of neurogenesis. NSCs can be differentiated to replace lost or injured neurons or in many cases even glial cells. NSCs are stimulated to begin differentiation via exogenous cues from their microenvironment, or the neural stem cell niche. This niche defines a zone in which stem cells are retained after embryonic development for the production of new cells of the nervous system. This continual supply of new neurons and glia then provides the postnatal and adult brain with an added capacity for cellular plasticity. Critical to the maintenance of the stem cell niche are microenvironmental cues and cell-cell interactions that act to balance stem cell quiescence with proliferation and to direct neurogenesis versus gliogenesis lineage decisions. Several proteins like different growth factors are involved in the mechanisms of the neural stem cell niche as well as in the maintenance and growth of the newly formed neurons. These include the BMPs, FGFs, PDGF, VEGF, TGF β, BDNF and others. Neuron-related neoplastic diseases include, but are not limited to, Anaplastic astrocytoma, Astrocytoma, Central neurocytoma, Choroid plexus carcinoma, Choroid plexus papilloma, Choroid plexus tumor, Dysembryoplastic neuroepithelial tumour, Ependymal tumor, Fibrillary astrocytoma, Giant-cell glioblastoma, Glioblastoma multiforme, Gliomatosis cerebri, Gliosarcoma, Hemangiopericytoma,Medulloblastoma, Medulloepithelioma, Meningeal carcinomatosis, Neuroblastoma, Neurocytoma, Oligoastrocytoma, Oligodendroglioma, Optic nerve sheath meningioma, Pediatric ependymoma, Pilocytic astrocytoma, Pinealoblastoma, Pineocytoma, Pleomorphic anaplastic neuroblastoma, Pleomorphic xanthoastrocytoma, Primary central nervous system lymphoma, Sphenoid wing meningioma, Subependymal giant cell astrocytoma, Subependymoma, and Trilateral retinoblastoma.

**Neuronal cell lineage:** As used herein, unless indicated otherwise or contradictory in context, the term "neuron lineage" refers to brain cells at any stage of their development and thus include, but are not limited to, neural stem cells, neuroblast, neurocyte and neuroglial cells or any precursors thereof.

**Eye retina:** The vertebrate retina is a light-sensitive layer of tissue, lining the inner surface of the eye. Retinal development involves a complex progression of tissue induction, proliferation of retinal progenitor cell (RPC) populations and terminal differentiation of these cells into specific functional types. Bone morphogenetic protein (BMP), is a member of the transforming growth factor (TGF)-β family of signaling molecules plays an important role in the retinal cell development. BMP-2, -4, and -7 and their receptors (BMPRs) are expressed in the eye during embryogenesis and are essential for multiple aspects of retinal development. Retina-related neoplastic diseases include, but are not limited to, Retinoblastoma. It should also be noted that eye cancers can be primary (starts within the eye) and metastatic (spread to the eye from another organ). The two most common cancers that would spread to the eyes from another organ are breast cancer and lung cancer. Other, less common, sites of origin include prostate, kidney, thyroid, skin, colon and blood or bone marrow.

**Retinal cell lineage:** As used herein, unless indicated otherwise or contradictory in context, the term "retinal cell lineage" refers to eye retina cells at any stage of their development and thus include, but are not limited to, photoreceptor, bipolar cells, rod and cone cells or any precursors thereof.

**Kidneys:** The kidneys are composed of complex tissues consisting of several different cell types including glomerular podocytes, endothelial cells, mesangial cells, interstitial cells, tubular epithelial cells, and connecting duct cells. These cell types interact to establish a precise cellular environment that functions as an efficient tissue. Kidneys-related neoplastic diseases include, but are not limited to, Squamous cell carcinoma, Juxtaglomerular cell, tumor (reninoma), Angiomyolipoma, Renal oncocytoma, Bellini duct carcinoma, Clear-cell sarcoma of the kidney, Mesoblastic nephroma, Wilms' tumor, Mixed epithelial stromal tumor, Clear cell adenocarcinoma, Transitional cell carcinoma, Inverted papilloma, Renal lymphoma, Teratoma, Carcinosarcoma, and Carcinoid tumor.

**Renal cell lineage:** As used herein, unless indicated otherwise or contradictory in context, the term "renal cell lineage" refers to renal cells at any stage of their development and thus include, but are not limited to, mesenchymal stem cells, podocytes, or any precursors thereof.

**Ligaments and Tendons:** Tendons and ligaments (T/L) are dense connective tissues of mesodermal origin. They connect and transmit force from muscle to bone and bone to bone, respectively. Both tissues are able to store elastic energy and withstand hightensile forces, on which locomotion is entirely dependent. T/L are predominantly composed of collagen type I fibrils organized in a highly hierarchical manner that is unique for the T/L. Other collagens (types III-VI, XI,

XII, XIV, and XV) and various proteoglycans (decorin, cartilage oligomeric matrix protein (COMP), byglican, lumican, fibromodulin, tenascin-C, etc.) are building the remaining T/L substance. The cellular content of T/L is dominated by tendon-specific fibroblasts named tenocytes. During embryonic development, the tendon-specific cells descend from a sub-set of mesenchymal progenitors condensed in the syndetome, a dorsolateral domain of the sclerotome. L/T-related neoplastic diseases include, but are not limited to, Fibrosarcoma, Malignant fibrous, Hystiocytoma, and Dermatofibrosarcoma.

**Ligament and tendon cell lineage:** As used herein, unless indicated otherwise or contradictory in context, the term "ligament and tendon cell lineage" or "L/T cell lineage" refers to bone or cartilage cells at any stage of their development and thus include, but are not limited to, mesenchymal stem cells, fibroblasts, fibrocytes, or any precursors thereof.

**Skin:** The skin constantly renews itself throughout adult life. Stem cells (SCs) residing in the epidermis ensure the maintenance of adult skin homeostasis, but they also participate in the repair of the epidermis after injuries. The skin protects the body from dehydration, injury and infection. The skin consists of an underlying dermis, separated by a basement membrane from the multilayered overlaying epidermis. The dermis is of mesodermal embryonic origin and contains as adult stem cells fibroblastic mesenchymal stem-cell-like cells. These cells have a multi-lineage differentiation potential, being also able to form adipose tissue or bones. Skin-related neoplastic diseases include, but are not limited to, basal cell carcinoma, squamous cell carcinoma, malignant melanoma, dermatofibrosarcoma protuberans, Merkel cell carcinoma, Kaposi's sarcoma, keratoacanthoma, spindle cell tumors, sebaceous carcinomas, microcystic adnexal carcinoma, Paget's disease of the breast, atypical fibroxanthoma, leiomyosarcoma, and angiosarcoma.

**Fibroblast lineage:** As used herein, unless indicated otherwise or contradictory in context, the term "fibroblast lineage" refers to skin cells at any stage of their development and thus include, but are not limited to, mesenchymal stem cells, fibroblasts, keratinocytes, Merkel cells, melanocytes, Langerhans cells, and any precursor cells thereof.

**Reproduction:** Reproduction (or procreation) is the biological process by which new offspring individual organisms are produced from their parents. Sexual reproduction is a biological process by which organisms create descendants that have a combination of genetic material contributed from two (usually) different members of the species. The development and physiological functions of basic structures in the mammalian reproductive system are influenced by the tissue-specific expression of members of different growth factors families like the BMP family. The establishment of the germ line is a fundamental aspect of reproduction. Germ cell determination is induced in epiblast cells by the extraembryonic ectoderm, and is not acquired through the inheritance of preformed germ plasma. There is some strong evidence that BMP-4 and -8b play a central role in determining primordial germ cell (PGC) formation in the embryo. The genes encoding BMP-4 and -8b have overlapping expression in the extraembryonic ectoderm before gastrulation, i.e., before PGCs are seen. Thus, PGC formation requires BMP-4 expression. There is also evidence from knockout mammals that BMP-8b is required for PGC formation. Furthermore, there is increasing evidence that locally produced BMPs play a major role in the differentiation of the pituitary gonadotrope. Reproduction-related neoplastic diseases include, but are not limited to, Prostate cancer, Ovary cancer (adenocarcinoma, or glandular cancer) also known as carcinoma of the prostate or prostatic intraepithelial neoplasia.

**Reproduction system lineage:** As used herein, unless indicated otherwise or contradictory in context, the term "reproduction system lineage" refers to Sertoli cells, Leydig cell and Germ cell at any stage of their development, in particular, mesenchymal stem cells.

**Blood:** Blood is a bodily fluid in animals that delivers necessary substances such as nutrients and oxygen to the cells and transports metabolic waste products away from those cells. When it reaches the lungs, gas exchange occurs wherein carbon dioxide is diffused out of the blood into the alveoli and oxygen is diffused into the blood. This oxygenated blood is pumped to the left hand side of the heart in the pulmonary vein and enters the left atrium. From here it passes through the bicuspid valve, through the ventricle and taken all around the body by the aorta. Blood contains antibodies, nutrients, oxygen and much more to help the body work. In vertebrates, it is composed of blood cells suspended in blood plasma. Plasma, which constitutes 55% of blood fluid, is mostly water (92% by volume), and contains dissipated proteins, glucose, mineral ions, hormones, carbon dioxide (plasma being the main medium for excretory product transportation), and blood cells themselves. Albumin is the main protein in plasma, and it functions to regulate the colloidal osmotic pressure of blood. Hematopoietic stem cells (HSCs) are the blood cells that give rise to all the other blood cells and are derived from the mesoderm. They are located in the red bone marrow, which is contained in the core of most bones. The HSCs give rise to the myeloid lineage (monocytes and macrophages, neutrophils, basophils, eosinophils, erythrocytes, megakaryocytes/platelets, dendritic cells), and to the lymphoid lineages (T-cells, B-cells, NK-cells). The most abundant cells in the vertebrate blood are red blood cells (also called RBSs or erythrocytes). These contain hemoglobin, an iron-containing protein, which facilitates oxygen transport by reversibly binding to this respiratory gas and greatly increasing its solubility in blood. Blood-related neoplastic diseases include, but are not limited to, acute lymphoblastic leukemia (ALL), acute myeloid leukemia (AML), chronic lymphocytic leukemia (CLL) and chronic myeloid leukemia (CML).

**Blood cell lineages (myeloid lineage and lymphoid lineage):** As used herein, unless indicated otherwise or contradictory in context, the term "blood cell lineages" refers to blood cells at any stage of their development from the

myeloid or from the lymphoid lineage, and thus include, but are not limited to, hematopoietic stem cells (HSC), myeloid progenitors, lymphoid progenitors, mast cells, myeloblasts, monocytes, macrophages, neutrophils, basophils, eosinophils, erythrocytes, megakaryocytes, thrombocytes, dendritic cells, small lymphocytes, T-lymphocytes (T-cells), B-lymphocytes (B-cells), natural killer (NK)-cells, and any precursor cells thereof.

**Adipose tissue:** Adipose tissue is loose connective tissue composed mostly of adipocytes. In addition to adipocytes, adipose tissue contains the stromal vascular fraction (SVF) of cells including preadipocytes, fibroblasts, vascular endothelial cells and a variety of immune cells (i.e. adipose tissue macrophages (ATMs)). Adipose tissue is derived from preadipocytes. Its main role is to store energy in the form of lipids, although it also cushions and insulates the body. Pre-adipocytes are thought to be undifferentiated fibroblasts that can be stimulated to form adipocytes. The pre-adipocytes originate from mesenchymal stem cells. Areolar connective tissue is composed of adipocytes. The term "lipoblast" is used to describe the precursor of the adult cell. The term "lipoblastoma" is used to describe a tumor of this cell type. Adipose tissue-related neoplastic diseases include, but are not limited to, lipoma, Adenolipomas, Angiolipoleiomyomas, Angiolipomas, Corpus callosum lipoma, Cerebellar pontine angle and internal auditory canal lipomas, Chondroid lipomas, Hibernomas, Intradermal spindle cell lipomas, Neural fibrolipomas, Pleomorphic lipomas, Spindle-cell lipomas, Superficial subcutaneous lipomas, Lipoblastoma, Liposarcoma.

**Adipocyte lineage:** As used herein, unless indicated otherwise or contradictory in context, the term "adipocyte cell lineage" refers to adipocyte cells at any stage of their development and thus include, but are not limited to, mesenchymal stem cells, areolar connective cells, adipocytes, pre-adipocytes/lipoblasts, and any precursor cells thereof.

**Digestive system:** The human digestive system is composed mainly of the gastrointestinal tract (including the esophagus, stomach, small intestine, large intestine, rectum and anus) and the accessory digestive glands (the liver, the gall bladder and the pancreas). To achieve the goal of providing energy and nutrients to the body, six major functions take place in the digestive system: ingestion, secretion, mixing and movement, digestion, absorption, excretion. The gastrointestinal wall refers to the specialized series of tissue layers surrounding the lumen of the gastrointestinal tract. The general structure involves the four following layers (ordered from the lumen outward): mucosa, submucosa, muscularis externa, serosa (if the tissue is intraperitoneal) / adventitia (if the tissue is retroperitoneal). Gastrointestinal cancer refers to malignant conditions of the gastrointestinal tract (GI tract) and accessory organs of digestion. The symptoms relate to the organ affected and can include obstruction (leading to difficulty swallowing or defecating), abnormal bleeding or other associated problems. Gastrointestinal tissue-related neoplastic diseases include, but are not limited to, Esophageal cancer, Stomach cancer, Pancreatic cancer, Liver cancer, Gallbladder cancer, MALT lymphoma, Gastrointestinal stromal tumors and Cancers of the biliary tree, including cholangiocarcinoma.

**Gastrointestinal cell lineages:** As used herein, unless indicated otherwise or contradictory in context, the term " gastrointestinal cell lineages" refers to gastrointestinal cells and cells of the digestive accessory organs at any stage of their development and thus include, but are not limited to interstitial cells of Cajal, gastrointestinal epithelial cells, parietal cells, acinar cells, chief cells, mucus cells, goblet cells, G cells, endocrine I cells, endocrine S cells, endocrine K cells, endocrine M cells, ECL (enterochromaffin) cells, D cells, enteroendocrine cells, APUD cells, hepatocytes, sinusoidal hepatic endothelial cells, Kupffer cells, hepatic stellate cells, centroacinar cells, pancreatic stellate cells, $\alpha$-cells, $\gamma$-cells, $\beta$-cells, $\delta$-cells, centroacinar cells, basophilic cells, ductal cells, columnar cells, cholecystocytes and any precursor cells thereof.

**Lung:** The lung is the essential respiration organ in many air-breathing animals. In mammals the two lungs are located near the backbone on either side of the heart. Their principal function is to transport oxygen from the atmosphere into the bloodstream, and to release carbon dioxide from the bloodstream into the atmosphere. A large surface area is needed for this exchange of gases, which is accomplished by the mosaic of specialized cells that form millions of tiny, exceptionally thin-walled air sacs called alveoli. Lung cells include, but are not limited to, type I pneumocytes, type II pneumocytes, clara cells and goblet cells. Lung tissue-related neoplastic diseases include, but are not limited to, lung cancer also known as carcinoma of the lung or pulmonary carcinoma, Epithelial cells or small-cell lung carcinoma (SCLC) and non-small-cell lung carcinoma (NSCLC).

**Lung cell Lineages:** As used herein, unless indicated otherwise or contradictory in context, the term "lung cell Lineage" refers to lung cells at any stage of their development and thus include, but are not limited to, epithelial cells, erythrocytes, alveolar cells and any precursor cells thereof.

**Head and neck cancer:** As used herein, unless indicated otherwise or contradictory in context, the term "head and neck cancer" refers to a group of cancers that usually starts in the lip, oral cavity, nasal cavity, paranasal sinuses, pharynx, and larynx. About 90% of head and neck cancers are squamous cell carcinomas. Thus, neoplastic diseases such as head and neck cancers include, but are not limited to, oral cancer, nasopharynx cancer, oropharyngeal cancer, hypopharynx cancer and laryngeal cancer.

The cell lineage involved in head and neck cancers includes all the cells involved in the formation of such cancers at any stage of their development and thus include, but are not limited to, cells of the oral cavity, cells of the pharynx, cells of the larynx, cells of the paranasal sinuses and nasal cavity, cells of the salivary glands and any precursor cells thereof.

**Ratio:** As used herein, unless indicated otherwise or contradictory in context, the term "ratio", when used in

relation to GFR-binding compound with respect to the bioactive carrier in the pharmaceutical association or composition disclosed herein, refers to the (molar, weight or part as specified) ratio between the quantity of GFR-binding compound and the quantity of bioactive carrier. The ratio may be a molar ratio, a weight ratio or a part ratio and will be specified as needed on a case by case basis. Quantity units may conventionally be mole, millimole, gram, milligram or parts. For example, in certain embodiments, it is convenient to express the relative quantity between GFR-binding compounds and bioactive carriers using densities. It shall be understood that this ratio may be varied according to the neoplastic cell type to be treated.

**Density:** As used herein, unless indicated otherwise or contradictory in context, the term "density", when used in relation to GFR-binding compound with respect to the bioactive carrier in the pharmaceutical composition disclosed herein, refers to the quantity of GFR-binding compounds, expressed in *e.g.* mole, millimole, gram, or milligram, with respect to one standardised surface unit *e.g.* squared millimetre ($mm^2$), squared micrometre ($\mu m^2$), or squared nanometre ($nm^2$)). For example, in certain embodiments, the ratio between a GFR-binding compound and a bioactive carrier in the pharmaceutical association or composition disclosed herein may be expressed in pmol per $mm^2$ or pmol/$mm^2$.

**Cell cycle:** As used herein, unless indicated otherwise or contradictory in context, the term "cell cycle" refers to the process through which a vertebrate cell self-replicate. In eukaryote cells, cell cycle consists of four discrete phases: G1, S, G2, and M. Together, the G1, S, and G2 phases make up the period known as interphase. During the S or "synthesis" phase, the cell replicates its DNA creating an exact copy of all of its chromosomes. In the M or "mitotic" phase, the cell division actually occurs and separates the chromosomes in its cell nucleus into two identical sets in two nuclei. The first phase within interphase, from the end of the previous M phase until the beginning of DNA synthesis, is called G1. During this phase the biosynthetic activities of the cell, which are considerably slowed down during M phase, resume at a high rate. This phase is marked by the formation of millions of proteins and later on enzymes that are required in S phase, mainly those needed for DNA replication. The G2 phase, or pre-mitotic phase, is the third and final sub-phase of Interphase in the cell cycle directly preceding Mitosis. It follows the successful completion of S phase and ends with the onset of prophase, the first phase of mitosis. In order to move from one phase of the cell cycle to the next, a cell must "validate" numerous checkpoints. At each checkpoint, specialized proteins determine whether the necessary conditions exist. If so, the cell is free to enter into the next phase. If not, progression through the cell cycle is halted. For example, in certain embodiments, during G1, the cell passes through a "validation" window punctuated by the restriction point R. During the "validation" phase, different checkpoints ensure that environmental conditions are favourable for replication. If conditions are not favourable, the cell may enter a resting state known as G0. The G0 phase or resting phase is a period in the cell cycle in which cells exist in a quiescent state. G0 phase is viewed as either an extended G1 phase, where the cell is neither dividing nor preparing to divide, or a distinct quiescent stage that occurs outside of the cell cycle. Typically, a healthy vertebrate cell undergoes cell division when needed *e.g.* to regenerate damaged tissues or simply replace old tissues by new ones, by switching from a G0 resting state into the G1 state of the cell cycle and resume cell division. In contrast, neoplastic cells (such as cancer cells) have lost their ability to suspend cell division and switch to the G0 phase therefore undergoing permanent cell division which is generally referred to uncontrolled cell division or proliferation. Another "validation" window takes place later in the cell cycle, just before a cell moves from G2 to mitosis. Here, a number of proteins scrutinize the cell's DNA ensuring proper replication has taken place. Finally, another cell cycle "validation" window takes place during mitosis in which different checkpoints determines whether chromosomes are correctly attached to the spindle, and to the network of microtubules that will separate them during cell division.

**Quiescence:** As used herein, unless indicated otherwise or contradictory in context, the term "quiescence", when used in relation to a cell, refers to a resting state during which the cell does not divide, duplicate or proliferate. This state is also called the G0 state. As used herein, "inducing quiescence of a neoplastic cell" thus means to provoke the passage from G1 to G0 of a neoplastic cell which usually has lost the ability to do so. One method to detect the passage from G1 to G0 is, for instance, to monitor the state of phosphorylation of the protein Rb via Western Blot. The Rb protein is normally not phosphorylated during the G0 phase but becomes phosphorylated or even hyper-phosphorylated during the rest of the cell cycle. Consequently, observing the absence (or substantial reduction *i.e.* at least 90% reduction, in comparison with the quantity present in the G1 phase) of phosphorylated Rb protein on a western blot confirms that a neoplastic cell has undergone (at some point in time) a switch to the G0 phase.

**Cell division or cell proliferation:** As used herein, unless indicated otherwise or contradictory in context, the term "cell division" or "cell proliferation", refers to the process by which a cell self-replicate, replicate or is caused to replicate.

**Proliferate:** As used herein, unless indicated otherwise or contradictory in context, the term "proliferate" means to grow, expand or increase or cause to grow, expand or increase. "Proliferative" means having the ability to proliferate. "Anti-proliferative" means having properties to counter, reduce, or inhibit proliferation.

**Hyperproliferation:** As used herein, unless indicated otherwise or contradictory in context, the term "hyperproliferation" or "uncontrolled proliferation" means the abnormal growth, expansion or increase or causing the abnormal growth, expansion or increase. Abnormal growth typically originates from the abnormal regulation of the cell cycle preventing the cell to reach the G0 state and stop cell division and replication. One consequence of hyper or uncontrolled

proliferation is the development of neoplastic diseases such as tumours and cancers.

**Hyperproliferative diseases:** As used herein, unless indicated otherwise or contradictory in context, the term "hyperproliferative diseases" is used interchangeably with "neoplastic diseases" and refers to diseases that are characterized by uncontrolled cellular proliferation and/or disruption in programmed cell death. The loss of a cell's ability to control cellular proliferation is often caused by genetic damage to the cellular pathways responsible for regulating cellular functions, including but not limited to, for example, in certain embodiments, metabolism, cell cycle progression, cell adhesion, vascular function, apoptosis, and angiogenesis.

**Anti-mitogen activity:** As used herein, unless indicated otherwise or contradictory in context, the term "anti-mitogen activity", refers to biological pathways that down-regulate, partially inhibit or suppress cell mitosis *i.e.* cell division. As used herein, a substance or pharmaceutical association which promotes, induces or favours anti-mitogen activity thus means a substance or pharmaceutical association which provides at least part of its effective biologic or therapeutic action by down-regulating, partially inhibiting or suppressing mitosis of the neoplastic cell to be treated.

**Tumor suppressor pathways:** As used herein, unless indicated otherwise or contradictory in context, the term "tumor suppressor pathways" refers to biological pathways that down-regulate, partially inhibit or suppress tumor activity *i.e.* protect the cell against cell defects which could cause tumors or cancers. As used herein, a substance or pharmaceutical association which promotes, induces or favours tumor suppressor pathways thus means a substance or pharmaceutical association which provides at least part of its effective biologic or therapeutic action by up-regulating, activating or promoting the tumor suppressor genes or proteins of the neoplastic cell to be treated. Known tumor suppressor proteins which have a dampening or repressive effect on the regulation of the cell cycle or promote apoptosis include, but are not limited to, p53, pRb, pVHL, APC, CD95, ST5, YPEL3, ST7, and ST14.

**Anti-oncogenic activity:** As used herein, unless indicated otherwise or contradictory in context, the term "anti-oncogenic activity" refers to any molecule having the ability to inhibit, repress or down-regulate the gene or protein expression of oncogenes. An oncogene is a gene that has the potential to cause neoplastic diseases such as cancer. Examples of anti-oncogene molecules include tumor suppressor proteins.

By **"without temporarily or permanently damaging or killing a neoplastic cell",** is meant, unless indicated otherwise or contradictory in context, that a neoplastic disease, such as cancer, is treated without inducing the entry of the neoplastic cells into apoptosis as it may be assessed by the positive expression of proteins caspase 3, 6 and 7.

By **"without permanently inducing a quiescent state in a neoplastic cell",** is meant, unless indicated otherwise or contradictory in context, that a neoplastic disease, such as cancer, is treated by inducing a temporary quiescent state in the neoplastic cells (the cells enter the G0 phase) and then differentiate into a more specialised state.

**Non-mutagenic:** As used herein, unless indicated otherwise or contradictory in context, the term "non-mutagenic", when used in relation to a therapy or treatment, refers to a therapy which does not involve the alteration or modification of a cell's genome.

**Recoding:** As used herein, unless indicated otherwise or contradictory in context, the term "recoding" or "converting", when used in relation to a cell (in particular a neoplastic cell such as a cancer cell), refers to the action of providing, to a neoplastic cell to be treated, a suitable extracellular micro-environment (*e.g.* in the form of a pharmaceutical association or composition as defined herein) providing appropriate extracellular signals so that the cell may undergo self-recovery or -healing and be converted into a partially or fully differentiated non-neoplastic cell.

**Recoding therapy:** As used herein, unless indicated otherwise or contradictory in context, the term "recoding therapy" refers to a therapy that promotes and stimulates the cell's natural abilities to redirect its own fate by integrating accurate micro-environmental recoding signals.

**Extracellular micro-environment:** As used herein, unless indicated otherwise or contradictory in context, the term "extracellular micro-environment" refers to the environment surrounding (in functional proximity with) a specific cell which is characterized by biophysical, mechanical and biochemical properties specific for each tissue and is able to regulate cell behavior. Modification of the extracellular micro-environment of a neoplastic cell using, for instance, pharmaceutical associations or compositions as defined herein allows for the conversion or recoding of said neoplastic cell into a healthy, functional, non-neoplastic cell.

**Self-recovery or self-healing:** As used herein, unless indicated otherwise or contradictory in context, the term "self-recovery" or "self-healing", when used in relation to a neoplastic cell such as a cancer cell, means that the neoplastic cell operates its own internal biological changes once it has been recoded or treated using a pharmaceutical association or composition as defined herein, so that it becomes a functional, healthy, non-neoplastic cell. The cell heal itself when in contact with the pharmaceutical composition or association as defined herein and, in contrast with previously reported methods wherein the neoplastic cell is forced to die or maintained in a temporarily reduced or non-proliferative state.

**Physiologically functional cell:** As used herein, unless indicated otherwise or contradictory in context, the term "physiologically functional cell" refers to a cell which is able to perform normally all of the cell functions associated with a particular cell type and necessary for the normal physiology of a cell. These functions include all of the intracellular molecular mechanisms but also all of the activities necessary for a normal communication between the cell and its microenvironment. One method which may be used to verify if a cell is physiologically functional is the grafting of the cell,

after the introduction of fluorescent markers, in other mammalian model organisms such as mouse models. The cell is grafted in the tissue corresponding to its cell type. The cell characteristics and normal functions are monitored after a period of time with various methods such as in vivo microscopy or histological staining. The term "functional" when used in relation to a molecule, compound or substance refers to a biological molecule in a form in which it exhibits a property and/or activity by which it is characterized.

**Healthy cell:** As used herein, unless indicated otherwise or contradictory in context, the term "healthy cell" refers to a cell which presents a normal morphology, normal cell functions and normal cell growth which are not damaged, altered or inactivated by a neoplastic disease.

**Shorter period of time:** As used herein, unless indicated otherwise or contradictory in context, the term "shorter period of time", when used in relation to conversion or recoding duration, means substantially shorter to provide a substantial benefit for the treated patient in comparison with existing treatments. In certain embodiments, a shorter period of time includes at least 1.5-fold, at least 2-fold, at least 2.5-fold, at least 3-fold, at least 3.5-fold, at least 4-fold, at least 4.5-fold, at least 5-fold, at least 6-fold, at least 7-fold, at least 8-fold, at least 9-fold or at least 10-fold reduction with respect to an existing treatment.

**Exogenous:** As used herein, unless indicated otherwise or contradictory in context, the term "exogenous" refers to a substance coming from outside a living system such as a cell, an organ, or an individual organism. For example, in certain embodiments, exogenous factors in medicine include pathogens and therapeutics. DNA introduced into a cell via transfection or viral infection may be considered as an exogenous factor. Carcinogens are also commonly referred to as exogenous factors.

**Endogenous:** As used herein, unless indicated otherwise or contradictory in context, the term "endogenous" refers to substances that originate from within an organism, tissue, or cell.

**Intracellular:** As used herein, unless indicated otherwise or contradictory in context, the term "intracellular" generally means "inside the cell". In vertebrates, such as animals, the cell membrane is the barrier between the inside of the cell and the outside of the cell (the extracellular milieu). Thus, treatments and therapies in which at least one substance, compound, pharmaceutical association, combination or composition penetrates the cell wall of a cell to be treated in order to produce/deliver its (effective) biological effect are considered as intracellular treatments and therapies.

**Extracellular:** As used herein, unless indicated otherwise or contradictory in context, the term "extracellular" means "outside the cell". In vertebrates, such as animals, the cell membrane is the barrier between the inside of the cell (the intracellular milieu) and the outside of the cell. Thus, treatments and therapies in which no substance, compound, pharmaceutical association, combination or composition requires penetration of the cell membrane in order to produce/deliver its (effective) biological effect *(e.g.* by interacting with trans-membrane receptors) are considered as extracellular treatments and therapies. In other words, a therapy using a plurality of substances in order to provide the desired biological effect wherein one or more of these substances require the entry into the intracellular compartment to provide (or deliver) its biological effect is not considered as an extracellular therapy in the sense of the present disclosure.

**Cytostatic:** As used herein, unless indicated otherwise or contradictory in context, the term "cytostatic" refers to inhibiting, reducing, or suppressing the growth, division, or multiplication of a cell (*e.g.,* a mammalian cell (*e.g.,* a human cell)), bacterium, virus, fungus, protozoan, parasite, prion, or a combination thereof.

**Cytotoxic:** As used herein, unless indicated otherwise or contradictory in context, the term "cytotoxic" refers to killing or causing injurious, toxic, or deadly effect on a cell (*e.g.,* a mammalian cell (e.g., a human cell)), bacterium, virus, fungus, protozoan, parasite, prion, or a combination thereof.

**In vitro:** As used herein, unless indicated otherwise or contradictory in context, the term "in vitro" refers to events that occur in an artificial environment, *e.g.,* in a test tube or reaction vessel, in cell culture, in a Petri dish, etc., rather than within an organism (*e.g.,* animal, plant, or microbe).

**In vivo:** As used herein, unless indicated otherwise or contradictory in context, the term "in vivo" refers to events that occur within an organism (*e.g.,* animal, plant, or microbe or cell or tissue thereof).

**Ex vivo:** As used herein, unless indicated otherwise or contradictory in context, the term "ex vivo" refers to events that occur in an external environment on tissues sourced from an organism (*e.g.,* animal, plant, or microbe) in an attempt to replicate natural living conditions outside such an organism.

**Syndecans:** As used herein, unless indicated otherwise or contradictory in context, the term "syndecans" refers to single transmembrane domain proteins that are thought to act as co-receptors, especially for G protein-coupled receptors. These core proteins carry three to five heparan sulfate and chondroitin sulfate chains, which allow for interaction with a large variety of ligands including fibroblast growth factors, vascular endothelial growth factor, transforming growth factor-beta, fibronectin and antithrombin-1. Interactions between fibronectin and some syndecans can be modulated by the extracellular matrix protein tenascin C. The syndecan protein family has four members. Syndecans 1 and 3 and syndecans 2 and 4, making up separate subfamilies, arose by gene duplication and divergent evolution from a single ancestral gene. The syndecan numbers reflect the order in which the cDNAs for each family member were cloned. All syndecans have an N-terminal signal peptide, an ectodomain, a single hydrophobic transmembrane domain, and a short C-terminal cytoplasmic domain. All syndecans are anchored to plasma membrane via a 24-25 amino acid long

hydrophobic transmembrane domain. In mammalian cells, syndecans are expressed by unique genes located on different chromosomes. All members of the syndecan family have 5 exons. The difference in size of the syndecans is credited to the variable length of exon 3, which encodes a spacer domain.In humans, the amino acid length of syndecan 1, 2,3 and 4 is 310, 201, 346 and 198 respectively. Glycosaminoglycan chains, a member of the heparan sulfate group, are an important component of syndecans and are responsible for a diverse set of syndecan functions. The addition of glycosaminoglycans to syndecan is controlled by a series of post- translational events.

**Cyclin-dependent kinases:** As used herein, unless indicated otherwise or contradictory in context, the term "Cyclin-dependent kinases" or "CDKs" refers to a family of protein involved in the regulation of the cell cycle. They are present in all known eukaryotes, and their regulatory function in the cell cycle has been evolutionarily conserved. By definition, a CDK binds a regulatory protein called a cyclin. It is reported that, without cyclin, CDK has little kinase activity; only the cyclin-CDK complex is considered to be an active kinase. CDKs phosphorylate their substrates on serines and threonines, so they can be said to belong to the serine-threonine kinase family. CDKs and cyclins are thus highly conserved across species and are present in all cell types including those having a neoplastic phenotype (*e.g.* cancer cells). Most of the known cyclin-CDK complexes regulate the progression through the cell cycle. Animal cells contain at least nine CDKs, four of which, CDK 1, 2, 3, 4 and 6, are reported to be directly involved in cell cycle regulation: CDK1 regulated by cyclin A, cyclin B; CDK2 regulated by cyclin A, cyclin E; CDK3 regulated by cyclin C; CDK4 regulated by cyclin D1, cyclin D2, cyclin D3; CDK5 regulated by CDK5R1, CDK5R2; CDK6 regulated by cyclin D1, cyclin D2, cyclin D3; CDK7 regulated by cyclin H; CDK8 regulated by cyclin C; CDK9 regulated by cyclin T1, cyclin T2a, cyclin T2b, cyclin K.

**Cyclins:** As used herein, unless indicated otherwise or contradictory in context, the term "cyclins" refers to a family of proteins that control the progression of cells through the cell cycle by activating cyclin-dependent kinase (CDK) enzymes. Cyclin D is one of the major cyclins produced in terms of its functional importance. It is known to interact with four CDKs: CDK2, 4, 5, and 6. In proliferating cells, cyclin D-CDK4/6 complex accumulation is of great importance for cell cycle progression. For instance, cyclin D-CDK4/6 complexes partially phosphorylates retinoblastoma tumor suppressor protein (Rb), whose inhibition can induce expression of some genes important for S phase progression.

**Patient/subject:** As used herein, unless indicated otherwise or contradictory in context, the term "patient" or "subject", which are used interchangeably, refers to any organism to which a composition in accordance with the invention may be administered, e.g., for experimental, diagnostic, prophylactic, and/or therapeutic purposes. Typical subjects include animals (e.g., mammals such as mice, rats, rabbits, non-human primates, and humans) and/or plants. As used herein, patients/subjects include those individuals who may seek or be in need of treatment, requires treatment, is receiving treatment, will receive treatment, or a subject who is under care by a trained professional for a particular disease or condition.

**Purified:** As used herein, unless indicated otherwise or contradictory in context, the term "purify," "purified," "purification" means to make substantially pure or clear from unwanted components, material defilement, admixture or imperfection.

**Targeted Cells:** As used herein, unless indicated otherwise or contradictory in context, the term "targeted cells" refers to any one or more cells of interest. The cells may be found in vitro, in vivo, in situ or in the tissue or organ of an organism. The organism may be an animal, preferably a mammal, more preferably a human and most preferably a patient.

**Molecule length:** As used herein, unless indicated otherwise or contradictory in context, the term molecule or peptide "length" or "size" means the longest 2D or 3D distance which may possibly be measured within the molecule. For cyclic molecules, "length" or "size" means the longest measurable distance across the cyclic structure. Throughout the present disclosure, when a molecule size or length is given (in general using the nanometre, nm, unit), the following procedures were used to calculate them:

- The so-called « 2D » procedure: a 2D chemical structure was drawn in e.g. the ChemDraw® Software. Then, size measurement was carried out via the available ChemDraw length measurement tools. The length value given herein corresponds to the longest 2D length of the molecule using the default settings 2D bond sizes and angles of the software.
- Alternatively, the so-called "3D" procedure may be followed:

  (1) Drawing of the chemical structure of the molecule using suitable softwares (such as ChemDraw).

  (2) Creating a 3D structure model of the molecule hereby drawn using SCWRL (Protein Sci. 2003; 12(9):2001-14) or MODELLER (Current Protocols in Bioinformatics. 15:5.6:5.6.1-5.6.30),

  (3) Incubating the obtained 3D structure model in a box simulation containing water for few milliseconds using AMBER (J. Computat. Chem. 2005; 26, 1668-1688),

  (4) Measuring the size of the molecule hereby obtained using softwares such as Pymol® using available Pymol

length measurement tools (DeLano Scientific LLC, http://www.pymol.org).

**Root Mean Square Deviation:** As used herein, unless indicated otherwise or contradictory in context, the term "Root Mean Square Deviation" or "RMSD" is well known in the art and means the square root of the arithmetic mean of the square of the distances between certain matched atoms. One can represent a molecular conformation as a vector whose components are the Cartesian coordinates of the molecule's atoms. Therefore, a conformation for a molecule with N atoms can be represented as a 3N-dimensional vector of real numbers. To calculate the RMSD of a pair of peptides or peptidomimetics (e.g. x and y), each one of them must be represented as a 3N-length (assuming N atoms) vector of coordinates. The RMSD is therefore the square root of the arithmetic mean of the square of the distances between corresponding atoms of x and y. It is a measure of the average atomic displacement between the conformations of the two structures:

$$\sqrt{\frac{1}{N}\sum_{i=1}^{N}|xi - yi|1^2}$$

In other words, the RMSD is the measure of the average distance between the atoms (usually the backbone atoms) of superimposed polypeptides or peptidomimetics. In the study of globular protein conformations, one customarily measures the similarity in three-dimensional structure by the RMSD of the C$\alpha$ atomic coordinates after optimal rigid body superposition.

The RMSD value of a given peptide or peptidomimetic with respect to a specifically selected reference structure (hereinafter may also be referred to as "PEPREF") may be calculated using various methods all well know by the skilled person. However, for the purpose of the present disclosure and for the avoidance of doubts, the RMSD of a given peptide or peptidomimetic as used in the present disclosure is obtained precisely using the following procedure:

<u>STEP 1:</u> Creating a 3-dimensional model of (i.e. obtaining 3D structure coordinates for) a peptide or peptidomimetic for which the RMSD is to be calculated, by:

<u>STEP 1.1:</u> Obtaining a set of polypeptide 3D structure coordinates based on the alignment with the sequence of a peptide or peptidomometic for which the RMSD value is to be calculated, using the BLAST algorithm according to the following procedure:

1. Open the following link to access the "Standard Protein Blast" tool: **http://blast.ncbi.nlm.nih.gov/Blast.cgi?PROGRAM=blastp&PAGE_TYPE=BlastSearch&LINK_L OC=-blasthome**
2. Enter the amino acid sequence of the peptide or peptidomimetic of interest in the "Enter Query Sequence" section. The alignment is performed one sequence after the other (this is not a multiple alignment tool).
3. In the section "Choose Search Set", choose the following database: Protein Data Bank Proteins (pdb).
4. In the section "Choose Search Set", do not exclude « Models (XM/XP) » and do not Exclude « Uncultured /environmental sample sequences ».
5. In the section "Program Selection", choose the following algorithm: blastp (protein-protein BLAST)
6. Leave other fields as shown on the screenshot in Figure 20.
7. Run BLAST.
8. The results obtained from the query are presented in the form of several pdb files.
9. From this output results, the first ten (10) PDB files corresponding to the best sequence alignments are retained. This set of 10 PDB files or structures will be used in the next step (Step 2: structural alignments with STAMP).
10. Finally, clean up the 10 structures contained in the 10 PDB files by removing all *e.g.* additional small molecules, receptors or portions thereof, dimers or portions thereof, so as to retain only the polypeptide chain of interest.

The set of pdf files contains the polypeptide 3D structure coordinates of the 10 structures having the highest sequence homology with the peptide or peptidomometic for which the RMSD value is to be calculated.

<u>STEP 1.2:</u> Performing the structural alignment of the set of 3D structure coordinates obtained in STEP 1.1, thereby obtaining a set of aligned polypeptide 3D structure coordinates, by using STAMP (Structural Alignment of Multiple Proteins Version 4.2) according to the following procedure:

1. Open the following link to access the "STAMP superposition" tool: **http://www.russelllab.org/cgi-bin/pdc/stamp.pl**
2. In the section entitled "Structure A", input the PDB file corresponding to the first structure from the set of ten 3D structure coordinates obtained in STEP 1.1, which corresponds to the best sequence alignment with BLAST.

3. In the section entitled "Structure B", input the PDB file corresponding to the second structure from the set of ten 3D structure coordinates obtained in STEP 1.1, which corresponds to the second best sequence alignment with BLAST.
4. Run STAMP.
5. Repeat steps 2 to 4 with the other eight pdb files from the set of ten 3D structure coordinates identified in STEP 1.1 by successively entering the PDB files in the field "Structure B".
6. As a result, the structural alignment of the 10 structures contained in the set of PDB files obtained in STEP 1.1 is obtained in the form of 9 disctinct PDB files each containing a pair of aligned polypeptide 3D structure (structure 1 with structure 2, structure 1 with structure 3, structure 1 with structure 4, ... , structure 1 with structure 10).
7. From these 9 "pair" PDB files, 10 PDB files each containing one of structures 1 to 10 are created.
8. 10 PDB files containing the aligned 3D structure coordinates are thus obtained from STEP 1.2. for use in the next step.

STEP 1.3: Modelling the sequence of peptide or peptidomometic for which the RMSD value is to be calculated against the set of aligned polypeptide 3D structure coordinates obtained in STEP 1.2, thereby obtaining a set of 3D structure coordinates for the peptide or peptidomometic for which the RMSD value is to be calculated, using SCWRL (reference: "SCWRL and MolIDE: computer programs for side-chain conformation prediction and homology modeling", Nature Protocols VOL.3 NO.12 2008, Qiang Wang et al.; according to the following procedure:

1. Insert the input sequence of a peptide or peptidomometic for which the RMSD value is to be calculated in Fasta format.
2. Import the first PDB file containing the aligned polypeptide 3D structure coordinates obtained in STEP 1.2.
3. Run SCWRL by typing the following command for Unix based systems: "scwrl_path/scwrl3 -i inputpdbfile -o outputpdbfile -s sequencefile 4 logfile".
4. As a result, a first PBD file is obtained containing the predicted 3D structure coordinates of the peptide or peptidomometic for which the RMSD value is to be calculated.
5. Repeat steps 1 to 3 using the 9 remaining PDB files obtained in STEP 1.2.
6. 10 PDB files are obtained from STEP 1.3 for use in the following STEP 1.4.

STEP 1.4: Minimizing the free energy (ΔG) of the set of 3D structure coordinates for the peptide or peptidomometic for which the RMSD value is to be calculated obtained in STEP 1.3 using GROMACS (Reference: Hess B, Kutzner C, Van Der Spoel D, Lindahl E (2008). "GROMACS 4: Algorithms for Highly Efficient, Load-Balanced, and Scalable Molecular Simulation". J Chem Theory Comput 4 (2): according to the following procedure:

1. Create a Gromacs topology (gmx) file from the first PDB file of the modeled peptide or peptidomometic for which the RMSD value is to be calculated obtained in STEP 1.3, by using the command « pdb2gmx -f NOMDUFICHIERPDB.pdb -water spc». "NOMDUFICHIERPDB" is the name of the input PDB file.
2. Create a box around the imported modeled peptide or peptidomometic by using the command « editconf -f conf.gro -bt cubic d 0.7 o box.gro».
3. Add solvent (water) molecules into the box by using the command « genbox -cp box.gro -cs spc216.gro -p topol.top -o solvated.gro».
4. Prepare the input for the molecular dynamics (MD) run with the command « vim em.mdp ». Default run is set to 1000 nsteps.
5. Create an input for the MD run by using the command « grompp -f em.mdp -p topol.top -c solvated.gro -o em.tpr».
6. Run the command « mdrun -v -deffnm em» to perform the actual energy minimization.
7. Run the command « g energy -f em.edr -s em.tpr -o em.xvg » and then run option « 7 ».
8. As a result, a first XMG file is obtained. To view the XMG file run the command « xmgrace em.xvg ».
9. Repeat steps 1 to 8 with the 9 remaining structures obtained in STEP 1.3. 10 XMG files are thus obtained.
10. The structure of lowest energy is obtained from each XMG file in the form of a PDB file. 10 PDB files each containing one structure of lowest energy are thus obtained from STEP 1.4 for use in the next step.

STEP 2: Calculating the RMSD of the peptide or peptidomimetic for which the RMSD value is to be calculated by comparing the 3D structure coordinates of the peptide or peptidomimetic obtained in STEP 1.4 with the 3D structure coordinates of PEPREF to obtain the lowest possible RMSD value using FATCAT (Flexible structure AlignmenT by Chaining Aligned fragment pairs allowing Twists) according to the following procedure:

1. Open the following link to access the "FATCAT" software: **http://fatcat.burnham.org**
2. Open the "pairwise alignment" tool.
3. Import the PDB file containing the structure coordinates of PEPREF in the "Get the 1st structure" section.

4. Import the first PDB file of the peptide or peptidomimetic for which the RMSD value is to be calculated with minimized energy obtained in STEP 1.4.
5. Run FATCAT.
6. As a result, a first RMSD value of the first structure of the peptide or peptidomimetic for which the RMSD value is to be calculated as obtained in STEP 1.4 will be obtained in the output report.
7. Repeat steps 1 to 5 with the 9 remaining structures (PDB files) obtained from STEP 1.4.
8. The peptide or peptidomimetic structure with the lowest RMSD (out of the ten RMSD values successively obtained) is the value taken into account in the present application.

**3D structure coordinates of PEPREF:** As used herein, unless indicated otherwise or contradictory in context, the 3D structure coordinates of PEPREF are as follows:

| | | | | | | | | |
|---|---|---|---|---|---|---|---|---|
| ATOM | 511 | N | LYS | A | 1 | -14.570 | 46.437 | 27.424 |
| ATOM | 512 | CA | LYS | A | 1 | -13.512 | 45.748 | 28.151 |
| ATOM | 513 | C | LYS | A | 1 | -13.655 | 44.259 | 27.884 |
| ATOM | 514 | O | LYS | A | 1 | -12.769 | 43.463 | 28.197 |
| ATOM | 515 | CB | LYS | A | 1 | -13.605 | 46.029 | 29.652 |
| ATOM | 516 | CG | LYS | A | 1 | -13.640 | 47.509 | 29.991 |
| ATOM | 517 | CD | LYS | A | 1 | -12.615 | 48.297 | 29.183 |
| ATOM | 518 | CE | LYS | A | 1 | -12.625 | 49.768 | 29.575 |
| ATOM | 519 | NZ | LYS | A | 1 | -13.994 | 50.369 | 29.497 |
| ATOM | 520 | N | ILE | A | 2 | -14.792 | 43.890 | 27.309 |
| ATOM | 521 | CA | ILE | A | 2 | -15.051 | 42.499 | 26.967 |
| ATOM | 522 | C | ILE | A | 2 | -14.911 | 42.370 | 25.444 |
| ATOM | 523 | O | ILE | A | 2 | -15.531 | 43.125 | 24.683 |
| ATOM | 524 | CB | ILE | A | 2 | -16.466 | 42.065 | 27.401 |
| ATOM | 525 | CG1 | ILE | A | 2 | -16.630 | 42.238 | 28.915 |
| ATOM | 526 | CG2 | ILE | A | 2 | -16.710 | 40.629 | 26.985 |
| ATOM | 527 | CD1 | ILE | A | 2 | -15.631 | 41.478 | 29.30 |
| ATOM | 528 | N | PRO | A | 3 | -14.085 | 41.411 | 24.989 |
| ATOM | 529 | CA | PRO | A | 3 | -13.789 | 41.109 | 23.588 |
| ATOM | 530 | C | PRO | A | 3 | -14.998 | 40.695 | 22.768 |
| ATOM | 531 | O | PRO | A | 3 | -15.969 | 40.164 | 23.305 |
| ATOM | 532 | CB | PRO | A | 3 | -12.785 | 39.968 | 23.688 |
| ATOM | 533 | CG | PRO | A | 3 | -12.156 | 40.166 | 25.007 |
| ATOM | 534 | CD | PRO | A | 3 | -13.330 | 40.506 | 25.867 |
| ATOM | 535 | N | LYS | A | 4 | -14.937 | 40.937 | 21.463 |
| ATOM | 536 | CA | LYS | A | 4 | -16.023 | 40.529 | 20.590 |
| ATOM | 537 | C | LYS | A | 4 | -15.886 | 39.015 | 20.391 |
| ATOM | 538 | O | LYS | A | 4 | -14.903 | 38.415 | 20.831 |
| ATOM | 539 | CB | LYS | A | 4 | -15.926 | 41.244 | 19.245 |
| ATOM | 540 | CG | LYS | A | 4 | -15.802 | 42.751 | 19.355 |
| ATOM | 541 | CD | LYS | A | 4 | -16.292 | 43.433 | 18.083 |
| ATOM | 542 | CE | LYS | A | 4 | -16.162 | 44.943 | 18.177 |

(continued)

| | | | | | | | | |
|---|---|---|---|---|---|---|---|---|
| ATOM | 543 | NZ | LYS | A | 4 | -16.825 | 45.628 | 17.019 |
| ATOM | 544 | N | ALA | A | 5 | -16.85 | 38.393 | 19.759 |
| ATOM | 545 | CA | ALA | A | 5 | -16.811 | 36.955 | 19.507 |
| ATOM | 546 | C | ALA | A | 5 | -15.772 | 36.771 | 18.416 |
| ATOM | 547 | O | ALA | A | 5 | -15.727 | 37.534 | 17.455 |
| ATOM | 548 | CB | ALA | A | 5 | -18.168 | 36.419 | 19.043 |
| ATOM | 549 | N | CYS | A | 6 | -14.935 | 35.756 | 18.562 |
| ATOM | 550 | CA | CYS | A | 6 | -13.887 | 35.518 | 17.584 |
| ATOM | 551 | C | CYS | A | 6 | -14.347 | 34.765 | 16.338 |
| ATOM | 552 | O | CYS | A | 6 | -15.327 | 34.018 | 16.368 |
| ATOM | 553 | CB | CYS | A | 6 | -12.743 | 34.768 | 18.241 |
| ATOM | 554 | SG | CYS | A | 6 | -11.198 | 34.959 | 17.353 |
| ATOM | 555 | N | CYS | A | 7 | -13.623 | 34.973 | 15.243 |
| ATOM | 556 | CA | CYS | A | 7 | -13.931 | 34.328 | 13.969 |
| ATOM | 557 | C | CYS | A | 7 | -13.091 | 33.071 | 13.798 |
| ATOM | 558 | O | CYS | A | 7 | -11.961 | 33.123 | 13.302 |
| ATOM | 559 | CB | CYS | A | 7 | -13.653 | 35.290 | 12.824 |
| ATOM | 560 | SG | CYS | A | 7 | -13.930 | 34.633 | 11.154 |
| ATOM | 561 | N | VAL | A | 8 | -13.654 | 31.941 | 14.209 |
| ATOM | 562 | CA | VAL | A | 8 | -12.949 | 30.684 | 14.110 |
| ATOM | 563 | C | VAL | A | 8 | -13.653 | 29.733 | 13.157 |
| ATOM | 564 | O | VAL | A | 8 | -14.759 | 30.016 | 12.687 |
| ATOM | 565 | CB | VAL | A | 8 | -12.814 | 30.038 | 15.492 |
| ATOM | 566 | CG1 | VAL | A | 8 | -11.807 | 30.825 | 16.337 |
| ATOM | 567 | CG2 | VAL | A | 8 | -14.161 | 30.006 | 16.170 |
| ATOM | 568 | N | PRO | A | 9 | -13.003 | 28.601 | 12.828 |
| ATOM | 569 | CA | PRO | A | 9 | -13.593 | 27.615 | 11.918 |
| ATOM | 570 | C | PRO | A | 9 | -14.726 | 26.886 | 12.631 |
| ATOM | 571 | O | PRO | A | 9 | -14.581 | 26.476 | 13.780 |
| ATOM | 572 | CB | PRO | A | 9 | -12.423 | 26.676 | 11.601 |
| ATOM | 573 | CG | PRO | A | 9 | -11.204 | 27.487 | 11.925 |
| ATOM | 574 | CD | PRO | A | 9 | -11.620 | 28.226 | 13.163 |
| ATOM | 575 | N | THR | A | 10 | -15.847 | 26.721 | 11.942 |
| ATOM | 576 | CA | THR | A | 10 | -16.999 | 26.060 | 12.527 |
| ATOM | 577 | C | THR | A | 10 | -17.334 | 24.767 | 11.804 |
| ATOM | 578 | O | THR | A | 10 | -18.097 | 23.943 | 12.303 |
| ATOM | 579 | CB | THR | A | 10 | -18.211 | 27.010 | 12.523 |
| ATOM | 580 | OG1 | THR | A | 10 | -18.491 | 27.445 | 11.185 |
| ATOM | 581 | CG2 | THR | A | 10 | -17.902 | 28.230 | 13.375 |
| ATOM | 582 | N | GLU | A | 11 | -16.750 | 24.586 | 10.627 |

(continued)

| | | | | | | | | |
|---|---|---|---|---|---|---|---|---|
| ATOM | 583 | CA | GLU | A | 11 | -16.980 | 23.377 | 9.848 |
| ATOM | 584 | C | GLU | A | 11 | -15.643 | 22.935 | 9.246 |
| ATOM | 585 | O | GLU | A | 11 | -15.029 | 23.666 | 8.464 |
| ATOM | 586 | CB | GLU | A | 11 | -17.981 | 23.624 | 8.715 |
| ATOM | 587 | CG | GLU | A | 11 | -19.421 | 23.807 | 9.163 |
| ATOM | 588 | CD | GLU | A | 11 | -19.686 | 25.166 | 9.770 |
| ATOM | 589 | OE1 | GLU | A | 11 | -19.478 | 26.175 | 9.073 |
| ATOM | 590 | OE2 | GLU | A | 11 | -20.111 | 25.227 | 10.939 |
| ATOM | 591 | N | LEU | A | 12 | -15.183 | 21.749 | 9.622 |
| ATOM | 592 | CA | LEU | A | 12 | -13.923 | 21.254 | 9.104 |
| ATOM | 593 | C | LEU | A | 12 | -14.062 | 19.912 | 8.386 |
| ATOM | 594 | O | LEU | A | 12 | -15.136 | 19.299 | 8.359 |
| ATOM | 595 | CB | LEU | A | 12 | -12.893 | 21.144 | 10.230 |
| ATOM | 596 | CG | LEU | A | 12 | -12.660 | 22.422 | 11.054 |
| ATOM | 597 | CD1 | LEU | A | 12 | -13.475 | 22.350 | 12.337 |
| ATOM | 598 | CD2 | LEU | A | 12 | -11.181 | 22.586 | 11.399 |
| ATOM | 599 | N | SER | A | 13 | -12.971 | 19.476 | 7.771 |
| ATOM | 600 | CA | SER | A | 13 | -12.964 | 18.218 | 7.046 |
| ATOM | 601 | C | SER | A | 13 | -11.568 | 17.628 | 7.164 |
| ATOM | 602 | O | SER | A | 13 | -10.613 | 18.320 | 7.550 |
| ATOM | 603 | CB | SER | A | 13 | -13.346 | 18.435 | 5.578 |
| ATOM | 604 | OG | SER | A | 13 | -12.404 | 19.261 | 4.923 |
| ATOM | 605 | N | ALA | A | 14 | -11.449 | 16.352 | 6.818 |
| ATOM | 606 | CA | ALA | A | 14 | -10.179 | 15.665 | 6.949 |
| ATOM | 607 | C | ALA | A | 14 | -9.421 | 15.471 | 5.652 |
| ATOM | 608 | O | ALA | A | 14 | -9.941 | 15.720 | 4.563 |
| ATOM | 609 | CB | ALA | A | 14 | -10.413 | 14.306 | 7.626 |
| ATOM | 610 | N | ILE | A | 15 | -8.171 | 15.046 | 5.783 |
| ATOM | 611 | CA | ILE | A | 15 | -7.343 | 14.746 | 4.623 |
| ATOM | 612 | C | ILE | A | 15 | -6.475 | 13.559 | 5.004 |
| ATOM | 613 | O | ILE | A | 15 | -6.212 | 13.316 | 6.183 |
| ATOM | 614 | CB | ILE | A | 15 | -6.401 | 15.916 | 4.183 |
| ATOM | 615 | CG1 | ILE | A | 15 | -5.284 | 16.106 | 5.200 |
| ATOM | 616 | CG2 | ILE | A | 15 | -7.188 | 17.211 | 3.982 |
| ATOM | 617 | CD1 | ILE | A | 15 | -4.173 | 16.973 | 4.696 |
| ATOM | 618 | N | SER | A | 16 | -6.045 | 12.806 | 3.999 |
| ATOM | 619 | CA | SER | A | 16 | -5.187 | 11.662 | 4.242 |
| ATOM | 620 | C | SER | A | 16 | -3.740 | 12.089 | 4.217 |
| ATOM | 621 | O | SER | A | 16 | -3.360 | 13.020 | 3.508 |
| ATOM | 622 | CB | SER | A | 16 | -5.416 | 10.584 | 3.185 |

(continued)

| | | | | | | | | |
|---|---|---|---|---|---|---|---|---|
| ATOM | 623 | OG | SER | A | 16 | -6.667 | 9.971 | 3.401 |
| ATOM | 624 | N | MET | A | 17 | -2.933 | 11.409 | 5.012 |
| ATOM | 625 | CA | MET | A | 17 | -1.518 | 11.700 | 5.047 |
| ATOM | 626 | C | MET | A | 17 | -0.778 | 10.414 | 5.244 |
| ATOM | 627 | O | MET | A | 17 | -1.137 | 9.594 | 6.078 |
| ATOM | 628 | CB | MET | A | 17 | -1.170 | 12.694 | 6.164 |
| ATOM | 629 | CG | MET | A | 17 | -1.848 | 14.042 | 5.974 |
| ATOM | 630 | SD | MET | A | 17 | -1.017 | 15.431 | 6.760 |
| ATOM | 631 | CE | MET | A | 17 | -0.799 | 14.823 | 8.475 |
| ATOM | 632 | N | LEU | A | 18 | 0.238 | 10.231 | 4.426 |
| ATOM | 633 | CA | LEU | A | 18 | 1.077 | 9.065 | 4.508 |
| ATOM | 634 | C | LEU | A | 18 | 2.289 | 9.610 | 5.264 |
| ATOM | 635 | O | LEU | A | 18 | 2.939 | 10.565 | 4.818 |
| ATOM | 636 | CB | LEU | A | 18 | 1.461 | 8.608 | 3.100 |
| ATOM | 637 | CG | LEU | A | 18 | 2.324 | 7.355 | 2.955 |
| ATOM | 638 | CD1 | LEU | A | 18 | 1.553 | 6.145 | 3.445 |
| ATOM | 639 | CD2 | LEU | A | 18 | 2.723 | 7.190 | 1.492 |
| ATOM | 640 | N | TYR | A | 19 | 2.581 | 9.029 | 6.418 |
| ATOM | 641 | CA | TYR | A | 19 | 3.706 | 9.501 | 7.196 |
| ATOM | 642 | C | TYR | A | 19 | 4.434 | 8.333 | 7.835 |
| ATOM | 643 | O | TYR | A | 19 | 4.081 | 7.186 | 7.603 |
| ATOM | 644 | CB | TYR | A | 19 | 3.222 | 10.458 | 8.281 |
| ATOM | 645 | CG | TYR | A | 19 | 2.386 | 9.782 | 9.346 |
| ATOM | 646 | CD1 | TYR | A | 19 | 1.029 | 9.527 | 9.147 |
| ATOM | 647 | CD2 | TYR | A | 19 | 2.961 | 9.379 | 10.550 |
| ATOM | 648 | CE1 | TYR | A | 19 | 0.273 | 8.894 | 10.128 |
| ATOM | 649 | CE2 | TYR | A | 19 | 2.218 | 8.745 | 11.526 |
| ATOM | 650 | CZ | TYR | A | 19 | 0.877 | 8.508 | 11.317 |
| ATOM | 651 | OH | TYR | A | 19 | 0.134 | 7.922 | 12.318 |
| ATOM | 652 | N | LEU | A | 20 | 5.439 | 8.651 | 8.650 |
| ATOM | 653 | CA | LEU | A | 20 | 6.255 | 7.661 | 9.347 |
| ATOM | 654 | C | LEU | A | 20 | 6.210 | 7.946 | 10.847 |
| ATOM | 655 | O | LEU | A | 20 | 6.685 | 8.992 | 11.288 |
| ATOM | 656 | CB | LEU | A | 20 | 7.701 | 7.763 | 8.871 |
| ATOM | 657 | CG | LEU | A | 20 | 7.901 | 7.850 | 7.359 |
| ATOM | 658 | CD1 | LEU | A | 20 | 9.300 | 8.379 | 7.039 |
| ATOM | 659 | CD2 | LEU | A | 20 | 7.669 | 6.482 | 6.748 |

**Structure coordinates:** As used herein, unless indicated otherwise or contradictory in context, the "structure coordinates" refers to Cartesian coordinates derived from mathematical equations related to the patterns obtained on diffraction of a monochromatic beam of X-rays by the atoms (scattering centers) of a protein, protein complex or peptide in

crystal form. The diffraction data are used to calculate an electron density map of the repeating unit of the crystal. The electron density maps are then used to establish the positions of the individual atoms of the molecule or molecular complex.

**STAMP:** STAMP (Structural Alignment of Multiple Proteins) is a tool for aligning protein sequences based on a three-dimensional structure. Its algorithm minimizes the C$\alpha$ distance between aligned residues of each molecule by applying globally optimal rigid-body rotations and translations. This program provides some information on the equivalence of the residues between the selected models.

**SCWRL:** This program predicts and optimizes the protein side-chain conformations. It is using the backbone of a support protein and a backbone-dependent rotamer library. The possible conformations are explored by minimizing the steric hindrance between the side-chains and between the side-chains and the backbone.

**GROMACS:** GROMACS is a molecular dynamics package. The "gmx rms" tool included in GROMACS compares two structures by computing the root mean square deviation (RMSD).

For the avoidance of doubts, the term "pharmaceutical association" or "pharmaceutical combination" as used herein refers to a compound or substance comprising at least two components, *i.e.* a (modified) GFR-binding compound and a bioactive carrier, linked, connected or bound through at least one covalent or non-covalent link, connection or bond.

The present disclosure describes non-mutagenic extracellular therapies having the ability to direct cell fate. It is thus possible to convert or recode a neoplastic cell by modifying its surrounding extracellular micro-environment, in-vitro, ex-vivo or in-vivo, so that the cell operates self-recovery or self-healing and a subject possessing such a neoplastic cell may be protected from a neoplastic disease.

In one example, a neoplastic cell may operate self-recovery or self-healing *e.g.* by inducing a quiescence state so that the neoplastic cell may remain inactive or dormant for seconds, minutes, hours, days, weeks, months or years, in particular, will never resume neoplasia; and/or by preventing, reducing or suppressing cell division and/or cell proliferation, preferably uncontrolled cell division and/or cell proliferation of said neoplastic cell; and/or by regulating or promoting anti-mitogen activity and/or tumour suppressor pathways and/or anti-oncogenic activity in said neoplastic cell; and/or by inducing cytostaticity and not cytotoxicity in the neoplastic cell; and/or by inducing differentiation; and/or by regulating and/or modulating the adhesion or interactions between the cell and its micro-environment (i.e. the surrounding ECM) so as activate, reactivate or restore cell adhesion checkpoints in said neoplastic cell.

In one aspect, the present disclosure provides a pharmaceutical association or combination having the ability to convert or recode, extracellularly, a neoplastic cell, in-vitro, ex-vivo or in-vivo, so that it may be used in the treatment, prevention and/or diagnostic of a neoplastic disease, said association comprising at least one growth factor receptor-binding compound which activates at least one growth factor receptor of a neoplastic cell and at least one bioactive carrier which forms at least one covalent or non-covalent association with said at least one growth factor receptor-binding compound, and wherein said association reduces or suppresses (i) the gene expression of at least one cyclin D in the neoplastic cell and/or (ii) reduces or suppresses the formation of at least one complex formed between said at least one cyclin D and at least one of cyclin dependent-kinase (CDK) 4 or 6 in the neoplastic cell; wherein said growth factor receptor-binding compound comprises a peptide with four amino acids PEP1; wherein PEP1 is SAIS; wherein said growth factor receptor-binding compound is a peptide or a peptidomimetic; wherein said growth factor receptor-binding compound is a non-cyclic peptide with between 8 and 25 amino acids or a non-cyclic peptidomimetic having a molecular weight comprised between 600 and 4,000 Daltons and comprises between 8 and 25 amino acids; wherein said growth factor receptor-binding compound is a cyclic peptide with between 10 and 60 amino acids or a cyclic peptidomimetic comprising between 10 and 60 amino acids having a molecular weight comprised between 1,000 and 7,000 Da; and wherein said bioactive carrier is a biomaterial.

## II. Growth factor receptor-binding compounds

The present disclosure describes pharmaceutical associations or combinations comprising at least one growth factor receptor-binding compound as defined herein and a bioactive carrier as defined herein, said associations or combinations having the ability to convert or recode a neoplastic cell into a non-neoplastic cell.

As used herein, the term "Growth factor receptor-binding compound" or "GFR-binding compound" refers to an exogenous or endogenous compound, molecule or substance (a) having an (binding) affinity for a growth factor receptor as defined herein, (b) comprising the ability to associate or combine with a bioactive carrier as defined herein, and (c) comprising the ability to activate a growth factor receptor as defined herein.

There are many ways to test, measure and present the binding affinity of a given substance for a given receptor, but for the purpose of the present disclosure, and for the avoidance of any doubts, the (binding) affinity values of a given GFR-binding compound to a given GFR are provided using the method of fluorescence anisotropy. In this method, a GFR-binding compound is fluorescently labelled using technics well established in the art. Binding of the resulting labelled compound to a growth factor receptor results in a fluctuation of fluorescence anisotropy which is used to construct an affinity binding curve from which the GFR-binding compound binding affinity value is derived. Using this technique, binding

affinity values are given in the form of dissociation constants Kd. In certain embodiments, GFR-binding compounds of the present disclosure have Kd values as measured by fluorescence anisotropy of more than 1 (one) picomolar (pM). In certain embodiments, GFR-binding compounds of the present disclosure have Kd values as measured by fluorescence anisotropy of more than 1 (one) nanomolar (nM). In certain embodiments, GFR-binding compounds of the present disclosure have Kd values as measured by fluorescence anisotropy of more than 10 (ten) nanomolar (nM). In certain embodiments, GFR-binding compounds of the present disclosure have Kd values as measured by fluorescence anisotropy of more than 100 (one hundred) nanomolar (nM). In certain embodiments, GFR-binding compounds of the present disclosure have Kd values as measured by fluorescence anisotropy of more than 1 (one) micromolar (μM). In certain embodiments, GFR-binding compounds of the present disclosure have Kd values as measured by fluorescence anisotropy of more than 10 (ten) micromolar (μM). In certain embodiments, GFR-binding compounds of the present disclosure have Kd values as measured by fluorescence anisotropy of more than 100 (one hundred) micromolar (μM).

There are many ways to test and measure the ability of a given substance to activate a given receptor, but for the purpose of the present disclosure, and for the avoidance of any doubts, a given GFR-binding compound activates a growth factor receptor if it induces growth factor receptor phosphorylation as measured by the western blot method. There are many distinct phosphorylation sites on growth factor receptors and they may widely vary according to the type of growth factor receptor as reported in the published scientific article from Mark A. Lemmon and Joseph Schlessinger, "Cell Signaling by Receptor Tyrosine Kinases", Cell. 2010; 141(7), 1117-1134,

A GFR-binding compound is said to possess the ability to associate or combine with a bioactive carrier if it comprises a functional chemical element, function or group allowing for the covalent or non-covalent assembly of the GFR-binding compound and the bioactive carrier. Such a functional chemical element, function or group, also referred to as a bioactive carrier-affinity-contaning group or bioactive carrier-high-affinity-containing group, include, but is not limited to, a thiol-containing compound, a cysteine-containing compound, a cysteine, or a GTPGP or a WWFWG peptide fragment.

**Growth factor receptor:** As used herein, unless indicated otherwise or contradictory in context, the term "growth factor receptor" or "GFR" is a receptor which binds to growth factors which are naturally occurring substances capable of stimulating, for instance, cellular growth, proliferation, healing, and cellular differentiation. Suitable as growth factor receptors for implementing embodiments of the present invention include epidermal growth factor receptors (EGFR), fibroblast growth factor receptors (FGFR), vascular endothelial growth factor receptors (VEGFR), nerve growth factor receptors (NGFR), Insulin receptor family, Trk receptor family, Eph receptor family, AXL receptor family, LTK receptor family, TIE receptor family, ROR receptor family, DDR receptor family, RET receptor family, KLG receptor family, RYK receptor family, MuSK receptor family, hepatocyte growth factor receptors (HGFR), somatomedin or insulin-like growth factor receptors (SGFR), platelet-derived growth factor receptors (PDGFR), transforming growth factor beta (TGF-β) superfamily proteins such as AMH, ARTN, BMP10, BMP15, BMP2, BMP3, BMP4, BMP5, BMP6, BMP7, BMP8A, BMP8B, GDF1, GDF10, GDF11, GDF15, GDF2, GDF3, GDF3A, GDF5, GDF6, GDF7, GDF8, GDF9, GDNF, INHA, INHBA, INHBB, INHBC, INHBE, LEFTY1, LEFTY2, MSTN, NODAL, NRTN, PSPN, TGFB1, TGFB2 and TGFB3, and any combination thereof.

**Growth factor:** As used herein, unless indicated otherwise or contradictory in context, the term "growth factor" refers to any substance(s) having the ability to bind to a growth factor receptor and produce (a) biological effect(s) or reaction(s), such as promoting the growth of tissues, by activating such a growth factor receptor. Exemplary growth factors include, but are not limited to, platelet-derived growth factor (PDGF), platelet-derived angiogenesis factor (PDAF), vascular endotheial growth factor (VEGF), platelet-derived epidermal growth factor (PDEGF), transforming growth factor beta (TGF-β), transforming growth factor A (TGF-A), epidermal growth factor (EGF), fibroblast growth factor (FGF), acidic fibroblast growth factor (FGF-A), basic fibroblast growth factor (FGF-B), insulin-like growth factors 1 and 2 (IGF-I and IGF-2), keratinocyte growth factor (KGF), tumor necrosis factor (TNF), fibroblast growth factor (FGF) and interleukin-1 (IL-I), Keratinocyte Growth Factor-2 (KGF-2), and combinations thereof.

**Activation of growth factor receptors:** As used herein, unless indicated otherwise or contradictory in context, the term "activating" or "activation of", when used in relation to a growth factor receptor, refers to the phosphorylation of the tyrosine kinase domain of such a growth factor receptor.

In one aspect, the present disclosure provides a GFR-binding compound, as part of a pharmaceutical association, combination or composition as defined herein, as an active principle for use in methods and uses described herein.

In one particular example, the growth factor receptor involved in the interaction with said GFR-binding compound is an epidermal growth factor receptor. In one particular example, the growth factor receptor involved in the interaction with said GFR-binding compound is a fibroblast growth factor receptor. In one particular example, the growth factor receptor involved in the interaction with said GFR-binding compound is a vascular endothelial growth factor receptor. In one particular example, the growth factor receptor involved in the interaction with said GFR-binding compound is a nerve growth factor receptor. In one particular example, the growth factor receptor involved in the interaction with said GFR-binding compound is a hepatocyte growth factor receptor. In one particular example, the growth factor receptor involved in the interaction with said GFR-binding compound is a somatomedin or insulin-like growth factor receptor. In one particular example, the growth factor receptor involved in the interaction with said GFR-binding compound is a platelet-derived

growth factor receptor. In one particular example, the growth factor receptor involved in the interaction with said GFR-binding compound is a protein from the transforming growth factor beta (TGF-β) superfamily.

In one particular example, the growth factor receptor(s) involved in the interaction with said GFR-binding compound is (are) preferably selected from epidermal growth factor receptors, fibroblast growth factor receptors, vascular endothelial growth factor receptors, nerve growth factor receptors, hepatocyte growth factor receptors, somatomedin or insulin-like growth factor receptors, platelet-derived growth factor receptors, and transforming growth factor beta (TGF-β) superfamily proteins.

In one particular example, the gene expression of cyclin-D, in a neoplastic cell, is reduced, downregulated, inhibited or suppressed during phase G1 of the cell cycle. In one particular example, the gene expression of cyclin-D is reduced or suppressed for substantially at least the entire duration of phase G1 of a cell cycle. In one particular example, the gene expression of cyclin-D is reduced by at least 20%. In one particular example, the gene expression of cyclin-D is reduced by at least 30%. In one particular example, the gene expression of cyclin-D is reduced by at least 40%. In one particular example, the gene expression of cyclin-D is reduced by at least 50%. In one particular example, the gene expression of cyclin-D is reduced by at least 60%. In one particular example, the gene expression of cyclin-D is reduced by at least 70%. In one particular example, the gene expression of cyclin-D is reduced by at least 80%. At least 40% is particularly preferred. Reduction of cyclin D gene expression is assessed with respect to the wild-type gene expression of cyclin-D and is measured by Quantitative Real Time Polymerase Chain Reaction (Q-PCR or RT-PCR) by (i) extracting RNA from the treated cells, (ii) converting the extracted RNA into the corresponding cDNA, (iii) subjecting the obtained cDNA to a real-time PCR amplification, (iv) analysing the data obtained from the real-time PCR amplification and comparing them with the data obtained by the ΔΔCt method, and (v) comparing the obtained values to the wild-type value.

In more details, Quantitative Real Time Polymerase Chain Reaction is carried out by (i) extracting RNA from the treated cells using the RNeasy total RNA kit from Qiagen®, (ii) converting the extracted RNA into the corresponding cDNA using a reverse transcription reaction (Gibco Brl®) and random primers from Invitrogen®, (iii) subjecting the obtained cDNA to a real-time PCR amplification in the presence of SYBR green reagents from Bio-Rad® in a thermocycler (iCycler, Biorad®), (iv) analysing the data obtained from the real-time PCR amplification with the iCycler IQ™ software following the iCycler iQ™ Real-Time PCR Detection System's instruction manual (Catalog Number 170-8740) and using weighted mean as a digital filter, PCR Baseline Subtracted Curve Fit as the analysis mode, FAM/490 as a fluorophore, the automatic calculation of the baseline cycles and the threshold, and the default settings for all other parameters, and comparing them with the data obtained by the ΔΔCt method as reported and described in Livak, K. J. and T. D. Schmittgen. "Analysis of relative gene expression data using real-time quantitative PCR and the 2(-Delta Delta C(T)) Method", Methods (2001) 25(4): 402-408, and (v) comparing the obtained values to the wild-type value.

In one particular example, the gene expression of cyclin-D is maintained at such a reduced level (at least 20%, at least 30%, at least 40%, at least 50%, at least 60%, at least 70%, or at least 80%) during phase G1 of a cell cycle of the treated neoplastic cell. In one example, the gene expression of cyclin-D is maintained at such a reduced level during substantially the entire duration of the G1 phase. In one example, the gene expression of cyclin-D is maintained at such a reduced level during substantially the entire duration of the G1 and S phases. In one example, the gene expression of cyclin-D is maintained at such a reduced level during substantially the entire duration of the G1, S and G2 phases. In one example, the gene expression of cyclin-D is maintained at such a reduced level during substantially the entire duration of the G1, S, G2 and M phases *i.e.* during substantially the entire duration of a cell cycle of a treated neoplastic cell. Reduction of the gene expression level of cyclin D during substantially the entire duration of G1 is preferred.

As used herein, unless indicated otherwise or contradictory in context, the term "wild-type expression" of a protein or a gene, refers to the expression of a protein or a gene observed in normal, standard biological conditions i.e., in the present disclosure, without the presence of, or prior to the provision or administration to a neoplastic cell of a pharmaceutical association, combination or composition as defined herein. In-vitro, ex-vivo or in-vivo natural expression level of a protein or a gene in a neoplastic cell may thus be used as a comparative data (or control) to assess and quantify the effect of the presence or administration of a pharmaceutical association, combination or composition as defined herein on the expression level of such a protein or gene in that cell.

Suitable GFR-binding compounds for implementing certain embodiments of the invention include, without being limited to, linear (i.e. non-cyclic) GFR-binding compounds such as peptides or peptidomimetics, and cyclic GFR-binding compounds such as cyclic peptides or cyclic peptidomimetics.

### Non-cyclic GFR-binding compounds

In one particular example, said non-cyclic GFR-binding compound has a molecular weight comprised between 600 and 4,000 Daltons. In one particular example, said non-cyclic GFR-binding compound has a molecular weight comprised between 800 and 4,000 Daltons. In one particular example, said non-cyclic GFR-binding compound has a molecular weight comprised between 600 and 3,000 Daltons. In one particular example, said non-cyclic GFR-binding compound has a molecular weight comprised between 800 and 3,000 Daltons. Between 800 and 3,000 Daltons is

particularly preferred.

Said GFR-binding compound is a (non-cyclic) peptide with between 8-25 amino acids or between 8-22 amino acids, more particularly between 18-22 amino acids, even more particularly between 19-21 or 20 amino acids, having growth factor receptor-binding capability or capabilities.

Said GFR-binding compound may also be a (non-cyclic) peptidomimetic as defined herein, comprising (consecutively or non-consecutively) between 8-25 amino acids or between 8-22 amino acids, more particularly between 18-22 amino acids, even more particularly between 19-21 or 20; wherein said GFR-binding compound has a molecular weight comprised between 600 and 4,000 Daltons (in particular, between 800-4,000 Da, 600-3,000 Da, more particularly between 800-3,000 Da);

In one particular example, said GFR-binding compound is a peptide or a peptidomimetic as defined herein, having growth factor receptor-binding capability or capabilities, having a molecular weight of between 600-3,000 Da, or 800-4,000 Da, in particular between 800 and 3,000 Da.

Said GFR-binding compound is a peptide or a peptidomimetic having a molecular weight comprised between 600 and 4,000 Daltons as defined herein, having growth factor receptor-binding capability or capabilities, with between 8 and 25 (in particular between 8-22, more particularly between 18-22, even more particularly between 19-21 or 20) amino acids, comprising a peptide with four amino acids (PEP1).

In one particular example, said GFR-binding compound comprises a peptide with eight amino acids (PEP12).

In one particular example, said GFR-binding compound comprises a peptide with four amino acids (PEP1); wherein said GFR-binding compound further comprises a peptide with three amino acids (PEP3).

In one particular example, said GFR-binding compound comprises a peptide with eight amino acids (PEP12); wherein said GFR-binding compound further comprises a peptide with three amino acids (PEP3).

In one particular example, said GFR-binding compound comprises a peptide with four amino acids (PEP1); wherein said GFR-binding compound further comprises a peptide with five amino acids (PEP5).

In one particular example, said GFR-binding compound comprises a peptide with eight amino acids (PEP12); wherein said GFR-binding compound further comprises a peptide with five amino acids (PEP5).

In one particular example, said GFR-binding compound comprises a peptide with four amino acids (PEP1); wherein said GFR-binding compound further comprises a peptide with between six and twelve amino acids (PEP9).

In one particular example, said GFR-binding compound comprises a peptide with eight amino acids (PEP12); wherein said GFR-binding compound further comprises a peptide with between six and twelve amino acids (PEP9).

In one particular example, said GFR-binding compound comprises a peptide with four amino acids (PEP1); wherein said GFR-binding compound further comprises a peptide with three amino acids (PEP3), an amino acid or a peptide with between two and seven amino acids (PEP7).

In one particular example, said GFR-binding compound comprises a peptide with four amino acids (PEP12); wherein said GFR-binding compound further comprises a peptide with three amino acids (PEP3), an amino acid or a peptide with between two and seven amino acids (PEP7).

In one particular example, said GFR-binding compound comprises a peptide with four amino acids (PEP1); wherein said GFR-binding compound further comprises a peptide with five amino acids (PEP5), an amino acid or a peptide with between two and seven amino acids (PEP7).

In one particular example, said GFR-binding compound comprises a peptide with four amino acids (PEP12); wherein said GFR-binding compound further comprises a peptide with five amino acids (PEP5), an amino acid or a peptide with between two and seven amino acids (PEP7).

In one particular example, said GFR-binding compound has the following general formula (I) (hereinafter may also be referred to as compound (I) or peptide (I)):

PEP(C)-PEP12 (I)

wherein PEP12 is a peptide with 8 amino acids of formula PEP1-$AA^{17}$-PEP11 as defined herein; wherein one end of PEP(C) interacts covalently with PEP12 via one end of PEP1; wherein PEP(C) is a peptide with at least 5 amino acids, in particular a peptide with between 5 and 12 amino acids.

In one particular example, the present disclosure provides a GFR-binding compound of general formula (I), wherein PEP(C) comprises PEP3.

In one particular example, the present disclosure provides a GFR-binding compound of general formula (I), wherein PEP(C) comprises PEP5. In one particular example, PEP(C) is PEP5.

In one particular example, the present disclosure provides a GFR-binding compound of general formula (I), wherein PEP(C) comprises PEP9. In one particular example, PEP(C) is PEP9.

In one particular example, the present disclosure provides a GFR-binding compound of general formula (I), wherein PEP(C) comprises PEP3 and PEP7.

In one particular example, the present disclosure provides a GFR-binding compound of general formula (I),

wherein PEP(C) comprises PEP5 and PEP7.

In one particular example, said GFR-binding compound has the following general formula (II) (hereinafter may also be referred to as compound (II) or peptide (II)):

PEP7-PEP5-PEP12 (II)

wherein PEP12 is a peptide with 8 amino acids of formula PEP1-$AA^{17}$-PEP11 as defined herein; wherein PEP5 is a peptide with five amino acids as defined herein; wherein PEP7 is an amino acid or a peptide with between two and seven amino acids as defined herein; wherein one end of PEP5 interacts covalently with one end of PEP12 via one end of PEP1; wherein another end of PEP5 interacts covalently with one end of PEP7 via $AA^7$.

PEP1 is SAIS.

In certain embodiments, PEP3 is selected from the group consisting of VPT, VPE, APT, TPT, VPA, APV, VPQ, VSQ, SRV and TQV.

In certain embodiments, PEP5 is a peptide of general formula PEP3-$AA^{11}$-$AA^{12}$; wherein PEP3 is selected from the group consisting of VPT, VPE, APT, TPT, VPA, APV, VPQ, VSQ, SRV and TQV; wherein $AA^{11}$ is selected from the group consisting of E, K, Q, R, A, D, G and H; and wherein $AA^{12}$ is selected from the group consisting of L, M, T, E, Q and H. In one particular example, PEP5 is selected from the group consisting of VPTEL, VPEKM, APTKL, APTQL, VPTKL, TPTKM, VPARL, VPTRL, APVKT, VPQAL, VSQDL, VPQDL, VPTEE, VPTGQ, SRVHH and TQVQL.

In certain embodiments, PEP7 is an amino acid or a peptide with between two and seven amino acids of general formula $AA^1$-$AA^2$-$AA^3$-$AA^4$-$AA^5$-$AA^6$-$AA^7$; wherein wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R; wherein $AA^7$ is absent or is selected from the group consisting of S, T, C, E, Q, P and R, and wherein at least one of $AA^1$, $AA^2$, $AA^3$, $AA^4$, $AA^5$, $AA^6$ or $AA^7$ is not absent. In one particular example, PEP7 is selected from the group consisting of KIPKAXX, GIPEPXX, SIPKAXX, HVTKPTX, YVPKPXX, TVPKPXX, AVPKAXX, KVGKAXX, KASKAXX, GSAGPXX, AAPASXX, STPPTXX, HVPKPXX, RVPSTXX, ASAAPXX, ASASPXX, NDEGLEX, SSVKXQP and RNVQXRP, wherein X is C or S throughout the present description.

In certain embodiments, PEP9 is a peptide of general formula PEP7-PEP5; wherein PEP5 is a peptide of formula PEP3-$AA^{11}$-$AA^{12}$; wherein PEP3 is selected from the group consisting of VPT, VPE, APT, TPT, VPA, APV, VPQ, VSQ, SRV and TQV; wherein $AA^{11}$ is selected from the group consisting of E, K, Q, R, A, D, G and H; and wherein $AA^{12}$ is selected from the group consisting of L, M, T, E, Q and H; wherein PEP7 is an amino acid or a peptide with between two and seven amino acids of general formula $AA^1$-$AA^2$-$AA^3$-$AA^4$-$AA^5$-$AA^6$-$AA^7$; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R; wherein $AA^7$ is absent or is selected from the group consisting of S, T, C, E, Q, P and R. In one particular example, PEP9 is selected from the group consisting of KIPKAXXVPTEL, GIPEPXXVPEKM, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKM, AAPASXXVPARL, STPPTXXVPTRL, HVPKPXXAPTKL, RVPSTXXAPVKT, ASAAPXXVPQAL, ASASPXXVSQDL, ASASPXXVPQDL, NDEGLEXVPTEE, NDEGLEXVPTGQ, SSVKXQPSRVHH and RNVQXRPTQVQL, wherein X is C or S throughout the present description.

In certain embodiments, PEP12 is a peptide of general formula PEP1-$AA^{17}$-PEP11; wherein $AA^{17}$ is selected from the group consisting of G, A, V, L, I, P, F, M, W, T and S (in particular is selected from the group consisting of M, I, L, V and T); wherein PEP1 is SAIS.

In particular, in certain embodiments, the pair PEP3:PEP1 is selected from the group consisting of VPT:SAIS, VPE:SAIS, APT:SAIS, TPT:SAIS, VPA:SAIS, APV:SAIS, VPQ:SAIS, VSQ:SAIS, and SRV:SAIS, TQV:SAIS.

In particular, in certain embodiments, the pair PEP5:PEP1 is selected from the group consisting of VPTKM:SAIS, VPTKL:SAIS, VPTQL:SAIS, VPTRL:SAIS, VPTKT:SAIS, VPTAL:SAIS, VPTDL:SAIS, VPEKM:SAIS, APTKL:SAIS, APTQL:SAIS, TPTKM:SAIS, VPARL:SAIS, APVKT:SAIS, VPQAL:SAIS, VSQDL:SAIS, VPQDL:SAIS, SRVHH:SAIS, and TQVQL:SAIS.

In particular, in certain embodiments, the pair PEP7:PEP1 is selected from the group consisting of GIPEPXX:SAIS, HVTKPTX:SAIS, YVPKPXX:SAIS, TVPKPXX:SAIS, AVPKAXX:SAIS, KVGKAXX:SAIS, KASKAXX:SAIS, GSAGPXX:SAIS, AAPASXX:SAIS, STPPTXX:SAIS, HVPKPXX:SAIS, RVPSTXX:SAIS, ASAAPXX:SAIS, ASASPXX:SAIS, SSVKXQP:SAIS, and RNVQXRP:SAIS.

In particular, in certain embodiments, the pair PEP9:PEP1 is selected from the group consisting of GIPEPXXVPTKM:SAIS, HVTKPTXVPTKL:SAIS, YVPKPXXVPTKL:SAIS, TVPKPXXVPTQL:SAIS, AVPKAXXVPTKL:SAIS, KVGKAXXVPTKL:SAIS, KASKAXXVPTKL:SAIS, GSAGPXXVPTKM:SAIS, AAPASXXVPTRL:SAIS, STPPTXXVPTRL:SAIS, HVPKPXXVPTKL:SAIS, RVPSTXXVPTKT:SAIS, ASAAPXXVPTAL:SAIS, ASASPXXVPTDL:SAIS, GIPEPXXVPEKM:SAIS, HVTKPTXAPTKL:SAIS, YVPKPXXAPTKL:SAIS, TVPKPXXAPTQL:SAIS, AVPKAXXAPTKL:SAIS, GSAGPXXTPTKM:SAIS, AAPASXXVPARL:SAIS, HVPKPXXAPTKL:SAIS, RVPSTXXAPVKT:SAIS, ASAAPXXVPQAL:SAIS, ASASPXXVSQDL:SAIS,

ASASPXXVPQDL:SAIS, SSVKXQPSRVHH:SAIS, and RNVQXRPTQVQL:SAIS.

In particular, in certain embodiments, the pair PEP3:PEP12 is selected from the group consisting of VPT:SAIS-$AA^{17}$-LYL, VPE:SAIS-$AA^{17}$-LYL, APT:SAIS-$AA^{17}$ -LYL, TPT:SAIS-$AA^{17}$-LYL, VPA:SAIS-$AA^{17}$-LYL, APV:SAIS-$AA^{17}$-LYL, VPQ:SAIS-$AA^{17}$-LYL, VSQ:SAIS-$AA^{17}$-LYL, SRV:SAIS-$AA^{17}$-LYL, and TQV:SAIS-$AA^{17}$-LYL; and wherein $AA^{17}$ is selected from the group consisting of G, A, V, L, I, P, F, M, W, T and S (in particular is selected from the group consisting of M, I, L, V and T).

In particular, in certain embodiments, the pair PEP12:PEP5 is selected from the group consisting of VPTKM:SAIS-$AA^{17}$-LYL, VPTKL:SAIS-$AA^{17}$-LYL, VPTQL:SAIS-$AA^{17}$-LYL, VPTRL:SAIS-$AA^{17}$-LYL, VPTKT:SAIS-$AA^{17}$-LYL, VPTAL:SAIS-$AA^{17}$-LYL, VPTDL:SAIS-$AA^{17}$-LYL, VPEKM:SAIS-$AA^{17}$-LYL, APTKL:SAIS-$AA^{17}$-LYL, APTQL:SAIS-$AA^{17}$-LYL, TPTKM:SAIS-$AA^{17}$-LYL, VPARL:SAIS-$AA^{17}$-LYL, APVKT:SAIS-$AA^{17}$-LYL, VPQAL:SAIS-$AA^{17}$-LYL, VSQDL:SAIS-$AA^{17}$-LYL, VPQDL:SAIS-$AA^{17}$-LYL, SRVHH:SAIS-$AA^{17}$-LYL, and TQVQL:SAIS-$AA^{17}$-LYL; and wherein $AA^{17}$ is selected from the group consisting of G, A, V, L, I, P, F, M, W, T and S (in particular is selected from the group consisting of M, I, L, V and T).

In particular, in certain embodiments, the pair PEP12:PEP7 is selected from the group consisting of GIPEPXX:SAIS-$AA^{17}$-LYL, HVTKPTX:SAIS-$AA^{17}$-LYL, YVPKPXX:SAIS-$AA^{17}$-LYL, TVPKPXX:SAIS-$AA^{17}$-LYL, AVPKAXX:SAIS-$AA^{17}$-LYL, KVGKAXX:SAIS-$AA^{17}$-LYL, KASKAXX:SAIS-$AA^{17}$-LYL, GSAGPXX:SAIS-$AA^{17}$-LYL, AAPASXX:SAIS-$AA^{17}$-LYL, STPPTXX:SAIS-$AA^{17}$-LYL, HVPKPXX:SAIS-$AA^{17}$-LYL, RVPSTXX:SAIS-$AA^{17}$-LYL, ASAAPXX:SAIS-$AA^{17}$-LYL, ASASPXX:SAIS-$AA^{17}$-LYL, SSVKXQP:SAIS-$AA^{17}$-LYL, and RNVQXRP:SAIS-$AA^{17}$-LYL; and wherein $AA^{17}$ is selected from the group consisting of G, A, V, L, I, P, F, M, W, T and S (in particular is selected from the group consisting of M, I, L, V and T).

In particular, in certain embodiments, the pair PEP12:PEP9 is selected from the group consisting of GIPEPXXVPTKM:SAIS-$AA^{17}$-LYL, HVTKPTXVPTKL:SAIS-$AA^{17}$-LYL, YVPKPXXVPTKL:SAIS-$AA^{17}$-LYL, TVPKPXXVPTQL:SAIS-$AA^{17}$-LYL, AVPKAXXVPTKL:SAIS-$AA^{17}$-LYL, KVGKAXXVPTKL:SAIS-$AA^{17}$-LYL, KASKAXXVPTKL:SAIS-$AA^{17}$-LYL, GSAGPXXVPTKM:SAIS-$AA^{17}$-LYL, AAPASXXVPTRL:SAIS-$AA^{17}$-LYL, STPPTXXVPTRL:SAIS-$AA^{17}$-LYL, HVPKPXXVPTKL:SAIS-$AA^{17}$-LYL, RVPSTXXVPTKT:SAIS-$AA^{17}$-LYL, ASAAPXXVPTAL:SAIS-$AA^{17}$-LYL, ASASPXXVPTDL:SAIS-$AA^{17}$-LYL, GIPEPXXVPEKM:SAIS-$AA^{17}$-LYL, HVTKPTXAPTKL:SAIS-$AA^{17}$-LYL, YVPKPXXAPTKL:SAIS-$AA^{17}$-LYL, TVPKPXXAPTQL:SAIS-$AA^{17}$-LYL, AVPKAXXAPTKL:SAIS-$AA^{17}$-LYL, GSAGPXXTPTKM:SAIS-$AA^{17}$-LYL, AAPASXXVPARL:SAIS-$AA^{17}$-LYL, HVPKPXXAPTKL:SAIS-$AA^{17}$-LYL, RVPSTXXAPVKT:SAIS-$AA^{17}$-LYL, ASAAPXXVPQAL:SAIS-$AA^{17}$-LYL, ASASPXXVSQDL:SAIS-$AA^{17}$-LYL, ASASPXXVPQDL:SAIS-$AA^{17}$-LYL, SSVKXQPSRVHH:SAIS-$AA^{17}$-LYL, and RNVQXRPTQVQL:SAIS-$AA^{17}$-LYL; and wherein $AA^{17}$ is selected from the group consisting of G, A, V, L, I, P, F, M, W, T and S (in particular is selected from the group consisting of M, I, L, V and T).

In certain embodiments, the triplet PEP7:PEP3:PEP1 is selected from the group consisting of GIPEPXX:VPT:SAIS, HVTKPTX:VPT:SAIS, YVPKPXX:VPT:SAIS, TVPKPXX:VPT:SAIS, AVPKAXX:VPT:SAIS, KVGKAXX:VPT:SAIS, KASKAXX:VPT:SAIS, GSAGPXX:VPT:SAIS, AAPASXX:VPT:SAIS, STPPTXX:VPT:SAIS, HVPKPXX:VPT:SAIS, RVPSTXX:VPT:SAIS, ASAAPXX:VPT:SAIS, ASASPXX:VPT:SAIS, GIPEPXX:VPE:SAIS, HVTKPTX:APT:SAIS, YVPKPXX:APT:SAIS, TVPKPXX:APT:SAIS, AVPKAXX:APT:SAIS, GSAGPXX:TPT:SAIS, AAPASXX:VPA:SAIS, HVPKPXX:APT:SAIS, RVPSTXX:APV:SAIS, ASAAPXX:VPQ:SAIS, ASASPXX:VSQ:SAIS, ASASPXX:VPQ:SAIS, SSVKXQP:SRV:SAIS, and RNVQXRP:TQV:SAIS.

In certain embodiments, the triplet PEP7:PEP3:PEP12 is selected from the group consisting of GIPEPXX:VPT:SAIS-$AA^{17}$-LYL, HVTKPTX:VPT:SAIS-$AA^{17}$-LYL, YVPKPXX:VPT:SAIS-$AA^{17}$-LYL, TVPKPXX:VPT:SAIS-$AA^{17}$-LYL, AVPKAXX:VPT:SAIS-$AA^{17}$-LYL, KVGKAXX:VPT:SAIS-$AA^{17}$-LYL, KASKAXX:VPT:SAIS-$AA^{17}$-LYL, GSAGPXX:VPT:SAIS-$AA^{17}$-LYL, AAPASXX:VPT:SAIS-$AA^{17}$-LYL, STPPTXX:VPT:SAIS-$AA^{17}$-LYL, HVPKPXX:VPT:SAIS-$AA^{17}$-LYL, RVPSTXX:VPT:SAIS-$AA^{17}$ -LYL, ASAAPXX:VPT:SAIS-$AA^{17}$-LYL, ASASPXX:VPT:SAIS-$AA^{17}$-LYL, GIPEPXX:VPE:SAIS-$AA^{17}$-LYL, HVTKPTX:APT:SAIS-$AA^{17}$-LYL, YVPKPXX:APT:SAIS-$AA^{17}$-LYL, TVPKPXX:APT:SAIS-$AA^{17}$-LYL, AVPKAXX:APT:SAIS-$AA^{17}$-LYL, GSAGPXX:TPT:SAIS-$AA^{17}$-LYL, AAPASXX:VPA:SAIS-$AA^{17}$-LYL, HVPKPXX:APT:SAIS-$AA^{17}$-LYL, RVPSTXX:APV:SAIS-$AA^{17}$-LYL, ASAAPXX:VPQ:SAIS-$AA^{17}$-LYL, ASASPXX:VSQ:SAIS-$AA^{17}$-LYL, ASASPXX:VPQ:SAIS-$AA^{17}$-LYL, SSVKXQP:SRV:SAIS-$AA^{17}$-LYL, and RNVQXRP:TQV:SAIS-$AA^{17}$-LYL; and wherein $AA^{17}$ is selected from the group consisting of G, A, V, L, I, P, F, M, W, T and S (in particular is selected from the group consisting of M, I, L, V and T).

In certain embodiments, the triplet PEP7:PEP5:PEP1 is selected from the group consisting of GIPEPXX:VPTKM:SAIS, HVTKPTX:VPTKL:SAIS, YVPKPXX:VPTKL:SAIS, TVPKPXX:VPTQL:SAIS, AVPKAXX:VPTKL:SAIS, KVGKAXX:VPTKL:SAIS, KASKAXX:VPTKL:SAIS, GSAGPXX:VPTKM:SAIS, AAPASXX:VPTRL:SAIS, STPPTXX:VPTRL:SAIS, HVPKPXX:VPTKL:SAIS, RVPSTXX:VPTKT:SAIS, ASAAPXX:VPTAL:SAIS, ASASPXX:VPTDL:SAIS, GIPEPXX:VPEKM:SAIS, HVTKPTX:APTKL:SAIS, YVPKPXX:APTKL:SAIS, TVPKPXX:APTQL:SAIS, AVPKAXX:APTKL:SAIS, GSAGPXX:TPTKM:SAIS, AAPASXX:VPARL:SAIS, HVPKPXX:APTKL:SAIS, RVPSTXX:APVKT:SAIS, ASAAPXX:VPQAL:SAIS, ASASPXX:VSQDL:SAIS, ASASPXX:VPQDL:SAIS, SSVKXQP:SRVHH:SAIS, and RNVQXRP:TQVQL:SAIS.

In certain embodiments, the triplet PEP7:PEP5:PEP12 is selected from the group consisting of GIPEPXX:VPTKM:SAIS-$AA^{17}$-LYL, HVTKPTX:VPTKL:SAIS-$AA^{17}$-LYL, YVPKPXX:VPTKL:SAIS-$AA^{17}$-LYL, TVPKPXX:VPTQL:SAIS-$AA^{17}$-LYL, AVPKAXX:VPTKL:SAIS-$AA^{17}$-LYL, KVGKAXX:VPTKL:SAIS-$AA^{17}$-LYL, KASKAXX:VPTKL:SAIS-$AA^{17}$-LYL, GSAGPXX:VPTKM:SAIS-$AA^{17}$-LYL, AAPASXX:VPTRL:SAIS-$AA^{17}$-LYL, STPPTXX:VPTRL:SAIS-$AA^{17}$-LYL, HVPKPXX:VPTKL:SAIS-$AA^{17}$-LYL, RVPSTXX:VPTKT:SAIS-$AA^{17}$-LYL, ASAAPXX:VPTAL:SAIS-$AA^{17}$-LYL, ASASPXX:VPTDL:SAIS-$AA^{17}$-LYL, GIPEPXX:VPEKM:SAIS-$AA^{17}$-LYL, HVTKPTX:APTKL:SAIS-$AA^{17}$-LYL, YVPKPXX:APTKL:SAIS-$AA^{17}$-LYL, TVPKPXX:APTQL:SAIS-$AA^{17}$-LYL, AVPKAXX:APTKL:SAIS-$AA^{17}$-LYL, GSAGPXX:TPTKM:SAIS-$AA^{17}$-LYL, AAPASXX:VPARL:SAIS-$AA^{17}$-LYL, HVPKPXX:APTKL:SAIS-$AA^{17}$-LYL, RVPSTXX:APVKT:SAIS-$AA^{17}$-LYL, ASAAPXX:VPQAL:SAIS-$AA^{17}$-LYL, ASASPXX:VSQDL:SAIS-$AA^{17}$-LYL, ASASPXX:VPQDL:SAIS-$AA^{17}$ -LYL, SSVKXQP:SRVHH:SAIS-$AA^{17}$-LYL, and RNVQXRP:TQVQL:SAIS-$AA^{17}$-LYL; and wherein $AA^{17}$ is selected from the group consisting of G, A, V, L, **I, P,** F, M, W, T and S (in particular is selected from the group consisting of M, I, L, V and T).

In certain embodiments, PEP11 is a peptide with 3 amino acids of general formula $AA^{18}$-$AA^{19}$-$AA^{20}$; wherein $AA^{18}$ is selected from the group consisting of L, V, Q, A and R; wherein $AA^{19}$ is selected from the group consisting of F, W, H, Y, I and K; wherein $AA^{20}$ is selected from the group consisting of L, F, Y, K, I, V and M. In one particular example, PEP11 is selected from the group consisting of LYL, LFF, LYF, LYY, LYK, LYI, LFI, LYV, VYY, QIM, AKV and RKI.

In certain embodiments, the pair PEP1:PEP11 is SAIS:LYL.

In one particular example, said GFR-binding compound has the following general formula (III) (hereinafter may also be referred to as compound (III) or peptide (III)):

$$AA^1\text{-}AA^2\text{-}AA^3\text{-}AA^4\text{-}AA^5\text{-}AA^6\text{-}AA^7\text{-}AA^8\text{-}AA^9\text{-}AA^{1°}\text{-}AA^{11}\text{-}AA^{12}\text{-}AA^{13}\text{-}AA^{14}\text{-}AA^{15}\text{-}AA^{16}\text{-}AA^{17}\text{-}AA^{18}\text{-}AA^{19}\text{-}AA^{20} \quad \text{(III)}$$

wherein $AA^1$-$AA^2$-$AA^3$-$AA^4$-$AA^5$-$AA^6$-$AA^7$ is PEP7 as defined herein; wherein $AA^{13}$-$AA^{14}$-$AA^{15}$-$AA^{16}$-$AA^{17}$-$AA^{18}$-$AA^{19}$-$AA^{20}$ is PEP12 as defined herein; wherein $AA^8$-$AA^9$-$AA^{10}$ is PEP3 as defined herein; wherein $AA^{11}$ and $AA^{12}$ are as defined herein; wherein $AA^1$ may be an N-terminal amino acid or a C-terminal amino acid; wherein $AA^{20}$ may be an N-terminal amino acid or a C-terminal amino acid.

In one most particular example, the RMSD value of the three dimensional (3D) atomic coordinates of said GFR-binding compound as defined herein with respect to PEPREF is 2.45Å (Angstroms) or less, in particular is 2Å or less, and more particularly is 1.79Å or less, and wherein PEPREF is the set of 3D atomic coordinates already defined herein (hereinafter may be referred to as "wherein the RMSD is 2.45Å or less" for the sake of conciseness).

In one example, said GFR-binding compound is a synthetic molecule as defined herein in the definition section.

In one particular example, said GFR-binding compound is a synthetic peptide or a peptidomimetic.

In one most particular example, said GFR-binding compound is a non-cyclic synthetic peptide.

In one example, a length of said GFR-binding compound, in solution, such as in a physiologically acceptable solvent such as water or PBS, is comprised between about 6 and about 20 nm, preferably between about 6 and about 16 nm, as determined using the standard « 3D » procedure described above.

In one particular example, said GFR-binding compounds may be any one of peptides comprising the 4mer peptide SAIS among SEQ ID NO: 1 to 4802

**Cyclic GFR-binding compounds**

In one example, said cyclic GFR-binding compound has a molecular weight of less than 7,000 Daltons. In one example, said cyclic GFR-binding compound has a molecular weight of less than 6,000 Daltons. In one example, said cyclic GFR-binding compound has a molecular weight of less than 5,000 Daltons. In one particular example, said cyclic GFR-binding compound has a molecular weight comprised between 1,000 and 7,000 Daltons. In one particular example, said cyclic GFR-binding compound has a molecular weight comprised between 1,000 and 6,000 Daltons. In one particular example, said cyclic GFR-binding compound has a molecular weight comprised between 2,000 and 7,000 Daltons. In one particular example, said cyclic GFR-binding compound has a molecular weight comprised between 2,000 and 6,000 Daltons.

In one particular example, the growth factor receptor involved in the interaction with said cyclic GFR-binding compound is an epidermal growth factor receptor. In one particular example, the growth factor receptor involved in the interaction with said cyclic GFR-binding compound is a fibroblast growth factor receptor. In one particular example, the growth factor receptor involved in the interaction with said cyclic GFR-binding compound is a vascular endothelial growth factor receptor. In one particular example, the growth factor receptor involved in the interaction with said cyclic GFR-binding compound is a nerve growth factor receptor. In one particular example, the growth factor receptor involved in the interaction with said cyclic GFR-binding compound is a hepatocyte growth factor receptor. In one particular example, the growth factor receptor involved in the interaction with said cyclic GFR-binding compound is a somatomedin or insulin-like

growth factor receptor. In one particular example, the growth factor receptor involved in the interaction with said cyclic GFR-binding compound is a platelet-derived growth factor receptor. In one particular example, the growth factor receptor involved in the interaction with said cyclic GFR-binding compound is a protein from the transforming growth factor beta (TGF- β) superfamily.

In one particular example, the growth factor receptor(s) involved in the interaction with said cyclic GFR-binding compound is (are) preferably selected from epidermal growth factor receptors, fibroblast growth factor receptors, vascular endothelial growth factor receptors, nerve growth factor receptors, hepatocyte growth factor receptors, somatomedin or insulin-like growth factor receptors, platelet-derived growth factor receptors, and transforming growth factor beta (TGF-β) superfamily proteins.

Said cyclic GFR-binding compound is a peptide having growth factor receptor-binding capability or capabilities, with between 10-60 amino acids, in particular between 10-55 amino acids, more particularly between 15-60 amino acids, and even more particularly between 15-55 amino acids, or between 10-35 amino acids, in particular between 15-35 amino acids, more particularly between 10-30 amino acids, and even more particularly between 15-30 amino acids.

Said cyclic GFR-binding compound may also be a cyclic peptidomimetic as defined herein, having growth factor receptor-binding capability or capabilities, comprising (consecutively or non consecutively) between 10-60 amino acids, in particular between 10-55 amino acids, more particularly between 15-60 amino acids, and even more particularly between 15-55 amino acids, or between 10-35 amino acids, in particular between 15-35 amino acids, more particularly between 10-30 amino acids, and even more particularly between 15-30 amino acids; wherein said cyclic GFR-binding compound has a molecular weight comprised between 1,000 and 7,000 Daltons (in particular, between 1,000 and 6,000 Da).

In one particular example, said cyclic GFR-binding compound is a cyclic peptidomimetic as defined herein, having growth factor receptor-binding capability or capabilities, comprising (consecutively or non consecutively) between 10-60 amino acids, in particular between 10-55 amino acids, more particularly between 15-60 amino acids, and even more particularly between 15-55 amino acids, or between 10-35 amino acids, in particular between 15-35 amino acids, more particularly between 10-30 amino acids, and even more particularly between 15-30 amino acids; and containing at least one peptide portion or fragment with between 5-20 amino acids (in particular containing one peptide portion or fragment with between 5-20 amino acids); wherein said cyclic GFR-binding compound has a molecular weight comprised between 1,000 and 7,000 Daltons (in particular, between 1,000 and 6,000 Da).

Said cyclic GFR-binding compound is a cyclic peptide or a cyclic peptidomimetic as defined herein, having growth factor receptor-binding capability or capabilities, with between 10-60 (in particular between 15-60, more particularly between 10-55, and even more particularly between 15-55) amino acids or with between 10-35 (in particular between 15-35, more particularly between 10-30, and even more particularly between 15-30) amino acids, comprising a peptide with four amino acids (PEP1).

In one particular example said cyclic GFR-binding compound is a cyclic peptide or a cyclic peptidomimetic as defined herein, with between 10-60 (in particular between 15-60, more particularly between 10-55, and even more particularly between 15-55) amino acids or with between 10-35 (in particular between 15-35, more particularly between 10-30, and even more particularly between 15-30) amino acids, comprising a peptide with eight amino acids (PEP12).

In one particular example said cyclic GFR-binding compound comprises a peptide with four amino acids (PEP1); wherein said cyclic GFR-binding compound further comprises a peptide with three amino acids (PEP3).

In one particular example said cyclic GFR-binding compound comprises a peptide with eight amino acids (PEP12); wherein said cyclic GFR-binding compound further comprises a peptide with three amino acids (PEP3).

In one particular example said cyclic GFR-binding compound comprises a peptide with four amino acids (PEP1); wherein said cyclic GFR-binding compound further comprises a peptide with five amino acids (PEP5).

In one particular example said cyclic GFR-binding compound comprises a peptide with eight amino acids (PEP12); wherein said cyclic GFR-binding compound further comprises a peptide with five amino acids (PEP5).

In one particular example said cyclic GFR-binding compound comprises a peptide with four amino acids (PEP1); wherein said cyclic GFR-binding compound further comprises a peptide with between six and twelve amino acids (PEP9).

In one particular example said cyclic GFR-binding compound comprises a peptide with eight amino acids (PEP12); wherein said cyclic GFR-binding compound further comprises a peptide with between six and twelve amino acids (PEP9).

In one particular example said cyclic GFR-binding compound comprises a peptide with four amino acids (PEP1); wherein said cyclic GFR-binding compound further comprises a peptide with three amino acids (PEP3), and an amino acid or a peptide with between two and seven amino acids (PEP7).

In one particular example said cyclic GFR-binding compound comprises a peptide with four amino acids (PEP12); wherein said cyclic GFR-binding compound further comprises a peptide with three amino acids (PEP3), and an amino acid or a peptide with between two and seven amino acids (PEP7).

In one particular example said cyclic GFR-binding compound comprises a peptide with four amino acids (PEP1); wherein said cyclic GFR-binding compound further comprises a peptide with five amino acids (PEP5), and an amino acid or a peptide with between two and seven amino acids (PEP7).

In one particular example said cyclic GFR-binding compound comprises a peptide with four amino acids (PEP12); wherein said cyclic GFR-binding compound further comprises a peptide with five amino acids (PEP5), and an amino acid or a peptide with between two and seven amino acids (PEP7).

In one particular example said cyclic GFR-binding compound comprises a peptide or a peptidomimetic having the following general formula (IIIa) (hereinafter may also be referred to as compound (IIIa) or peptide (IIIa)):

PEP(A)-LINKER (IIIa)

wherein one end of LINKER interacts covalently with one end of PEP(A); wherein PEP(A) comprises PEP1 or PEP12; wherein LINKER is a linear or branched organic divalent radical, moiety or compound having a molecular weight (Mw) comprised between 450 and 4,500 Daltons, in particular comprised between about 600 and about 4,500 Da, more particularly between about 600 and about 4,000 Da, and even more particularly between about 600 and about 3,500 Da.

In one particular example said cyclic GFR-binding compound comprises a peptide or a peptidomimetic having the following general formula (IIIb) (hereinafter may also be referred to as compound (IIIb) or peptide (IIIb)):

LINKER-PEP(A)-LINKER (IIIb)

wherein one end of a first LINKER interacts covalently with one end of PEP(A); wherein one end of a second LINKER interacts covalently with another end of PEP(A); wherein another end of a first LINKER interacts covalently with another end of a second LINKER; wherein PEP(A) comprises PEP1 or PEP12; wherein LINKER are independently a linear or branched organic divalent radical, moiety or compound having a molecular weight (Mw) comprised between 450 and 4,500 Daltons, in particular comprised between about 600 and about 4,500 Da, more particularly between about 600 and about 4,000 Da, and even more particularly between about 600 and about 3,500 Da.

In the present description, the molecular weight of LINKER refer to the calculated molecular weight prior to being connected to / reacted with any of the elements it is configured to connect to or react with e.g. PEP(A), or any other groups defined herein.

In one particular example said cyclic GFR-binding compound comprises compounds (IIIa) or (IIIb), wherein PEP(A) further comprises PEP3.

In one particular example said cyclic GFR-binding compound comprises compounds (IIIa) or (IIIb), wherein PEP(A) further comprises PEP5.

In one particular example said cyclic GFR-binding compound comprises compounds (IIIa) or (IIIb), wherein PEP(A) further comprises PEP9.

In one particular example said cyclic GFR-binding compound comprises compounds (IIIa) or (IIIb), wherein PEP(A) further comprises PEP3 and PEP7.

In one particular example said cyclic GFR-binding compound comprises compounds (IIIa) or (IIIb), wherein PEP(A) further comprises PEP5 and PEP7.

In one particular example said cyclic GFR-binding compound comprises a peptide or a peptidomimetic having the following general formula (IVa) (hereinafter may also be referred to as compound (IVa) or peptide (IVa)):

PEP(C)-PEP12-LINKER (IVa)

wherein LINKER is a linear or branched organic divalent radical, moiety or compound having a molecular weight (Mw) comprised between 450 and 4,500 Daltons, in particular comprised between about 600 and about 4,500 Da, more particularly between about 600 and about 4,000 Da, and even more particularly between about 600 and about 3,500 Da; wherein PEP12 is a peptide with 8 amino acids of formula PEP1-$AA^{17}$-PEP11 as defined herein; wherein PEP2 is a peptide with five amino acids as already defined herein; wherein one end of PEP(C) interacts covalently with PEP12 via one end of PEP1; wherein one end of LINKER interacts covalently with one end of PEP12 via one end of PEP11; wherein PEP(C) is a peptide with at least 5 amino acids, in particular a peptide with between 5 and 12 amino acids.

In one particular example said cyclic GFR-binding compound comprises a peptide or a peptidomimetic having the following general formula (IVb) (hereinafter may also be referred to as compound (IVb) or peptide (IVb)):

LINKER-PEP(C)-PEP12-LINKER (IVb)

wherein LINKER are independently a linear or branched organic divalent radical, moiety or compound having a molecular weight (Mw) comprised between 450 and 4,500 Daltons, in particular comprised between about 600 and about 4,500 Da, more particularly between about 600 and about 4,000 Da, and even more particularly between about 600 and about 3,500 Da; wherein PEP12 is a peptide with 8 amino acids of formula PEP1-AA"-PEP11 as defined herein; wherein PEP2 is a peptide with five amino acids as already defined herein; wherein one end of PEP(C) interacts covalently with PEP12 via

one end of PEP1; wherein one end of a first LINKER interacts covalently with one end of PEP12 via one end of PEP11; wherein one end of a second LINKER interacts covalently with another end of PEP(C); wherein another end of a first LINKER interacts covalently with another end of a second LINKER; wherein PEP(C) is a peptide with at least 5 amino acids, in particular a peptide with between 5 and 12 amino acids.

In one particular example said cyclic GFR-binding compound comprises compound (IVa) or (IVb), wherein PEP(C) comprises PEP3.

I In one particular example said cyclic GFR-binding compound comprises compound (IVa) or (IVb), wherein PEP(C) comprises PEP5. In one particular example, PEP(C) is PEP5.

In one particular example said cyclic GFR-binding compound comprises compound (IVa) or (IVb), wherein PEP(C) comprises PEP9. In one particular example, PEP(C) is PEP9.

In one particular example said cyclic GFR-binding compound comprises compound (IVa) or (IVb), wherein PEP(C) comprises PEP3 and PEP7.

In one particular example said cyclic GFR-binding compound comprises compound (IVa) or (IVb), wherein PEP(C) comprises PEP5 and PEP7.

In one particular example said cyclic GFR-binding compound comprises compound (IVa) or (IVb), wherein PEP(C) is PEP5 or PEP9.

In one particular example said cyclic GFR-binding compound comprises a peptide or a peptidomimetic having the following general formula (Va) (hereinafter may also be referred to as compound (Va) or peptide (Va)):

PEP7-PEP5-PEP12-LINKER (Va)

wherein LINKER is a linear or branched organic divalent radical, moiety or compound having a molecular weight (Mw) comprised between 450 and 4,500 Daltons, in particular comprised between about 600 and about 4,500 Da, more particularly between about 600 and about 4,000 Da, and even more particularly between about 600 and about 3,500 Da; wherein PEP12 is a peptide with 8 amino acids of formula PEP1-$AA^{17}$-PEP11 as defined herein; wherein PEP5 is a peptide with five amino acids as already defined herein; wherein PEP7 an amino acid or a peptide with between two and seven amino acids as already defined herein; wherein one end of LINKER interacts covalently with one end of PEP12 via $AA^{20}$; wherein one end of PEP5 interacts covalently with another end of PEP12 via $AA^{12}$; wherein another end of PEP5 interacts covalently with one end of PEP7 via $AA^{8}$.

In one particular example said cyclic GFR-binding compound comprises a peptide or a peptidomimetic having the following general formula (Vb) (hereinafter may also be referred to as compound (Vb) or peptide (Vb)):

LINKER-PEP7-PEP5-PEP12-LINKER (Vb)

wherein LINKER are independently a linear or branched organic divalent radical, moiety or compound having a molecular weight (Mw) comprised between 450 and 4,500 Daltons, in particular comprised between about 600 and about 4,500 Da, more particularly between about 600 and about 4,000 Da, and even more particularly between about 600 and about 3,500 Da; wherein PEP12 is a peptide with 8 amino acids of formula PEP1-$AA^{17}$-PEP11 as defined herein; wherein PEP5 is a peptide with five amino acids as already defined herein; wherein PEP7 an amino acid or a peptide with between two and seven amino acids as already defined herein; wherein one end of PEP5 interacts covalently with another end of PEP12 via $AA^{12}$; wherein another end of PEP5 interacts covalently with one end of PEP7 via $AA^{8}$; wherein one end of a first LINKER interacts covalently with one end of PEP12 via $AA^{20}$; wherein one end of a second LINKER interacts covalently with another end of PEP7; wherein another end of a first LINKER interacts covalently with another end of a second LINKER.

In one particular example said cyclic GFR-binding compound comprises a peptide or a peptidomimetic having the following general formula (VIa) (hereinafter may also be referred to as compound (VIa) or peptide (VIa)):

$AA^{1}$-$AA^{2}$-$AA^{3}$-$AA^{4}$-$AA^{5}$-$AA^{6}$-$AA^{7}$-$AA^{8}$-$AA^{9}$-$AA^{10}$-$AA^{11}$-$AA^{12}$-$AA^{13}$-$AA^{14}$-$AA^{15}$-$AA^{16}$-$AA^{17}$-$AA^{18}$-$AA^{19}$-$AA^{20}$-LINKER (VIa)

wherein LINKER is a linear or branched organic divalent radical, moiety or compound having a molecular weight (Mw) comprised between 450 and 4,500 Daltons, in particular comprised between about 600 and about 4,500 Da, more particularly between about 600 and about 4,000 Da, and even more particularly between about 600 and about 3,500 Da; wherein $AA^{1}$-$AA^{2}$-$AA^{3}$-$AA^{4}$-$AA^{5}$-$AA^{6}$-$AA^{7}$ is PEP7 as defined herein; wherein $AA^{13}$-$AA^{14}$-$AA^{15}$-$AA^{16}$-$AA^{17}$-$AA^{18}$-$AA^{19}$-$AA^{20}$ is PEP12 as defined herein; wherein $AA^{8}$-$AA^{9}$-$AA^{10}$ is PEP3 as defined herein; wherein $AA^{11}$ and $AA^{12}$ are as defined herein; wherein one end of LINKER interacts covalently with $AA^{20}$; wherein $AA^{1}$ may be an N-terminal amino acid or a C-terminal amino acid; wherein $AA^{20}$ may be an N-terminal amino acid or a C-terminal amino acid.

In one particular example said cyclic GFR-binding compound comprises a peptide or a peptidomimetic having the following general formula (VIb) (hereinafter may also be referred to as compound (VIb) or peptide (VIb)):

$$\text{LINKER-}\\ AA^1\text{-}AA^2\text{-}AA^3\text{-}AA^4\text{-}AA^5\text{-}AA^6\text{-}AA^7\text{-}AA^8\text{-}AA^9\text{-}AA^{10}\text{-}AA^{11}\text{-}AA^{12}\text{-}A^{13}\text{-}AA^{14}\text{-}AA^{15}\text{-}AA^{16}\text{-}AA^{17}\text{-}AA^{18}\text{-}AA^{19}\text{-}AA^{20}\text{-LINKER}\quad (VIb)$$

wherein LINKER are independently a linear or branched organic divalent radical, moiety or compound having a molecular weight (Mw) comprised between 450 and 4,500 Daltons, in particular comprised between about 600 and about 4,500 Da, more particularly between about 600 and about 4,000 Da, and even more particularly between about 600 and about 3,500 Da; wherein $AA^1$-$AA^2$-$AA^3$-$AA^4$-$AA^5$-$AA^6$-$AA^7$ is PEP7 as defined herein; wherein $AA^{13}$-$AA^{14}$-$AA^{15}$-$AA^{16}$-$AA^{17}$-$AA^{18}$-$AA^{19}$-$AA^{20}$ is PEP12 as defined herein; wherein $AA^8$-$AA^9$-$AA^{10}$ is PEP3 as defined herein; wherein $AA^{11}$ and $AA^{12}$ are as defined herein; wherein one end of a first LINKER interacts covalently with $AA^{20}$; wherein one end of a second LINKER interacts covalently with $AA^1$; wherein another end of a first LINKER interacts covalently with another end of a second LINKER; wherein one end of the first LINKER may be an N-terminal amino acid or a C-terminal amino acid.

In one particular example said cyclic GFR-binding compound comprises two LINKERs.

In one particular example said cyclic GFR-binding compound has any one of the following schematic general formulae (VII) to (XX) (hereinafter may also be referred to as compounds (VII) to (XX) or peptides (VII) to (XX)):

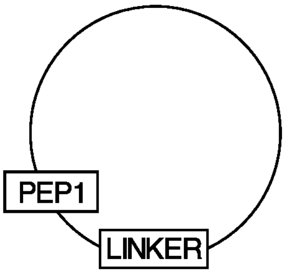


(VII)

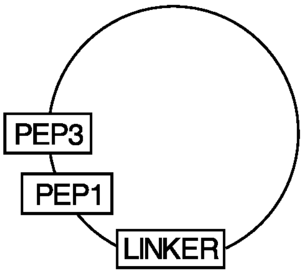


(VIII)

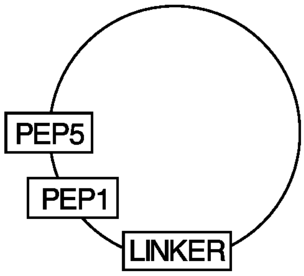


(IX)

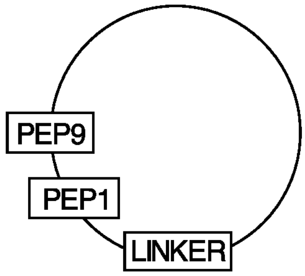


(X)

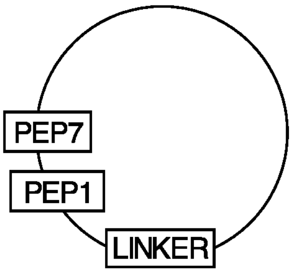


(IXI)

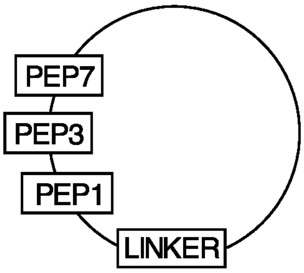


(XII)

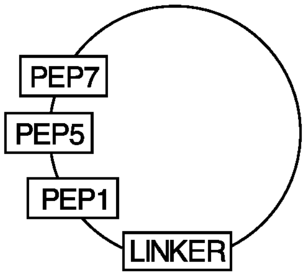


(XIII)

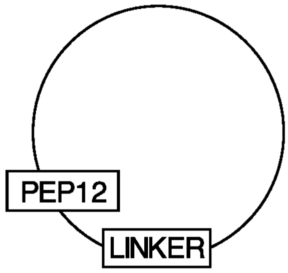


(XIV)

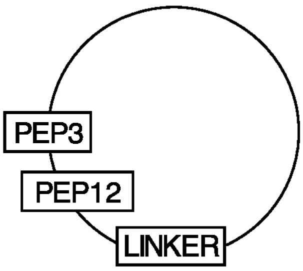


(XV)

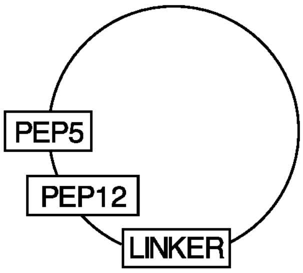


(XVI)

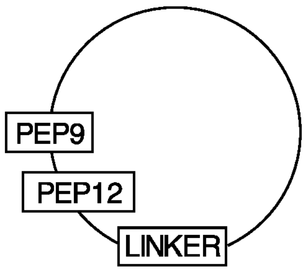


(XVII)

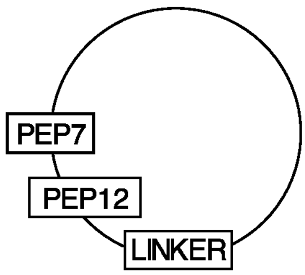


(XVIII)

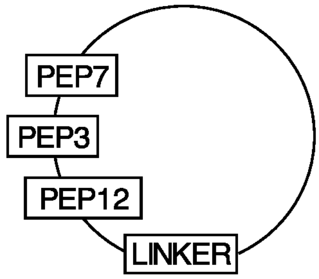


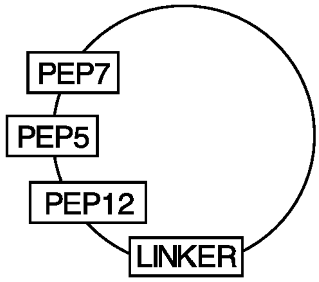


(XIX) (XX)

wherein LINKER is a linear or branched organic divalent radical, moiety or compound having a molecular weight (Mw) comprised between 450 and 4,500 Daltons, in particular comprised between about 600 and about 4,500 Da, more particularly between about 600 and about 4,000 Da, and even more particularly between about 600 and about 3,500 Da; wherein PEP12 is a peptide with 8 amino acids of formula PEP1-$AA^{17}$-PEP11 as defined herein; wherein PEP5 is a peptide with five amino acids as already defined herein; wherein PEP7 an amino acid or a peptide with between two and seven amino acids as already defined herein; wherein PEP9 is a peptide with between six and twelve amino acids; wherein curved lines represents covalent bonds between LINKER and PEP1 to PEP12. Curved lines' lengths may not be representative of the actual relative distance between the LINKERs and PEP1 to PEP12.

In one particular example said cyclic GFR-binding compound has any one of the following schematic general formulae (XXI) to (XXIII) (hereinafter may also be referred to as compounds (XXI) to (XXIII) or peptides (XXI) to (XXIII)):

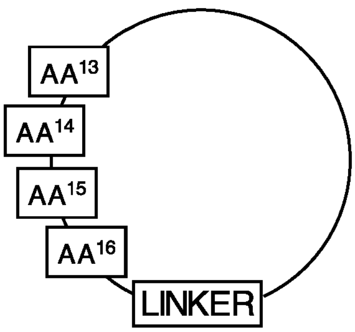


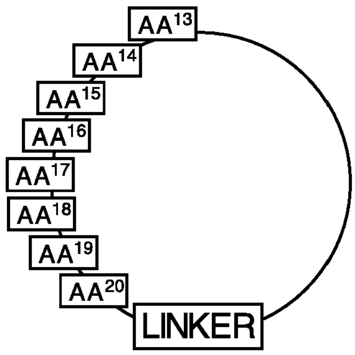


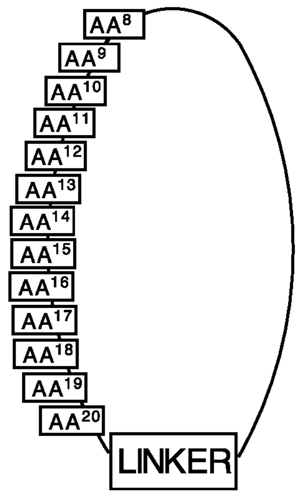


(XXI) (XXII) (XXIII)

wherein LINKER is a linear or branched organic divalent radical, moiety or compound having a molecular weight (Mw) comprised between 450 and 4,500 Daltons, in particular comprised between about 600 and about 4,500 Da, more particularly between about 600 and about 4,000 Da, and even more particularly between about 600 and about 3,500 Da; wherein $AA^{13}$-$AA^{14}$-$AA^{15}$-$AA^{16}$-$AA^{17}$-$AA^{16}$-$AA^{19}$-$AA^{20}$ is PEP12 as defined herein; wherein $AA^{6}$-$AA^{9}$-$AA^{10}$ is PEP3 as defined herein; wherein $AA^{11}$ and $AA^{12}$ are as defined herein; wherein one end of LINKER interacts covalently with $AA^{16}$ or $AA^{20}$; wherein another end of LINKER interacts covalently with $AA^{8}$ or $AA^{13}$; wherein curved lines represents covalent bonds between LINKER and AAs. Curved lines' lengths may not be representative of the actual relative distance between the LINKER and the AAs.

PEP1 is SAIS

In certain embodiments, PEP3 is selected from the group consisting of VPT, VPE, APT, TPT, VPA, APV, VPQ, VSQ, SRV and TQV.

In certain embodiments, PEP5 is a peptide of general formula PEP3-$AA^{11}$-$AA^{12}$; wherein PEP3 is selected from the group consisting of VPT, VPE, APT, TPT, VPA, APV, VPQ, VSQ, SRV and TQV; wherein $AA^{11}$ is selected from the group consisting of E, K, Q, R, A, D, G and H; and wherein $AA^{12}$ is selected from the group consisting of L, M, T, E, Q and H. In one particular example, PEP5 is selected from the group consisting of VPTEL, VPEKM, APTKL, APTQL, VPTKL, TPTKM,

VPARL, VPTRL, APVKT, VPQAL, VSQDL, VPQDL, VPTEE, VPTGQ, SRVHH and TQVQL.

In certain embodiments, PEP7 is an amino acid or a peptide with between two and seven amino acids of general formula $AA^1$-$AA^2$-$AA^3$-$AA^4$-$AA^5$-$AA^6$-$AA^7$; wherein wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R; wherein $AA^7$ is absent or is selected from the group consisting of S, T, C, E, Q, P and R, and wherein at least one of $AA^1$, $AA^2$, $AA^3$, $AA^4$, $AA^5$, $AA^6$ or $AA^7$ is not absent. In one particular example, PEP7 is selected from the group consisting of KIPKAXX, GIPEPXX, SIPKAXX, HVTKPTX, YVPKPXX, TVPKPXX, AVPKAXX, KVGKAXX, KASKAXX, GSAGPXX, AAPASXX, STPPTXX, HVPKPXX, RVPSTXX, ASAAPXX, ASASPXX, NDEGLEX, SSVKXQP and RNVQXRP, wherein X is C or S throughout the present description.

In certain embodiments, PEP9 is a peptide of general formula PEP7-PEP5; wherein PEP5 is a peptide of formula PEP3-$AA^{11}$-$AA^{12}$; wherein PEP3 is selected from the group consisting of VPT, VPE, APT, TPT, VPA, APV, VPQ, VSQ, SRV and TQV; wherein $AA^{11}$ is selected from the group consisting of E, K, Q, R, A, D, G and H; and wherein $AA^{12}$ is selected from the group consisting of L, M, T, E, Q and H; wherein PEP7 is an amino acid or a peptide with between two and seven amino acids of general formula $AA^1$-$AA^2$-$AA^3$-$AA^4$-$AA^5$-$AA^6$-$AA^7$; wherein $AA^1$, $AA^2$, $AA^3$, $AA^4$, and $AA^5$ are independently absent or $AA^1$ as defined herein; wherein $AA^6$ is absent or selected from the group consisting of S, T, C, E, Q, P and R; wherein $AA^7$ is absent or is selected from the group consisting of S, T, C, E, Q, P and R. In one particular example, PEP9 is selected from the group consisting of KIPKAXXVPTEL, GIPEPXXVPEKM, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKM, AAPASXXVPARL, STPPTXXVPTRL, HVPKPXXAPTKL, RVPSTXXAPVKT, ASAAPXXVPQAL, ASASPXXVSQDL, ASASPXXVPQDL, NDEGLEXVPTEE, NDEGLEXVPTGQ, SSVKXQPSRVHH and RNVQXRPTQVQL, wherein X is C or S throughout the present description.

In certain embodiments, PEP12 is a peptide of general formula PEP1-$AA^{17}$-PEP11; wherein $AA^{17}$ is selected from the group consisting of G, A, V, L, I, P, F, M, W, T and S (in particular is selected from the group consisting of M, I, L, V and T); wherein PEP1 is SAIS.

In certain embodiments, PEP11 is a peptide with 3 amino acids of general formula $AA^{18}$-$AA^{19}$-$AA^{20}$; wherein $AA^{18}$ is selected from the group consisting of L, V, Q, A and R; wherein $AA^{19}$ is selected from the group consisting of F, W, H, Y, I and K; wherein $AA^{20}$ is selected from the group consisting of L, F, Y, K, I, V and M. In one particular example, PEP11 is selected from the group consisting of LYL, LFF, LYF, LYY, LYK, LYI, LFI, LYV, VYY, QIM, AKV and RKI.

In certain embodiments, PEP7 is selected from the group consisting of KIPKAXX, GIPEPXX, SIPKAXX, HVTKPTX, YVPKPXX, TVPKPXX, AVPKAXX, KVGKAXX, KASKAXX, GSAGPXX, AAPASXX, STPPTXX, HVPKPXX, RVPSTXX, ASAAPXX, ASASPXX, NDEGLEX, SSVKXQP and RNVQXRP; wherein PEP8 is selected from the group consisting of GXGXR, SXAXR, SXGXH, AXGXH, XGXR, EXGXR, RXGXS, AXGXR, SXGXR, XGXL, XKXS, KXEXR, QXEXR, LEXAXA and LAXKXE; and wherein the pair PEP7:PEP8 is selected from the group consisting of KIPKAXX:GXGXR, GIPEPXX:SXAXR, SIPKAXX:GXGXR, HVTKPTX:SXGXH, YVPKPXX:SXGXH, TVPKPXX:AXGXH, AVPKAXX:AXGXH, KVGKAXX:XGXR, KASKAXX:EXGXR, GSAGPXX:RXGXS, AAPASXX:AXGXR, STPPTXX:SXGXR, HVPKPXX:SXGXH, RVPSTXX:XGXL, ASAAPXX:XKXS, ASASPXX:XKXS, NDEGLEX:KXEXR, NDEGLEX:QXEXR, SSVKXQP:LEXAXA and RNVQXRP:LAXKXE.

In certain embodiments, the pair PEP1:PEP11 is SAIS:LYL.

In particular, in certain embodiments, the pair PEP3:PEP1 is selected from the group consisting of VPT:SAIS, VPE:SAIS, APT:SAIS, TPT:SAIS, VPA:SAIS, APV:SAIS, VPQ:SAIS, VSQ:SAIS, SRV:SAIS, and TQV:SAIS.

In particular, in certain embodiments, the pair PEP5:PEP1 is selected from the group consisting of VPTKM:SAIS, VPTKL:SAIS, VPTQL:SAIS, VPTRL:SAIS, VPTKT:SAIS, VPTAL:SAIS, VPTDL:SAIS, VPEKM:SAIS, APTKL:SAIS, APTQL:SAIS, TPTKM:SAIS, VPARL:SAIS, APVKT:SAIS, VPQAL:SAIS, VSQDL:SAIS, VPQDL:SAIS, SRVHH:SAIS, and TQVQL:SAIS.

In particular, in certain embodiments, the pair PEP7:PEP1 is selected from the group consisting of GIPEPXX:SAIS, HVTKPTX:SAIS, YVPKPXX:SAIS, TVPKPXX:SAIS, AVPKAXX:SAIS, KVGKAXX:SAIS, KASKAXX:SAIS, GSAGPXX:SAIS, AAPASXX:SAIS, STPPTXX:SAIS, HVPKPXX:SAIS, RVPSTXX:SAIS, ASAAPXX:SAIS, ASASPXX:SAIS, SSVKXQP:SAIS, and RNVQXRP:SAIS.

In particular, in certain embodiments, the pair PEP9:PEP1 is selected from the group consisting of GIPEPXXVPTKM:SAIS, HVTKPTXVPTKL:SAIS, YVPKPXXVPTKL:SAIS, TVPKPXXVPTQL:SAIS, AVPKAXXVPTKL:SAIS, KVGKAXXVPTKL:SAIS, KASKAXXVPTKL:SAIS, GSAGPXXVPTKM:SAIS, AAPASXXVPTRL:SAIS, STPPTXXVPTRL:SAIS, HVPKPXXVPTKL:SAIS, RVPSTXXVPTKT:SAIS, ASAAPXXVPTAL:SAIS, ASASPXXVPTDL:SAIS, GIPEPXXVPEKM:SAIS, HVTKPTXAPTKL:SAIS, YVPKPXXAPTKL:SAIS, TVPKPXXAPTQL:SAIS, AVPKAXXAPTKL:SAIS, GSAGPXXTPTKM:SAIS, AAPASXXVPARL:SAIS, HVPKPXXAPTKL:SAIS, RVPSTXXAPVKT:SAIS, ASAAPXXVPQAL:SAIS, ASASPXXVSQDL:SAIS, ASASPXXVPQDL:SAIS, SSVKXQPSRVHH:SAIS, and RNVQXRPTQVQL:SAIS.

In particular, in certain embodiments, the pair PEP3:PEP12 is selected from the group consisting of VPT:SAIS-$AA^{17}$-LYL, VPE:SAIS-$AA^{17}$-LYL, APT:SAIS-$AA^{17}$-LYL, TPT:SAIS-$AA^{17}$-LYL, VPA:SAIS-$AA^{17}$-LYL, APV:SAIS-$AA^{17}$-LYL,

VPQ:SAIS-$AA^{17}$-LYL, VSQ:SAIS-$AA^{17}$-LYL, SRV:SAIS-$AA^{17}$-LYL, and TQV:SAIS-$AA^{17}$-LYL; and wherein $AA^{17}$ is selected from the group consisting of G, A, V, L, I, P, F, M, W, T and S (in particular is selected from the group consisting of M, I, L, V and T).

In particular, in certain embodiments, the pair PEP12:PEP5 is selected from the group consisting of VPTKM:SAIS-$AA^{17}$-LYL, VPTKL:SAIS-$AA^{17}$-LYL, VPTQL:SAIS-$AA^{17}$-LYL, VPTRL:SAIS-$AA^{17}$-LYL, VPTKT:SAIS-$AA^{17}$-LYL, VPTAL:SAIS-$AA^{17}$-LYL, VPTDL:SAIS-$AA^{17}$-LYL, VPEKM:SAIS-$AA^{17}$-LYL, APTKL:SAIS-$AA^{17}$-LYL, APTQL:SAIS-$AA^{17}$-LYL, TPTKM:SAIS-$AA^{17}$-LYL, VPARL:SAIS-$AA^{17}$-LYL, APVKT:SAIS-$AA^{17}$-LYL, VPQAL:SAIS-$AA^{17}$-LYL, VSQDL:SAIS-$AA^{17}$-LYL, VPQDL:SAIS-$AA^{17}$-LYL, SRVHH:SAIS-$AA^{17}$-LYL, and TQVQL:SAIS-$AA^{17}$-LYL; and wherein $AA^{17}$ is selected from the group consisting of G, A, V, L, I, P, F, M, W, T and S (in particular is selected from the group consisting of M, I, L, V and T).

In particular, in certain embodiments, the pair PEP12:PEP7 is selected from the group consisting of GIPEPXX:SAIS-$AA^{17}$-LYL, HVTKPTX:SAIS-$AA^{17}$-LYL, YVPKPXX:SAIS-$AA^{17}$-LYL, TVPKPXX:SAIS-$AA^{17}$-LYL, AVPKAXX:SAIS-$AA^{17}$-LYL, KVGKAXX:SAIS-$AA^{17}$-LYL, KASKAXX:SAIS-$AA^{17}$-LYL, GSAGPXX:SAIS-$AA^{17}$-LYL, AAPASXX:SAIS-$AA^{17}$-LYL, STPPTXX:SAIS-$AA^{17}$-LYL, HVPKPXX:SAIS-$AA^{17}$-LYL, RVPSTXX:SAIS-$AA^{17}$-LYL, ASAAPXX:SAIS-$AA^{17}$-LYL, ASASPXX:SAIS-$AA^{17}$-LYL, SSVKXQP:SAIS-$AA^{17}$-LYL, and RNVQXRP:SAIS-$AA^{17}$-LYL; and wherein $AA^{17}$ is selected from the group consisting of G, A, V, L, I, P, F, M, W, T and S (in particular is selected from the group consisting of M, I, L, V and T).

In particular, in certain embodiments, the pair PEP12:PEP9 is selected from the group consisting of GIPEPXXVPTKM:SAIS-$AA^{17}$-LYL, HVTKPTXVPTKL:SAIS-$AA^{17}$-LYL, YVPKPXXVPTKL:SAIS-$AA^{17}$-LYL, TVPKPXXVPTQL:SAIS-$AA^{17}$-LYL, AVPKAXXVPTKL:SAIS-$AA^{17}$-LYL, KVGKAXXVPTKL:SAIS-$AA^{17}$-LYL, KASKAXXVPTKL:SAIS-$AA^{17}$-LYL, GSAGPXXVPTKM:SAIS-$AA^{17}$-LYL, AAPASXXVPTRL:SAIS-$AA^{17}$-LYL, STPPTXXVPTRL:SAIS-$AA^{17}$-LYL, HVPKPXXVPTKL:SAIS-$AA^{17}$-LYL, RVPSTXXVPTKT:SAIS-$AA^{17}$-LYL, ASAAPXXVPTAL:SAIS-$AA^{17}$-LYL, ASASPXXVPTDL:SAIS-$AA^{17}$-LYL, GIPEPXXVPEKM:SAIS-$AA^{17}$-LYL, HVTKPTXAPTKL:SAIS-$AA^{17}$-LYL, YVPKPXXAPTKL:SAIS-$AA^{17}$-LYL, TVPKPXXAPTQL:SAIS-$AA^{17}$-LYL, AVPKAXXAPTKL:SAIS-$AA^{17}$-LYL, GSAGPXXTPTKM:SAIS-$AA^{17}$-LYL, AAPASXXVPARL:SAIS-$AA^{17}$-LYL, HVPKPXXAPTKL:SAIS-$AA^{17}$-LYL, RVPSTXXAPVKT:SAIS-$AA^{17}$-L YL, ASAAPXXVPQAL:SAIS-$AA^{17}$-LYL, ASASPXXVSQDL:SAIS-$AA^{17}$-LYL, ASASPXXVPQDL:SAIS-$AA^{17}$-LYL, SSVKXQPSRVHH:SAIS-$AA^{17}$-LYL, and RNVQXRPTQVQL:SAIS-$AA^{17}$-LYL; and wherein $AA^{17}$ is selected from the group consisting of G, A, V, L, I, P, F, M, W, T and S (in particular is selected from the group consisting of M, I, L, V and T).

In certain embodiments, the triplet PEP7:PEP3:PEP1 is selected from the group consisting of GIPEPXX:VPT:SAIS, HVTKPTX:VPT:SAIS, YVPKPXX:VPT:SAIS, TVPKPXX:VPT:SAIS, AVPKAXX:VPT:SAIS, KVGKAXX:VPT:SAIS, KASKAXX:VPT:SAIS, GSAGPXX:VPT:SAIS, AAPASXX:VPT:SAIS, STPPTXX:VPT:SAIS, HVPKPXX:VPT:SAIS, RVPSTXX:VPT:SAIS, ASAAPXX:VPT:SAIS, ASASPXX:VPT:SAIS, GIPEPXX:VPE:SAIS, HVTKPTX:APT:SAIS, YVPKPXX:APT:SAIS, TVPKPXX:APT:SAIS, AVPKAXX:APT:SAIS, GSAGPXX:TPT:SAIS, AAPASXX:VPA:SAIS, HVPKPXX:APT:SAIS, RVPSTXX:APV:SAIS, ASAAPXX:VPQ:SAIS, ASASPXX:VSQ:SAIS, ASASPXX:VPQ:SAIS, SSVKXQP:SRV:SAIS, and RNVQXRP:TQV:SAIS.

In certain embodiments, the triplet PEP7:PEP3:PEP12 is selected from the group consisting of GIPEPXX:VPT:SAIS-$AA^{17}$-LYL, HVTKPTX:VPT:SAIS-$AA^{17}$-LYL, YVPKPXX:VPT:SAIS-$AA^{17}$-LYL, TVPKPXX:VPT:SAIS-$AA^{17}$-LYL, AVPKAXX:VPT:SAIS-$AA^{17}$-LYL, KVGKAXX:VPT:SAIS-$AA^{17}$-LYL, KASKAXX:VPT:SAIS-$AA^{17}$-LYL, GSAGPXX:VPT:SAIS-$AA^{17}$-LYL, AAPASXX:VPT:SAIS-$AA^{17}$-LYL, STPPTXX:VPT:SAIS-$AA^{17}$-LYL, HVPKPXX:VPT:SAIS-$AA^{17}$-LYL, RVPSTXX:VPT:SAIS-$AA^{17}$-LYL, ASAAPXX:VPT:SAIS-$AA^{17}$-LYL, ASASPXX:VPT:SAIS-$AA^{17}$-LYL, GIPEPXX:VPE:SAIS-$AA^{17}$-LYL, HVTKPTX:APT:SAIS-$AA^{17}$-LYL, YVPKPXX:APT:SAIS-$AA^{17}$-LYL, TVPKPXX:APT:SAIS-$AA^{17}$-LYL, AVPKAXX:APT:SAIS-$AA^{17}$-LYL, GSAGPXX:TPT:SAIS-$AA^{17}$-LYL, AAPASXX:VPA:SAIS-$AA^{17}$-LYL, HVPKPXX:APT:SAIS-$AA^{17}$-LYL, RVPSTXX:APV:SAIS-$AA^{17}$-LYL, ASAAPXX:VPQ:SAIS-$AA^{17}$-LYL, ASASPXX:VSQ:SAIS-$AA^{17}$-LYL, ASASPXX:VPQ:SAIS-$AA^{17}$-LYL, SSVKXQP:SRV:SAIS-$AA^{17}$-LYL, and RNVQXRP:TQV:SAIS-$AA^{17}$-LYL; and wherein $AA^{17}$ is selected from the group consisting of G, A, V, L, I, P, F, M, W, T and S (in particular is selected from the group consisting of M, I, L, V and T).

In certain embodiments, the triplet PEP7:PEP5:PEP1 is selected from the group consisting of GIPEPXX:VPTKM:SAIS, HVTKPTX:VPTKL:SAIS, YVPKPXX:VPTKL:SAIS, TVPKPXX:VPTQL:SAIS, AVPKAXX:VPTKL:SAIS, KVGKAXX:VPTKL:SAIS, KASKAXX:VPTKL:SAIS, GSAGPXX:VPTKM:SAIS, AAPASXX:VPTRL:SAIS, STPPTXX:VPTRL:SAIS, HVPKPXX:VPTKL:SAIS, RVPSTXX:VPTKT:SAIS, ASAAPXX:VPTAL:SAIS, ASASPXX:VPTDL:SAIS, GIPEPXX:VPEKM:SAIS, HVTKPTX:APTKL:SAIS, YVPKPXX:APTKL:SAIS, TVPKPXX:APTQL:SAIS, AVPKAXX:APTKL:SAIS, GSAGPXX:TPTKM:SAIS, AAPASXX:VPARL:SAIS, HVPKPXX:APTKL:SAIS, RVPSTXX:APVKT:SAIS, ASAAPXX:VPQAL:SAIS, ASASPXX:VSQDL:SAIS, ASASPXX:VPQDL:SAIS, SSVKXQP:SRVHH:SAIS, and RNVQXRP:TQVQL:SAIS.

In certain embodiments, the triplet PEP7:PEP5:PEP12 is selected from the group consisting of GIPEPXX:VPTKM:SAIS-$AA^{17}$-LYL, HVTKPTX:VPTKL:SAIS-$AA^{17}$-LYL, YVPKPXX:VPTKL:SAIS-$AA^{17}$-LYL, TVPKPXX:VPTQL:SAIS-$AA^{17}$-LYL, AVPKAXX:VPTKL:SAIS-$AA^{17}$-LYL, KVGKAXX:VPTKL:SAIS-$AA^{17}$-LYL, KAS-

KAXX:VPTKL:SAIS-AA$^{17}$-LYL, GSAGPXX:VPTKM:SAIS-AA$^{17}$-LYL, AAPASXX:VPTRL:SAIS-AA$^{17}$-LYL, STPPTXX:VPTRL:SAIS-AA$^{17}$-LYL, HVPKPXX:VPTKL:SAIS-AA$^{17}$-LYL, RVPSTXX:VPTKT:SAIS-AA$^{17}$-LYL, ASAAPXX:VPTAL:SAIS-AA$^{17}$-LYL, ASASPXX:VPTDL:SAIS-AA$^{17}$-LYL, GIPEPXX:VPEKM:SAIS-AA$^{17}$-LYL, HVTKPTX:APTKL:SAIS-AA$^{17}$-LYL, YVPKPXX:APTKL:SAIS-AA$^{17}$-LYL, TVPKPXX:APTQL:SAIS-AA$^{17}$-LYL, AVPKAX-X:APTKL:SAIS-AA$^{17}$-LYL, GSAGPXX:TPTKM:SAIS-AA$^{17}$-LYL, AAPASXX:VPARL:SAIS-AA$^{17}$-LYL, HVPKPXX:APTKL:SAIS-AA$^{17}$-LYL, RVPSTXX:APVKT:SAIS-AA$^{17}$-LYL, ASAAPXX:VPQAL:SAIS-AA$^{17}$-LYL, ASASPXX:VSQDL:SAIS-AA$^{17}$-LYL, ASASPXX:VPQDL:SAIS-AA$^{17}$-LYL, SSVKXQP:SRVHH:SAIS-AA$^{17}$-LYL, and RNVQXRP:TQVQL:SAIS-AA$^{17}$-LYL; and wherein AA$^{17}$ is selected from the group consisting of G, A, V, L, I, P, F, M, W, T and S (in particular is selected from the group consisting of M, I, L, V and T).

In one example, said cyclic GFR-binding compound is a synthetic molecule as defined herein in the definition section.

In one particular example, said cyclic GFR-binding compound is a synthetic peptide or a cyclic peptidomimetic.

In one most particular example, said cyclic GFR-binding compound is a cyclic synthetic peptide.

In one example, a length of said cyclic GFR-binding compound, in solution, such as in a physiologically acceptable solvent such as water or PBS, is comprised between about 6 and about 20 nm, preferably between about 6 and about 16 nm, as determined using the standard « 3D » procedure described above.

In one particular example, said cyclic GFR-binding compounds may be any one of peptides comprising the 4mer peptide SAIS among SEQ **ID** NO: 4803 to 13564.

*LINKER suitable for implementing cyclic GFR-compounds' embodiments*

In one particular example, said LINKER has a Mw comprised between 450 and 4,500 Daltons, in particular comprised between about 600 and about 4,500 Da, more particularly between about 600 and about 4,000 Da, and even more particularly between about 600 and about 3,500 Da.

The chemical nature of said LINKER is not meant to be particularly limited and may be any organic molecule capable of covalently connecting two ends of a peptide or a peptidomimetic such as PEP(A) or PEP(C)-PEP12 so as to form a cyclic compound and so long as LINKER provides sufficient cycle stability for the pharmaceutical association, combination or composition as defined herein to promote the conversion or recoding of a neoplastic cell into a non-neoplastic cell. LINKER may thus be, for example, in certain embodiments, a peptide, or variant, analog or peptidomimetic thereof, a polysaccharide, a polynucleotide, a saturated or unsaturated hydrocarbon chain, or a mixture thereof.

For example, in certain embodiments, LINKER is a peptide with 6 to 31 amino acids. In one particular example, LINKER is a peptide with 6 to 25 amino acids. In one particular example, LINKER is a peptide with 8 to 25 amino acids. In one most particular example, LINKERL is a peptide with 8 to 20 amino acids.

Thus, in one particular example, said cyclic GFR-binding compound is a peptide, a variant or analog thereof as defined herein, with between 10-60 (in particular between 15-60, more particularly between 10-55, and even more particularly between 15-55) amino acids or with between 10-35 (in particular between 15-35, more particularly between 10-30, and even more particularly between 15-30) amino acids, comprising a peptide, a variant or analog thereof of general formula (IIIa); wherein one end of LINKER interacts covalently with one end of PEP(A); wherein PEP(A) comprises PEP1 or PEP12; wherein LINKER is a peptide comprising 6 to 31 amino acids (in particular 6 to 25, 8 to 25, or 8 to 20 amino acids).

Thus, in one particular example, said cyclic GFR-binding compound is a cyclic peptide or a cyclic peptidomimetic as defined herein, with between 10-60 (in particular between 15-60, more particularly between 10-55, and even more particularly between 15-55) amino acids or with between 10-35 (in particular between 15-35, more particularly between 10-30, and even more particularly between 15-30) amino acids, comprising a peptide or a peptidomimetic having the following general formula (IIIb); wherein one end of a first LINKER interacts covalently with one end of PEP(A); wherein one end of a second LINKER interacts covalently with another end of PEP(A); wherein another end of a first LINKER interacts covalently with another end of a second LINKER; wherein PEP(A) comprises PEP1 or PEP12; wherein LINKER are independently a peptide comprising 6 to 31 amino acids (in particular 6 to 25, 8 to 25, or 8 to 20 amino acids).

Thus, in one particular example, said cyclic GFR-binding compound is a peptide, a variant or analog thereof as defined herein, with between 10-60 (in particular between 15-60, more particularly between 10-55, and even more particularly between 15-55) amino acids or with between 10-35 (in particular between 15-35, more particularly between 10-30, and even more particularly between 15-30) amino acids, comprising a peptide, a variant or analog thereof of general formula (IVa); wherein LINKER is a peptide comprising 6 to 31 amino acids (in particular 6 to 25, 8 to 25, or 8 to 20 amino acids); wherein PEP12 is a peptide with 8 amino acids of formula PEP1-AA$^{17}$-PEP11 as already defined herein; wherein PEP1, PEP11 and PEP(C) are as already defined herein; wherein AA$^{13}$ may be an N-terminal amino acid or a C-terminal amino acids; wherein AA$^{20}$ may be an N-terminal amino acid or a C-terminal amino acid; wherein one end of LINKER interacts covalently with one end of PEP12 via AA$^{20}$; wherein another end of LINKER interacts covalently with one end of PEP(C); wherein another end of PEP(C) interacts covalently with PEP12 via AA$^{13}$.

Thus, in one particular example, said cyclic GFR-binding compound is a cyclic peptide or a cyclic peptidomimetic as defined herein, with between 10-60 (in particular between 15-60, more particularly between 10-55, and even more particularly between 15-55) amino acids or with between 10-35 (in particular between 15-35, more particularly between 10-30, and even more particularly between 15-30) amino acids, comprising a peptide or a peptidomimetic having the following general formula (IVb); wherein LINKER are independently a peptide comprising 6 to 31 amino acids (in particular 6 to 25, 8 to 25, or 8 to 20 amino acids); wherein PEP12 is a peptide with 8 amino acids of formula PEP1-$AA^{17}$-PEP11 as defined herein; wherein PEP2 is a peptide with five amino acids as already defined herein; wherein one end of PEP(C) interacts covalently with PEP12 via one end of PEP1; wherein one end of a first LINKER interacts covalently with one end of PEP12 via one end of

**PEP11; wherein** one end of a second LINKER interacts covalently with another end of PEP(C); wherein another end of a first LINKER interacts covalently with another end of a second LINKER; wherein PEP(C) is a peptide with at least 5 amino acids, in particular a peptide with between 5 and 12 amino acids.

Thus, in one particular example, said cyclic GFR-binding compound is a peptide, a variant or analog thereof as defined herein, with between 10-60 (in particular between 15-60, more particularly between 10-55, and even more particularly between 15-55) amino acids or with between 10-35 (in particular between 15-35, more particularly between 10-30, and even more particularly between 15-30) amino acids, having any one of the general formula (VII) to (XXIII); wherein LINKER is a peptide comprising 6 to 31 amino acids (in particular 6 to 25, 8 to 25, or 8 to 20 amino acids).

In one particular example, LINKER comprises (or is) a peptide of general formula (XXIV):

$$*AA^{201}-AA^{202}-AA^{203}-AA^{204}-AA^{205}-AA^{206}-AA^{207}-AA^{208}-AA^{209}** \qquad (XXIV)$$

wherein said peptide of formula (XXIV) may be selected from the group consisting of $*AA^{III}-AA^{I}-AA^{I}-AA^{II}-AA^{VII}-AA^{XII}-AA^{XII}-AA^{XIII}-AA^{XIII}**$, $*AA^{III}-AA^{I}-AA^{VI}-AA^{II}-AA^{VII}-AA^{XII}-AA^{XII}-AA^{XIII}-AA^{XIII}**$, $*AA^{III}-AA^{I}-AA^{I}-AA^{II}-AA^{II}-AA^{XII}-AA^{XII}-AA^{XIII}-AA^{XIII}**$, $*AA^{III}-AA^{I}-AA^{II}-AA^{II}-AA^{VII}-AA^{XII}-AA^{XII}-AA^{XIII}-AA^{XIII}**$, $*AA^{III}-AA^{I}-AA^{II}-AA^{IV}-AA^{IV}-AA^{XII}-AA^{XII}-AA^{XIII}-AA^{XIII}**$, $*AA^{II}-AA^{I}-AA^{II}-AA^{II}-AA^{II}-AA^{XII}-AA^{XII}-AA^{XIII}-AA^{XIII}**$, $*AA^{III}-AA^{I}-AA^{II}-AA^{VII}-AA^{II}-AA^{XII}-AA^{XII}-AA^{XIII}-AA^{XIII}**$, $*AA^{III}-AA^{I}-AA^{I}-AA^{II}-AA^{II}-AA^{XII}-AA^{XII}-AA^{XIII}-AA^{XIII}**$, $*AA^{V}-AA^{V}-AA^{VII}-AA^{VII}-AA^{XII}-AA^{XII}-AA^{XIII}-AA^{XIII}**$, $*AA^{VIII}-AA^{V}-AA^{VII}-AA^{II}-AA^{XII}-AA^{XII}-AA^{XIII}-AA^{XIII}**$ and $*AA^{V}-AA^{V}-AA^{VII}-AA^{II}-AA^{XII}-AA^{XII}-AA^{XIII}-AA^{XIII}**$; wherein $AA^{I}$ is any amino acid as defined herein, AA" is any polar amino acid as defined herein, $AA^{III}$ is any acidic amino acid as defined herein, $AA^{IV}$ is any aliphatic amino acid as defined herein, $AA^{V}$ is any apolar amino acid as defined herein, $AA^{VI}$ is any aromatic amino acid as defined herein, $AA^{VII}$ is any basic amino acid as defined herein, $AA^{VII}$ is L or I as defined herein, $AA^{XII}$ is an amino acid selected from the group consisting of G, A, V, L, I, P, M, K, R, H, Y and E, wherein $AA^{XIII}$ is absent, $AA^{II}$ or $AA^{VII}$, preferably absent; and wherein any one of the fragment $AA^{201}$, $AA^{201}-AA^{202}$ $AA^{201}-AA^{202}-AA^{203}$, $AA^{201}-AA^{202}-AA^{203}-AA^{204}$, $AA^{201}-AA^{202}-AA^{203}-AA^{204}-AA^{205}$, $AA^{201}-AA^{202}-AA^{203}-AA^{204}-AA^{205}-AA^{206}$, $AA^{201}-AA^{202}-AA^{203}-AA^{204}-AA^{205}-AA^{206}-AA^{207}$, $AA^{203}-AA^{204}-AA^{205}-AA^{206}-AA^{207}-AA^{208}-AA^{209}$, $AA^{204}-AA^{205}-AA^{206}-AA^{207}-AA^{208}-AA^{209}$, $AA^{205}-AA^{206}-AA^{207}-AA^{208}-AA^{209}$, $AA^{206}-AA^{207}-AA^{208}-AA^{209}$, $AA^{207}-AA^{208}-AA^{209}$, $AA^{208}-AA^{209}$ or $AA^{209}$, may be absent. In the peptides of formula (XXIV), any one of the amino acid labelled "*" or the amino acid labelled "**" is an N-terminal amino acid and the other is a C-terminal amino acid.

For instance, in the peptide of formula $*AA^{III}-AA^{I}-AA^{I}-AA^{II}-AA^{VII}-AA^{XII}AA^{XII}-AA^{XII}-AA^{XIII}-AA^{XIII}**$ (XXIV-1), $AA^{III}$ occupies position $AA^{201}$, $*AA^{I}$ occupies position $AA^{202}$, $AA^{I}$ occupies position $AA^{203}$, $AA^{II}$ occupies position $AA^{204}$, $AA^{VII}$occupies position $AA^{205}$, $AA^{XII}$ occupies position $AA^{206}$, $AA^{XII}$ occupies position $AA^{207}$, $AA^{XIII}$ occupies position $AA^{208}$ and $AA^{XIII}**$ occupies position $AA^{209}$.

Likewise, in the peptide of formula $*AA^{V}-AA^{V}-AA^{VII}-AA^{II}-AA^{XII}-AA^{XII}-AA^{XIII}-AA^{XIII}**$ (XXIV-1) $AA^{V}$ occupies position $AA^{201}$, $AA^{V}$ occupies position $AA^{202}$, $AA^{VII}$ occupies position $AA^{203}$, $AA^{II}$ occupies position $AA^{204}$, $AA^{XII}$ occupies position $AA^{205}$, $AA^{XII}$ occupies position $AA^{206}$, $AA^{XIII}$ occupies position $AA^{207}$, $AA^{XIII}$ occupies position $AA^{208}$ and position $AA^{209}$ is vacants.

In one example, LINKER may thus comprise or be any one of the following peptides: $*AA^{201}-AA^{202}-AA^{203}-AA^{204}-AA^{205}-AA^{206}-AA^{207}-AA^{208}**$ (XXIV-1); $*AA^{201}-AA^{202}-AA^{203}-AA^{204}-AA^{205}-AA^{206}-AA^{207}**$ (XXIV-2); $*AA^{201}-AA^{202}-AA^{203}-AA^{204}-AA^{205}-AA^{206}**$ (XXIV-3); $*AA^{201}-AA^{202}-AA^{203}-AA^{204}-AA^{205}**$ (XXIV-4); $*AA^{201}-AA^{202}-AA^{203}-AA^{204}**$ (XXIV-5); $*AA^{201}-AA^{202}-AA^{203}**$ (XXIV-6); $*AA^{201}-AA^{202}**$ (XXIV-7); $*AA^{202}-AA^{203}-AA^{204}-AA^{205}-AA^{206}-AA^{207}-AA^{208}-AA^{209}**$ (XXIV-8); $*AA^{203}-AA^{204}-AA^{205}-AA^{206}-AA^{207}-AA^{208}-AA^{209}**$ (XXIV-9); $*AA^{204}-AA^{205}-AA^{206}-AA^{207}-AA^{208}-AA^{209}**$ (XXIV-10); $*AA^{205}-AA^{206}-AA^{207}-AA^{208}-AA^{209}**$ (XXIV-11); $*AA^{206}-AA^{207}-AA^{208}-AA^{209}**$ (XXIV-12); $*AA^{207}-AA^{208}-AA^{209}**$ (XXIV-13); $*AA^{208}-AA^{209}**$ (XXIV-14); wherein, for instance, said peptide of formulae (XXIV-1) may thus be selected from the group consisting of $*AA^{III}-AA^{I}-AA^{I}-AA^{II}-AA^{VII}-AA^{XII}-AA^{XII}-AA^{XIII}**$, $*AA^{III}-AA^{I}-AA^{VI}-AA^{II}-AA^{VII}-AA^{XII}-AA^{XII}-AA^{XIII}**$, $*AA^{III}-AA^{I}-AA^{I}-AA^{II}-AA^{II}-AA^{XII}-AA^{XII}-AA^{XIII}**$,$*AA^{III}-AA^{I}-AA^{II}-AA^{II}-AA^{VII}-AA^{XII}-AA^{XII}-AA^{XIII}**$,$*AA^{III}-AA^{I}-AA^{II}-AA^{IV}-AA^{IV}-AA^{II}-AA^{XII}-AA^{XII}-AA^{XIII}**$, $*AA^{II}-AA^{I}-AA^{II}-AA^{II}-AA^{II}-AA^{XII}-AA^{XII}-AA^{XIII}**$,$*AA^{III}-AA^{III}-AA^{II}-AA^{VII}-AA^{II}-AA^{XIII}-AA^{XII}-AA^{XIII}**$, $*AA^{III}-AA^{I}-AA^{I}-AA^{II}-AA^{II}-AA^{XII}-AA^{XII}-AA^{XIII}**$,$AA^{V}-AA^{V}-AA^{VII}-AA^{VII}-AA^{XII}-AA^{XII}-AA^{XIII}**$, $*AA^{VII}-AA^{V}-AA^{VII}-AA^{II}-AA^{XII}-AA^{XII}-AA^{XIII}**$, and $AA^{V}-AA^{V}-AA^{VII}-AA^{II}-AA^{XII}-AA^{XII}-AA^{XIII}**$.

In one particular example, LINKER comprises a peptide of formula (XXIV), (XXIV-2) or (XXIV-4).

In one example, LINKER comprises (or is) a poly-(aliphatic amino acid) peptide such as poly-alanine peptide $(A)_n$, or a poly-glycine $(G)_n$, n being an integer comprised between 2 and 31, in particular between 2 to 25, more particularly between 2 and 20, such as A-A-A-A-A-A-A-A-A-A, A-A-A-A-A-A-A-A, A-A-A-A-A, G-G-G-G-G-G-G-G-G, G-G-G-G-G-G-G or G-G-G-G-G.

In one particular example, LINKER comprises (or is) a peptide of general formulae (XXIV) to (XXIV-14), more particularly (XXIV), (XXIV-2) or (XXIV-4), and/or a poly-(aliphatic amino acid)$_n$ peptide as defined herein.

More particularly, LINKER is a octapeptide (8 amino acids). More particularly, LINKER is a nonapeptide (9 amino acids). More particularly, LINKER is a decapeptide (10 amino acids). More particularly, LINKER is a hendecapeptide (11 amino acids). More particularly, LINKER is a dodecapeptide (12 amino acids). More particularly, LINKER is a tridecapeptideo (13 amino acids). More particularly, LINKER is a tetradecapeptide (14 amino acids). More particularly, LINKER is a pentadecapeptide (15 amino acids). More particularly, LINKER is a hexadecapeptide (16 amino acids). More particularly, LINKER is a heptadecapeptide (17 amino acids). More particularly, LINKER is an octadecapeptide (18 amino acids). More particularly, LINKER is an enneadecapeptide (19 amino acids). More particularly, LINKER is an icosapeptide (20 amino acids).

In one particular example, LINKER comprises one or more of a peptide selected from the group consisting of DENEKVV, DENKNVV, DEYDKVV, DDSSNVI, DSSNNVI, DDMGVPT, DKGVVTY, NDKQQII, DAANNVV, DSANNW, DDSSNVI, DNGRVLL, VGRKPKV, IGKTPKI, VGRTPKV, RIKPHQGQH, EYVRKKPKL, EIVRKKPIF, EYVRKKP, EIVRKKP, polyalanine $(A_{1-12})$ (preferably $A_{2-8}$) and polyglycine $(G_{1-12})$ (preferably $G_{2-8}$).

For example, in certain embodiments, to synthetise cyclic GFR-binding compounds of the present disclosure, the covalent bonds between e.g. LINKER, PEP(A), PEP(C) or PEP1 to PEP12, may be created through the chemical reaction between a free amine moiety e.g. of a N-terminal amino acid ($-NH_2$ or $-NH_3X$, X generally being a halide anion selected from the group consisting of $F^-$, $Cl^-$ and $Br^-$), typically acting as a nucleophile, and an electrophile moiety of e.g. a C-terminal amino acid. Such an electrophile moiety includes, but is not limited to, alkyl halides ($-CR_2-X$), alcohols ($-CR_2-OH$), acid chlorides ($-C(=O)X$), esters, ($-C(=O)OR$), phosphate ($-OP(OR)_3$), phosphinate ($-OP(OR)R_2$), phosphonates ($-OP(OR)_2R$), phosphonite ($-P(OR)_2R$) or sulfonic esters ($-SO_2OR$). More particularly, this covalent bond is an amide bond (in particular a peptide bond) formed through conventional peptide synthesis using conventional coupling reagents as already defined herein.

For example, in certain embodiments, to synthetise cyclic GFR-binding compounds of the present disclosure, the covalent bonds between e.g. LINKER, PEP(A), PEP(C) or PEP1 to PEP12, may be created through the chemical reaction between a free carboxylic acid moiety e.g. of a C-terminal amino acid ($-CO_2H$ or $-CO_2X$, X generally being an inorganic cation such as alkaline cations (*e.g.* $Li^+$, $Na^+$ or $K^+$) or an organic cation such as ammonium cations), typically acting as an electrophile, and a nucleophile moiety of e.g. an N-terminal amino acid. Such a nucleophile moiety includes, but is not limited to, alcohols (-OH), amines ($-NH_2$), phosphines ($-PR_3$), thiols (-SH). More particularly, this covalent interaction is a peptide bond formed through conventional peptide synthesis using conventional coupling reagents as already defined herein.

Cyclisation of a cyclic GFR-binding compound of the present disclosure may be carried out as described above using conventional peptide bond formation procedures, click chemistry, formation of disulphide bonds, etc.

The present disclosure provides pharmaceutical associations, combinations and compositions, and uses for converting or recoding any neoplastic cell into a non-neoplastic cell (in particular, a functional and healthy cell) of any type. In some cases, the cell type of the converted or recoded non-neoplastic cell may be selected/chosen by *e.g.* a person providing the treatment to a subject.

In one example, said pharmaceutical association, combination or composition induces the conversion of a neoplastic cell into a physiologically functional and/or healthy cell of the cell lineage includingof bone cell, chondrocytic cell lineages. Such cells include any progenitor or precursor cells or any partially or fully differentiated cells of these lineages. More generally, the present disclosure includes the treatment of any neoplastic cell of any type and cell lineage using the associations, combinations, compositions, methods and uses as defined herein.

For example, in certain embodiments, a neoplastic cell of a bone tissue *(i.e.* a neoplastic cell from the bone cell lineage) has been, may be or will be the cause of the development of a neoplastic bone disease *(e.g.* bone cancer). Inducing the conversion of this neoplastic bone cell into a physiologically functional and/or healthy cell (any physiologically functional and/or healthy cell) of the bone cell lineage may protect a subject/patient carrying this cell from bone cancer. In some cases, it may be preferable and/or satisfying and/or more practical to induce the conversion of a neoplastic bone cell into a functional and/or healthy cell of a different cell lineage *i.e.* not a cell from a bone lineage, such as, for example, in certain embodiments, a cell from a chondrocytic cell lineage, a fibroblast lineage or a ligament/tendon cell lineage.

In one example, a neoplastic cell of a soft tissue such as a muscle, vascular, skin or kidney tissue *(i.e.* a neoplastic cell from the muscle, vascular, skin or kidney cell lineage, respectively) has been, may be or will be the cause of the development of a neoplastic muscle, vascular, skin or kidney disease *(e.g.* Rhabdomyosarcoma, Hemangiosarcoma, basal cell carcinoma or Squamous cell carcinoma, respectively). Inducing the conversion of any of these neoplastic cells

into a physiologically functional and/or healthy cell (any physiologically functional and/or healthy cell) of the muscle, vascular, skin or kidney cell lineage, respectively, may protect a subject carrying any of these cells from the diseases mentioned above. In some cases, it may be preferable and/or satisfying and/or more practical to induce the conversion of, *e.g.* a neoplastic muscle, vascular, skin or kidney cell into a functional and/or healthy cell of a different cell lineage *i.e.* not a cell from a muscle, vascular, skin or kidney lineage, such as, for example, in certain embodiments, a cell from a bone cell lineage so that a medical practitioner such as a surgeon, may be able to surgically remove the newly converted bone cells more conveniently. The surgery may be carried out purely for aesthetic reasons and/or because of a discomfort and/or to avoid the possibility of *e.g.* infections, complications, etc.

For example, in certain embodiments, a neoplastic cell of an adipose tissue (*i.e.* a neoplastic cell from the adipocyte lineage) has been, may be or will be the cause of the development of a neoplastic adipose tissue disease *(e.g.* adipose tissue cancer or lipoma). Several treatment routes may be envisaged to induce the conversion of this neoplastic adipocyte into a physiologically functional and/or healthy cell and thus protect a subject/patient carrying this cell from a lipoma. In some cases, it may be preferable and/or satisfying and/or more practical to induce the conversion of the neoplastic adipocyte into a functional and/or healthy cell of a bone cell lineage. Once the treatment has been completed *i.e.* after the conversion of the neoplastic adipocyte into a non-neoplastic bone cell (e.g. an osteocyte) has taken place, surgical removal of the newly converted bone cells may be performed for *e.g.* aesthetic reasons and/or because of a discomfort and/or to avoid the possibility of *e.g.* infections, complications, etc. In other cases, it may be preferable and/or satisfying and/or more practical to induce the conversion of the neoplastic adipocyte into a functional and/or healthy cell of a muscle cell lineage. Because the conversion of the neoplastic adipocyte into a non-neoplastic muscle cell (e.g. a myocyte) does not yield hard tissues such as osteocytes, the patient would generally not feel any substantial discomfort and therefore, the surgical removal of the newly converted muscle cells may be avoided, although it may still be performed if the patient were to feel even the slight discomfort. The necessity of performing surgery following the presently defined recoding treatment would generally be left to the appreciation of the patient upon supervision of a medical practitioner.

The present disclosure thus provides uses to convert or recode a neoplastic cell of a specific tissue/cell type *(e.g.* bone, cartilage, neuron, prostate, ovary, muscle, skin, vascular, ligament, tendon, eye retina, kidney, head, neck, blood, gastrointestinal, lung and adipose tissues) into a non-neoplastic cell of any tissue/cell type among bone, cartilage, and adipose tissue).

**Induction into a cell of a specific cell lineage**

*Bone cell lineage*

Certain embodiments of the invention are particularly useful for inducing the conversion of a neoplastic cell into a cell (any cell) of a bone cell lineage.

In certain embodiments useful for inducing the conversion of a neoplastic cell into a cell (any cell) of a bone cell lineage, PEP1 is SAIS.

In certain embodiments useful for inducing the conversion of a neoplastic cell into a cell (any cell) of a bone cell lineage, PEP3 is selected from the group consisting of VPT, APT, VPQ, VSQ and TQV.

In certain embodiments useful for inducing the conversion of a neoplastic cell into a cell (any cell) of a bone cell lineage, PEP5 is a peptide of general formula PEP3-$AA^{11}$-$AA^{12}$; wherein PEP3 is selected from the group consisting of VPT, APT, VPQ, VSQ and TQV; wherein $AA^{11}$ is selected from the group consisting of E, K, Q, R, A, D, G and H, in particular E, K, Q, A and D; wherein $AA^{12}$ is selected from the group consisting of L, M, T, E, Q and H, in particular L. In one particular example, PEP5 is selected from the group consisting of VPTEL, APTKL, APTQL, VPTKL, VPQAL, VSQDL, VPQDL and TQVQL.

In certain embodiments useful for inducing the conversion of a neoplastic cell into a cell (any cell) of a bone cell lineage, PEP7 is an amino acid or a peptide with between two and seven amino acids of general formula $AA^1$-$AA^2$-$AA^3$-$AA^4$-$AA^5$-$AA^6$-$AA^7$; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q, P and R, preferably C, S, T or R; wherein $AA^7$ is absent or is selected from the group consisting of S, T, C, E, Q, P and R, preferably is selected from the group consisting of C, S and P; and wherein at least one of $AA^1$, $AA^2$, $AA^3$, $AA^4$, $AA^5$, $AA^6$ or $AA^7$ is not absent. In one particular example, PEP7 is selected from the group consisting of KIPKAXX, SIPKAXX, HVTKPTX, YVPKPXX, TVPKPXX, AVPKAXX, KVGKAXX, ASAAPXX, ASASPXX and RNVQXRP.

In certain embodiments useful for inducing the conversion of a neoplastic cell into a cell (any cell) of a bone cell lineage, PEP9 is a peptide of general formula $AA^1$-$AA^2$-$AA^3$-$AA^4$-$AA^5$-$AA^6$-$AA^7$-PEP5; wherein PEP5 is a peptide of formula PEP3-$AA^{11}$-$AA^{12}$; wherein PEP3 is selected from the group consisting of VPT, APT, VPQ, VSQ and TQV; wherein $AA^{11}$ is selected from the group consisting of E, K, Q, R, A, D, G and H, in particular E, K, Q, A and D; wherein $AA^{12}$ is selected from the group consisting of L, M, T, E, Q and H, in particular L; wherein $AA^1$, $AA^2$, $AA^3$, $AA^4$, and $AA^5$ are independently absent or $AA^I$ as defined herein; wherein $AA^6$ is absent or selected from the group consisting of S, T, C, E, Q,

P and R, preferably C, S, T or R; wherein $AA^7$ is selected from the group consisting of S, T, C, E, Q, P and R, preferably is selected from the group consisting of C, S and P. In one particular example, PEP9 is selected from the group consisting of KIPKAXXVPTEL, SIPKAXXVPTEL, HVTKPTXAPTKL, YVPKPXXAPTKL, TVPKPXXAPTQL, AVPKAXXAPTKL, KVGKAXXVPTKL, ASAAPXXVPQAL, ASASPXXVSQDL, ASASPXXVPQDL and RNVQXRPTQVQL.

In certain embodiments useful for inducing the conversion of a neoplastic cell into a cell (any cell) of a bone cell lineage, PEP12 is a peptide of general formula PEP1-$AA^{17}$-PEP11; wherein $AA^{17}$ is selected from the group consisting of G, A, V, L, I, P, F, M, W, T and S (in particular is selected from the group consisting of M, I, L, V and T); wherein PEP1 is SAIS.

In certain embodiments useful for inducing the conversion of a neoplastic cell into a cell (any cell) of a bone cell lineage, PEP11 is a peptide with 3 amino acids of general formula $AA^{18}$-$AA^{19}$-$AA^{20}$; wherein $AA^{18}$ is selected from the group consisting of L, V, Q, A and R, in particular is L; wherein $AA^{19}$ is selected from the group consisting of F, W, H and Y (in particular is an aromatic, polar amino acid such as Y); wherein $AA^{20}$ is selected from the group consisting of L, F, Y, K, I, V and M, in particular is selected from the group consisting of L, F, Y, and K. In one particular example, PEP11 is selected from the group consisting of LYL, LYF, LYY and LYK.

In certain embodiments useful for inducing the conversion of a neoplastic cell into a cell (any cell) of a bone cell lineage, the pair PEP1:PEP11 is SAIS:LYL.

The definitions of "PEP" pairs and triplets e.g. PEP3:PEP1, PEP5:PEP12, or PEP7:PEP5:PEP1, most particularly useful for inducing the conversion of a neoplastic cell into a cell (any cell) of a bone cell lineage, are as already defined herein to the extent that PEP1, PEP3, PEP5, PEP7, PEP9, PEP11 and PEP12 are particularly useful for these applications as defined in the present bone section.

In certain embodiments useful for inducing the conversion of a neoplastic cell into a cell (any cell) of a bone cell lineage, said GFR-binding compound is a synthetic molecule as defined herein in the definition section.

In certain embodiments useful for inducing the conversion of a neoplastic cell into a cell (any cell) of a bone cell lineage, said GFR-binding compound is a synthetic peptide or a peptidomimetic.

*Chondrocytic cell lineage*

Certain embodiments of the invention are particularly useful for inducing the conversion of a neoplastic cell into a cell (any cell) of a chondrocytic cell lineage.

In certain embodiments useful for inducing the conversion of a neoplastic cell into a cell (any cell) of a chondrocytic cell lineage, PEP1 is SAIS.

In certain embodiments useful for inducing the conversion of a neoplastic cell into a cell (any cell) of a chondrocytic cell lineage, PEP3 is selected from the group consisting of VPT, APT, VPQ and VSQ.

In certain embodiments useful for inducing the conversion of a neoplastic cell into a cell (any cell) of a chondrocytic cell lineage, PEP5 is a peptide of general formula PEP3-$AA^{11}$-$AA^{12}$; wherein PEP3 is selected from the group consisting of VPT, APT, VPQ and VSQ; wherein $AA^{11}$ is selected from the group consisting of E, K, Q, R, A, D, G and H, in particular E, K, Q, R, A and D; wherein $AA^{12}$ is selected from the group consisting of L, M, T, E, Q and H, in particular is L. In one particular example, PEP5 is selected from the group consisting of VPTEL, APTKL, APTQL, VPTRL, VPQAL, VSQDL and VPQDL.

In certain embodiments useful for inducing the conversion of a neoplastic cell into a cell (any cell) of a chondrocytic cell lineage, PEP7 is an amino acid or a peptide with between two and seven amino acids of general formula $AA^1$-$AA^2$-$AA^3$-$AA^4$-$AA^5$-$AA^6$-$AA^7$; wherein $AA^1$, $AA^2$, $AA^3$, $AA^4$, and $AA^5$ are independently absent or $AA^l$ as defined herein; wherein $AA^6$ is absent or selected from the group consisting of S, T, C, E, Q, P and R, preferably is S, C or T; wherein $AA^7$ is absent or is selected from the group consisting of S, T, C, E, Q, P and R, preferably is S or C; and wherein at least one of $AA^1$, $AA^2$, $AA^3$, $AA^4$, $AA^5$, $AA^6$ or $AA^7$ is not absent. In one particular example, PEP7 is selected from the group consisting of KIPKAXX, SIPKAXX, HVTKPTX, YVPKPXX, TVPKPXX, STPPTXX, ASAAPXX and ASASPXX.

In certain embodiments useful for inducing the conversion of a neoplastic cell into a cell (any cell) of a chondrocytic cell lineage, PEP9 is a peptide of general formula $AA^1$-$AA^2$-$AA^3$-$AA^4$-$AA^5$-$AA^6$-$AA^7$-PEP5; wherein PEP5 is a peptide of formula PEP3-$AA^{11}$-$AA^{12}$; wherein PEP3 is selected from the group consisting of VPT, APT, VPQ and VSQ; wherein $AA^{11}$ is selected from the group consisting of E, K, Q, R, A, D, G and H, in particular E, K, Q, R, A and D; wherein $AA^{12}$ is selected from the group consisting of L, M, T, E, Q and H, in particular is L; wherein $AA^1$, $AA^2$, $AA^3$, $AA^4$, and $AA^5$ are independently absent or $AA^l$ as defined herein; wherein $AA^6$ is absent or selected from the group consisting of S, T, C, E, Q, P and R, preferably is S, C or T; wherein $AA^7$ is selected from the group consisting of S, T, C, E, Q, P and R, preferably is S or C. In one particular example, PEP9 is selected from the group consisting of KIPKAXXVPTEL, SIPKAXXVPTEL, HVTKPTXAPTKL, YVPKPXXAPTKL, TVPKPXXAPTQL, STPPTXXVPTRL, ASAAPXXVPQAL, ASASPXXVSQDL and ASASPXXVPQDL.

In certain embodiments useful for inducing the conversion of a neoplastic cell into a cell (any cell) of a chondrocytic cell lineage, PEP12 is a peptide of general formula PEP1-$AA^{17}$-PEP11; wherein $AA^{17}$ is selected from the group consisting of G, A, V, L, I, P, F, M, W, T and S (in particular is selected from the group consisting of M, I, L, V and T);

wherein PEP1 is SAIS.

In certain embodiments useful for inducing the conversion of a neoplastic cell into a cell (any cell) of a chondrocytic cell lineage, PEP11 is a peptide with 3 amino acids of general formula $AA^{18}$-$AA^{19}$-$AA^{20}$; wherein $AA^{18}$ is selected from the group consisting of L, V, Q, A and R, in particular is L or V; wherein $AA^{19}$ is selected from the group consisting of F, W, H and Y, in particular is Y or F; wherein $AA^{20}$ is selected from the group consisting of L, F, Y and I. In one particular example, PEP11 is selected from the group consisting of LYL, LYF, LFI, VYY and LYY.

In certain embodiments useful for inducing the conversion of a neoplastic cell into a cell (any cell) of a chondrocytic cell lineage, the pair PEP1:PEP11 is SAIS:LYL.

The definitions of "PEP" pairs and triplets e.g. PEP3:PEP1, PEP5:PEP12, or PEP7:PEP5:PEP1, most particularly useful for inducing the conversion of a neoplastic cell into a cell (any cell) of a chondrocytic cell lineage, are as already defined herein to the extent that PEP1, PEP3, PEP5, PEP7, PEP9, PEP11 and PEP12 are particularly useful for these applications as defined in the present cartilage section.

In certain embodiments useful for inducing the conversion of a neoplastic cell into a cell (any cell) of a chondrocytic cell lineage, said GFR-binding compound is a synthetic molecule as defined herein in the definition section.

In certain embodiments useful for inducing the conversion of a neoplastic cell into a cell (any cell) of a chondrocytic cell lineage, said GFR-binding compound is a synthetic peptide or a peptidomimetic.

*Adipocyte cell lineage*

Certain embodiments of the invention are particularly useful for inducing the conversion of a neoplastic cell into a cell (any cell) of the adipocyte lineage.

In certain embodiments useful for inducing the conversion of a neoplastic cell into a cell (any cell) of the adipocyte lineage, PEP1 is SAIS.

In certain embodiments useful for inducing the conversion of a neoplastic cell into a cell (any cell) of the adipocyte lineage, PEP3 is selected from the group consisting of VPT, VPE, APT, TPT, VPA, APV, VPQ and VSQ.

In certain embodiments useful for inducing the conversion of a neoplastic cell into a cell (any cell) of the adipocyte lineage, PEP5 is a peptide of general formula PEP3-$AA^{11}$-$AA^{12}$; wherein PEP3 is selected from the group consisting of VPT, VPE, APT, TPT, VPA, APV, VPQ and VSQ; wherein $AA^{11}$ is selected from the group consisting of E, K, Q, R, A, D, G and H, in particular is selected from the group consisting of E, K, Q, R, A and D; wherein $AA^{12}$ is selected from the group consisting of L, M, T, E, Q and H, in particular selected from the group consisting of L, M and T. In one particular example, PEP5 is selected from the group consisting of VPTEL, VPEKM, APTKL, APTQL, VPTKL, TPTKM, VPARL, VPTRL, APVKT, VPQAL, VSQDL and VPQDL.

In certain embodiments useful for inducing the conversion of a neoplastic cell into a cell (any cell) of the adipocyte lineage, PEP7 is an amino acid or a peptide with between two and seven amino acids of general formula $AA^1$-$AA^2$-$AA^3$-$AA^4$-$AA^5$-$AA^6$-$AA^7$; wherein $AA^1$, $AA^2$, $AA^3$, $AA^4$, and $AA^5$ are independently absent or $AA^l$ as defined herein; wherein $AA^6$ is absent or selected from the group consisting of S, T, C, E, Q, P and R, preferably is selected from the group consisting of C, S and T; wherein $AA^7$ is absent or is selected from the group consisting of S, T, C, E, Q, P and R, preferably is C or S; and wherein at least one of $AA^1$, $AA^2$, $AA^3$, $AA^4$, $AA^5$, $AA^6$ or $AA^7$ is not absent. In one particular example, PEP7 is selected from the group consisting of KIPKAXX, GIPEPXX, SIPKAXX, HVTKPTX, YVPKPXX, TVPKPXX, AVPKAXX, KVGKAXX, KASKAXX, GSAGPXX, AAPASXX, STPPTXX, HVPKPXX, RVPSTXX, ASAAPXX and ASASPXX.

In certain embodiments useful for inducing the conversion of a neoplastic cell into a cell (any cell) of the adipocyte lineage, PEP9 is a peptide of general formula $AA^1$-$AA^2$-$AA^3$-$AA^4$-$AA^5$-$AA^6$-$AA^7$-PEP5; wherein PEP5 is a peptide of formula PEP3-$AA^{11}$-$AA^{12}$; wherein PEP3 is selected from the group consisting of VPT, VPE, APT, TPT, VPA, APV, VPQ and VSQ; wherein $AA^{11}$ is selected from the group consisting of E, K, Q, R, A, D, G and H, in particular E, K, Q, R, A and D; wherein $AA^{12}$ is selected from the group consisting of L, M, T, E, Q and H, in particular selected from the group consisting of L, M and T; wherein $AA^1$, $AA^2$, $AA^3$, $AA^4$, and $AA^5$ are independently absent or $AA^l$ as defined herein; wherein $AA^6$ is absent or selected from the group consisting of S, T, C, E, Q, P and R, preferably is selected from the group consisting of C, S and T; wherein $AA^7$ is selected from the group consisting of S, T, C, E, Q, P and R, preferably is C or S. In one particular example, PEP9 is selected from the group consisting of KIPKAXXVPTEL, GIPEPXXVPEKM, SIPKAXXVPTEL, HVTKPTXAPTKL, YVPKPXXAPTKL, TVPKPXXAPTQL, AVPKAXXAPTKL, KVGKAXXVPTKL, KASKAXXVPTKL, GSAGPXXTPTKM, AAPASXXVPARL, STPPTXXVPTRL, HVPKPXXAPTKL, RVPSTXXAPVKT, ASAAPXXVPQAL, ASASPXXVSQDL and ASASPXXVPQDL.

In certain embodiments useful for inducing the conversion of a neoplastic cell into a cell (any cell) of the adipocyte lineage, PEP12 is a peptide of general formula PEP1-$AA^{17}$-PEP11; wherein $AA^{17}$ is selected from the group consisting of G, A, V, L, I, P, F, M, W, T and S (in particular is selected from the group consisting of M, V and T); wherein PEP1 is SAIS.

In certain embodiments useful for inducing the conversion of a neoplastic cell into a cell (any cell) of the adipocyte lineage, PEP11 is a peptide with 3 amino acids of general formula $AA^{18}$-$AA^{19}$-$AA^{20}$; wherein $AA^{18}$ is selected from the

group consisting of L, V, Q, A and R, in particular is L; wherein $AA^{19}$ is selected from the group consisting of F, W, H and Y (in particular is a polar aromatic amino acid such as Y); wherein $AA^{20}$ is selected from the group consisting of L, F, Y, K, I, V and M, in particular is L or F. In one particular example, PEP11 is LYL or LYF.

In certain embodiments useful for inducing the conversion of a neoplastic cell into a cell (any cell) of the adipocyte lineage, the pair PEP1:PEP11 is SAIS:LYL.

The definitions of "PEP" pairs and triplets e.g. PEP3:PEP1, PEP5:PEP12, or PEP7:PEP5:PEP1, useful for inducing the conversion of a neoplastic cell into a cell (any cell) of the adipocyte lineage, are as already defined herein to the extent that PEP1, PEP3, PEP5, PEP7, PEP9, PEP11 and PEP12 are particularly useful for these applications as defined in the present adipose tissue section.

In certain embodiments useful for inducing the conversion of a neoplastic cell into a cell (any cell) of the adipocyte lineage, said GFR-binding compound is a synthetic molecule as defined herein in the definition section.

In certain embodiments useful for inducing the conversion of a neoplastic cell into a cell (any cell) of the adipocyte lineage, said GFR-binding compound is a synthetic peptide or a peptidomimetic.

## III. Bioactive carriers

The present invention achieves its intended therapeutic action(s) *i.e.* the treatment of a neoplastic disease via extracellular, non-mutagenic, recoding or conversion of neoplastic cells, by functional combination (or association) of at least two bioactive substances, namely, a GFR-binding compound (e.g. non-cyclic or cyclic) as described above and a bioactive carrier.

In one example, said GFR-binding compound and said bioactive carrier are thus operably associated, combined, linked or connected as defined herein and thus form a pharmaceutical association or combination for uses and methods of the present invention.

In one aspect, the present disclosure provides a bioactive carrier, as part of a pharmaceutical association as defined herein, as an active principle for use in methods and uses described herein.

Suitable bioactive carriers for implementing embodiments of the invention include any substance (i) which can interact and/or be compatible with a biological system and (ii) participate to the intended biological activity of a treatment as described in the present application.

As may be used herein, the term "bioactive carrier", "biocompatible carrier", "bioactive material", "biocompatible material", "bioactive substance", "bio-substance", "biocompatible substance", are used interchangeably.

A suitable bioactive carrier is compatible with living cells, tissues, organs or systems posing little to no risk of injury, toxicity or rejection by the immune system. Bioactive carriers suitable for implementing embodiments of the present invention include, but are not limited to, (a) a biopolymer such as (a1) collagen, (a2) fibrin; (b) a synthetic polymer such as (b1) ultra-high molecular weight polyethylene (UHMWPE), (b2) polyurethane (PE), (b3) polyurethane (PU), (b4) polytetrafuoroethylene (PTFE), (b5) polyacetal (PA), (b6) polymethylmethacrylate (PMMA), (b7) polyethylene terepthalate (PET), (b8) silicone rubber (SR), (b9) polyetheretherketone (PEEK), (b10) poly(lactic acid) (PLA), (b11) polysulfone (PS), (b12) PLLA, (b13) PLGA or (b14) PLDA; (c) metals and metal oxides such as (c1) gold and gold alloys, (c2) silver and silver alloys, (c3) platinum and platinum alloys, (c4) tantalum, (c5) Ti6Al4V, (c6) 316L stainless steel, (c7) Co-Cr Alloys, (c8) titanium alloys such as such as $\alpha$-type, $\beta$-type, $\alpha+\beta$-type Ti alloy, Ti-Nb alloys such as Ti29Nb13Ta4.6Zr or Ti35Nb4Sn); (d) metallic glasses; (e) amorphous alloys such as Zr-based alloys; (f) porous metals such as the ones reported in Ryan et al., 2006, Biomaterials, 27, 2651; Lopez-Heredia et al. 2008, Biomaterials, 29, 2608; Ryan et al., 2008, Biomaterials, 29, 3625; Li et al., 2007, Biomaterials, 28, 2810; or Hollander et al., 2006, Biomaterials, 27, 955; (g) gel or solid ceramics such as (g1) alumina, (g2) zirconia, (g3) carbon, (g4) titania, (g5) bioglass, or (g6) hydroxyapatite (HA); (h) composites such as (h1) silica/SR, (h2) CF/UHMWPE, (h3) CF/PTFE, (h4) HA/PE, (h5) CF/epoxy, (h6) CF/PEEK, (h7) CF/C or (h8) $Al_2O_3$/PTFE; (i) hydrogels such as (i1) polyisocyanopeptide hydrogels such as oligo(ethylene)glycol polyisocyanopeptides as described, for instance, in Van Buul, et al.; Chem. Sci. 4, 2357-2363 (2013), (i2) polysaccharides such as alginates, chitosans, chitins, guar gums, pectins, gellan gums, heparins, carrageenans, hyaluronans, starches, agars, xanthan gums, methylcellulose, carboxymethylcellulose, hydroxypropyl methyl cellulose, (i3) polyglycols such as polyethyleneglycol or polypropyleneglycol, (i4) polyvinylpyrrolidone, (i5) poly(vinylalcohol), (i6) polyacrylic acids, (i7) glycerophosphates, (i8) 2-acrylamido-2-methylpropanesulfonic acid, (i9) polyphosphazenes; (j) other suitable materials such as demineralized bone matrix; and any combinations thereof.

Suitable sources of bioactive carriers for implementing embodiments of the present invention include, but are not limited to, autographs, allographs, xenographs, plants, solutions, excipients, ceramics, metals, metal alloys, organic and inorganic polymers, bioglasses, carbon-containing structures, or combination thereof.

Particularly suitable as bioactive carriers for implementing embodiments of the present invention include bioactive carriers comprising at least one naturally occurring hydroxyl group on at least one surface thereof and bioactive carriers which do not naturally comprise at least one hydroxyl group on a surface thereof but which have been modified using conventional surface treatment techniques such that at least one hydroxyl group is present on a surface of the

bioactive carrier. In one example, said hydroxyl group is an available hydroxyl group *i.e.* it is not prevented from interacting and/or reacting with a compound of the present disclosure. Suitable as bioactive carriers naturally containing hydroxyl groups on a surface thereof for implementing embodiments of the invention specifically include metal oxides such as titanium oxides and non-metal oxides such ceramics. Also suitable as bioactive carriers for implementing embodiments of the invention include bioactive carriers comprising at least one naturally occurring carboxylate group (-COOH) or amine group (-$NH_2$) on at least one of a surface thereof and bioactive carriers which do not naturally comprise at least one carboxylate group (-COOH) or amine group (-$NH_2$) onto a surface thereof but which have been modified using conventional surface treatment techniques such that at least one carboxylate group (-COOH) or amine group (-$NH_2$) is present on a surface of the bioactive carrier.

In one example, said bioactive carrier includes a biomaterial. Suitable biomaterials for implementing certain embodiments of the present disclosure may be derived from nature or synthesized in the laboratory using a variety of chemical approaches utilizing metallic components, polymers, ceramics or composite materials. They are often used and/or adapted for a medical application, and thus comprise whole or part of a living structure or biomedical device. Suitable biomaterials for implementing certain embodiments of the present disclosure are commonly used in joint replacements, bone plates, bone cement, artificial ligaments and tendons, dental implants for tooth fixation, blood vessel prostheses, heart valves, skin repair devices (artificial tissue), cochlear replacements, contact lenses, breast implants, drug delivery mechanisms, sustainable materials, vascular grafts, stents, nerve conduits. Particularly suitable biomaterials for implementing certain embodiments of the present disclosure such as metals and alloys (pages 94-95),ceramics (pages 95-97), polymeric biomaterials (pages 97-98) and biocomposite materials (pages 98-99) are described in Nitesh et al., International Journal of Emerging Technology and Advanced Engineering, ISSN 2250-2459, Volume 2, Issue 4, 2012,

In one particular example, said bioactive carrier is a biomaterial.

In certain embodiments, particularly suitable bioactive carriers are selected from the group consisting of bioinert biomaterials, bioactive biomaterials and bioresorbable biomaterials.

The nature of the biomaterial is an important parameter. Particularly good results have been obtained using bioactive carriers composed mostly with the main material component of the tissue where the cells need to be recoded. For example, it was discovered that particularly good results may be obtained when a solid ceramic component (granulated ceramic powder or ceramic scaffolds) or a gel ceramic component is used in combination of a GFR-binding peptide of the present disclosure to recode osteosarcoma cells and thus protect from bone cancers. For example, it was also discovered that particularly good results may be obtained when collagen, in particular collagen types I, II, III and XI, is used in combination of a GFR-binding peptide of the present disclosure to recode chondrosarcomas and thus protect from cartilage cancers. For example, it was also discovered that particularly good results may be obtained when collagen, in particular collagen types I and III, or a biodegradable hydrogel is used in combination with a GFR-binding peptide of the present disclosure to recode disfunctioning muscle, skin, tendon and ligament cells. For example, it was also discovered that particularly good results may be obtained when a collagen or a biodegradable hydrogel is used in combination with a GFR-binding peptide of the present disclosure to recode cells and/or restore functions of vascular, neuron, eye retina, renal, wound healing, hair, fertility and reproduction, lung, and adipose tissues.

**Bioinert biomaterials:** As used herein, unless indicated otherwise or contradictory in context, the term "bioinert biomaterials" refers to any material that once placed in the human body has minimal interaction with its surrounding tissue. Examples of these are stainless steel, titanium, alumina, partially stabilised zirconia, and ultra-high molecular weight polyethylene. Generally a fibrous capsule might form around bioinert implants hence its biofunctionality relies on tissue integration through the implant.

**Bioactive biomaterial:** As used herein, unless indicated otherwise or contradictory in context, the term "bioactive biomaterial" refers to a material which, upon being placed within the human body, interacts with the surrounding bone and in some cases, even soft tissue. This occurs through a time-dependent kinetic modification of the surface, triggered by their implantation within the living bone. An ion-exchange reaction between the bioactive implant and surrounding body fluids, results in the formation of a biologically active carbonate apatite (CHAp) layer on the implant that is chemically and crystallographically equivalent to the mineral phase in bone. Examples of these materials are synthetic hydroxyapatite [$Ca_{10}(PO_4)_6(OH)_2$], glass ceramic A-W and bioglass®.

**Bioresorbable Biomaterials:** As used herein, unless indicated otherwise or contradictory in context, the term "bioresorbable biomaterials" refers to a material which, upon placement within the human body, starts to dissolve (resorbed) and slowly replaced by advancing tissue (such as bone). Examples of bioresorbable materials include, but are not limited to, tricalcium phosphate [$Ca_3(PO_4)_2$], polylactic-polyglycolic acid copolymers, calcium oxide, calcium carbonate and gypsum.

Therefore, no particular limitation should be ascribed to the substance, material or molecule suitable as being bioactive carriers for implementing embodiments of the present invention insofar as said substance, material or molecule is (a) biocompatible as defined herein and (b) combinable or associable with a GFR-binding compound as defined herein. In one preferred example, said bioactive carrier has a stiffness of at least 5 kPa, more preferably at least 35 kPa and preferably not more than 3 or 5 GPa as measured using conventional Dynamic Mechanical Analysis such as described in

details in Gong JP et al., Double-network hydrogels with extremely high mechanical strength, Adv Mater 2003, 15(14), 1155e8,

In one particular example, a biomaterial as defined herein for use in neuron-related applications has a stiffness comprised between about 0.01 kPa and about 3 kPa, preferably between about 0.01 kPa and about 1 kPa. In one particular example, a biomaterial as defined herein for use in muscle, cartilage and tendon/ligament -related applications has a stiffness comprised between about 3 kPa and about 200 kPa, preferably between about 10 kPa and about 30 kPa. In one particular example, a biomaterial as defined herein for use in bone-related applications has a stiffness comprised between about 30 kPa and about 2 GPa, preferably between about 70 kPa and about 200 kPa. In one particular example, a biomaterial as defined herein for use in hair-related applications has a stiffness comprised between about 0.01 kPa and about 200 kPa, preferably between about 3 kPa and about 70 kPa. In one particular example, a biomaterial as defined herein for use in endothelization-related applications has a stiffness comprised between about 0,01 kPa and about 500 kPa. In one particular example, a biomaterial as defined herein for use in angiogenesis-related applications has a stiffness comprised between about 0.5 kPa and about 100 kPa. In one particular example, a biomaterial as defined herein for use in wound healing and skin-related applications has a stiffness comprised between about 0.01 kPa and about 70 kPa.

**Available hydroxyl groups:** As used herein, unless indicated otherwise or contradictory in context, the term "free hydroxyl" or "available hydroxyl" means an hydroxyl group, which may be -OH or a radical ($-O^{\cdot}$) or an anion ($-O^{-}$) fully or partially ionised, which is able to / free to act as a nucleophile in a reaction with an electrophile such as compound (A) or compound (B) defined below.

**Available hydroxyl-containing surface:** As used herein, unless indicated otherwise or contradictory in context, the term "available hydroxyl-containing surface" or "free hydroxyl-containing surface" means a surface containing at least one free or available hydroxyl group as defined herein.

**Ceramics:** As used herein, unless indicated otherwise or contradictory in context, the term "ceramic" refers to an inorganic material with a high melting point, above 1000°C. Most typically, materials referred to as "ceramics" are obtained by a process in which raw material solid particles are heated in order to sinter them. Materials referred to as "ceramics" may broadly be split into two groups, these being "oxide ceramics" and "non-oxide ceramics". "Oxide ceramics" include, but are not limited to, alkaline earth oxides such as MgO and BaO, $Al_2O_3$ and aluminates, $TiO_2$ and titanates, $ZrO_2$ and zirconates, silicates such as clays and clay-derived materials. Since the term "ceramics" may encompass crystalline, partially amorphous and fully amorphous materials, the term "oxide ceramics" may also be interpreted as covering fully amorphous silicate glasses. "Non-oxide ceramics" include, but are not limited to, carbides and nitrides, and also borides and silicides, for example silicon carbide and silicon nitride, and also metal carbides and nitrides. In one particular example, solid ceramics e.g. in granulated powder or as a scaffold, is used as a bioactive carrier in the meaning of the present disclosure in bone-related applications. In one particular example, gel ceramics is used as a bioactive carrier in the meaning of the present disclosure in bone-related applications.

**Metal oxides:** As used herein, unless indicated otherwise or contradictory in context, the term "metal oxide" means a chemical compound that contains at least one oxygen atom and one other element in its chemical formula. Metal oxides typically contain an anion of oxygen in the oxidation state of -2. They can be obtained by hydrolysis or air/oxygen oxidation. Examples of such metal oxides are titanium oxides (e.g. TiO, $Ti_2O_3$, $TiO_2$), silicon oxide ($SiO_2$), aluminum oxide ($Al_2O_3$), iron (II, III) oxides such as $Fe_2O_3$, and zinc oxide (ZnO).

**Biopolymer:** As used herein, unless indicated otherwise or contradictory in context, the term "biopolymer" refers to a polymer produced by living organisms and includes, but is not limited to, polypeptides and proteins (such as collagen and fibrin), polysaccharides (such as cellulose, starch, chitin and chitosan), nucleic acids (such as DNA and RNA), and hydrides thereof.

**Hydrogel:** As used herein, unless indicated otherwise or contradictory in context, the term "hydrogel" refers to "Hydrogel" refers to a class of polymeric materials which are swollen in an aqueous medium, but which do not dissolve in water. Hydrogels are highly absorbent (they can contain over 99% water) natural or synthetic polymers. Hydrogels also possess a degree of flexibility very similar to natural tissue, due to their significant water content. U.S. Patent No. 6,475,516, for example, provides hydrogels being covalently bound to the surface of an in-dwelling medical device such as an implant, which may be functionalized with a GFR-binding compound of the present disclosure using, for instance, a process as described herein. In one particular example, biodegradable hydrogels are used as bioactive carriers in the meaning of the present disclosure.

**Collagen:** As used herein, unless indicated otherwise or contradictory in context, the term "collagen" refers to the main structural protein of the various connective tissues in animals which is mostly found in fibrous tissues such as tendons, ligaments and skin, and is also abundant in corneas, cartilage, bones, blood vessels, the gut, and intervertebral discs. Collagen is typically composed of a triple helix and generally contains high hydroxyproline content. The most common motifs in its amino acid sequence glycine-proline-X and glycine-X-hydroxyproline, where X is any amino acid other than glycine, proline or hydroxyproline. 28 types of collagen have been identified and described in the literature, which are all presently contemplated to be suitable for implementing embodiments of the invention. The five most common types are: Collagen I which may be found in skin, tendon, vascular ligature, organs, bone (main component of the organic

part of bone); Collagen II which may be found in cartilage (main component of cartilage); Collagen III which may be found in reticulate (main component of reticular fibers); Collagen IV which may be found in the basal lamina, the epithelium-secreted layer of the basement membrane; Collagen V which may be found on cell surfaces, hair and placenta. For example, in certain embodiments, suitable collagens for implementing embodiments of the present invention particularly include collagen type-I and type-IV. In one particular example, collagen, in particular collagen types I, II, III and XI, is used as a bioactive carrier in the meaning of the present disclosure in cartilage-related applications. In one particular example, collagen, in particular collagen types I and III, is used as a bioactive carrier in the meaning of the present disclosure in muscle-related applications, skin-related applications, and T/L-related applications. In one particular example, any type of collagen is used as a bioactive carrier in the meaning of the present disclosure in vascular, neuron, eye retina, renal, wound healing, hair, fertility and reproduction, lung, adipose -related applications.

In certain embodiments, said association, combination, linkage or connection between said GFR-binding compound and a bioactive carrier may occur via a bioactive carrier-affinity-containing group as defined herein.

## IV. Bioactive carrier-affinity-containing group

In certain embodiments, said GFR-binding compound as already defined herein is modified or functionalised with at least one bioactive carrier-affinity-containing group. Said at least one bioactive carrier-affinity-containing group provides said GFR-binding compound with an ability to, covalently or non-covalently, interact with, or be connected to, a bioactive carrier as defined herein (in particular, a biomaterial as defined herein).

In such embodiments where affinity is required via covalent interaction or binding, said bioactive carrier-affinity-containing group may be a thiol (SH)-containing group or a cysteine-containing group, in particular, a thiol (SH)-containing peptide or a cysteine-containing peptide. In such embodiments where affinity is required via covalent interaction or binding, said bioactive carrier-affinity-containing group may particularly be a cysteine.

In such embodiments where affinity is required via non-covalent interaction or binding, said bioactive carrier-affinity-containing group may comprise (or be) a peptide group such as any one of the peptide groups disclosed in US patent application No. 2008/0268015 A1, In particular, peptides containing amino acid sequences rich in large aromatic amino acid residues (aromatic amino acid-containing peptides or peptidomimetics) that include one or more of Phe, Trp, Tyr such as sequence number 1 to 45 described in US 2008/0268015 A1 are suitable as a biomaterial-affinity-containing fragment for implementing embodiments of the present invention. Said fragment may also be a peptide fragment such as any one of the peptide fragments disclosed in US patent No. 6,818,620 B2, In particular, peptides of sequence number 1 to 7 described in US 6,818,620 B2 are suitable as a biomaterial-affinity-containing fragment for implementing embodiments of the present invention. Thus, said biomaterial affinity-containing group may be a peptide with 3 to 25 amino acids comprising one or more of Phe, Trp or Tyr.

In one particular example, said bioactive carrier-affinity-containing group is a bioactive carrier high-affinity-containing group such as a biomaterial high-affinity-containing group.

In certain embodiments, said bioactive carrier-affinity-containing group has some affinity (preferably high affinity) with a given bioactive carrier (in particular, a biomaterial) such as collagen, apatite, titanium or any of those listed in *e.g.* US patent application No. 2008/0268015 A1. For instance, a group having some affinity with a biomaterial is any group capable to non-covalently interact/bind to a biomaterial with an affinity/specificity selected from at least 10%, at least 20%, at least 30%, at least 40%, at least 50%, at least 60%, at least 70%, at least 80%, at least 90%, at least 100%, at least 200%, at least 300%, at least 400%, at least 500%, or a higher percentage, with respect to an affinity where said group binds to an appropriate control such as, for example, a different material or surface, or a protein typically used for such comparisons such as bovine serum albumin. In one example, a biomaterial-affinity-containing group has a binding specificity that is characterized by a relative binding affinity as measured by an EC50 of 10 μM or less, and in certain emdiments, less than 1 μM. In certain embodiments, a relative affinity comprised between 1 pM and 100 μM, between 1 pM and 10 μM, or between 1 pM and 1 μM is particularly suitable. The EC50 is determined using any number of methods known in the art. In this case, the EC50 represents the concentration of fragment producing 50% of the maximal binding observed for that fragment in the assay.

In one particular example, said bioactive carrier-affinity-containing group is selected from the group consisting of GTPGP, which may preferably non-covalently interact with a bioactive carrier such as an apatite, and WWFWG, which may preferably non-covalently interact with a bioactive carrier such as a collagen.

In one particular example, said bioactive carrier-affinity-containing group is covalently or non-covalently (in particular, covalently) attached at an end (or extremity) of said GFR-binding compound.

## V. Modified non-cyclic GFR-binding compound

Thus, in one aspect, the present disclosure provides a pharmaceutical association or combination comprising a modified (non-cyclic) GFR-binding compound and a bioactive carrier, wherein said modified (non-cyclic) GFR-binding

compound comprises a (non-cyclic) GFR-binding compound as defined in the present disclosure and a bioactive carrier-affinity-containing group also as defined herein.

For example, in certain embodiments, said modified GFR-binding compound comprises a GFR-binding compound as defined in the present disclosure and a bioactive carrier-affinity-containing group; wherein said bioactive carrier-affinity-containing group is selected from the group consisting of a thiol-containing group (in particular, a thiol-containing peptide), a cysteine-containing group (in particular, a cysteine-containing peptide and more particularly, a cysteine), and an aromatic amino acid-containing peptide or peptidomimetic.

For example, in certain embodiments, said modified GFR-binding compound comprises a GFR-binding compound and a bioactive carrier-affinity-containing group; wherein said GFR-binding compound is a peptide or a peptidomimetic as defined herein, with between 8-25 amino acids or between 8-22 amino acids, more particularly between 18-22 amino acids, even more particularly between 19-21 or 20, comprising a peptide w SAIS; wherein said growth factor receptor-binding compound is a peptide or a peptidomimetic; wherein said growth factor receptor-binding compound is a non-cyclic peptide with between 8 and 25 amino acids or a non-cyclic peptidomimetic having a molecular weight comprised between 600 and 4,000 Daltons and comprises between 8 and 25 amino acids; wherein said growth factor receptor-binding compound is a cyclic peptide with between 10 and 60 amino acids or a cyclic peptidomimetic comprising between 10 and 60 amino acids having a molecular weight comprised between 1,000 and 7,000 Da; wherein said bioactive carrier-affinity-containing group is selected from the group consisting of a thiol-containing group (in particular, a thiol-containing peptide), a cysteine-containing group (in particular, a cysteine-containing peptide and more particularly, a cysteine), and an aromatic amino acid-containing peptide or peptidomimetic.

For example, in certain embodiments, said modified GFR-binding compound comprises a GFR-binding compound and a bioactive carrier-affinity-containing group; wherein said GFR-binding compound is a peptide or a peptidomimetic as defined herein, with between 8-25 amino acids or between 8-22 amino acids, more particularly between 18-22 amino acids, even more particularly between 19-21 or 20, comprising a peptide with height amino acids of general formula (PEP12): PEP1-$AA^{17}$-PEP11; wherein PEP1 is SAIS; wherein PEP11 is a peptide with 3 amino acids of formula $AA^{18}$-$AA^{19}$-$AA^{20}$; wherein $AA^{17}$ is selected from the group consisting of G, A, V, L, I, P, F, M, W, T and S (in particular is selected from the group consisting of M, I, L, V and T); wherein $AA^{18}$ is selected from the group consisting of L, V, Q, A and R; wherein $AA^{19}$ is selected from the group consisting of F, W, H and Y (in particular is an aromatic, polar amino acid such as Y); wherein $AA^{20}$ is selected from the group consisting of L, F, Y, K, I, V and M; wherein said bioactive carrier-affinity-containing group is selected from the group consisting of a thiol-containing group (in particular, a thiol-containing peptide), a cysteine-containing group (in particular, a cysteine-containing peptide and more particularly, a cysteine), and an aromatic amino acid-containing peptide or peptidomimetic.

For example, in certain embodiments, said modified GFR-binding compound comprises a GFR-binding compound and a bioactive carrier-affinity-containing group; wherein said GFR-binding compound is a peptide or a peptidomimetic as defined herein, with between 8-25 or between 8-22, more particularly between 18-22, even more particularly between 19-21 or 20 amino acids, having the following general formula (I) (hereinafter may also be referred to as compound (I) or peptide (I)):

PEP(C)-PEP12 (I)

wherein PEP12 is a peptide with 8 amino acids of formula PEP1-$AA^{17}$-PEP11 as defined herein; wherein one end of PEP(C) interacts covalently with PEP12 via one end of PEP1; wherein PEP(C) is a peptide with at least 5 amino acids, in particular a peptide with between 5 and 12 amino acids; wherein said bioactive carrier-affinity-containing group is selected from the group consisting of a thiol-containing group (in particular, a thiol-containing peptide), a cysteine-containing group (in particular, a cysteine-containing peptide and more particularly, a cysteine), and an aromatic amino acid-containing peptide or peptidomimetic.

For example, in certain embodiments, said modified GFR-binding compound comprises a GFR-binding compound and a bioactive carrier-affinity-containing group; wherein said GFR-binding compound is a peptide or a peptidomimetic as defined herein, with between 8-25 or between 8-22, more particularly between 18-22, even more particularly between 19-21 or 20 amino acids, having the following general formula (II) (hereinafter may also be referred to as compound (II) or peptide (II)):

PEP7-PEP5-PEP12 (II)

wherein PEP12 is a peptide with 8 amino acids of formula PEP1-$AA^{17}$-PEP11 as defined herein; wherein PEP5 is a peptide with five amino acids as defined herein; wherein PEP7 is an amino acid or a peptide with between two and seven amino acids as defined herein; wherein one end of PEP5 interacts covalently with one end of PEP12 via one end of PEP1; wherein another end of PEP5 interacts covalently with one end of PEP7 via $AA^7$; wherein said bioactive carrier-affinity-containing group is selected from the group consisting of a thiol-containing group (in particular, a thiol-containing peptide),

a cysteine-containing group (in particular, a cysteine-containing peptide and more particularly, a cysteine), and an aromatic amino acid-containing peptide or peptidomimetic.

For example, in certain embodiments, said modified GFR-binding compound comprises a GFR-binding compound and a bioactive carrier-affinity-containing group; wherein said GFR-binding compound is a peptide or a peptidomimetic as defined herein, with between 8-25 or between 8-22, more particularly between 18-22, even more particularly between 19-21 or 20 amino acids, having the following general formula (III) (hereinafter may also be referred to as compound (III) or peptide (III)):

$$AA^1\text{-}AA^2\text{-}AA^3\text{-}AA^4\text{-}AA^5\text{-}AA^6\text{-}AA^7\text{-}AA^8\text{-}AA^9\text{-}AA^{1°}\text{-}AA^{11}\text{-}AA^{12}\text{-}AA^{13}\text{-}AA^{14}\text{-}AA^{15}\text{-}AA^{16}\text{-}AA^{17}\text{-}AA^{18}\text{-}AA^{19}\text{-}AA^{20} \quad \text{(III)}$$

wherein $AA^1$-$AA^2$-$AA^3$-$AA^4$-$AA^5$-$AA^6$-$AA^7$ is PEP7 as defined herein; wherein $AA^{13}$-$AA^{14}$-$AA^{15}$-$AA^{16}$-$AA^{17}$-$AA^{18}$-$AA^{19}$-$AA^{20}$ is PEP12 as defined herein; wherein $AA^8$-$AA^9$-$AA^{10}$ is PEP3 as defined herein; wherein $AA^{11}$ and $AA^{12}$ are as defined herein; wherein $AA^1$ may be an N-terminal amino acid or a C-terminal amino acid; wherein $AA^{20}$ may be an N-terminal amino acid or a C-terminal amino acid; wherein said bioactive carrier-affinity-containing group is selected from the group consisting of a thiol-containing group (in particular, a thiol-containing peptide), a cysteine-containing group (in particular, a cysteine-containing peptide and more particularly, a cysteine), and an aromatic amino acid-containing peptide or peptidomimetic.

In certain embodiments, said modified GFR-binding compound comprises a GFR-binding compound and a bioactive carrier-affinity-containing group; wherein the RMSD value of the three dimensional (3D) atomic coordinates of said GFR-binding compound with respect to PEPREF is 2.45Å (Angstroms) or less, in particular is 2Å or less, and more particularly is 1.79Å or less, and wherein PEPREF is the set of 3D atomic coordinates already defined herein.

## VI. Modified cyclic GFR-binding compound

Thus, in one aspect, the present disclosure provides a pharmaceutical association or combination comprising a modified cyclic GFR-binding compound and a bioactive carrier, wherein said modified cyclic GFR-binding compound comprises a cyclic GFR-binding compound as defined in the present disclosure and a bioactive carrier-affinity-containing group also as defined herein.

For example, in certain embodiments, said modified cyclic GFR-binding compound comprises a cyclic GFR-binding compound as defined in the present disclosure and a bioactive carrier-affinity-containing group; wherein said bioactive carrier-affinity-containing group is selected from the group consisting of a thiol-containing group (in particular, a thiol-containing peptide), a cysteine-containing group (in particular, a cysteine-containing peptide and more particularly, a cysteine), and an aromatic amino acid-containing peptide or peptidomimetic.

For example, in certain embodiments, the present disclosure provides a modified cyclic GFR-binding compound comprising a cyclic GFR-binding compound and a bioactive carrier-affinity-containing group; wherein said cyclic GFR-binding compound is a cyclic peptide, a variant or analog thereof, or a cyclic peptidomimetic as defined herein, with (comprising, or exclusively consisting of, or constituted of) between 10-60 (in particular between 15-60, more particularly between 10-55, and even more particularly between 15-55) amino acids or with between 10-35 (in particular between 15-35, more particularly between 10-30, and even more particularly between 15-30) amino acids; comprising a peptide SAIS; wherein said bioactive carrier-affinity-containing group is selected from the group consisting of a thiol-containing group (in particular, a thiol-containing peptide), a cysteine-containing group (in particular, a cysteine-containing peptide and more particularly, a cysteine), and an aromatic amino acid-containing peptide or peptidomimetic.

For example, in certain embodiments, the present disclosure provides a modified cyclic GFR-binding compound comprising a cyclic GFR-binding compound and a bioactive carrier-affinity-containing group; wherein said cyclic GFR-binding compound is a cyclic peptide or a cyclic peptidomimetic as defined herein, with between 10-60 (in particular between 15-60, more particularly between 10-55, and even more particularly between 15-55) amino acids or with between 10-35 (in particular between 15-35, more particularly between 10-30, and even more particularly between 15-30) amino acids; comprising a peptide with height amino acids of general formula (PEP12): PEP1-$AA^{17}$-PEP11; wherein PEP1 is SAIS; wherein PEP11 is a peptide with 3 amino acids of formula $AA^{18}$-$AA^{19}$-$AA^{20}$; wherein $AA^{17}$ is selected from the group consisting of G, A, V, L, I, P, F, M, W, T and S (in particular is selected from the group consisting of M, I, L, V and T); wherein $AA^{18}$ is selected from the group consisting of L, V, Q, A and R; wherein $AA^{19}$ is selected from the group consisting of F, W, H and Y (in particular is an aromatic, polar amino acid such as Y); wherein $AA^{20}$ is selected from the group consisting of L, F, Y, K, I, V and M; wherein said bioactive carrier-affinity-containing group is selected from the group consisting of a thiol-containing group (in particular, a thiol-containing peptide), a cysteine-containing group (in particular, a cysteine-containing peptide and more particularly, a cysteine), and an aromatic amino acid-containing peptide or peptidomimetic.

For example, in certain embodiments, the present disclosure provides a modified cyclic GFR-binding compound comprising a cyclic GFR-binding compound and a bioactive carrier-affinity-containing group, wherein said cyclic GFR-binding compound is a cyclic peptide or a cyclic peptidomimetic as defined herein, with between 10-60 (in particular between 15-60, more particularly between 10-55, and even more particularly between 15-55) amino acids or with between 10-35 (in particular between 15-35, more particularly between 10-30, and even more particularly between 15-30) amino acids, comprising a peptide or a peptidomimetic having the following general formula (IIIa):

PEP(A)-LINKER (IIIa)

wherein one end of LINKER interacts covalently with one end of PEP(A); wherein PEP(A) comprises PEP1 or PEP12; wherein LINKER is a linear or branched organic divalent radical, moiety or compound having a molecular weight (Mw) comprised between 450 and 4,500 Daltons, in particular comprised between about 600 and about 4,500 Da, more particularly between about 600 and about 4,000 Da, and even more particularly between about 600 and about 3,500 Da; wherein said bioactive carrier-affinity-containing group is selected from the group consisting of a thiol-containing group (in particular, a thiol-containing peptide), a cysteine-containing group (in particular, a cysteine-containing peptide and more particularly, a cysteine), and an aromatic amino acid-containing peptide or peptidomimetic.

For example, in certain embodiments, the present disclosure provides a modified cyclic GFR-binding compound comprising a cyclic GFR-binding compound and a bioactive carrier-affinity-containing group, wherein said cyclic GFR-binding compound is a cyclic peptide or a cyclic peptidomimetic as defined herein, with between 10-60 (in particular between 15-60, more particularly between 10-55, and even more particularly between 15-55) amino acids or with between 10-35 (in particular between 15-35, more particularly between 10-30, and even more particularly between 15-30) amino acids, comprising a peptide or a peptidomimetic having the following general formula (IIIb):

LINKER-PEP(A)-LINKER (IIIb)

wherein one end of a first LINKER interacts covalently with one end of PEP(A); wherein one end of a second LINKER interacts covalently with another end of PEP(A); wherein another end of a first LINKER interacts covalently with another end of a second LINKER; wherein PEP(A) comprises PEP1 or PEP12; wherein LINKER are independently a linear or branched organic divalent radical, moiety or compound having a molecular weight (Mw) comprised between 450 and 4,500 Daltons, in particular comprised between about 600 and about 4,500 Da, more particularly between about 600 and about 4,000 Da, and even more particularly between about 600 and about 3,500 Da; and wherein said bioactive carrier-affinity-containing group is selected from the group consisting of a thiol-containing group (in particular, a thiol-containing peptide), a cysteine-containing group (in particular, a cysteine-containing peptide and more particularly, a cysteine), and an aromatic amino acid-containing peptide or peptidomimetic.

For example, in certain embodiments, the present disclosure provides a modified cyclic GFR-binding compound comprising a cyclic GFR-binding compound and a bioactive carrier-affinity-containing group; wherein said cyclic GFR-binding compound is a cyclic peptide or a cyclic peptidomimetic as defined herein, with between 10-35 (in particular between 15-35, more particularly between 10-30, and even more particularly between 15-30) amino acids, comprising a peptide or a peptidomimetic having the following general formula (IVa):

PEP(C)-PEP12-LINKER (IVa)

wherein LINKER is a linear or branched organic divalent radical, moiety or compound having a molecular weight (Mw) comprised between 450 and 4,500 Daltons, in particular comprised between about 600 and about 4,500 Da, more particularly between about 600 and about 4,000 Da, and even more particularly between about 600 and about 3,500 Da; wherein PEP12 is a peptide with 8 amino acids of formula PEP1-$AA^{17}$-PEP11 as defined herein; wherein PEP2 is a peptide with five amino acids as already defined herein; wherein one end of PEP(C) interacts covalently with PEP12 via one end of PEP1; wherein one end of LINKER interacts covalently with one end of PEP12 via one end of PEP11; wherein PEP(C) is a peptide with at least 5 amino acids, in particular a peptide with between 5 and 12 amino acids; and wherein said bioactive carrier-affinity-containing group is selected from the group consisting of a thiol-containing group (in particular, a thiol-containing peptide), a cysteine-containing group (in particular, a cysteine-containing peptide and more particularly, a cysteine), and an aromatic amino acid-containing peptide or peptidomimetic.

For example, in certain embodiments, the present disclosure provides a modified cyclic GFR-binding compound comprising a cyclic GFR-binding compound and a bioactive carrier-affinity-containing group; wherein said cyclic GFR-binding compound is a cyclic peptide or a cyclic peptidomimetic as defined herein, with between 10-60 (in particular between 15-60, more particularly between 10-55, and even more particularly between 15-55) amino acids or with between 10-35 (in particular between 15-35, more particularly between 10-30, and even more particularly between 15-30) amino acids, comprising a peptide or a peptidomimetic having the following general formula (IVb):

LINKER-PEP(C)-PEP12-LINKER (IVb)

wherein LINKER are independently a linear or branched organic divalent radical, moiety or compound having a molecular weight (Mw) comprised between 450 and 4,500 Daltons, in particular comprised between about 600 and about 4,500 Da, more particularly between about 600 and about 4,000 Da, and even more particularly between about 600 and about 3,500 Da; wherein PEP12 is a peptide with 8 amino acids of formula PEP1-$AA^{17}$-PEP11 as defined herein; wherein PEP2 is a peptide with five amino acids as already defined herein; wherein one end of PEP(C) interacts covalently with PEP12 via one end of PEP1; wherein one end of a first LINKER interacts covalently with one end of PEP12 via one end of PEP11; wherein one end of a second LINKER interacts covalently with another end of PEP(C); wherein another end of a first LINKER interacts covalently with another end of a second LINKER; wherein PEP(C) is a peptide with at least 5 amino acids, in particular a peptide with between 5 and 12 amino acids; and wherein said bioactive carrier-affinity-containing group is selected from the group consisting of a thiol-containing group (in particular, a thiol-containing peptide), a cysteine-containing group (in particular, a cysteine-containing peptide and more particularly, a cysteine), and an aromatic amino acid-containing peptide or peptidomimetic.

For example, in certain embodiments, the present disclosure provides a modified cyclic GFR-binding compound comprising a cyclic GFR-binding compound and a bioactive carrier-affinity-containing group, wherein said cyclic GFR-binding compound is a cyclic peptide or a cyclic peptidomimetic as defined herein, with between 10-35 (in particular between 15-35, more particularly between 10-30, and even more particularly between 15-30) amino acids, comprising a peptide or a peptidomimetic having the following general formula (Va):

PEP7-PEP5-PEP12-LINKER (Va)

wherein LINKER is a linear or branched organic divalent radical, moiety or compound having a molecular weight (Mw) comprised between 450 and 4,500 Daltons, in particular comprised between about 600 and about 4,500 Da, more particularly between about 600 and about 4,000 Da, and even more particularly between about 600 and about 3,500 Da; wherein PEP12 is a peptide with 8 amino acids of formula PEP1-$AA^{17}$-PEP11 as defined herein; wherein PEP5 is a peptide with five amino acids as already defined herein; wherein PEP7 an amino acid or a peptide with between two and seven amino acids as already defined herein; wherein one end of LINKER interacts covalently with one end of PEP12 via $AA^{20}$; wherein one end of PEP5 interacts covalently with another end of PEP12 via $AA^{12}$; wherein another end of PEP5 interacts covalently with one end of PEP7 via $AA^{8}$; and wherein said bioactive carrier-affinity-containing group is selected from the group consisting of a thiol-containing group (in particular, a thiol-containing peptide), a cysteine-containing group (in particular, a cysteine-containing peptide and more particularly, a cysteine), and an aromatic amino acid-containing peptide or peptidomimetic.

For example, in certain embodiments, the present disclosure provides a modified cyclic GFR-binding compound comprising a cyclic GFR-binding compound and a bioactive carrier-affinity-containing group, wherein said cyclic GFR-binding compound is a cyclic peptide or a cyclic peptidomimetic as defined herein, with between 10-60 (in particular between 15-60, more particularly between 10-55, and even more particularly between 15-55) amino acids or with between 10-35 (in particular between 15-35, more particularly between 10-30, and even more particularly between 15-30) amino acids, comprising a peptide or a peptidomimetic having the following general formula (Vb):

LINKER-PEP7-PEP5-PEP12-LINKER (Vb)

wherein LINKER are independently a linear or branched organic divalent radical, moiety or compound having a molecular weight (Mw) comprised between 450 and 4,500 Daltons, in particular comprised between about 600 and about 4,500 Da, more particularly between about 600 and about 4,000 Da, and even more particularly between about 600 and about 3,500 Da; wherein PEP12 is a peptide with 8 amino acids of formula PEP1-$AA^{17}$-PEP11 as defined herein; wherein PEP5 is a peptide with five amino acids as already defined herein; wherein PEP7 an amino acid or a peptide with between two and seven amino acids as already defined herein; wherein one end of PEP5 interacts covalently with another end of PEP12 via $AA^{12}$; wherein another end of PEP5 interacts covalently with one end of PEP7 via $AA^{8}$; wherein one end of a first LINKER interacts covalently with one end of PEP12 via $AA^{20}$; wherein one end of a second LINKER interacts covalently with another end of PEP7; wherein another end of a first LINKER interacts covalently with another end of a second LINKER; and wherein said bioactive carrier-affinity-containing group is selected from the group consisting of a thiol-containing group (in particular, a thiol-containing peptide), a cysteine-containing group (in particular, a cysteine-containing peptide and more particularly, a cysteine), and an aromatic amino acid-containing peptide or peptidomimetic.

For example, in certain embodiments, the present disclosure provides a modified cyclic GFR-binding compound comprising a cyclic GFR-binding compound and a bioactive carrier-affinity-containing group, wherein said cyclic GFR-binding compound is a cyclic peptide or a cyclic peptidomimetic as defined herein, with between 10-35 (in particular between 15-35, more particularly between 10-30, and even more particularly between 15-30) amino acids, comprising a peptide or a peptidomimetic having the following general formula (Via):

$AA^1$-$AA^2$-$AA^3$-$AA^4$-$AA^5$-$AA^6$-$AA^7$-$AA^8$-$AA^9$-$AA^{1°}$-
$AA^{11}$-$AA^{12}$-$AA^{13}$-$AA^{14}$-$AA^{15}$-$AA^{16}$-$AA^{17}$-$AA^{18}$-$AA^{19}$-$AA^{20}$-LINKER (VIa)

wherein LINKER is a linear or branched organic divalent radical, moiety or compound having a molecular weight (Mw) comprised between 450 and 4,500 Daltons, in particular comprised between about 600 and about 4,500 Da, more particularly between about 600 and about 4,000 Da, and even more particularly between about 600 and about 3,500 Da; wherein $AA^1$-$AA^2$-$AA^3$-$AA^4$-$AA^5$-$AA^6$-$AA^7$ is PEP7 as defined herein; wherein $AA^{13}$-$AA^{14}$-$AA^{15}$-$AA^{16}$-$AA^{17}$-$AA^{18}$-$AA^{19}$-$AA^{20}$ is PEP12 as defined herein; wherein $AA^8$-$AA^9$-$AA^{10}$ is PEP3 as defined herein; wherein $AA^{11}$ and $AA^{12}$ are as defined herein; wherein one end of LINKER interacts covalently with $AA^{20}$; wherein $AA^1$ may be an N-terminal amino acid or a C-terminal amino acid; wherein $AA^{20}$ may be an N-terminal amino acid or a C-terminal amino acid; and wherein said bioactive carrier-affinity-containing group is selected from the group consisting of a thiol-containing group (in particular, a thiol-containing peptide), a cysteine-containing group (in particular, a cysteine-containing peptide and more particularly, a cysteine), and an aromatic amino acid-containing peptide or peptidomimetic.

For example, in certain embodiments, the present disclosure provides a modified cyclic GFR-binding compound comprising a cyclic GFR-binding compound and a bioactive carrier-affinity-containing group, wherein said cyclic GFR-binding compound is a cyclic peptide or a cyclic peptidomimetic as defined herein, with between 10-60 (in particular between 15-60, more particularly between 10-55, and even more particularly between 15-55) amino acids or with between 10-35 (in particular between 15-35, more particularly between 10-30, and even more particularly between 15-30) amino acids, comprising a peptide or a peptidomimetic having the following general formula (VIb) (hereinafter may also be referred to as compound (VIb) or peptide (VIb)):

LINKER-
$AA^1$-$AA^2$-$AA^3$-$AA^4$-$AA^5$-$AA^6$-$AA^7$-$AA^8$-$AA^9$-$AA^{10}$-$AA^{11}$-$AA^{12}$-$AA^{13}$-$AA^{14}$-$AA^{15}$-$AA^{16}$-$AA^{17}$-$AA^{18}$-$AA^{19}$-$AA^{20}$-LINKER
(VIb)

wherein LINKER are independently a linear or branched organic divalent radical, moiety or compound having a molecular weight (Mw) comprised between 450 and 4,500 Daltons, in particular comprised between about 600 and about 4,500 Da, more particularly between about 600 and about 4,000 Da, and even more particularly between about 600 and about 3,500 Da; wherein $AA^1$-$AA^2$-$AA^3$-$AA^4$-$AA^5$-$AA^6$-$AA^7$ is PEP7 as defined herein; wherein $AA^{13}$-$AA^{14}$-$AA^{15}$-$AA^{16}$-$AA^{17}$-$AA^{18}$-$AA^{19}$-$AA^{20}$ is PEP12 as defined herein; wherein $AA^3$-$AA^9$-$AA^{10}$ is PEP3 as defined herein; wherein $AA^{11}$ and $AA^{12}$ are as defined herein; wherein one end of a first LINKER interacts covalently with $AA^{20}$; wherein one end of a second LINKER interacts covalently with $AA^1$; wherein another end of a first LINKER interacts covalently with another end of a second LINKER; wherein one end of the first LINKER may be an N-terminal amino acid or a C-terminal amino acid; and wherein said bioactive carrier-affinity-containing group is selected from the group consisting of a thiol-containing group (in particular, a thiol-containing peptide), a cysteine-containing group (in particular, a cysteine-containing peptide and more particularly, a cysteine), and an aromatic amino acid-containing peptide or peptidomimetic.

In one particular example, said bioactive carrier-affinity-containing group is comprised within said cyclic GFR-binding compound e.g. is comprised in at least one LINKER, or is at least one LINKER. For example, in certain embodiments, said modified cyclic GFR-binding compound may have any one of the following general schematic formulae:

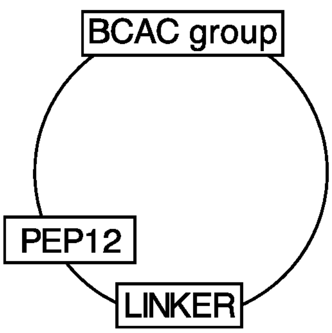


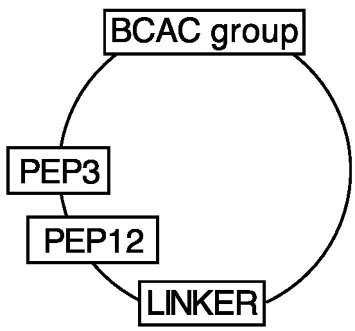


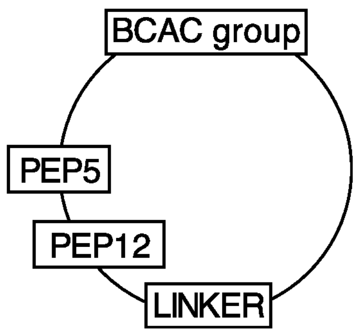


(XXV) (XXVI) (XXVII)

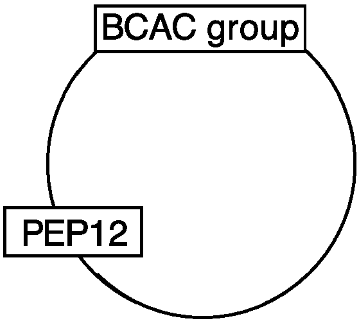


(XXVIII)

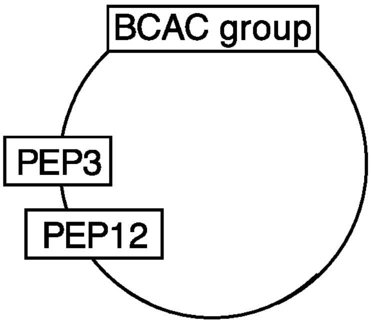


(XXIX)

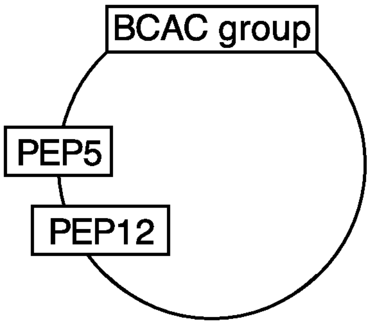


(XXX)

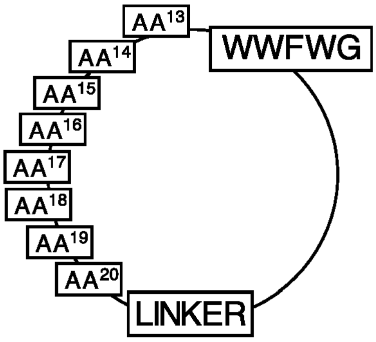


(XXXI)

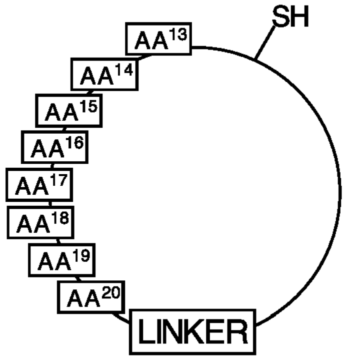


(XXXII)

wherein curved lines represents covalent bonds between LINKER, PEP3, PEP5, PEP12 and AAs "boxes". Curved lines' lengths may not be representative of the actual relative distance between the LINKER, PEP3, PEP5, PEP12 and AAs.

**VII. Pharmaceutical associations or combinations**

In one aspect, the present disclosure provides a pharmaceutical association or combination, which may be used for converting or recoding, in-vitro, ex-vivo or in-vivo, a neoplastic cell into a non-neoplastic cell, comprising at least one (modified) GFR-binding compound and a bioactive carrier, both as defined in the present disclosure. In one example, said (modified) GFR-binding compound and bioactive carrier are both active principles/ingredients. In one example, said GFR-binding compound and bioactive carrier are functionally associated/combined as defined herein. In certain embodiments, said pharmaceutical association or combination is a modified, functionalised, coated or grafted biomaterial as defined herein.

The present pharmaceutical associations or combinations may thus also be used for protecting a subject carrying a neoplastic cell from a neoplastic disease.

In one aspect, the present disclosure provides a pharmaceutical association or combination for the uses disclosed herein, substantially free from any cell adhesion promoter. As used herein, the term "substantially free", as applied to a given component such as a cell adhesion promoter, means that the amount of such a component is less than 20%, 15%, 10%, 5%, 1%, 0.5%, 0.1% or less in mole with respect to the mole content of (modified) GFR-binding compound unless otherwise indicated, self-evident or contradictory in context.

IIn one particular example, the pharmaceutical association as defined herein further comprising (another) anti-cancer agent thus forming a pharmaceutical composition of the invention. In one example, said pharmaceutical composition further comprises at least one pharmaceutically acceptable excipient. In one example, said further anti-cancer agent is functionally associated with said GFR-binding compound and/or said bioactive carrier. Suitable further anti-cancer agents include but are not limited to, agents that inhibit the synthesis of DNA molecule building blocks, agents that directly damage DNA in the cell nucleus, agents that affect the synthesis or breakdown of mitotic spindles, and agents that inhibit kinase proteins by interacting with the kinase active site. Preferred examples of agents that inhibit the synthesis of DNA molecule building blocks include, but are not limited to, methotrexate (Abitrexate®), fluorouracil (Adrucil®), gemcitabine (Gemzar®), arabinosylcytosine (araC), hydroxyurea (Hydrea®), and mercaptopurine (Purinethol®). Preferred examples of agents that directly damage DNA in the cell nucleus include, but are not limited to, carboplatin (Paraplatin® and paraplatin-AQ®), cisplatin (Platinol®) and antibiotics such as daunorubicin (Cerubidine®), doxorubicin (Adriamycin®), and etoposide (VePesid®). Preferred examples of agents that affect the synthesis or breakdown of mitotic spindles include, but are not limited to, miotic disrupters such as Vinblastine (Velban®), Vincristine (Oncovin®) and Pacitaxel (Taxol®). Preferred examples of agents that inhibit kinase proteins include, but are not limited to, Afatinib®, Axitinib®, Bosulif®, Bosutinib®, Cabozantinib®, Caprelsa®, Cometriq®, Crizotinib®, Dasatinib®, Erlotinib®, Gilotrif®, Gleevec®, Ibrutinib®, Iclusig®, Imatinib®, Imbruvica®, Inlyta®, Lapatinib®, Nexavar®, Nilotinib®, Pazopanib®, Ponatinib®, Regorafenib®, Sorafenib®, Sprycel®, Stivarga®, Sunitinib®, Sutent®, Tarceva®, Tasigna®, Tivopath®, Tivozanib®, Tykerb®, Vandetanib®, Votrient®, Xalkori®, Zaltrap®, and ziv-aflibercept®.

In one example, the L-asparaginase enzyme may also be used as a further agent in combination with the pharmaceutical association or composition as defined herein. L-asparaginase enzyme has been reported *e.g.* in L-Asparaginase: A Promising Enzyme for Treatment of Acute Lymphoblastic Leukiemia, People's Journal of Scientific Research, Vol. 5(1), Jan. 2012, to act by depriving cancer cells (such as leukemia cells) of asparagine thus inducing their death.

Other anti-cancer agents may also be used such as nitrogen mustards, ethylenimes, alkylsulfonates, triazenes, piperazines, nitrosureas and antibiotics such as anthracyclines, dactinomycin, bleomycin, adriamycin, or mithramycin.

IIn one particular example, the pharmaceutical association as defined herein further comprising an adhesion protein inhibitor thus forming a pharmaceutical composition of the invention. In one example, said pharmaceutical composition further comprises at least one pharmaceutically acceptable excipient. In one example, said adhesion protein inhibitor is functionally associated with said GFR-binding compound and/or said bioactive carrier. Suitable adhesion protein inhibitors include, but are not limited to, siRNA, mRNA or microRNAs which inhibits or down-regulates the gene or protein expression of at least one integrin, syndecan, selectin or dystroglycan, anti-integrin antibodies, anti-syndecan antibodies, anti-selectin antibodies, anti-dystroglycan antibodies, foldamers, or dendrimers.

In one example, said pharmaceutical association or combination comprises one (modified) GFR-binding compound. In one example, said pharmaceutical association or combination comprises two or more distinct (modified) GFR-binding compounds. In one example, said pharmaceutical association or combination comprises three or more distinct (modified) GFR-binding compounds. In one example, said pharmaceutical association or combination comprises four or more distinct (modified) GFR-binding compounds.

Said pharmaceutical association or composition substantially down-regulates, reduces, inhibits or suppresses the gene and/or protein expression of at least one of cyclin-D1, cyclin-D2 or cyclin-D3. Because cyclins D1, D2 and D3 regulate the activity of Cyclin-dependent kinases (CDKs) 4 and 6 by forming cyclin-CDK protein complexes including Cyclin D1-CDK4 complex, Cyclin D1-CDK6 complex, Cyclin D2-CDK4 complex, Cyclin D2-CDK6 complex, Cyclin D3-CDK4 complex and Cyclin D3-CDK6 complex, said pharmaceutical association or composition was also observed to substantially reduce, inhibit, suppress or destabilise the formation of any one of such complexes.

There are many ways to test, measure and represent the inhibitory effect of a given substance on the gene or protein expression of cyclins D, but for the purpose of the present disclosure, and for the avoidance of any doubts, the inhibition values of a given pharmaceutical association or composition as defined herein is a measure of the gene expression of cyclins D as provided by RT-PCR. It is to be understood that the values of the gene expression of cyclins D disclosed herein correspond to the total gene expression of all cyclins D present in the tested cell *i.e.* D1, D2 and D3 as known at the date of the present disclosure.

As already stated above, pharmaceutical associations or compositions suitable for implementing embodiments of the present invention reduce the gene expression of cyclins D by at least 20% during at least one part of the G1 phase of the cell cycle as compared to the wild-type expression. The absolute and relative duration of each cell cycle phase (in other words, the cell cycle duration profile) usually varies (some phases such as the Gap phases may "shrink") amongst healthy cells of different types *(e.g.* bone cells, skin cells, etc...) and between healthy cells and neoplastic cells of the same type *(e.g.* healthy bone cells and neoplastic bone cells).Typical average cell cycle duration is commonly accepted to be about 24 hours. Typically accepted phase durations for a healthy cell are 11 to 14 hours for the G1 phase, 5 to 12 hours for the S phase, 3 to 12 hours for the G2 phase and about 1 hour for the M phase. In neoplastic cells, such as in cancer cells, it is commonly admitted that, although the duration of the S and M phases may generally be conserved, the length of the G1 and G2 phases is generally shortened in order to increase cell division. Cell cycle phase's duration may thus vary significantly from one cell type to another. Consequently, conventionally and for the purpose of facilitating the comparative representation of the inhibition of the gene expression of cyclins D for different cell types, the gene expression is represented as a function of the cell cycle progression (starting from G1 and finishing with M) and not as a direct function of time.

**Active or bioactive principles or ingredients:** In the present description and unless otherwise indicated or contradictory in context, the term "(bio)active principle" or "(bio)active ingredient" generally refers to a molecule, compound or substance which is responsible for providing the desired biological effect. Without said active ingredient, the formulation or composition containing it, would not provide the desired biological effect. For example, in certain

embodiments, formulation excipients are not considered as active ingredients in the pharmaceutical composition as defined herein.

In one example, said (modified) GFR-binding compound and bioactive carrier are both active principles/ingredients.

In one aspect, the present disclosure also provides a GFR-binding compound modified to be associated with a bioactive carrier (i.e. a modified GFR-binding compound) to form a pharmaceutical association all as defined herein, for use in the prevention or treatment of a neoplastic disease.

**Neoplastic disease medicament:** In the present description and unless otherwise indicated or contradictory in context, the term "neoplastic disease medicament" means a substance, compound, pharmaceutical association, combination, composition or formulation which is suitable for treating or preventing a neoplastic disease in a subject.

**Subject carrying a neoplastic cell:** In the present description and unless otherwise indicated or contradictory in context, the term "subject carrying a neoplastic cell" means that at least one cell constitutive of the subject is a neoplastic cell as defined herein.

In the present description and unless otherwise indicated or contradictory in context, the terms "functionally associated", "functionally combined", "functionalized", "immobilized", "deposited", "coated", or "grafted" all refer to the action of associating or functionalising at least one part of a bioactive carrier with a (modified) GFR-binding compound so that the desired biological, therapeutic and/or cosmetic effect *e.g.* inducing tissue formation, is obtained. The association or combination may be covalent and form, between said (modified) GFR-binding compound and said bioactive carrier, a covalent interaction as already defined herein, or, the association or combination may be non-covalent and form, between said (modified) GFR-binding compound and said bioactive carrier, a non-covalent interaction as already defined herein.

For example, in certain embodiments, a (modified) GFR-binding compound interacts covalently (makes at least one functional covalent interaction) with said bioactive carrier.

In one aspect, the present disclosure thus provides a pharmaceutical association or combination comprising a (modified) GFR-binding compound and a bioactive carrier for use in converting or recoding a neoplastic cell into a non-neoplastic cell, wherein said GFR-binding compound (before any modifications) and said bioactive carrier are both as defined herein.

In one particular example, said pharmaceutical association or combination comprises at least one GFR-binding compound selected from the group consisting of peptides comprising the 4mer peptide SAIS among SEQ ID NO: 1 to 13564.

The present disclosure provides a pharmaceutical association or combination comprising a (modified) GFR-binding compound, wherein all of PEP1, PEP3, PEP5, PEP9, PEP11, PEP12, $AA^{17}$, pairs and triplets thereof, disclaimers and provisos, are as already defined herein.

Suitable covalent association or functionalization techniques for implementing embodiments of the present invention include, but are not limited to, reductive amination coupling or photo-grafting such as described in H. Freichel et al., Macromol. Rapid Commun. 2011, 32, 616-621 and V. Pourcelle et al., Biomacromol. 2009, 10, 966-974,

In one aspect, the present disclosure provides a production method or process useful for producing a pharmaceutical association or combination according to the present disclosure wherein said bioactive carrier is a biomaterial such as a ceramic or a titanium, comprising, or exclusively consisting of, the contacting of a compound of formula (C-I) and a bioactive carrier as defined herein under suitable covalent-bond formation conditions thereby forming at least one covalent bond between said compound (C-I) and said bioactive carrier thus forming a pharmaceutical association or combination according to the present disclosure:

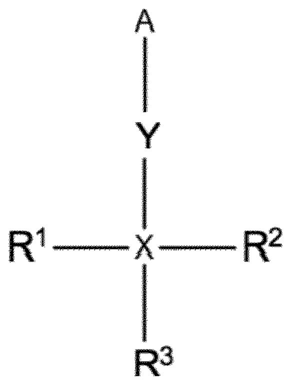

(C-I)

wherein X is Si; wherein Y is a divalent organic linker; wherein A is a (modified) GFR-binding compound according to the

present disclosure, wherein $R^1$ and $R^2$ are both independently an organic spacing-compound other than a leaving group as defined herein, and wherein $R^3$ is a leaving group as defined herein;

In one particular example, a process or method which may be used to functionally associate or combine a (modified) GFR-binding compound with a bioactive carrier such as a ceramic or a titanium is shown in Scheme 1:

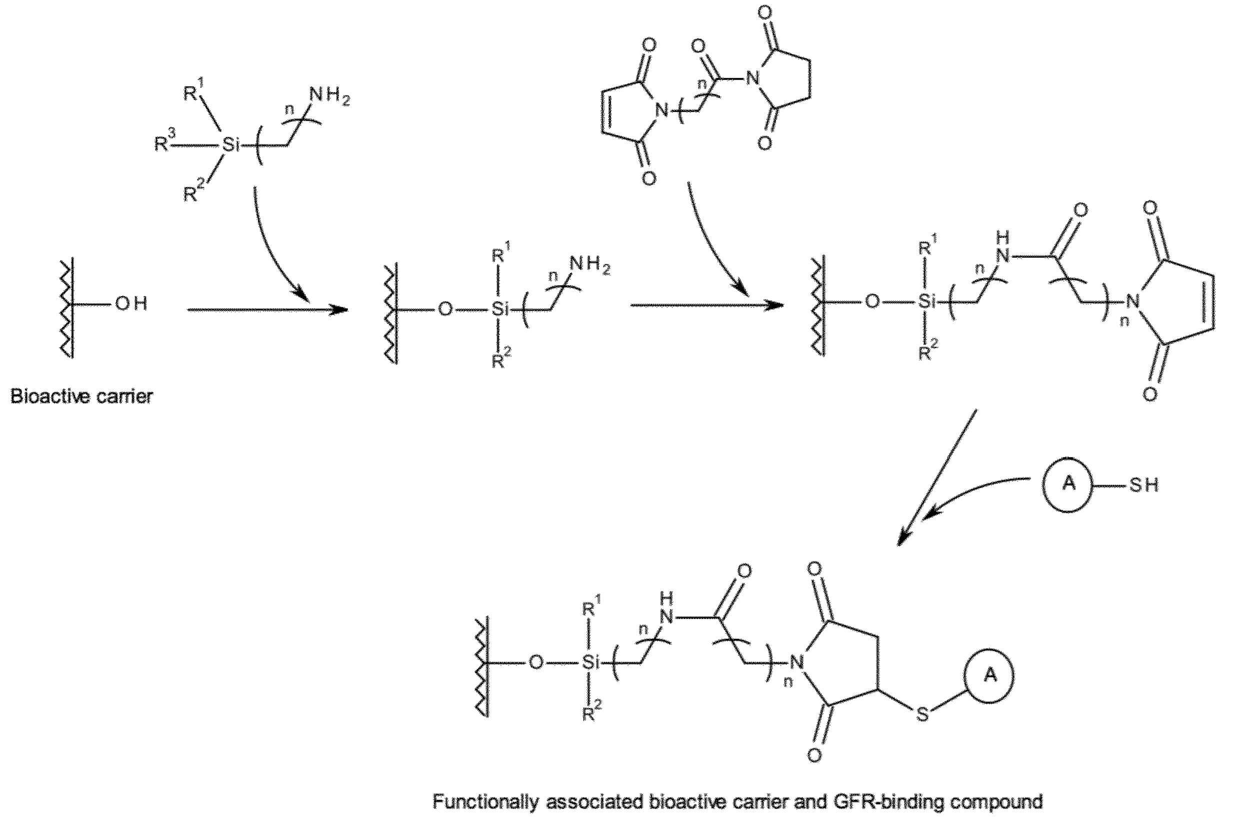


Scheme 1

Such syntheses involve the formation of a covalent interaction (or association) between a (modified) GFR-binding compound (represented as (A)-SH in Scheme 1) and a bioactive carrier as defined herein.

In one particular example, a process or method which may be used to functionally associate or combine a (modified) GFR-binding compound with a bioactive carrier is a method for covalent functionalization or depositing of a (modified) GFR-binding compound onto a polyetheretherketone polymer (PEEK) surface wherein (i) the polymer is treated with ethylene diamine ($NH_2=NH_2$) to create $NH_2$ functions on a PEEK surface from ketone (=O) functions and (ii) the hereby modified PEEK-$NH_2$ polymer is immersed in a solution of a chosen hetero-bifunctional cross-linker such as 3-succinimidyl-3-maleimidopropionate thereby reacting the maleimide group with a (modified) GFR-binding compound through *e.g.* a thiol group thereof.

In one particular example, a process or method which may be used to functionally associate or combine a (modified) GFR-binding compound with a bioactive carrier is a method for covalent functionalization or depositing of a (modified) GFR-binding compound onto a polylactic acid (PLLA) polymer wherein (i) the polymer is immersed in a solution containing, for instance, (dimethylaminopropyl)-3-ethylcarbodiimide hydrochloride + N-hydroxysuccinimide in (2-(N-morpholino)-ethanesulfonic acid and then (ii) rinsed using *e.g.* MilliQ water.

**Leaving groups:** As used herein, unless indicated otherwise or contradictory in context, the term "leaving group" means a molecular fragment which possesses the ability to depart with a pair of electrons in a heterolytic bond cleavage. Leaving groups are anions or neutral molecules and possess the ability to stabilize the additional electron density that results from bond heterolysis. Common anionic leaving groups are halogen atoms such as chlorine (Cl), bromine (Br), and iodine (I), which leaves as a chloride ion ($Cl^-$), a bromide ion ($Br^-$) and an iodide ion ($I^-$), respectively. Other leaving groups include sulfonate esters, such as tosylate ($TsO^-$). Conventional neutral molecule leaving groups are water and ammonia. Suitable as leaving groups for implementing embodiments of the invention preferably include the group consisting of a halogen, a substituted or unsubstituted alkoxy group (-OR), a substituted or unsubstituted aryloxy or heteroaryloxy group (-OAr), a substituted or unsubstituted alkylcarbonyloxy group (-$O_2CR$), a substituted or unsubstituted arylcarbonyloxy or heteroarylcarbonyloxy group (-$O_2CAr$), a substituted or unsubstituted alkylsulfonyloxy group (-$O_3SR$), a substituted or

unsubstituted arylsulfonyloxy or heteroarylsulfonyloxy group ($-O_3SAr$). Substituents of leaving groups include halogens, alkyl (preferably C1 to C5-alkyl) groups and alkoxy (preferably C1 to C5-alkoxy) groups.

**Y group**

In the present disclosure, the Y group is not aimed at being particularly limited and any moiety comprising at least one atom and having the ability to covalently or non-covalently, preferably covalently, link or interact with the X and A groups as defined herein thereby providing a stable connection between an active substance A and the X group as defined herein, is, unless contradictory or non-adapted in context, suitable for implementing embodiments of the present disclosure and is comprised within the scope of the invention.

Thus, in the present description and unless otherwise indicated, the term "linker", when used in relation to a Y group, means any organic moiety comprising at least one atom and having the ability to interact covalently or non-covalently with an active substance A and covalently interact with an X group as defined herein.

In one example, Y groups include divalent organic radicals selected from the group consisting of a saturated or unsaturated, preferably saturated, hydrocarbon chain comprising between 1 and 30 carbon atoms, wherein said hydrocarbon chain is optionally interrupted by one or more non-carbon atom, preferably between 1 and 16, between 1 and 12 or between 1 and 8 non-carbon atoms as appropriate, wherein said non-carbon atom is selected, for instance, from the group consisting of -O-, -S-, -C(=O), - $SO_2$-, -N(Ri)(C=O)-, -N(Ri)-, and the following radical:

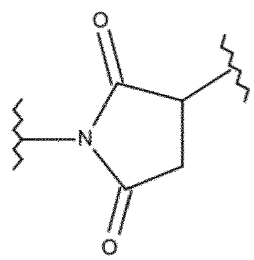

wherein Ri is selected from the group consisting of a hydrogen atom, a C1-C6 alkyl group and a aryl group, and wherein said hydrocarbon chain is non-substituted or substituted, by at least one radical selected from the group consisting of a halogen, a hydroxyl group, a C1-C20 alkyl group and a aryl group.

Suitable as Y groups for implementing embodiments of the invention include saturated or unsaturated hydrocarbon chains comprising between 1 and 20 carbon atoms, saturated or unsaturated hydrocarbon chains comprising between 1 and 10 carbon atoms, saturated or unsaturated hydrocarbon chains comprising between 1 and 5 carbon atoms, saturated or unsaturated hydrocarbon chains comprising 1, 2 or 3 carbon atoms, all of which being specifically and individually preferred.

Also suitable as Y groups for implementing embodiments of the invention include saturated or unsaturated hydrocarbon chains comprising between 1 and 20 carbon atoms, saturated or unsaturated hydrocarbon chains comprising between 1 and 10 carbon atoms, saturated or unsaturated hydrocarbon chains comprising between 1 and 5 carbon atoms, saturated or unsaturated hydrocarbon chains comprising 1, 2 or 3 carbon atoms, and in which said hydrocarbon chain is optionally interrupted by one or more, preferably between 1 and 16, between 1 and 12 or between 1 and 8, non-carbon atom, selected from the group consisting of an oxygen atom, a nitrogen atom, a carbonyl group and/or the following radical:

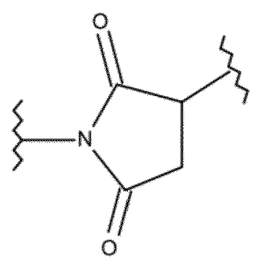

, all of which being specifically preferred and individually contemplated.

Also suitable as Y groups for implementing embodiments of the invention is:

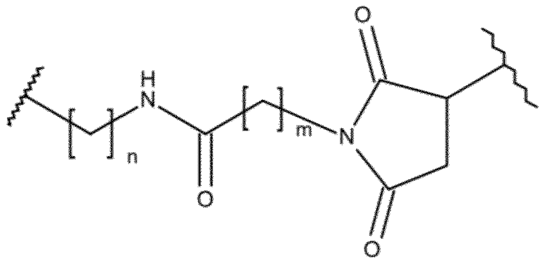

wherein n is comprised betwwen 1 and 29, in particular between 1 and 5; and wherein m is comprised between 1 and 29, in particular between 1 and 5.

**Suitable covalent-bond formation conditions:** As used herein, unless indicated otherwise or contradictory in context, the term "suitable covalent-bond formation conditions" means reaction conditions such as pressure, temperature, reagent quantities, solvent's type and quantity, or stirring, under which starting materials may contact and provide at least one further material resulting from the formation of at least one covalent bond between said starting materials. Suitable as covalent-bond formation conditions for implementing embodiments of the present invention preferably include substantially atmospheric conditions.

**Organic spacing-compound:** In the present description and unless otherwise indicated, the term "organic spacing compound" means an organic chemical radical (preferably monofunctional radical) having the ability to create a steric effect/hindrance and/or electronic effect/hindrance in a direct vicinity of a (modified) GFR-binding compound of the present disclosure. Suitable organic spacing compounds include, but are not limited to, monovalent organic radicals independently selected from the group consisting of a saturated or unsaturated hydrocarbon chain of at most 20 nanometres (nm) in length, preferably at most 10 nm, 5 nm, 1 nm, 0.5 nm, 0.1 nm, 0.05 nm or 0.01 nm, wherein said hydrocarbon chain is optionally interrupted by one or more, preferably between 1 and 16, between 1 and 12 or between 1 and 8 non-carbon atoms as appropriate, wherein said non-carbon atom is selected from the group consisting of -O-, -S-, -C(=O), $-SO_2-$, $-N(R^i)(C=O)-$, and $-N(R^i)-$, wherein $R^i$ is selected from the group consisting of a hydrogen atom, a C1-C6 alkyl group and an aryl group, and wherein said hydrocarbon chain is non-substituted or substituted by at least one radical selected from the group consisting of a halogen, a hydroxyl group, a C1-C20 alkyl group and an aryl group. In particular, organic spacing compounds include saturated or unsaturated hydrocarbon chains comprising between 1 and 80 carbon atoms, saturated or unsaturated hydrocarbon chains comprising between 1 and 60 carbon atoms, saturated or unsaturated hydrocarbon chains comprising between 1 and 40 carbon atoms, saturated or unsaturated hydrocarbon chains comprising between 1 and 20 carbon atoms, saturated or unsaturated hydrocarbon chains comprising between 1 and 10 carbon atoms, saturated hydrocarbon chains comprising 1, 2, 3, 4, 5 or 6 carbon atoms, all of which being specifically and individually preferred. In one example, the saturated hydrocarbon chain may be methyl, ethyl, propyl, butyl or pentyl. In one example, said unsaturated hydrocarbon chain may be ethylene, propene, 1- or 2-butene, 1-, 2- or 3-pentene, acetylene, propyne, 1- or 2-butyne, 1-, 2- or 3-pentyne.

**Saturated hydrocarbon chain:** In the present description and unless otherwise indicated, the terms "saturated hydrocarbon chain" means a chain of carbon atoms linked together by single bonds and has hydrogen atoms filling all of the other bonding orbitals of the carbon atoms.

**Unsaturated hydrocarbon chain:** In the present description and unless otherwise indicated, the terms "unsaturated hydrocarbon chain" means a chain of carbon that contains carbon-carbon double bonds or triple bonds, such as those found in alkenes or alkynes, respectively.

**Atmospheric conditions:** As used herein, unless indicated otherwise or contradictory in context, the term "atmospheric conditions" or "ambient conditions", which are interchangeably used, refers to conditions which may be found naturally at an experimentation location. For example, in certain embodiments, typical atmospheric conditions in a chemistry/biology laboratory are a temperature of between about 15°C and about 35°C and a pressure of about 1 atm.

**Solution:** As used herein, unless indicated otherwise or contradictory in context, the term "solution" means a homogeneous mixture composed of only one phase, which is stable, which does not allow beam of light to scatter, in which the particles of solute cannot be seen by naked eye and from which a solute cannot be separated by filtration.

**Suspension:** As used herein, unless indicated otherwise or contradictory in context, the term "suspension" means a heterogeneous mixture containing solid particles that are sufficiently large for sedimentation. Typically, said solid particles are larger than one micrometer. In general, the internal phase (solid) is dispersed throughout the external phase (fluid) through mechanical agitation, with the use of certain excipients or suspending agents.

Suitable non-covalent association or functionalization techniques for implementing embodiments of the present invention include, but are not limited to, association(s) between a bioactive carrier-affinity containing group as already defined herein and at least part of a bioactive carrier. Such association(s) involves the formation of at least one non-covalent interaction (or attachment) between a (modified) GFR-binding compound and a bioactive carrier as defined herein.

In one example, said pharmaceutical association or combination is functionally associated with at least two, at least three, at least four, at least five, at least six, at least seven, at least eight, at least nine or at least ten (modified) GFR-binding compounds, each possessing a different and distinct chemical structure.

In one example, said pharmaceutical association or combination does not comprise a layer of polysiloxane.

In an example, a bioactive carrier of the present invention (further) comprises at least one compound selected from the group consisting of anti-cancer agents as already defined herein, and an anti-inflammatory agent such as Celecoxib, Diclofenac, Diflunisal, Etodolac, Ibuprofen, Indomethacin, Ketoprofen, Ketorolac, Nabumetone, Naproxen, Oxaprozin, Piroxicam, Salsalate, Sulindac or Tolmetin.

For example, in certain embodiments, said further compounds interact covalently or non-covalently as already defined herein with said bioactive carrier.

For example, in certain embodiments, a pharmaceutical association or combination as defined herein comprises at least one (modified) GFR-binding compound, and at least one bioactive carrier, wherein said bioactive carrier:

- has a porosity (or average pore diameter) comprised between 1 nm and 1000 μm, as measured by scanning electronic microscopy for pore sizes within the supra-nanometre range and by atomic force microscopy for pore sizes within the nanometre range, and/or
- comprises a stiffness of at least 5 kPa, preferably at least 35 kPa, as measured by Dynamic Mechanical Analysis, and/or
- is selected from the group consisting of biopolymers (collagen, fibrin, ...etc), synthetic polymers (PEEK, PET, ...etc), solid materials (Titanium, Metals ...etc) and ceramics (Hydroxyapatite, Beta-tricalcium Phosphate, Biphasic Calcium Phosphate ...etc) , and/or
- comprises a density or concentration of associated compound (I) comprised between $0.05 \times 10^{-12}$ mol/mm$^2$ and $50 \times 10^{-12}$ mol/mm$^2$, as measured by conventional fluorescence microscopy or calculated theoretically on the basis of the peptide size, and/or
- does not comprise a layer of polysiloxane.

**Porosity:** As used herein, unless indicated otherwise or contradictory in context, the term "porosity" refers to the measure of the void spaces in a substance or material, and is a fraction of the volume of voids over the total volume, between 0 and 1, or as a percentage between 0 and 100%. There are many ways to test and measure the porosity of a substance or material, but for the purpose of the present disclosure, and for the avoidance of any doubts, porosity values are provided in manometers (nm) as obtained using atomic force microscopy for small pore diameters (up to 100 nm) and scanning electron microscopy for larger pore sizes.

**Stiffness:** As used herein, unless indicated otherwise or contradictory in context, the term "stiffness" refers to the rigidity of a substance or material *i.e.* the extent to which it resists deformation in response to an applied force. There are many ways to test and measure the stiffness of a substance or material, but for the purpose of the present disclosure, and for the avoidance of any doubts, stiffness values are provided in Pascal (Pa) as obtained using Dynamic Mechanical Analysis (DMA). Particularly preferred stiffness values are comprised between 1 kPa and 100 kPa and not more than 5 GPa depending on the tissue to be regenerated or repaired.

As already stated, the nature of the biomaterial is an important parameter. Particularly good results have been obtained using bioactive carriers composed mostly with the main material component of the tissue to be regenerated and/or repaired. This generally allows for a better integration of the bioactive carrier, a better resorption from the surrounding cells already present and therefore a better regeneration or repair of the targeted tissue to be achieved.

In one particular example, the concentration or density (as defined herein) of (modified) GFR-binding compounds in a pharmaceutical association, combination or composition as defined herein is comprised between 0.05 and 50 pmol/mm$^2$, in particular comprised between 0.1 and 30 pmol/mm$^2$, comprised between 0.1 and 10 pmol/mm$^2$, comprised between 0.1 and 5 pmol/mm$^2$, or comprised between 0.1 and 2 pmol/mm$^2$, each range being preferred and specifically contemplated to be combined with any other numerical or non-numerical ranges as described herein. Most particularly, the density is comprised between 0.2 and 2 pmol/mm$^2$.

## VIII. Pharmaceutical compositions

In one aspect, the present disclosure provides a composition such as a pharmaceutical, prophylactic, surgical, diagnostic, or imaging composition (hereinafter shorten as pharmaceutical or medical composition) for the uses and methods already disclosed herein comprising at least one pharmaceutical association or combination as defined herein and further comprising at least one pharmaceutically acceptable excipient carriers and/or vehicles.

Formulations of the pharmaceutical compositions described herein may be prepared by any method known or hereafter developed in the art of pharmacology. Generally, such methods of preparation include the step of bringing the active ingredient(s) into association with an excipient and/or one or more other accessory ingredients, and then, if

necessary and/or desirable, shaping and/or packaging the product into a desired single- or multi-dose unit.

For example, in certain embodiments, a pharmaceutical composition as defined herein may contain between 0.01% and 100% by weight (over the total weight of the pharmaceutical composition) of a (modified) GFR-binding compound or a pharmaceutical association or combination, both as defined herein, as a pharmaceutically effective amount. The pharmaceutical composition particularly comprises between 0.01% and 95%, between 0.01% and 90%, between 0.01% and 85%, between 0.01% and 80%, between 0.01% and 75%, between 0.01% and 70%, between 0.01% and 65%, between 0.01% and 60%, between 0.01% and 55%, between 0.01% and 50%, between 0.01% and 45%, between 0.01% and 40%, between 0.01% and 35%, between 0.01% and 30%, between 0.01% and 25%, between 0.01% and 20%, between 0.01% and 15%, between 0.01% and 10%, between 0.01% and 5%, between 0.1% and 100%, between 0.1% and 95%, between 0.1% and 90%, between 0.1% and 85%, between 0.1% and 80%, between 0.1% and 75%, between 0.1% and 70%, between 0.1% and 65%, between 0.1% and 60%, between 0.1% and 55%, between 0.1% and 50%, between 0.1% and 45%, between 0.1% and 40%, between 0.1% and 35%, between 0.1% and 30%, between 0.1% and 25%, between 0.1% and 20%, between 0.1% and 15%, between 0.1% and 10%, and between 0.1% and 5% by weight (over the total weight of the pharmaceutical composition) of any one of a (modified) GFR-binding compound or a pharmaceutical association or combination as defined herein.

Generally, the (modified) GFR-binding compounds or pharmaceutical association or combinations as defined herein may thus be administered as such or as part of a formulation in association with one or more pharmaceutically acceptable excipients, carriers and/or vehicles so as to form what is generally referred to as a pharmaceutical composition or pharmaceutical formulation.

**Pharmaceutical effective amount:** As used herein, unless indicated otherwise or contradictory in context, the term "pharmaceutical effective amount" or "therapeutically effective amount" refers to an amount of an agent to be delivered (e.g., nucleic acid, protein, peptide, drug, therapeutic agent, diagnostic agent, prophylactic agent, etc.) that is sufficient, when administered to a subject suffering from or susceptible to an infection, disease, disorder, condition and/or pathology, to produce/provide a therapeutically effective outcome. Thus, a "pharmaceutical effective amount" depends upon the context in which it is being applied. A pharmaceutical effective amount of a composition is provided based, at least in part, on the target tissue, target cell type, means of administration, physical characteristics of the pharmaceutical association or composition (e.g., size, 3D shape, etc.), and other determinants. For example, in certain embodiments, in the context of administering an agent that treats cancer, a pharmaceutical effective amount of an agent is, for example, in certain embodiments, an amount sufficient to achieve treatment, as defined herein, of cancer, as compared to the response obtained without administration of the agent. For example, in certain embodiments, a therapeutically effective amount as used herein is any of the herein disclosed weight or molar amounts, ratios or ranges of the (modified) GFR-binding compound, the bioactive carrier or the association/combination thereof.

**Therapeutically effective outcome:** As used herein, unless indicated otherwise or contradictory in context, the term "therapeutically effective outcome" refers to an outcome that is sufficient in a subject suffering from or susceptible to an infection, disease, disorder, condition and/or pathology, to treat, improve symptoms of, diagnose, prevent, and/or delay the onset of the infection, disease, disorder, condition and/or pathology.

**Therapeutic Agent:** As used herein, unless indicated otherwise or contradictory in context, the term "therapeutic agent" refers to any agent that, when administered to a subject/patient/individual, has a therapeutic, diagnostic, and/or prophylactic effect and/or elicits a desired biological and/or pharmacological effect.

**Pharmaceutically acceptable:** As used herein, unless indicated otherwise or contradictory in context, the term "pharmaceutically acceptable" refers to those compounds, materials, compositions, and/or dosage forms which are, within the ambit of sound medical judgment, suitable for use in contact with the tissues of human beings and animals without excessive toxicity, irritation, allergic response, or other problem or complication, commensurate with a reasonable benefit/risk ratio.

**Pharmaceutically acceptable excipients:** As used herein, unless indicated otherwise or contradictory in context, the term "pharmaceutically acceptable excipient" refers to any ingredient other than the compounds described herein (*i.e.* GFR-binding compounds, bioactive carriers as defined herein or any further active principles) and satisfying to the herein defined definition of pharmaceutically acceptable for a patient. Excipients may include, for example: inert diluents, dispersing and/or granulating agents, surface active agents and/or emulsifiers, disintegrating agents, binding agents, preservatives, buffering agents, lubricating agents, oils, printing inks, sweeteners, and/or waters of hydration. The choice of excipient(s) will largely depend on factors such as the particular mode of administration, the effect of the excipient(s) on solubility and stability, and the nature of the dosage form. In one embodiment, the pharmaceutically acceptable excipient is not a naturally occurring excipient.

**Diluents:** As used herein, unless indicated otherwise or contradictory in context, diluents include, but are not limited to, calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, powdered sugar and/or any combinations thereof.

**Buffering agents:** As used herein, unless indicated otherwise or contradictory in context, buffering agents

include, but are not limited to, citrate buffer solutions, acetate buffer solutions, phosphate buffer solutions, ammonium chloride, potassium acetate, potassium chloride, monobasic potassium phosphate, calcium carbonate, calcium chloride, calcium citrate, calcium gluconate, calcium lactate, propanoic acid, calcium levulinate, pentanoic acid, phosphoric acid, calcium hydroxide phosphate, sodium acetate, sodium bicarbonate, sodium chloride, sodium citrate, sodium lactate, magnesium hydroxide, aluminum hydroxide, alginic acid, pyrogen-free water, isotonic saline, Ringer's solution, ethyl alcohol and any combinations thereof.

**Granulating and/or dispersing agents:** As used herein, unless indicated otherwise or contradictory in context, granulating and/or dispersing agents include, but are not limited to, potato starch, corn starch, tapioca starch, sodium starch glycolate, clays, alginic acid, guar gum, citrus pulp, agar, bentonite, cellulose and wood products, natural sponge, cation-exchange resins, calcium carbonate, silicates, sodium carbonate, cross-linked poly(vinyl-pyrrolidone), sodium carboxymethyl starch, carboxymethyl cellulose, cross-linked sodium carboxymethyl cellulose, methylcellulose, pregelatinized starch, microcrystalline starch, water insoluble starch, calcium carboxymethyl cellulose, magnesium aluminum silicate, sodium lauryl sulfate, quaternary ammonium compounds and/or any combinations thereof.

**Surface active agents and/or emulsifiers:** As used herein, unless indicated otherwise or contradictory in context, surface active agents and/or emulsifiers include, but are not limited to, colloidal clays (such as aluminum silicates and magnesium aluminum silicates), natural emulsifiers (such as acacia, agar, sodium alginate, cholesterol, xanthan, pectin, gelatin, egg yolk, casein, cholesterol, wax, and lecithin), long chain amino acid derivatives, high molecular weight alcohols (such as stearyl, cetyl and oleyl alcohols, triacetin monostearate, ethylene glycol distearate and glyceryl monostearate), carbomers (such as carboxy polymethylene, polyacrylic acid, acrylic acid polymer, and carboxyvinyl polymer), diethylene glycol monolaurate, triethanolamine oleate, sodium oleate, potassium oleate, ethyl oleate, oleic acid, ethyl laurate, sodium lauryl sulfate, cetrimonium bromide, cetylpyridinium chloride, benzalkonium chloride, docusate sodium, carrageenan, cellulosic derivatives (such as carboxymethylcellulose sodium, hydroxymethyl cellulose, hydroxypropyl methylcellulose and methylcellulose), sorbitan fatty acid esters (such as polyoxyethylene sorbitan monolaurate, polyoxyethylene sorbitan, polyoxyethylene sorbitan monooleate, sorbitan monopalmitate and glyceryl monooleate), polyoxyethylene esters, sucrose fatty acid esters, polyethylene glycol fatty acid esters, polyoxyethylene ethers, poly(vinyl-pyrrolidone), and any combinations thereof.

**Binding agents:** As used herein, unless indicated otherwise or contradictory in context, binding agents include, but are not limited to, natural and synthetic gums (such as acacia, sodium alginate, carboxymethylcellulose, methylcellulose, ethylcellulose, hydroxyethylcellulose, hydroxypropyl cellulose, hydroxypropyl methylcellulose, microcrystalline cellulose, cellulose acetate and poly(vinyl-pyrrolidone), gelatin, starch, sugars (such as sucrose, dextrose, glucose, dextrin, lactose, and mannitol), alignates, magnesium aluminum silicates, polyethylene glycol, polyethylene oxide, inorganic calcium salts, water, alcohol, silicic acid, waxes, and any combinations thereof.

**Preservatives:** As used herein, unless indicated otherwise or contradictory in context, preservatives include, but are not limited to, antioxidants, chelating agents, antifungal preservatives, antimicrobial preservatives, acidic preservatives, and alcohol preservatives.

**Antioxidants:** As used herein, unless indicated otherwise or contradictory in context, antioxidants include, but are not limited to, alpha tocopherol, ascorbic acid, acorbyl palmitate, butylated hydroxyanisole, propionic acid, potassium metabisulfite, propyl gallate, sodium metabisulfite, sodium ascorbate, and sodium sulfite.

**Chelating agents:** As used herein, unless indicated otherwise or contradictory in context, chelating agents include ethylenediaminetetraacetic acid (EDTA), fumaric acid, malic acid, phosphoric acid, citric acid monohydrate and tartaric acid.

**Antimicrobial preservatives:** As used herein, unless indicated otherwise or contradictory in context, antimicrobial preservatives include, but are not limited to, benzalkonium chloride, benzethonium chloride, chlorhexidine, chlorobutanol, chlorocresol, chloroxylenol, benzyl alcohol, bronopol, cetylpyridinium chloride, cresol, ethyl alcohol, glycerin, hexetidine, imidurea, phenoxyethanol, phenylmercuric nitrate, phenylethyl alcohol, phenol, and propylene glycol.

**Antifungal preservatives:** As used herein, unless indicated otherwise or contradictory in context, antifungal preservatives include, but are not limited to, benzoic acid, hydroxybenzoic acid, butyl paraben, methyl paraben, ethyl paraben, propyl paraben, potassium benzoate, sodium propionate, potassium sorbate, and/or sorbic acid.

**Alcohol preservatives:** As used herein, unless indicated otherwise or contradictory in context, alcohol preservatives include, but are not limited to, phenol, phenolic compounds, bisphenol, ethanol, polyethylene glycol, chlorobutanol and hydroxybenzoate.

**Acidic preservatives:** As used herein, unless indicated otherwise or contradictory in context, acidic preservatives include, but are not limited to, vitamin A, vitamin C, vitamin E, beta-carotene, acetic acid, citric acid, dehydroacetic acid, and sorbic acid.

**Lubricating agents:** As used herein, unless indicated otherwise or contradictory in context, lubricating agents include, but are not limited to, magnesium stearate, calcium stearate, stearic acid, sodium benzoate, sodium acetate, sodium chloride, silica, talc, malt, glyceryl behanate, hydrogenated vegetable oils, polyethylene glycol, magnesium lauryl

sulphate and any combinations thereof.

**Sweeteners:** As used herein, unless indicated otherwise or contradictory in context, sweeteners include, but are not limited to, any natural or synthetic sugar substitutes. Natural sugar substitutes include, but are not limited to, brazzein, curculin, erythritol, glycyrrhizin, glycerol, hydrogenated starch hydrolysates, inulin, isomalt, lactitol, mogroside mix, mabinlin, maltitol, malto-oligosaccharide, mannitol, miraculin, monatin, monellin, osladin, pentadin, sorbitol, stevia, tagatose, thaumatin, and xylitol. Synthetic sugar substitutes include, but are not limited to, acesulfame potassium, advantame, alitame, aspartame, salt of aspartame-acesulfame, sodium cyclamate, dulcin, glucin, neohesperidin dihydrochalcone, neotame, P-4000, saccharin, Sucralose.

Exemplary excipients include, but are not limited to: butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch, stearic acid, sucrose, talc, titanium dioxide, vitamin A, vitamin E, vitamin C, and xylitol. Suitable excipients for use in the present invention also include, but are not limited to, water, phosphate buffered saline (PBS), Ringer's solution, dextrose solution, serum-containing solutions, Hank's solution, other aqueous physiologically balanced solutions, oils, esters and glycols. Aqueous excipients can contain suitable auxiliary substances required to approximate the physiological conditions of the recipient, for example, in certain embodiments, by enhancing chemical stability and isotonicity.

**Pharmaceutically/medically acceptable carriers:** As used herein, unless indicated otherwise or contradictory in context, the term "pharmaceutically acceptable carriers", "medically acceptable carrier" or "carriers" refers to pharmaceutically acceptable excipients and/or delivery vehicles suitable for delivering a pharmaceutical or therapeutic composition useful in a therapeutic method and uses of the present invention to a suitable in-vivo or ex-vivo site. Preferred pharmaceutically acceptable carriers are capable of maintaining a composition containing an active combination or association of a (modified) GFR-binding compound and a bioactive carrier as defined herein, in a form that, upon arrival of the combination to a target cell, site or tissue, the active combination is capable of performing one or more biological functions thereof the protein at the cell or tissue site. One type of pharmaceutically acceptable carrier includes a controlled release formulation that is capable of slowly releasing a composition or combination into an animal. In one example, a controlled release formulation comprises an active combination or association as defined herein in a controlled release vehicle. Suitable controlled release vehicles include, but are not limited to, microparticles, biocompatible polymers, other polymeric matrices, capsules, microcapsules, osmotic pumps, bolus preparations, diffusion devices, liposomes, lipospheres, and transdermal delivery systems. Such suitable controlled release vehicle may be combined with at least one targeting moiety. In one embodiment, the pharmaceutically acceptable carrier is not a naturally occurring carrier.

**Targeting Moieties:** In one example, the pharmaceutical association or combination disclosed herein includes at least one binding partner which functions to target the cell to a specific tissue space or to interact with a specific moiety, either in-vivo, ex-vivo or in-vitro. Suitable binding partners include antibodies and functional fragments thereof, scaffold proteins, or peptides.

In one example, said excipients, carriers or vehicles are compatible with the (modified) GFR-binding compounds or pharmaceutical association or combinations defined herein so that they do not disrupt, tamper,modify, de-organise, decombine or de-associate said the (modified) GFR-binding compounds or pharmaceutical association or combinations. In contrast, said excipients, carriers or vehicles preserves, maintains or reinforces the stability of the (modified) GFR-binding compounds or pharmaceutical association or combinations so as to preserve their biological activity.

In one example, the present pharmaceutical compositions also include pharmaceutically acceptable salts and/or solvates and/or prodrugs and/or isotopically-labelled derivatives of the substances and compounds described herein such as the (modified) GFR-binding compounds or any other active principles.

**Pharmaceutically acceptable salts:** As used herein, unless indicated otherwise or contradictory in context, the term "pharmaceutically acceptable salts" refers to derivatives of the disclosed substances and compounds wherein the parent substance or compound is modified by converting an existing acid or base moiety to its salt form (e.g., by reacting the free base group with a suitable organic acid). The degree of ionization in the salt may vary from completely ionized to almost non-ionized. Examples of pharmaceutically acceptable salts include, but are not limited to, mineral or organic acid salts of basic residues such as amines; alkali or organic salts of acidic residues such as carboxylic acids; and the like. Representative acid addition salts include acetate, adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include

sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. The pharmaceutically acceptable salts of the present disclosure include the conventional non-toxic salts of the parent compound formed, for example, in certain embodiments, from non- toxic inorganic or organic acids. The pharmaceutically acceptable salts of the present disclosure can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, ethanol, isopropanol, or acetonitrile are preferred. Lists of suitable salts are generally found in Remington's Pharmaceutical Sciences, 17th ed., Mack Publishing Company, Easton, Pa., 1985, p. 1418 and in Pharmaceutical Salts: Properties, Selection, and Use, P.H. Stahl and C.G. Wermuth (eds.), Wiley-VCH, 2008, In one embodiment, the pharmaceutically acceptable salt is not a naturally occurring salt.

**Pharmaceutically acceptable solvate:** As used herein, unless indicated otherwise or contradictory in context, the term "pharmaceutically acceptable solvate," refers to a compound, substance, association or combination wherein molecules of a suitable solvent are incorporated in the crystal lattice. A suitable solvent is physiologically tolerable at the dosage administered. For example, in certain embodiments, solvates may be prepared by crystallization, recrystallization, or precipitation from a solution that includes organic solvents, water, or a mixture thereof. Examples of suitable solvents are ethanol, water (For example, in certain embodiments, mono-, di-, and tri-hydrates), N-methylpyrrolidinone (NMP), dimethyl sulfoxide (DMSO), dimethylformamide (DMF), [Nu],[Nu]'-dimethylacetamide (DMAC), 1,3-dimethyl-2-imidazolidinone (DMEU), 1,3-dimethyl-3,4,5,6-tetrahydro-2-(1H)-pyrimidinone (DMPU), acetonitrile (ACN), propylene glycol, ethyl acetate, benzyl alcohol, 2-pyrrolidone, benzyl benzoate, and the like. When water is the solvent, the solvate is referred to as a "hydrate". In one embodiment, the pharmaceutically acceptable solvate is not a naturally occurring solvate.

**Pharmaceutically acceptable isotopically-labelled compounds:** In one example, the present invention also includes all pharmaceutically acceptable isotopically-labelled derivatives, which are identical to the compounds, substances, combinations or associations described herein but wherein one or more atoms are replaced by atoms having an atomic mass or mass number different from the atomic mass or mass number usually found in nature. Examples of isotopes that may be incorporated into GFR-binding compound(s) as defined herein include isotopes of hydrogen, carbon, chlorine, fluorine, iodine, nitrogen, oxygen, and sulfur, such as $^{2}H$, $^{3}H$, $^{11}C$, $^{13}C$, $^{14}C$, $^{36}Cl$, $^{18}F$, $^{123}I$, $^{13}N$, $^{15}N$, $^{17}O$, $^{18}O$, and $^{35}S$, respectively. It should be understood that compounds, substances, combinations, associations, prodrugs, and pharmaceutical acceptable salts thereof described herein which contain the aforementioned isotopes and/or other isotopes of other atoms are within the scope of the invention. Certain isotopically labeled of the compounds, substances, combinations, associations, prodrugs, and salts thereof such as, for example, in certain embodiments, those incorporating a radioactive isotope such as $^{3}H$ and $^{14}C$, are useful in drug and/or substrate tissue distribution studies. Tritium, i.e. $^{3}H$, and carbon-14, i.e. $^{14}C$, are particularly preferred due to their ease of preparation and detection. Further, substitution with heavier isotopes such as deuterium, i.e. $^{2}H$, can afford certain therapeutic advantages resulting from greater metabolic stability, for example, in certain embodiments, increased in vivo half-life or reduced dosage requirements, and hence may be preferred in some circumstances. Isotopically labeled compounds, substances, combinations, associations, prodrugs, and salts thereof can generally be prepared by carrying out the procedures disclosed in the Schemes and/or in the Examples by substituting a readily available non-isotopically labeled reagent for an isotopically labeled reagent.

**Prodrugs:** As used herein, unless indicated otherwise or contradictory in context, the term "prodrug" refers to a compound, substance, combination or association that is transformed in vivo to yield a compound, substance, combination or association as defined herein or a pharmaceutically acceptable salt or solvate thereof. The transformation may occur by various mechanisms, such as via hydrolysis in blood. A prodrug of a compound, substance, combination or association defined herein may be formed in a conventional manner with one or more functional groups in the compound, such as an amino, hydroxyl or carboxyl group. For example, in certain embodiments, if a compound defined herein contains a carboxylic acid functional group, a prodrug can comprise: (1) an ester formed by the replacement of a hydrogen of the acid group with a group such as (C1-C6)alkyl or (C6-C10) aryl; (2) an activated ester formed by the replacement of the hydrogen of the acid group with groups such as -($CR^2$)COOR', where $CR^2$ is a spacer and R can be groups such as H or methyl and R' can be groups such as (C1-C6)alkyl or

(C6-C10) aryl; and/or (3) a carbonate formed by the replacement of the hydrogen of the acid with groups such as CHROCOOR' where R can be groups such as H or methyl and R' can be groups such as (C1-C6)alkyl or (C6-C10)aryl. Similarly, if a compound defined herein contains an alcohol functional group, a prodrug can be formed via the replacement of the hydrogen of the alcohol with groups such as (C1-C6)alkanoyloxymethyl or (C1-C6)alkanoyloxyaryl or by forming an ester via condensation with, for example, in certain embodiments, an amino acid. Where a compound defined herein contains a primary or secondary amino group, a prodrug may comprise, for example, in certain embodiments, an amide formed by the replacement of one or both of the hydrogen atoms of the amino group with (C1-C10)alkanoyl or (C6-C10) aroyl. Other prodrugs of amines are well known to those skilled in the art. Alternatively, certain compounds defined herein may themselves act as prodrugs of other compounds defined herein. Discussions regarding prodrugs and their use can be

found in, for example, in certain embodiments, "Prodrugs as Novel Delivery Systems," T. Higuchi and W. Stella, Vol. 14 of the ACS Symposium Series, and Bioreversible Carriers in Drug Design, Pergamon Press, 1987 (ed. E B Roche, American Pharmaceutical Association). Examples of other prodrug types may be found in the aforementioned reference.

## IX. Administration routes and procedures

Compounds, substances, pharmaceutical associations or combinations to be delivered and/or pharmaceutical, dermatological, prophylactic, diagnostic, or imaging compositions or formulations thereof in accordance with the present disclosure may be administered by any route of administration effective for preventing, treating, diagnosing, or imaging a disease, disorder, and/or condition and/or treating or alleviating at least one symptoms thereof.

Suitable administration protocols include any in-vitro, in-vivo or ex-vivo administration protocol. The preferred types and routes of administration will be apparent to those of skill in the art, depending on the type of condition or disease to be prevented or treated; whether the composition is nucleic acid based, protein based, cell based or combinations or mixtures thereof; and/or the target cell/tissue.

Cells, tissues or organs can be contacted ex vivo or in vitro with a pharmaceutical association or combination for uses and methods of the invention by any suitable method, including mixing or the use of a delivery vehicle. Effective in vitro or ex vivo culture conditions include, but are not limited to, effective media, bioreactor, temperature, pH and oxygen conditions that permit cell culture. An effective medium refers to any medium in which a given host cell or tissue is typically cultured. Such medium typically comprises an aqueous medium having assimilable carbon, nitrogen and phosphate sources, and appropriate salts, minerals, metals and other nutrients, such as vitamins. Cells can be cultured in conventional fermentation bioreactors, shake flasks, test tubes, microtiter dishes, and petri plates. Culturing can be carried out at a temperature, pH and oxygen content appropriate for a cell or tissue. Such culturing conditions are within the expertise of one of ordinary skill in the art.

In one aspect, the present disclosure thus also provides a method for converting a neoplastic cell into a non-neoplastic cell, in-vitro or ex-vivo as defined herein, said method comprising the administration to a neoplastic cell of an effective amount of a pharmaceutical association, combination or composition as defined herein.

**Ex-vivo administration:** As used herein, unless indicated otherwise or contradictory in context, the term "ex-vivo administration" refers to performing the regulatory step outside of the subject/patient, such as administering a pharmaceutical association, combination or composition as defined herein to a population of cells (e.g., neoplastic cells) removed from a subject/patient for *e.g.* diagnostic, analysis and/or academic purposes.

**In-vivo administration:** In one example, pharmaceutical, prophylactic, diagnostic, or imaging associations, combinations or compositions are administered by one or more of a variety of routes, including oral, intravenous, intramuscular, intra-arterial, intramedullary, rectal, intravaginal, intrathecal, subcutaneous, intraventricular, transdermal, intradermal, intraperitoneal, topical (e.g. by ointments, creams, powders, lotions, gels, and/or drops), buccal, enteral, mucosal, nasal, vitreal, intratumoral, sublingual, by intra-tracheal instillation, bronchial instillation, and/or inhalation, as an oral spray, nasal spray, and/or aerosol, and/or through a portal vein catheter. In one example, pharmaceutical, prophylactic, diagnostic, or imaging associations, combinations or compositions are administered by systemic intravenous injection. In one example, pharmaceutical, prophylactic, diagnostic, or imaging associations, combinations or compositions may be administered in a way which allows them to cross the blood-brain barrier, vascular barrier, or other epithelial barrier. In one most particular example, pharmaceutical, prophylactic, diagnostic, or imaging associations, combinations or compositions may be administered locally by intratumoral administration.

**Delivery:** As used herein, unless indicated otherwise or contradictory in context, the term "delivery" refers to the act or manner of delivering a compound, substance, association, combination, composition, entity, moiety, cargo or payload.

**Delivery Agent:** As used herein, unless indicated otherwise or contradictory in context, the term "delivery agent" refers to any substance which facilitates, at least in part, the in vivo delivery of a pharmaceutical association, combination or composition defined herein to targeted cells.

**Forms suitable for oral administration:** A pharmaceutical association, combination or composition for uses and methods of the invention, for example, in certain embodiments, includes forms suitable for oral administration as a tablet, capsule, pill, powder, sustained release formulations, solution, suspension, or for parenteral injection as a sterile solution, suspension or emulsion. Pharmaceutical compositions suitable for the delivery of pharmaceutical associations or combinations defined herein and methods for their preparation will be readily apparent to those skilled in the art. Such compositions and methods for their preparation may be found, for example, in certain embodiments, in 'Remington's Pharmaceutical Sciences', 19th Edition (Mack Publishing Company, 1995), Oral administration may involve swallowing, so that the compounds or associations enters the gastrointestinal tract, or buccal or sublingual administration may be employed by which the compound enters the blood stream directly from the mouth. Formulations suitable for oral administration include solid formulations, such as tablets, capsules containing particulates, liquids, or powders; lozenges (including liquid-filled), chews; multi- and nano-particulates; gels, solid solution, liposome, films (including muco-adhesive), ovules, sprays and liquid formulations. Liquid formulations include suspensions, solutions, syrups and elixirs. Such formulations may be employed as fillers in soft or hard capsules and typically comprise a carrier, for example, in certain embodiments, water, ethanol, polyethylene glycol, propylene glycol, methylcellulose, or a suitable oil, and one or more emulsifying agents and/or suspending agents. Liquid formulations may also be prepared by the reconstitution of a solid, for example, in certain embodiments, from a sachet. The pharmaceutical associations defined herein may also be used in fast-dissolving, fast-disintegrating dosage forms such as those described in the art.

**Forms suitable for parenteral administration:** In one example, the pharmaceutical association, combination or composition for uses and methods of the invention may be administered by parenteral injection. Exemplary parenteral administration forms include sterile solutions, suspensions or emulsions of the pharmaceutical association defined herein in sterile aqueous media, for example, in certain embodiments, aqueous propylene glycol or dextrose. In another embodiment, the parenteral administration form is a solution. Such parenteral dosage forms can be suitably buffered, if desired. Preferred sterile solutions include sodium chloride, 0.9%, UPS solution. Injectable formulations can be sterilized, for example, in certain embodiments, by filtration through a bacterial- retaining filter, and/or by incorporating sterilizing agents in the form of sterile solid compositions which can be dissolved or dispersed in sterile water or other sterile injectable medium prior to use.

**Forms suitable for rectal and vaginal administration:** Compositions for rectal or vaginal administration are typically suppositories which can be prepared by mixing compositions with suitable non-irritating excipients such as cocoa butter, polyethylene glycol or a suppository wax which are solid at ambient temperature but liquid at body temperature and therefore melt in the rectum or vaginal cavity and release the active ingredient.

**Forms suitable for topical and/or transdermal administration:** Dosage forms for topical and/or transdermal administration of a composition may include ointments, pastes, creams, lotions, gels, powders, solutions, sprays, inhalants and/or patches. Generally, an active ingredient is admixed under sterile conditions with a pharmaceutically acceptable excipient and/or any needed preservatives and/or buffers as may be required.

**Forms suitable for pulmonary administration:** Dosage forms for pulmonary administration via the buccal cavity may comprise dry particles which comprise the active ingredient *(e.g.* the pharmaceutical association defined herein) and which have a diameter in the range from about 0.5 nm to about 7 nm. Such compositions are conveniently in the form of dry powders for administration using a device comprising a dry powder reservoir to which a stream of propellant may be directed to disperse the powder and/or using a self-propelling solvent/powder dispensing container such as a device comprising the active ingredient dissolved and/or suspended in a low-boiling propellant in a sealed container. Pharmaceutical compositions formulated for pulmonary delivery may provide an active ingredient in the form of droplets of a solution and/or suspension. Such formulations may be prepared, packaged, and/or sold as aqueous and/or dilute alcoholic solutions and/or suspensions, optionally sterile, comprising active ingredient, and may conveniently be administered using any nebulization and/or atomization device. Such formulations may further comprise one or more additional ingredients including, but not limited to, a flavoring agent such as saccharin sodium, a volatile oil, a buffering agent, a surface active agent, and/or a preservative such as methylhydroxybenzoate.

**Forms suitable for nasal administration:** Formulations described herein as being useful for pulmonary delivery are also useful for intranasal delivery of a pharmaceutical composition. Formulations suitable for nasal administration may, for example, in certain embodiments, comprise from about as little as 0.1% (w/w) and as much as 100% (w/w) of active ingredient *(e.g.* the pharmaceutical association defined herein), and may comprise one or more of the additional ingredients described herein. A pharmaceutical composition may be prepared, packaged, and/or sold in a formulation suitable for buccal administration. Such formulations may, for example, in certain embodiments, be in the form of tablets and/or lozenges made using conventional methods, and may, for example, in certain embodiments, 0.1 % to 20% (w/w) active ingredient, the balance comprising an orally dissolvable and/or degradable composition and, optionally, one or more of the additional ingredients described herein. Alternately, formulations suitable for buccal administration may comprise a powder and/or an aerosolized and/or atomized solution and/or suspension comprising active ingredient. Such powdered, aerosolized, and/or aerosolized formulations, when dispersed, may have an average particle and/or droplet size in the range from about 0.1 nm to about 200 nm, and may further comprise one or more of any additional ingredients described herein.

**Forms suitable for ophthalmic administration:** Dosage forms for ophthalmic administration include, for example, in certain embodiments, eye drops including, for example, in certain embodiments, a 0.1/1.0% (w/w) solution and/or suspension of the active ingredient *(e.g.* the pharmaceutical association defined herein) in an aqueous or oily liquid excipient. Such drops may further comprise buffering agents, salts, and/or one or more other of any additional ingredients described herein. Other opthalmically-administrable formulations which are useful include those which comprise the active ingredient in microcrystalline form and/or in a liposomal preparation. Ear drops and/or eye drops are contemplated as being within the scope of this present disclosure.

**Direct injection:** One preferred administration method for delivering a pharmaceutical association, combination or composition as defined herein is by local administration, in particular, by direct injection. Direct injection techniques are particularly useful for administering a composition to a cell or tissue that is accessible by surgery, and particularly, on or

near the surface of the body. Administration of a composition locally within the area of a target cell refers to injecting the composition centimeters and preferably, millimeters from the target cell or tissue.

**Dosage regimens:** The dosage regimen of the pharmaceutical associations or combinations and/or pharmaceutical compositions as defined herein may be adjusted to provide the optimum desired response. For example, in certain embodiments, a single bolus may be administered, several divided doses may be administered over time or the dose may be proportionally reduced or increased as indicated by the exigencies of the therapeutic situation. The appropriate dosing regimen, the amount of each dose administered and/or the intervals between doses will depend upon the pharmaceutical association being used, the type of pharmaceutical composition, the characteristics of the subject in need of treatment and the severity of the condition being treated. Thus, the skilled artisan would appreciate, based upon the disclosure provided herein, that the dose and dosing regimen is adjusted in accordance with methods well-known in the therapeutic arts. That is, the maximum tolerable dose can be readily established, and the effective amount providing a detectable therapeutic benefit to a patient may also be determined, as can the temporal requirements for administering each agent to provide a detectable therapeutic benefit to the patient. Accordingly, while certain dose and administration regimens are exemplified herein, these examples in no way limit the dose and administration regimen that may be provided to a patient in practicing the present invention. In general, pharmaceutical compositions in accordance with the present disclosure may be administered at dosage levels sufficient to deliver from about 0.0001 mg/kg to about 100 mg/kg, from about 0.01 mg/kg to about 50 mg/kg, from about 0.1 mg/kg to about 40 mg/kg, from about 0.5 mg/kg to about 30 mg/kg, from about 0.01 mg/kg to about 10 mg/kg, from about 0.1 mg/kg to about 10 mg/kg, or from about 1 mg/kg to about 25 mg/kg, of subject body weight per day, one or more times a day, to obtain the desired therapeutic, diagnostic, prophylactic, or imaging effect. The desired dosage may be delivered three times a day, two times a day, once a day, every other day, every third day, every week, every two weeks, every three weeks, or every four weeks. In certain embodiments, the desired dosage may be delivered using multiple administrations (e.g., two, three, four, five, six, seven, eight, nine, ten, eleven, twelve, thirteen, fourteen, or more administrations). It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the present invention. For example, in certain embodiments, doses may be adjusted based on pharmacokinetic or pharmacodynamic parameters, which may include clinical effects such as toxic effects and/or laboratory values. Thus, the present invention encompasses intra-patient dose-escalation as determined by the skilled artisan. Determining appropriate dosages and regiments for administration of the chemotherapeutic agent are well-known in the relevant art and would be understood to be encompassed by the skilled artisan once provided the teachings disclosed herein.

**Effective dose parameters:** The dosage regimen of the pharmaceutical associations or combinations and/or pharmaceutical compositions as defined herein may be adjusted to obtain effective dose parameters. Effective dose parameters can be determined using methods standard in the art for a particular disease or condition. In particular, the effectiveness of dose parameters of a therapeutic composition as defined herein when treating cancer can be determined by assessing response rates. Such response rates refer to the percentage of treated patients in a population of patients that respond with either partial or complete remission. Remission can be determined by, for example, in certain embodiments, measuring tumor size or microscopic examination for the presence of cancer cells in a tissue sample.

A pharmaceutical composition as defined herein may be prepared, packaged, or sold in bulk, as a single unit dose, or as a plurality of single unit doses.

**Unit dose:** As used herein, unless indicated otherwise or contradictory in context, the term "unit dose" refers to a discrete amount of the pharmaceutical composition comprising a predetermined amount of the active ingredient. The amount of the active ingredient is generally equal to the dosage of the active ingredient which would be administered to a subject or a convenient fraction of such a dosage such as, for example, in certain embodiments, one-half or one-third of such a dosage.

**Single unit dose:** As used herein, unless indicated otherwise or contradictory in context, the term "single unit dose" refers to a dose of any therapeutic association or composition administered in one dose/at one time/single route/single point of contact, *i.e.,* single administration event.

**Split dose:** As used herein, unless indicated otherwise or contradictory in context, the term "split dose" refers to the division of single unit dose or total daily dose into two or more doses.

**Total daily dose:** As used herein, unless indicated otherwise or contradictory in context, the term "total daily dose" refers to an amount given or prescribed in 24hr period. It may be administered as a single unit dose.

The relative amounts of the active ingredient(s), the pharmaceutically acceptable excipients, carriers or vehicles, and any additional ingredients in a pharmaceutical composition defined herein will vary, depending upon the identity, size, and condition of the subject treated and further depending upon the route by which the composition is to be administered. In addition to the active ingredient, a pharmaceutical composition of the invention may further comprise one or more additional pharmaceutically active agents.

**Combination therapy:** Compounds, associations, compositions or formulations defined herein may be used in

combination with one or more other therapeutic, prophylactic, diagnostic, or imaging agents. As used herein, the term "in combination with" is not intended to imply that the agents must be administered at the same time and/or formulated for delivery together, although these methods of delivery are within the scope of the present disclosure. Compositions can be administered concurrently with, prior to, or subsequent to, one or more other desired therapeutics or medical procedures. In some embodiments, they are administered within about 90, 60, 30, 15, 10, 5, or 1 minute of one another. In some embodiments, the administrations of the agents are spaced sufficiently closely together such that a combinatorial (e.g., a synergistic) effect is achieved. In general, each agent will be administered at a dose and/or on a time schedule determined for that agent. In one example, the present disclosure encompasses the delivery of pharmaceutical, prophylactic, diagnostic, or imaging compositions in combination with agents that improve their bioavailability, reduce and/or modify their metabolism, inhibit their excretion, and/or modify their distribution within the body. It will further be appreciated that therapeutically, prophylactically, diagnostically, or imaging active agents used in combination may be administered together in a single composition or administered separately in different compositions. In general, it is expected that agents used in combination with be used at levels that do not exceed the levels at which they are used individually. In one example, the levels used in combination will be lower than those utilized individually. The particular combination of therapies to employ in a combination regimen will take into account compatibility of the desired therapeutics and/or procedures and the desired therapeutic effect to be achieved. It will also be appreciated that the therapies employed may achieve a desired effect for the same disorder (For example, in certain embodiments, a composition useful for treating cancer in accordance with the present disclosure may be administered concurrently with a chemotherapeutic agent), or they may achieve different effects (e.g., control of any adverse effects).

Although the descriptions of pharmaceutical associations, combinations or compositions provided herein are principally directed to pharmaceutical associations, combinations or compositions which are suitable for administration to mammals, in particular humans, it will be understood by the skilled artisan that such compositions are generally suitable for administration to animals of all sorts, in particular to any member of the Vertebrate class. Modification of pharmaceutical compositions suitable for administration to humans in order to render the compositions suitable for administration to various animals is well understood, and the ordinarily skilled veterinary pharmacologist can design and/or perform such modification with merely ordinary, if any, experimentation. Subjects to which administration of the pharmaceutical compositions is contemplated include, but are not limited to, humans and/or other primates; mammals, including commercially relevant mammals such as cattle, pigs, horses, sheep, cats, dogs, mice, and/or rats; and/or birds, including commercially relevant birds such as chickens, ducks, geese, and/or turkeys.

## X. Dermatological applications

When the neoplastic disease to be prevented or treated involves neoplastic cells belonging to the skin cell lineage (mainly to the fibroblast lineage), the pharmaceutical composition defined herein may be a dermatological composition comprising a pharmaceutical association or combination of a GFR-binding compound and a bioactive carrier as all defined herein and at least one dermatologically acceptable excipient.

For example, in certain embodiments, a dermatological composition for the uses of the invention may contain between 0.01% and 100% by weight (over the total weight of the dermatological composition) of a GFR-binding compound or pharmaceutical association or combination, both as defined herein, as a dermatological effective amount. The dermatological composition particularly comprises between 0.01% and 95%, between 0.01% and 90%, between 0.01% and 85%, between 0.01% and 80%, between 0.01% and 75%, between 0.01% and 70%, between 0.01% and 65%, between 0.01% and 60%, between 0.01% and 55%, between 0.01% and 50%, between 0.01% and 45%, between 0.01% and 40%, between 0.01% and 35%, between 0.01% and 30%, between 0.01% and 25%, between 0.01% and 20%, between 0.01% and 15%, between 0.01% and 10%, between 0.01% and 5%, between 0.1% and 100%, between 0.1% and 95%, between 0.1% and 90%, between 0.1% and 85%, between 0.1% and 80%, between 0.1% and 75%, between 0.1% and 70%, between 0.1% and 65%, between 0.1% and 60%, between 0.1% and 55%, between 0.1% and 50%, between 0.1% and 45%, between 0.1% and 40%, between 0.1% and 35%, between 0.1% and 30%, between 0.1% and 25%, between 0.1% and 20%, between 0.1% and 15%, between 0.1% and 10%, and between 0.1% and 5% by weight (over the total weight of the dermatological composition) of any one of a GFR-binding compound or a pharmaceutical association or combination.

**Dermatologically acceptable:** As used herein, unless indicated otherwise or contradictory in context, the term "dermatologically acceptable" means that the compound(s) or pharmaceutical association(s) used are adapted for use in contact with human skin without undue toxicity, incompatibility, instability, allergic response, or their equivalents.

**Dermatological formulations:** Suitable formulation for implementing dermatological embodiments of the invention include an aqueous or oil-based solution, a water-based cream or gel or an oily gel, usually in a jar or a tube, particularly a shower gel, shampoo, milk, emulsion, microemulsion or nanoemulsion, particularly oil-in-water or water-in-oil or multiple of silicone-based; a lotion, particularly in a glass or plastic bottle of a spray or aerosol bottle, a blister-pack, liquid soap, a dermatological bar of soap, a pomade, mousse, an anhydrous product, preferably liquid, cream or solid, for

example in the form of a stick, particularly in the form of lipstick, a cataplasm or a patch.

Preferred administration routes include, but are not limited to, topical, intradermal and intra-tumoral as already defined herein.

**Dermatologically acceptable excipients:** Suitable dermatologically acceptable excipients for implementing embodiments of the invention include, but are not limited to, preservatives, emollients, emulsifiers, surfactants, moisturizers, thickeners, conditioners, mattifying agents, stabilizers, antioxidants, texturizing agents, shine agents, filmogenic agents, solubilizers, pigments, colorants, perfumes, and solar filters. These excipients are preferably chosen from among the group consisting of amino acids and their derivatives, polyglycerols, esters, polymers and cellulose derivatives, lanoline derivatives, phospholipids, lactoferrins, lactoperoxidases, sucrose-based stabilizers, vitamin E and its derivatives, natural and synthetic waxes, vegetable oils, triglycerides, insaponifiables, phytosterols, plant esters, silicones and their derivatives, protein hydrolysates, jojoba oil and its derivatives, lipo/hydrosoluble esters, betaines, aminoxides, saccharose ester plant extracts, titanium dioxides, glycines, parabens, and even more preferably from among the group consisting of butylene glycol, glycol-15 stearyl ether, cetearyl alcohol, phenoxyethanol, methylparaben, propylparaben, butylparaben, butylenes glycol, natural tocopherols, glycerine, dihydroxycetyl sodium phosphate, isopropyl hydroxycetyl ether, le glycol stearate, triisononanoin, octyl cocoate, polyacrylamide, isoparaffin, laureth-7, carbomer, propylene glycol, glycerol, bisabolol, dimethicone, sodium hydroxide, PEG 30-dipolyhydroxysterate, capric/caprylic triglycerides, cetearyl octanoate, dibutyl adipate, grapeseed oil, jojoba oil, magnesium sulfate, EDTA, cyclomethicone, xanthan gum, citric acid, sodium lauryl sulfate, mineral waxes and oils, isostearyl isostearate, dipelargonate of propylene glycol, isostearate of propylene glycol, PEG 8, beeswax, glyceride of hydrogenated palm kernel oil, lanolin oil, sesame oil, cetyl lactate, lanolin alcohol, titanium dioxide, lactose, saccharose, low-density polyethylene, and isotonic salt solution.

In one example, the dermatological composition as defined herein may contain at least one other active agents and/or excipients and/or additives of pharmaceutical, especially dermatological, interest such as agents with the following properties:

- wound-healing properties; such as panthenol and derivatives thereof, for example ethyl panthenol, aloe vera, pantothenic acid and derivatives thereof, allantoin, bisabolol, and dipotassium glycyrrhizinate;
- anti-inflammatory properties: such as steroidal and non-steroidal antiinflammatories, in particular Inhibitors of the production of cytokines and chemokines, of cyclooxygenase, of nitric oxide (NO) and nitric oxide synthase (NOS). As an example of anti-inflammatory products, mention may be made of extracts of Ginkgo biloba, trilactone terpenes such as ginkgolides, especially ginkgolide B and bilobalide known for their platelet- activating factor (PAF) antagonist properties.

The CTFA Cosmetic Ingredient Handbook, Second Edition (1992), describes different cosmetic and pharmaceutical ingredients currently used in the cosmetic and pharmaceutical industry that are particularly adapted to topical use and which may be used in a dermatological composition of the invention. Examples of these types of ingredients include but are not limited to the following compounds: abrasives, absorbent compounds, compounds with aesthetic purposes such as perfumes, pigments, colorants, essential oils, astringents, etc. (for example: clove oil, menthol, camphor, eucalyptus oil, eugenol, menthyl lactate, and hamelis distillate), anti-acne agents, anti-flocculant agents, anti-foaming agents, anti-microbial agents (for example iodopropyl butylcarbamate), les antioxidants, bonding agents, biological additives, tampon agents, swelling agents, chelatants, additives, biocidal agents, denaturants, external analgesics, film-forming materials, polymers, opacifying agents, pH adjusters, reducing agents, depigmenting or lightening agents (for example: hydroquinone, kojic acid, ascorbic acid, magnesium ascorbyl phosphate, ascorbyl glucosamine), conditioning agents (for example: humectants), calming agents for the skin and/or scarring agents (for example: panthenol and its derivatives, for example ethyl panthenol), aloe vera, pantothenic acid and its derivatives, allantoin, bisabolol and dipotassium glycyrrhizinate), thickeners, vitamins, and the derivatives or equivalents of these.

In one particular example, the pharmaceutical association or combination as defined herein or a dermatological composition as defined herein for use in protecting (i.e. preventing and/or treating) a subject or patient from a skin neoplastic disease, disorder or condition, in particular, but not limited to, basal and squamous cell skin cancers, melanoma skin cancer, Merkel cell carcinoma, lymphoma of the skin and Kaposi sarcoma.

Suitable as amounts of pharmaceutical association for implementing embodiments of the invention in the dermatological field include the group consisting of between about 0.0001 $\mu$g/day to about 5000 mg/day, between about 0.0001 $\mu$g/day to about 1000 mg/day, between about 0.0001 $\mu$g/day to about 10 mg/day, between about 0.0001 $\mu$g/day to about 1 mg/day, or between about 0.0001 $\mu$g/day to about 100 $\mu$g/day, all being preferred for implementing embodiments of the invention.

Advantageously, the subject who has need thereof is a subject chosen from a population having an average age of more than 30 years old or who has had sunlight over-exposure, has a family history of skin cancer, has or has had certain other skin conditions or previous radiotherapy, has been exposed to certain chemicals and has a weakened immune system.

## XI. Ophthalmic applications

Preferable dosage forms for the pharmaceutical association, combination or composition as defined herein for treating ophthalmic neoplastic diseases, disorders or conditions include, for example, in certain embodiments, eye drops and eye ointments. These can be prepared using conventional techniques. For instance, eye drops may be prepared, using isotonic agents such as sodium chloride, buffers such as sodium phosphate, and preservatives such as benzalkonium chloride. A suitable pH is within an ophthalmologically acceptable range. Preferred pH is within pH 4 to 8.
Particularly preferred administration routes include vitreal, intraocular and intra-tumoral.
A suitable dose of pharmaceutical association, combination or composition for treating eye disorders is appropriately selected, depending on the symptoms, age of patients, dosage form and the like. For eye drops, suitable concentration may be 0.0001 to 10 w/v %, preferably 0.0001 to 0.01 w/v % for administration into eyes once or several times a day.

## XII. Surgical treatments

In one particular example, the pharmaceutical association, combination or composition as defined herein may be used in a surgical method suitable for treating or preventing a neoplastic disease such as a tumour or a cancer.
For example, in certain embodiments, the surgical treatment of the invention may include the provision of a placement, insertion or depositing device and the contacting of said pharmaceutical association, combination or composition with a body part of a patient using said placement, insertion or depositing device.
In one example, said placement, insertion or depositing device comprises an injection device such as a syringe, and comprises the positioning of said pharmaceutical association, combination or composition inside said injection device for injection into a subject/patient or into a body part of a subject/patient.

## XIII. Pharmaceutical applications, uses and methods

The present invention provides for uses of converting or inducing the conversion or the recoding of a neoplastic cell *(e.g.* a cancer cell) into a non-neoplastic cell *(e.g.* a non-cancerous cell) through extracellular, non-mutagenic, conversion or recoding of said neoplastic cell. In other words, the present disclosure provides uses for a neoplastic cell to perform self-healing or self-recovery into a more functional, healthy, non-neoplastic cell.
Conveniently, such a self-healing process is generally achieved within less than 7 days. In particular, such a self-healing process is generally achieved within less than 6 days. In particular, such a self-healing process is generally achieved within less than 5 days. In particular, such a self-healing process is generally achieved within less than 4 days. In particular, such a self-healing process is generally achieved within less than 3 days. In particular, such a self-healing process is generally achieved within less than 2 days. In particular, such a self-healing process is generally achieved within less than 24 hours. In particular, such a self-healing process is generally achieved within less than 18 hours.
Conveniently, such a self-healing process is achieved with a cell conversion yield greater than about 50%. In particular, such a self-healing process is achieved with a cell conversion yield greater than about 60%. In particular, such a self-healing process is achieved with a cell conversion yield greater than about 70%. In particular, such a self-healing process is achieved with a cell conversion yield greater than about 80%. In particular, such a self-healing process is achieved with a cell conversion yield greater than about 90%. In particular, such a self-healing process is achieved with a cell conversion yield greater than about 95%. In particular, such a self-healing process is achieved with a cell conversion yield is about 100%.
**Cell conversion yield:** As used herein, "cell conversion yield" means the percentage of neoplastic cells that are transformed into non-neoplastic cells. It is considered significant when it is higher than 10%. There are many ways to measure a cell conversion yield, but for the purpose of the present disclosure, and for the avoidance of any doubts, the cell conversion yield is herein measured by using a haemocytometer for precise cell counting using, for instance, a haemocytometer from Baxter Scientific and following the standard procedure below:

(a) Cleaning the chamber and cover slip with alcohol. Drying and fixing the coverslip in position.
(b) Harvesting the cells. Adding 10 μL of the cells to the haemocytometer.
(c) Placing the chamber in the inverted microscope under a 10X objective. Using phase contrast to distinguish the cells.
(d) Counting the cells in the large, central gridded square (1 $mm^2$). The gridded square is circled in the graphic below. Multiplying by $10^4$ to estimate the number of cells per mL.
(e) Preparing duplicate samples and averaging the count.

In certain embodiments, the present disclosure provides uses that converts a neoplastic cell into a non-neoplastic cell, wherein said obtained neoplastic cell is homogeneous and/or of substantially the same differentiation state. Conveniently, the converted non-neoplastic cells obtained using a method as defined herein, have a homogeneity of greater than 20%. In particular, the converted non-neoplastic cells obtained using a method as defined herein, have a homogeneity of greater than 50%. In particular, the converted non-neoplastic cells obtained using a method as defined herein, have a homogeneity of greater than 70%. In particular, the converted non-neoplastic cells obtained using a method as defined herein, have a homogeneity of greater than 90%. In particular, the converted non-neoplastic cells obtained using a method as defined herein, have a homogeneity of greater than 99%.

**Homogeneous:** As used herein, "homogeneous", when used in relation to a non-neoplastic cell population obtained using methods as defined herein, means that substantially all of the obtained non-neoplastic cells within the population are in a G0 phase. There are many ways to test and measure the homogeneity of a cell population, but for the purpose of the present disclosure, and for the avoidance of any doubts, the cell homogeneity is herein measured using cell immunofluorescence staining by detecting phosphorylation of the Rb protein, an absence of phosphorylation meaning that substantially all cells are in a G0 phase.

Conveniently, the converted non-neoplastic cells obtained using a method as defined herein, are more than 20% identical. In particular, the converted non-neoplastic cells obtained using a method as defined herein, are more than 50% identical. In particular, the converted non-neoplastic cells obtained using a method as defined herein, are more than 70% identical. In particular, the converted non-neoplastic cells obtained using a method as defined herein, are more than 90% identical. In particular, the converted non-neoplastic cells obtained using a method as defined herein, are more than 99% identical.

As used herein, "substantially the same differentiation state" or "substantially identical differentiation state", when used in relation to non-neoplastic cells obtained using methods as defined herein, means exhibiting the same gene expression pattern. There are many ways to test and measure the differentiation state of a cell and group of cells, but for the purpose of the present disclosure, and for the avoidance of any doubts, the differentiation state of a cell or a group of cell is herein measured using RT-PCR for the quantification of the expression of well-defined marker genes for the particular differentiation state.

In certain aspects, a pharmaceutical association, combination or composition as defined herein is thus useful in the protection of a subject/patient from *(e.g.* in the treatment and/or prevention of) a neoplastic disease, condition, disorder or pathology and/or at least one symptom thereof such as tumors and cancers. In one aspect, the present disclosure provides a pharmaceutical association, combination or (therapeutic, dermatologic, ophthalmologic, diagnostic, etc.) composition defined herein for use in the treatment of a neoplastic disease, condition, disorder or pathology and/or at least one symptom thereof, said composition comprising a GFR-binding compound and a bioactive carrier, all as already defined herein.

In one example, the conversion or recoding of the neoplastic cell into a non-neoplastic cell is substantially permanent. As used herein, the term "permanent", when used in relation to the conversion or recoding of a neoplastic cell into a non-neoplastic cell, means physiologically permanent conversion as the neoplastic cell treated by the method of the invention then becomes a normal, functional, healthy cell just like any other non-neoplastic cell of the subject's body and is thus not prevented to develop a new neoplastic state or any other abnormal state in the future (like any normal cell could/would).

Also provided herein is a pharmaceutical association, combination or (therapeutic, dermatologic, ophthalmologic, diagnostic, etc.) composition defined herein for use in a method of restoring the ability of a neoplastic cell to undergo differentiation.

Also provided herein is a pharmaceutical association, combination or (therapeutic, dermatologic, ophthalmologic, diagnostic, etc.) composition defined herein for use in a method of converting and/or recoding a circulating or non-circulating neoplastic cell such as a metastatic or non-metastatic cancer cell, into a non-neoplastic cell.

Also provided herein is a pharmaceutical association, combination or (therapeutic, dermatologic, ophthalmologic, diagnostic, etc.) composition defined herein for use in a method of providing and/or producing and/or inducing the formation of a physiologically functional and/or healthy cell of the bone, cartilage, vascular, blood, fibroblast, muscle, neural, epithelial, renal, retinal cell lineage from a neoplastic cell.

Also provided herein is a pharmaceutical association, combination or (therapeutic, dermatologic, ophthalmologic, diagnostic, etc.) composition defined herein for use in a method of inducing and/or promoting and/or enhancing neoplastic cell differentiation.

Also provided herein is a pharmaceutical association, combination or (therapeutic, dermatologic, ophthalmologic, diagnostic, etc.) composition defined herein for use in a method of non-mutagenically protecting a subject from a neoplastic disease i.e. without modifying or altering the genome of the treated neoplastic cells.

Also provided herein is a pharmaceutical association, combination or (therapeutic, dermatologic, ophthalmologic, diagnostic, etc.) composition defined herein for use in a method of extracellular treatment of a neoplastic disease, disorder, condition, pathology, or any symptoms thereof.

An extracellular treatment as used herein implies a biological action/effect to be provided or to occur outside the

cell to be treated (*i.e.* a neoplastic cell). In other words, the biologically active agent *(e.g.* the pharmaceutical association, combination or composition as defined herein) delivers/provides its biological/pharmaceutical effect to the outside of the cell *(e.g.* on the cell's surface) without the need to penetrate through the cell membrane, inside the neoplastic cell to be treated. Once the extracellular action/effect has been administered/delivered to the cell to be treated, said active agent may be, for instance, excreted from the host organism with or without being metabolised, and/or tagged to be destroyed through apoptotic routes, and/or internalised by nearby cells, etc... Without being bound to any specific theory, it is believed that once the extracellular biological action/effect/message/signal has been delivered by the pharmaceutical association or composition defined herein to a neoplastic cell to be treated, said cell then undergo self-healing into a functional, healthy, non-neoplastic cell.

Data have shown (FIGURE 8) that the administration or action of the pharmaceutical association or composition as defined herein on neoplastic cells to be treated, implicates and down-regulates (in particular, reduces, substantially reduces or suppress) the Ras/MAP kinase, FAK/Src kinase and/or PIP2 signalling pathways. Without wishing to be bound to any specific theory, it is conventionally known that these pathways control the expression of cyclins D, which in turn controls the activity of CDKs 4 and/or 6 so that down-regulating (in particular, reducing, significantly reducing or suppressing) the Ras/MAP kinase, FAK/Src kinase and/or PIP2 pathways would inhibit, reduce, damper or suppress the expression of cyclins D and/or CDK4 and CDK6 and/or the formation of the cyclins D-CDK4/6 complexes.

As already stated herein, cell cycle comprises four main phases: The S phase for DNA duplication, followed by a G2 phase for preparation of the entry into the M phase, the M or mitotic phase wherein the cell divides and finally the G1 phase for cell growth. During the G1 phase the cell makes decisions on whether to continue the cell cycle and undergo cell growth and further divide, or to exit the cycle in the G0 phase and remains quiescent, die or initiate differentiation. The progression through the cell cycle is governed by phosphorylation signals emitted by different bimolecular complexes composed of CDKs and cyclin proteins, both as defined herein.

The cyclin D-CDK4/6 complexes are capable of leading a cell through the restriction (R)-point which is generally known for controlling the passage of the cell from the G1 phase to the S phase of the cell cycle. This control gate is generally thought to be located at the end of the cell cycle's G1 phase, just before entry into S phase, and is involved in the decision of whether the cell should divide or proliferate, delay division, or enter the G0 resting state.

This control gate ensures that a series of surveillance or monitoring mechanisms are completed successfully before proceeding through to the next phase. These monitoring mechanisms are also commonly termed "checkpoints" or "checkpoint controls". If a checkpoint is not validated by the control gate, the cell should halt further advance through the cell cycle and enter the G0 phase. However, neoplastic cells, such as cancer cells, have developed mechanisms that somehow "disconnect" or "impair" one or more checkpoints from the control gate so that the cell is "tricked to "believe that all checkpoints are validated (or, in other words, "tricked to ignore" the non-validated status of relevant checkpoints) and thus never or rarely enter into the G0 phase.

Without wishing to be bound to any specific theory, it is thought that the pharmaceutical associations, combination or composition as defined herein can restore or re-establish (the integrity of) one or more impaired cell cycle checkpoints so as to restore the lost ability of a neoplastic cell to detect a malfunction or a defect in the cell cycle regulation, induce the cell cycle arrest and exit the cell cycle in G0.

One particular checkpoint that the present invention is able to restore is the adhesion checkpoint. In one aspect, the present disclosure provides a pharmaceutical association, combination or (therapeutic, dermatologic, ophthalmologic, diagnostic, etc.) composition defined herein for use in a method of treating a neoplastic cell or a neoplastic disease by restoring the adhesion checkpoint of said neoplastic cell.

The cell adhesion checkpoint is known to be in charge of monitoring the cells attachments to the extracellular matrix (ECM). When a cell does not detect a "correct" attachment it should halt further advance through the cell cycle and enter the Go phase. The ECM attachment is normally achieved via cell transmembrane proteins, such as the integrins, syndecans and different proteoglycans. The most important among these are the integrins, which assemble as alpha-beta heterodimers. In addition to physically linking the cell to the extracellular matrix, the binding of the extracellular integrin domains to specific components of the ECM activates the integrins and allows binding of different signalling molecules to its intracellular domain. This activates various signalling pathways that mediate signals to the adhesion checkpoint including the Ras/MAP kinase, FAK/Src kinase, and PIP2 pathways. These pathways are involved in the regulation of the expression of cyclins D, which in turn controls the activity of CDK4/6. The cyclins D-CDK4/6 complexes are capable of leading the cell through the R-point gate.

Data have also shown (FIGURES 8 and 9) that the administration or action of the pharmaceutical association or composition as defined herein on neoplastic cells to be treated down-regulated (in particular, substantially reduced) the expression of FAK genes and proteins and/or MAP kinase and/or reduced (in particular, substantially reduced) the expression or activity of GTPase Ras, Rho (Rho, Rac, Cdc42).

Focal Adhesion Kinase (FAK) is a cytoplasmic tyrosine kinase that plays a role in integrin-mediated signal transductions and also participates in signalling via other cell surface receptors. FAK is typically located in structures known as focal adhesions. These are multi-protein structures that link the extracellular matrix (ECM) to the cytoplasmic

cytoskeleton. FAK is known to be phosphorylated in response to integrin engagement, growth factor stimulation, and the action of mitogenic neuropeptides. This cytosolic kinase has been reported to participate to diverse cellular mechanisms including cell locomotion, mitogen response and cell survival. FAK has four defined regions, or tertiary structure domains. Two of these domains, the N-terminal FERM domain and the Kinase domain, form an auto-inhibitory interaction. This interaction is thought to be the result of hydrophobic interactions between the two domains and prevents the activation of the Kinase domain, thereby preventing the signalling function of FAK. Release of this auto-inhibitory interaction has been shown to occur within focal adhesions but not in the cytoplasm and therefore is thought to require interaction with focal adhesion proteins.

MAP kinase Mitogen-activated protein kinases or MAP kinases (or MAPK) include a large group of related serine/threonine eukaryotic protein kinases whose regulatory cell functions include proliferation, gene expression, differentiation, mitosis, cell survival, and apoptosis. All have been reported to become activated when they have been phosphorylated at a Tyrosine and a Threonine amino acid. MAP kinases are catalytically inactive in their basic form. In order to become active, (potentially multiple) phosphorylation events is required to occur in their activation loops. MAP kinases typically form multistep pathways, receiving input signals at several levels above the actual MAP kinase. These pathways can effectively convey stimuli from the cell membrane to the nucleus or to many other subcellular targets.

GTPases (singular GTPase) are a large family of hydrolase enzymes that can bind and hydrolyze guanosine triphosphate (GTP). The GTP binding and hydrolysis takes place in the highly conserved G domain common to all GTPases. The hydrolysis of the $\gamma$ phosphate of GTP into guanosine diphosphate (GDP) and Pi, inorganic phosphate, occurs by the SN2 mechanism of nucleophilic substitution via a pentavalent intermediate state and is dependent on the magnesium ion $Mg^{2+}$. Regulatory GTPases, also called the GTPase superfamily, are GTPases used for regulation of other biochemical processes. Most prominent among the regulatory GTPases are the G proteins. Small GTPases have a molecular weight of about 21 kDa and generally serve as molecular switches for a variety of cellular signalling events.

According to their primary amino acid sequences and biochemical properties, the Ras superfamily is further divided into five subfamilies: Ras, Rho, Rab, Arf and Ran. The Rho subfamily is further divided into RHOA, RAC1, and CDC42.

Ras/MAP kinase signalling pathway (also known as the Ras-Raf-MEK-ERK pathway) is a chain of proteins in the cell that communicates a signal from a receptor on the surface of the cell to theDNA in the nucleus of the cell. The signal starts when a signalling molecule binds to the receptor on the cell surface and ends when the DNA in the nucleus expresses a protein and produces some change in the cell, such as cell division. The pathway includes many proteins, including MAPK (mitogen-activated protein kinases, originally called ERK, extracellular signal-regulated kinases), which communicate by adding phosphate groups to a neighbouring protein.

The FAK/Src kinase signalling pathway involves focal adhesion kinase (FAK) and steroid receptor coactivator (Src) which are intracellular (non-receptor) tyrosine kinases that physically and functionally interact to promote a variety of cellular responses. The linked activities of these two kinases are a common intracellular point of convergence in the signalling initiated by the integrin-extracellular matrix (ECM) interaction. Integrins, a family of transmembrane receptors, interact with the ECM and the intracellular actin-cytoskeleton. The integrins cluster upon binding to the ECM to form focal adhesion (FA) contacts. In response to this clustering, FAK associates to the cytoplasmic tail of the integrins and undergoes phosphorylation at its tyrosine 397 residue (Y397). This phosphorylated tyrosine provides a docking site for Src which is then able to phosphorylate additional sites on FAK, leading to a further increase in FAK activity and allowing the recruitment of proteins that contain Src homology 2 (SH2) domains such as Grb2 and PI3K.The mutually activated FAK/Src complex then initiates a cascade of phosphorylation events of new protein-protein interactions to trigger several signalling pathways that eventually leads to different cellular responses.

Phosphatidylinositol 4,5-bisphosphate or PIP2 is a minor phospholipid component of cell membranes. PIP2 is enriched at the plasma membrane where it is a substrate for a number of important signalling proteins. PIP2 is phosphorylated by the Class I PI 3-kinases. Class I PI 3-kinases are a subgroup of the enzyme family, phosphoinositide 3-kinase that possess a common protein domain structure, substrate specificity, and method of activation. Class I PI 3-kinases are further divided into two subclasses, class IA PI 3-kinases and class IB PI 3-kinases. Class IA PI 3-kinases are activated by receptor tyrosine kinases (RTKs). Class IB PI3-kinases are activated by G-protein-coupled receptors (GPCRs). After their activation these kinases phosphorylate PIP2 to form phosphatidylinositol (3,4,5)-trisphosphate PIP3. Both PIP2 and PIP3 not only act as substrates for enzymes but also serve as docking phospholipids that bind specific domains that promote the recruitment of proteins to the plasma membrane and subsequent activation of signalling cascades. PIP3 functions to activate downstream signalling components, the most notable one being the protein kinase AKT, which activates downstream anabolic signalling pathways required for cell growth and survival.

Also provided herein is a pharmaceutical association, combination or (therapeutic, dermatologic, ophthalmologic, diagnostic, etc.) composition defined herein for use in a method of treating a neoplastic cell or a neoplastic disease by down-regulating (in particular, substantially reducing) the expression of FAK genes and proteins and/or MAP kinase and/or reducing (in particular, substantially reducing) the expression or activity of GTPase Ras, Rho (Rho, Rac, Cdc42) in-vitro or in-vivo.

Also provided herein is a pharmaceutical association, combination or (therapeutic, dermatologic, ophthalmologic, diagnostic, etc.) composition defined herein for use in a method of converting a neoplastic cell into a non-neoplastic cell or treating/preventing a neoplastic disease by substantially inhibiting, down-regulating or dampening the gene expression or formation of at least one of, in particular a plurality of, the cyclin D proteins.

Also provided herein is a pharmaceutical association, combination or (therapeutic, dermatologic, ophthalmologic, diagnostic, etc.) composition defined herein for use in a method of converting a neoplastic cell into a non-neoplastic cell or treating/preventing a neoplastic disease by substantially inhibiting the formation of at least one of, in particular a plurality of, the protein complexes comprising at least one cyclin D and at least one CDK4 or CDK6.

Also provided herein is a pharmaceutical association, combination or (therapeutic, dermatologic, ophthalmologic, diagnostic, etc.) composition as defined herein for use in a method of converting a neoplastic cell into a non-neoplastic cell or treating/preventing a neoplastic disease without inducing the death of said neoplastic cell.

Also provided herein is a pharmaceutical association, combination or (therapeutic, dermatologic, ophthalmologic, diagnostic, etc.) composition as defined herein for use as a G0 cell cycle phase inducer.

Also provided herein is a pharmaceutical association, combination or (therapeutic, dermatologic, ophthalmologic, diagnostic, etc.) composition for use in a method of converting a neoplastic cell into a non-neoplastic cell or treating/preventing a neoplastic disease by dampening or arresting cell division and/or cell proliferation of said neoplastic cell.

Also provided herein is a pharmaceutical association, combination or (therapeutic, dermatologic, ophthalmologic, diagnostic, etc.) composition for use in a method of converting a neoplastic cell into a non-neoplastic cell or treating/preventing a neoplastic disease by activating and/or promoting anti-mitogen activity and/or tumor suppressor pathways and/or anti-oncogenic activity in said neoplastic cell.

Data have also shown (FIGURE 9) that the administration or action of the pharmaceutical association, combination or composition as defined herein on neoplastic cells to be treated, down-regulated (in particular, substantially reduced) the expression of paxillin and/or up-regulated (in particular, substantially increased) the expression of vinculin.

Paxillin is a signal transduction adaptor protein. The C-terminal region of paxillin contains four LIM domains that target paxillin to focal adhesions through a direct association with the cytoplasmic tail of beta-integrin. The N-terminal region of paxillin is rich in protein-protein interaction sites. The proteins that bind to paxillin are diverse and include protein tyrosine kinases, such as Src and focal adhesion kinase (FAK), structural proteins, such as vinculin and actopaxin, and regulators of actin organization, such as COOL/PIX and PKL/GIT. Paxillin is tyrosine-phosphorylated by FAK and Src upon integrin engagement or growth factor stimulation, creating binding sites for the adapter protein Crk.

Vinculin is a focal adhesion protein that is involved in linkage of integrin adhesion molecules to the actin cytoskeleton. Vinculin is a cytoskeletal protein associated with cell-cell and cell-matrix junctions, where it is thought to function as one of several interacting proteins involved in anchoring F-actin to the membrane. Vinculin is a 117-kDa cytoskeletal protein of 1066 amino acids. The protein contains an acidic N-terminal domain and a basic C-terminal domain separated by a proline-rich middle segment. Vinculin consists of a globular head domain that contains binding sites for talin and $\alpha$-actinin as well as a tyrosine phosphorylation site, while the tail region contains binding sites for F-actin, paxillin, and lipids.

Also provided herein is a pharmaceutical association, combination or (therapeutic, dermatologic, ophthalmologic, diagnostic, etc.) composition defined herein for use in a method of treating a neoplastic cell or a neoplastic disease by down-regulating the expression of paxillin and/or (preferably and) up-regulating the expression of vinculin in-vitro or in-vivo.

Data have also shown (FIGURES 3 to 6) that the administration or action of the pharmaceutical association, combination or composition as defined herein on neoplastic cells to be treated, up-regulated (in particular, substantially increased) the expression of at least one differentiation genes and proteins such as ADIPOQ (ACRP30), FABP4 and/or PPARG for adipocytes; ACAN (AGC1), COL10A1, COMP, Sox9, IBSP (Sialoprotein II) and/or COL4 for chondrocytes; CDH5, KDR (VEGFR3) and/or PECAM1 for the general endothelium; DLL4, EFNB2 and/or NRP1 for the arterial endothelium; LYVE1 and/or PROX1 for the lymphatic endothelium; NR2F2 and/or NRP2 for the venous endothelium; KRT1, KRT10 and/or KRT14 for the keratinocyte epithelium; PMEL (SILV), TYR and/or TYRP1 for the melanocyte epithelium; MMP-9, $\alpha$-SMA and/or Vimentin for the fibrocyte; BGLAP, COL2A1, IBSP, RANK-L, OCN, DMP-1, Sclerostin and/or MEPE for Osteoblasts/Osteocytes; CALCR and/or CTSK for Osteoclasts; ITGB4 and/or KRT19 for cholangiocytes; ALB, G6PC and/or TAT for Hepatocytes; CD79A for early B-cell development; CD3E and/or PTCRA for early T-cell development; CCR5, CXCR4 and/or EMR1 for macrophages; ITGAM for the monocytes; GALC and/or GFAP for glial cells; MAP2, NEFH and/or TUBB3 for mature neurons; CHAT for cholinergic neurons; TH for dopaminergic neurons; GAD1 and/or SLC32A1 for GABA neurons; SLC17A6 and/or SLC17A7 for glutamatergic neurons; ISL1 for motor neurons; MBP for oligodendrocytes; POU4F2 for ganglion cells; RLBP1 for Muller cells; PDE6B and/or RCVRN for photoreceptor cells; NPHS2 for podocytes; AQP1, CYP27B1 and/or MIOX for proximal tubule cells; AQP2 for collecting duct cells; UMOD for distal tubule cells; SFTPB, SFTPC and/or SFTPD for lung cells; MYH6, MYH7 and/or NPPA for cardiomyocytes; CAV3, MYH1, MYOD1, GATA4 and/or MLC1 for skeletal muscle cells; MYH11, SMTN and/or TAGLN for smooth muscle cells;

GCG, MAFB and/or POU3F4 for alpha cells; INS, MAFA and/or SLC2A2 for beta cells; SST for delta cells; GHRL (Ghrelin, Obestatin) for epsilon cells; PPY for pancreatic polypeptide producing (PP) cells; and/or CPA1 for exocrine cells.

Also provided herein is a pharmaceutical association, combination or (therapeutic, dermatologic, ophthalmologic, diagnostic, etc.) composition defined herein for use in a method of treating a neoplastic cell or a neoplastic disease by up-regulating (in particular, substantially increasing) the expression of differentiation genes and proteins such as ADIPOQ (ACRP30), FABP4, PPARG, ACAN (AGC1), COL10A1, COMP, Sox9, IBSP (Sialoprotein II), COL4, CDH5, KDR (VEGFR3), PECAM1, DLL4, EFNB2, NRP1, LYVE1, PROX1, NR2F2, NRP2, KRT1, KRT10, KRT14, PMEL (SILV), TYR, TYRP1, MMP-9, $\alpha$-SMA, Vimentin, BGLAP, COL2A1, IBSP, RANK-L, OCN, DMP-1, Sclerostin, MEPE, CALCR, CTSK, ITGB4, KRT19, ALB, G6PC, TAT, CD79A, CD3E, PTCRA, CCR5, CXCR4, EMR1, ITGAM, GALC, GFAP, MAP2, NEFH, TUBB3, CHAT, TH, GAD1, SLC32A1, SLC17A6, SLC17A7, ISL1, MBP, POU4F2, RLBP1, PDE6B, RCVRN, NPHS2, AQP1, CYP27B1, MIOX, AQP2, UMOD, SFTPB, SFTPC, SFTPD, MYH6, MYH7, NPPA, CAV3, MYH1, MYOD1, GATA4, MLC1, MYH11, SMTN, TAGLN, GCG, MAFB, POU3F4, INS, MAFA, SLC2A2, SST, GHRL (Ghrelin, Obestatin), PPY and/or CPA1, in-vitro or in-vivo.

Data have also shown (FIGURE 7) that the administration or action of the pharmaceutical association, combination or composition as defined herein on neoplastic cells to be treated increased the phosphorylation of the protein p53.

Data have also shown (FIGURE 7) that the administration or action of the pharmaceutical association, combination or composition as defined herein on neoplastic cells to be treated down-regulated (in particular, substantially decreased or suppressed) the phosphorylation of the protein pRb.

Data have also shown (FIGURE 8) that the administration or action of the pharmaceutical association, combination or composition as defined herein on neoplastic cells to be treated down-regulated the protein Src.

Tumor protein p53, also known as p53, cellular tumor antigen p53, phosphoprotein p53, or tumor suppressor p53, is a protein that in humans is encoded by the *TP53* gene. The p53 protein is involved in multicellular organisms, where it regulates the cell cycle and, thus, functions as a tumor suppressor, generally preventing neoplastic diseases such as cancers. As such, p53 has been described as "the guardian of the genome" because of its role in conserving stability by preventing genome mutation. *TP53* is thus said to be a tumor suppressor gene.

Src is a non-receptor protein tyrosine kinase that plays a multitude of roles in cell signalling. Src is involved in the control of many functions, including cell adhesion, growth, movement and differentiation. Src is widely expressed in many cell types, and can have different locations within a cell. It appears that the subcellular location of Src can affect its function. Src can associate with cellular membranes, such as the plasma membrane, the perinuclear membrane and the endosomal membrane. At the plasma membrane, Src can transduce signals from a variety of receptors to internal signalling pathways that convey these signals to the nucleus, cytoskeleton and other cellular components. For example, Src can act through growth factor receptors to affect cell growth and proliferation. Within the nucleus, Src is thought to help regulate the cell cycle and cell division by its interactions with other proteins. For example, Src can interact with Sam68 to help regulate gene expression. In bone osteoclasts, Src acts to regulate the respiratory enzyme cytochrome C oxidase (Cox), where Src-induced phosphorylation of Cox is required for maintaining high levels of ATP to meet the cells' high energy requirements. Src can also be found in the cytoplasm, and between cells at adherens junctions, where it takes on different roles.

pRb (Retinoblastoma protein) is a tumor suppressor protein that is dysfunctional in several major cancers. One function of pRb is to prevent excessive cell growth by inhibiting cell cycle progression until a cell is ready to divide. It is also a recruiter of several chromatin remodeling enzymes such as methylases and acetylases. Rb restricts the cell's ability to replicate DNA by preventing its progression from the G1 (first gap phase) to S (synthesis phase) phase of the cell cycle. Rb binds and inhibits transcription factors of the E2F family, which are composed of dimers of an E2F protein and a dimerization partner (DP) protein. When Rb is bound to E2F, the complex acts as a growth suppressor and prevents progression through the cell cycle. Rb is phosphorylated to pRb by certain Cyclin Dependent Kinases (CDKs). pRb is described as being hyperphosphorylated and when in this state, it is unable to complex E2F and therefore, unable to restrict progression from the G1 phase to the S phase of the cell cycle. During the M-to-G1 transition, pRb is progressively dephosphorylated by PP1, returning to its growth-suppressive hypophosphorylated state Rb.

Also provided herein is a pharmaceutical association, combination or (therapeutic, dermatologic, ophthalmologic, diagnostic, etc.) composition defined herein for use in a method of treating a neoplastic cell or a neoplastic disease by down-regulating (in particular, substantially decreasing or suppressing) the expression of genes and proteins p53 and/or Src.

Also provided herein is a pharmaceutical association, combination or (therapeutic, dermatologic, ophthalmologic, diagnostic, etc.) composition defined herein for use in a method of treating a neoplastic cell or a neoplastic disease by:

- down-regulating (in particular, substantially reducing) the expression of FAK genes and proteins and/or MAP kinase; and/or

- reducing (in particular, substantially reducing) the expression or activity of GTPase Ras, Rho (Rho, Rac, Cdc42); and/or
- down-regulating (in particular, reducing, significantly reducing or suppressing) the Ras/MAP kinase, FAK/Src kinase and/or PIP2 signalling pathways; and/or
- substantially inhibiting the formation of at least one of, in particular a plurality of, the protein complexes comprising at least one cyclin D and at least one CDK4 or CDK6; and/or
- substantially inhibiting, down-regulating or dampening the gene expression or formation of at least one of, in particular a plurality of, the cyclin D proteins; and/or
- down-regulating the expression of paxillin and/or (preferably and) up-regulating the expression of vinculin; and/or
- up-regulating (in particular, substantially increasing) the expression of at least one of differentiation genes and proteins such as ADIPOQ (ACRP30), FABP4, PPARG, ACAN (AGC1), COL10A1, COMP, Sox9, IBSP (Sialoprotein II), COL4, CDH5, KDR (VEGFR3), PECAM1, DLL4, EFNB2, NRP1, LYVE1, PROX1, NR2F2, NRP2, KRT1, KRT10, KRT14, PMEL (SILV), TYR, TYRP1, MMP-9, $\alpha$-SMA, Vimentin, BGLAP, COL2A1, IBSP, RANK-L, OCN, DMP-1, Sclerostin, MEPE, CALCR, CTSK, ITGB4, KRT19, ALB, G6PC, TAT, CD79A, CD3E, PTCRA, CCR5, CXCR4, EMR1, ITGAM, GALC, GFAP, MAP2, NEFH, TUBB3, CHAT, TH, GAD1, SLC32A1, SLC17A6, SLC17A7, ISL1, MBP, POU4F2, RLBP1, PDE6B, RCVRN, NPHS2, AQP1, CYP27B1, MIOX, AQP2, UMOD, SFTPB, SFTPC, SFTPD, MYH6, MYH7, NPPA, CAV3, MYH1, MYOD1, GATA4, MLC1, MYH11, SMTN, TAGLN, GCG, MAFB, POU3F4, INS, MAFA, SLC2A2, SST, GHRL (Ghrelin, Obestatin), PPY and/or CPA1; and/or
- increasing the phosphorylation of protein p53; and/or
- down-regulating (in particular, substantially decreasing or suppressing) the phosphorylation of the protein pRb; and/or
- down-regulating (in particular, substantially decreasing or suppressing) the gene and protein expression of Src; and/or
- not inducing the death of said neoplastic cell; and/or
- dampening or arresting cell division or cell proliferation of said neoplastic cell.

In one example, the pharmaceutical association as defined herein may thus be an animal or mammal (preferably a human) anti-neoplastic disease agent *(e.g.* anti-cancer agent) which has demonstrated the ability to convert a neoplastic cell into a non-neoplastic cell in vitro, ex-vivo and/or in vivo and/or to protect a subject from a neoplastic disease, disorder, condition, pathology or any symptoms thereof.

In one example, said administration induces the quiescence of the neoplastic cell. In one example, said administration prevents, reduces or suppresses the cell division and/or cell proliferation of said neoplastic cell. In one example, said administration regulates or promotes anti-mitogen activity and/or tumor suppressor activity and/or anti-oncogenic activity in said neoplastic cell. In one example, said method is cytostatic for the treated cell. In one example, said method is not cytotoxic for the treated cell. In one example, said method is non-mutagenic.

**Protected from a disease,** condition, disorder or pathology refers to the treatment of the underlying cause of the disease, condition, disorder or pathology as well as reducing the symptoms of the disease, condition, disorder or pathology; and/or reducing the occurrence of the disease, condition, disorder or pathology; and/or reducing the severity of the disease, condition, disorder or pathology. Protecting a patient can refer to the ability of a therapeutic composition of the present invention, when administered to a patient, to prevent a disease, condition, disorder or pathology from occurring and/or to cure or to alleviate disease, condition, disorder or pathology symptoms, signs or causes. As such, to protect a patient from a disease, condition, disorder or pathology includes both preventing disease, condition, disorder or pathology occurrence (prophylactic treatment) and treating a patient that has a disease, condition, disorder or pathology or that is experiencing initial symptoms or later stage symptoms of a disease, condition, disorder or pathology (therapeutic treatment).

**Treating:** As used herein, unless indicated otherwise or contradictory in context, the term "treating" or "treatment" refers to partially or completely alleviating, ameliorating, improving, relieving, delaying onset of, inhibiting progression of, reducing severity of, and/or reducing incidence of one or more symptoms or features of a particular disease, disorder, pathology and/or condition. For example, in certain embodiments, "treating" or "treatment of" cancer may refer to inhibiting survival, growth, and/or spread of a tumor. Treatment may be administered to a subject who does not exhibit signs of a disease, disorder, and/or condition and/or to a subject who exhibits only early signs of a disease, disorder, pathology and/or condition for the purpose of decreasing the risk of developing pathology associated with the disease, disorder, and/or condition.

As used herein, "treating a neoplastic cell" is used interchangeably with the expression "converting a neoplastic cell into a non-neoplastic cell".

**Preventing:** As used herein, unless indicated otherwise or contradictory in context, the term "preventing" refers to partially or completely delaying onset of an infection, disease, disorder and/or condition; partially or completely delaying onset of one or more symptoms, features, or clinical manifestations of a particular infection, disease, disorder, and/or condition; partially or completely delaying onset of one or more symptoms, features, or manifestations of a particular

infection, disease, disorder, and/or condition; partially or completely delaying progression from an infection, a particular disease, disorder and/or condition; and/or decreasing the risk of developing pathology associated with the infection, the disease, disorder, and/or condition.

**Substantially diminished, reduced or inhibited:** As used herein, unless indicated otherwise or contradictory in context, the term "substantially diminished" or "substantially inhibited" as it relates to cellular activity and/or function, protein expression or complexes formation, refers to a diminution or reduction in activity or function, in comparison with the activity and/or function of a normal or healthy cell (*e.g.* a non-neoplastic cell), of 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%, in particular of 50% to 99%, 55% to 99%, 60% to 99%, 65% to 99%, 70% to 99%, 75% to 99%, 80% to 99%, 85% to 99% or 90% to 99%. The same applies conversely to the term "substantially increased".

**Disease:** As used herein, unless indicated otherwise or contradictory in context, the term "disease" refers to any deviation from the normal health of a patient and includes a state when disease symptoms are present, as well as conditions in which a deviation has occurred, but symptoms are not yet manifested. A defective, ill-functioning, malfunctioning, dysfunctioning, and/or deleterious cell, such as *e.g.* a neoplastic cell, may be a cause of a disease. The same applies to "condition", "disorder" and "pathology".

**Neoplasm:** As used herein, unless indicated otherwise or contradictory in context, the term "neoplasm" (sometimes also referred to as tumor or tumour) refers to an abnormal growth of tissue. This abnormal growth usually but not always forms a mass. The World Health Organization classifies neoplasms into four main groups: benign neoplasms, in situ neoplasms, malignant neoplasms, and neoplasms of uncertain or unknown behavior. A malignant neoplasm is a cancer.

**Neoplastic cell:** As used herein, unless indicated otherwise or contradictory in context, the term "neoplastic cell" refers to a cell with abnormal proliferation, generally due the presence of a defective cell cycle, as well as cells which may become neoplastic such as cancer stem cell for which a definition is given in "Refining the role for adult stem cells as cancer cells of origin", White AC, Lowry WE, Trends Cell Biol. 2014 Sep 18. pii: S0962-8924(14)00145-7, and "review article Stem cells, cancer, and cancer stem cells", Tannishtha Reya et al., Nature 414, 105-111 (1 November 2001),

**Neoplastic disease:** As used herein, unless indicated otherwise or contradictory in context, the term "neoplastic disease", used interchangeably with "neoplastic conditions", "neoplastic disorder" and "neoplastic pathology", refers to a disease associated with an abnormal growth of tissue, generally due to the presence of a neoplastic cell.

**Circulating:** As used herein, unless indicated otherwise or contradictory in context, the term "circulating", when used in relation to a neoplastic cell, refers to a neoplastic cell that has detached itself from a primary tumor site and migrates to a different location in the body to become a metastatic tumor.

**Cancers:** As used herein, unless indicated otherwise or contradictory in context, the term "cancer" refers to a malign form of neoplasm which includes sarcomas, carcinomas, lymphomas, leukemias (or leukaemias), teratomas, mesotheliomas, myelomas and germinomas. Cancers include, but are not limited to, Acute lymphoblastic leukaemia (ALL), Acute myeloid leukaemia, Adrenocortical carcinoma, AIDS-related cancers, AIDS-related lymphoma, Anal cancer, Appendix cancer, Astrocytoma, childhood cerebellar or cerebral, Basal-cell carcinoma, Bile duct cancer, Bladder cancer, Bone tumor, osteosarcoma/malignant fibrous histiocytoma, Brainstem glioma, Brain cancer, Breast cancer, Bronchial adenomas/carcinoids, Burkitt’s lymphoma, Carcinoid tumor, Central nervous system lymphoma, Cerebellar astrocytoma, Cerebral astrocytoma/malignant glioma, Cervical cancer, Childhood cancers, Chronic bronchitis, Chronic lymphocytic leukaemia, Chronic myelogenous leukaemia, Chronic myeloproliferative disorders, Chronic obstructive pulmonary disease (COPD), Colon cancer, Cutaneous T-cell lymphoma, Desmoplastic small round cell tumor, Emphysema, Endometrial cancer, Ependymoma, Esophageal cancer, Ewing’s sarcoma in the Ewing family of tumors, Extracranial germ cell tumor, Extragonadal germ cell tumor, Extrahepatic bile duct cancer, Eye cancer, Gallbladder cancer, Gastric (stomach) cancer, Gastrointestinal carcinoid tumor, Gastrointestinal stromal tumor (GIST), Germ cell tumor: extracranial, extragonadal, or ovarian, Gestational trophoblastic tumor, Glioma of the brain stem, Glioma, Gastric carcinoid, Hairy cell leukemia, Head and neck cancer, Heart cancer, Hepatocellular (liver) cancer, Hodgkin lymphoma, Hypopharyngeal cancer, Hypothalamic and visual pathway glioma, childhood, Intraocular melanoma, Islet cell carcinoma (endocrine pancreas), Kaposi sarcoma, Kidney cancer (renal cell cancer), Laryngeal cancer, Leukemias, acute lymphoblastic leukemia, acute myeloid leukemia, chronic lymphocytic leukemia, chronic myelogenous leukemia, hairy cell Leukemia, Lip and oral cavity cancer, Liposarcoma, Liver cancer, Lung cancer, AIDS-related lymphoma, Burkitt lymphoma, cutaneous T-Cell lymphoma, Hodgkin lymphoma, Non-Hodgkin lymphoma, primary central nervous system lymphoma, Macroglobulinemia, Male breast cancer, Malignant fibrous histiocytoma of bone/osteosarcoma, Medulloblastoma, intraocular (eye) Melanoma, Merkel cell cancer, Mesothelioma, Metastatic squamous neck cancer with occult primary, Mouth cancer, Multiple endocrine neoplasia syndrome, Multiple myeloma/plasma cell neoplasm, Mycosis fungoides, Myelodysplastic syndromes, Myelodysplastic/myeloproliferative diseases, multiple Myeloma, Myeloproliferative disorders, Nasal cavity and paranasal sinus cancer, Nasopharyngeal carcinoma, Neuroblastoma, Oral cancer, Oropharyngeal cancer, Osteosarcoma/malignant fibrous histiocytoma of bone, Ovarian cancer, Ovarian epithelial cancer, Ovarian germ cell tumor, Ovarian low malignant potential tumor, Pancreatic cancer, Pancreatic cancer, islet cell Paranasal sinus and nasal cavity cancer, Parathyroid cancer, Penile cancer, Pharyngeal cancer, Pheochromocytoma, Pineal astrocytoma, Pineal germinoma, Pineoblastoma and supratentorial primitive neuroectodermal tumors, Pituitary adenoma, Plasma cell neoplasia/Multiple myeloma, Pleuropulmonary blastoma, Primary central nervous system lymphoma, Prostate cancer, Rectal cancer, Renal cell carcinoma (kidney cancer), Renal pelvis and ureter, transitional cell cancer, Retinoblastoma, Rhabdomyosarcoma, Salivary gland cancer, Ewing family of tumors Sarcoma, Kaposi Sarcoma, soft tissue Sarcoma, uterine Sarcoma, Sézary syndrome, Skin cancer (non-melanoma), Skin cancer (melanoma), Merkel cell Skin carcinoma, Small intestine cancer, Soft tissue sarcoma, metastatic squamous neck cancer with occult primary, Stomach cancer, Supratentorial primitive neuroectodermal tumor, cutaneous T-Cell lymphoma, Testicular cancer, Throat cancer, Thymoma and thymic carcinoma, Thyroid cancer, Transitional cell cancer of the renal pelvis and ureter, Trophoblastic tumor, transitional cell cancer, Urethral cancer, Uterine cancer, Uterine sarcoma, Vaginal cancer, Visual pathway and hypothalamic glioma, Vulvar cancer, Waldenström macroglobulinemia, and Wilms tumor (kidney cancer). In certain embodiments, cancers that may be treated using the recoding therapy disclosed herein are selected from the group consisting of sarcomas such as Osteosarcoma, Osteoma, Chondrosarcoma, Chondroma, Leiomyosarcoma, Leiomyoma, Rhabdomyosarcoma, Rhabdomyoma, Mesothelioma, Fibrosarcoma, Fibroma, Malignant Histiocytoma, Hisitocytoma, Angiosarcoma, Hemangiosarcoma, Hemangioma, Hemangiopericytoma, Lymphangiosarcoma, Lymphangioma, Liposarcoma, Lipid Cell Tumor, Lipoma, Myxosarcoma, Chordoma, Cystosarcoma Phylloides, Fibroadenoma, Seminoma, Dysgerminoma, Sertoli-Leydig Cell Tumor, Arrhenoblastoma, Granulose-Theca Cell Tumor, Hilar Cell Tumor, Prostate Cancer, Prostatic Hypertrophy, Wilms Tumor, Melanoma, Nevus, Neurofibrosarcoma, Glioma, Anaplastic Glioma, Glioblastoma, Neuroblastoma, Medulloblastoma, Ganglioneuroma, Malignant meningioma, Meningioma, Malignant Schwannoma, Schwannoma, Neurilemmoma, Neurofibroma and Mesenchymous Tumor; Carcinomas such as Squamous Cell Carcinoma, Merkel Cell Neoplasm, Epidermoid Carcinoma, malignant Skin Adnexal Tumor, Adenocarcinoma, Hepatocellular Carcinoma, Renal Cell Carcinoma, Choriocarcinoma, Hypernephroma, Cholangiocarcinoma, Transitional Cell Carcinoma, Seminoma, Embryonal Cell Carcinoma, Papilloma, Seborrheic Keratosis, Skin Adnexal Tumors, Hepatoma, Adenoma, Hepatic Adenoma, Renal Tubular Adenoma, Bile Duct Adenoma, Transitional Cell Papilloma, Hydatidiform Mole, Parathyroid Carcinoma, Parathyroid Adenoma, Medullary Carcinoma of Thyroid, C Cell Hyperplasia, Malignant Pheochromocytoma, Pheochromocytoma, Islet Cell Carcinoma, Islet Cell Adenoma, Insulinoma, Gastrinoma, Malignant Carcinoid, Carcinoid, Malignant Paraganglioma, Chemodectoma and Paraganglioma; Myelomas; Leukemias such as Myelogenous Leukemia, Lymphatic Leukemia, Policythemia, Aleukemic Leukemia, Preleukemia and Myeloproliferative Disorders; Lymphoma such as Myeloma, Hodgkin Lymphoma, Non-Hodgkin Lymphoma, Plasmacytoma and Plasmacytosis; Adenosquamous Carcinoma, Mesodermal Tumor, Carcinosarcoma and Teratocarcinoma.

**Symptoms of neoplastic diseases:** In one example, the present invention treats or alleviates at least one symptom selected from Angina, Bradypnea, Breathing difficulties, Gradual vision change in both eyes, Gradual vision changes in one eye, Head symptoms, Heartburn, Muscle symptoms, Nerve symptoms, Perforated gastric ulcer, Perforated ulcer, Respirations, shallow, Sexual symptoms, Weight loss, Oesophagus symptoms, Weight symptoms, Anaemia, Bad breath, Breath odors, Burning feet, Burning Legs, Cleft lip with or without cleft palate in children, Communication symptoms, Diarrhea, Dyspepsia, Easily fatigued, Enlarged liver, Face symptoms, Foot pain, Gastritis, Gastrooesophageal reflux in pregnancy, Glossitis, Hairy Tongue, Hyperlipidaemia, Hypertriglyceridemia, Impotence, Lack of energy, Lag in breathing, Male infertility, Meniere's disease, Movement symptoms, Musculoskeletal symptoms, Night sweats in children, Non-respiratory causes of shallow respiration, Raised ambulatory 24hr pH monitoring, Rapid heartbeat, Respiratory lag, Sleep symptoms, Sleeplessness, Speech symptoms, Sweat symptoms, Trauma-related symptoms, Voice symptoms, Vomiting, White tongue, Abdominal symptoms, Abnormal blood test symptoms, Acute reflux-like regurgitation in pregnancy, Acute reflux-like symptoms in pregnancy, Acute reflux-like vomiting in pregnancy, Acute stomach ulcer-like symptoms in pregnancy, Birth symptoms, Blanching in the fingers, Bleeding symptoms, Blood symptoms, Blood vessel damage, Chronic pain in multiple muscles, Digestive symptoms, Elevated blood pressure in pregnancy, Episodic arterial vasospasm, Female infertility, Female reproductive symptoms, Fertility symptoms, Heart enlargement, Hiatus hernia, Hiatus hernia related to chronic digestive disorders, High blood pressure, Hypertension-like symptoms in pregnancy, Impaired myocardial contractility, Increased Systolic pressure, Infection, Infertility, Intermittent claudication, Intermittent claudication of both lower limbs, Intermittent claudication of one lower limb, Intermittent pain of the lower limb, Lower abdominal symptoms, Lower blood pressure in the legs than in the arms, Mouth symptoms, Mycocardial ischemia, Noncompliance, Occlusion of renal arteries, Pain, Peripheral arterial trauma, Poor appetite, Poor wound healing, Pregnancy symptoms, Pulsatile Abdominal swelling, Red blood cell symptoms, Reflux-like symptoms, Respiratory symptoms, Sensory symptoms, Severe chronic pain in multiple bones, Sore throat, Sweating, Throat symptoms, Urinary incontinence, Venous insufficiency, Women's health symptoms, Birth defects, Eating symptoms, 1 litre of sweat per hour, Aberrant behaviour in pregnancy, Abnormal eye movements, Abnormal Liver Function Tests in Pregnancy, Abnormal thinking in pregnancy, Absenteeism, Accentuated fall in systolic pressure, Acidosis, Action tremor,

Acute epilepsy-like symptoms, Acute forgetfulness in pregnancy, Acute hemorrhagic pancreatitis, Acute intermittent forgetfulness in pregnancy, Acute onset of daytime sleepiness, Acute onset of daytime sleepiness in adults, Acute onset of daytime sleepiness in the elderly, Acute pancreatitis, Addiction symptoms, Aggression, Alcohol abuse, Alcohol breath odor, Alcohol use, Altered bladder habits in pregnancy, Altered mental state, Altered vital signs, Anxiety disorder in pregnancy, Anxiety in pregnancy, Apprehension in pregnancy, Arrhythmia, Arrhythmias, Atrial fibrillation, Balance symptoms, Behavior problems at school, Behavior problems at work, Behavior problems in adults, Behavior problems in children, Behavior problems in teens, Behavioral symptoms, Behaviour changes in pregnancy, Blackouts, Cerebellar ataxia, Chronic Diarrhea, Chronic orthostatic hypotension, Cleft palate in children, Clumsiness, CNS depression in children, Cognitive impairment, Coma, Confusion, Confusion in children, Coordination problems, De-personalization, Decreased level of consciousness, Deep stupor, Dehydration, Delayed ejaculation, Delirium, Delusions, Dementia, Depressed mental status, Depressive symptoms, Diabetic retinopathy, Diaphoresis of the feet, Diaphoresis of the forehead, Diaphoresis of the palms, Diaphoresis of the soles, Difficulty concentrating in adults, Disoriented state, Diuresis, Dramatic fall in blood pressure, Drowsiness, Drug abuse, Drug withdrawal causing new-onset seizures, Drugs, Drugs causing diarrhea, Drugs causing macrocytosis, Drugs causing persistent hypoglycemia, Drugs causing persistent hypoglycemia in adolescents, Drugs causing persistent hypoglycemia in children, Drugs causing persistent hypoglycemia in infants, Dysphagia due to esophagitis, Emotional symptoms, Energy symptoms, Epilepsy-like symptoms, Episodic Diaphoresis, Episodic Diaphoresis as in case of diabetes mellitus, Euphoria, Excessive diaphoresis, Eye symptoms, Fainting, Fainting episodes similar as in pulmonary hypertension, Falls, Fatigue, Feeling the heartbeat, Flushing, Forgetfulness, Forgetfulness in pregnancy, Frequency of urination in pregnancy, Frequent urination, Frequent urination in pregnancy, Generalised diaphoresis, Generalised diaphoresis with heat loss via evaporation, Hallucinations, Headache, Heart arrhythmia, Heart arrhythmias, Heart flutters, High ALP, High ALT, High AST, Hyperosmolarity, Hypoperfusion state, Hypothermia, Hypothermic attack, Imbalance, Impaired circulation, Inadequate intravascular pressure, Inattention, Inattention symptoms, Increased bilirubin, Increased GGT, Increased prothrombin time, Increased sweating, Intercourse symptoms, Intermittent diaphoresis, Intermittent epilepsy-like symptoms, Intermittent seizures in early childhood, Involuntary tonic movements, Irregular heartbeat, Irritability, Jaundice in pregnancy, Level of consciousness symptoms, Liver tenderness in pregnancy, Low albumin, Low blood platelet level in pregnancy, Low blood pressure, Low blood sugar, Low temperature, Major depression in pregnancy, Male impotence, Memory disturbances, Memory Disturbances in pregnancy, Memory loss in pregnancy, Memory symptoms, Memory symptoms in pregnancy, Mental changes in pregnancy, Mental depression, Mental depression in pregnancy, Mental problems, Mental problems in pregnancy, Mild depression in pregnancy, Mild depression-like symptoms in pregnancy, Mild personality changes, Moderate depression in pregnancy, Mood swings, Muscle pain, Muscle weakness, Myoclonus, Nerve damage, Nervousness, Nervousness in pregnancy, Neuromuscular irritability, Neuromuscular irritability of lower limbs, Neuropathy, Night time urination in pregnancy, Night urination in pregnancy, Nocturia in pregnancy, Numbness in Both Feet, Numbness in one foot, Nystagmus, Ocular myokymia, Orthostatic hypotension, Ototoxic medications, Palpitations, Pancreatitis, Paresthesias, Penile Burning Sensation, Persistent painful erection, Personality change, Personality symptoms, Phobia, Progressive mental deterioration, Prolonged exposure to cold, Psychiatric symptom, Pulse irregularity in pregnancy, Red face, Reduced immune response, Reduced intravascular pressure, Reduced sperm count, Retinopathy, School problems, Seizures, Seizures in infants and early childhood, Self-esteem symptoms, Severe epilepsy-like symptoms, Sexual dysfunction in pregnancy, Short-term memory loss, Signs of circulatory collapse, Signs of circulatory collapse as seen in intestinal hemorrhage, Sleeping problems, Slurred speech, Social problems, Society problems, Sole numbness, Standing symptoms, Stupor, Subfertility, Substance abuse causing school underachievement and academic failure, Substances of abuse causing delirium, Sudden onset of confusion in children, Syncopal episode, Thigh Burning Sensation, Thrombocytopenia, Thrombocytopenia in pregnancy, Tingling in Both Feet, Tingling in both hands, Tingling in one foot, Tingling in one hand, Tonic-clonic seizure, Toxins causing vomiting, Tremor, Unusual body odor in children, Urinary burning, Urinary urgency, Urine retention, Vascular collapse, Vertigo, Violent behaviour, Walking symptoms, Weight loss in pregnancy, Abnormal uterine bleeding, Abortion, Abruptio Placentae in Pregnancy, Abscessed teeth,

Acid Reflux in pregnancy, Acquired mental retardation, Acute acid reflux into mouth during pregnancy, Acute attack of asthma, Acute cerebral infarct, Acute seasonal asthma-like symptoms, Aphthous Ulcer, Arm burning sensation, Arm coldness, Arm infection, Arm inflammation, Arm numbness, Arm paresthesia, Arm redness, Arm sensitive, Arm spasm, Arm swelling, Atelectasis, Biceps burning sensation, Biceps cold, Biceps infection, Biceps inflammation, Biceps numb, Biceps redness, Biceps spasm, Biceps swelling, Biceps tingling, Bilateral cleft lip and palate, Bilateral complete cleft lip, Bilateral stroke, Black hairy tongue, Bladder symptoms, Blood clots, Blood lead concentration, Blood pressure symptoms, Blood vessel symptoms, Blue lips, Bone changes, Breath sound symptoms, Breath symptoms, Bronchial cancer, Bronchitis, Brown nails, Cancer-related symptoms, Cardiovascular symptoms, Cervix symptoms, Chronic bronchitis, Chronic cough, Chronic cough in adolescents, Circulation symptoms, Claudication, Cluster headache, Constant leg pain, Coracobrachialis infection, Coracobrachialis redness, Coracobrachialis swelling, Cough, Cramping leg pain, Cramping pain in both legs, Dark spots on teeth, Darkened tongue, Death, Death-related symptoms, Decreased salivary function, Digit burning sensation, Digit cold, Digit infection, Digit inflammation, Digit numb, Digit redness, Digit

sensitive, Digit spasm, Digit swelling, Digit tingling, Disc herniation, Disc prolapse, Discolored teeth in children, Dry cough, Dry Gangrene, Dry mucous membrane as in case of Sjogren's syndrome, Dry skin, Electrocardiogram changes, Eructation, Exercise symptoms, Female genital symptoms, Female sexual symptoms, Femur burning sensation, Femur numb, Femur tingling, Fetal symptoms, Fibula burning sensation, Fibula numb, Fibula tingling, Finger burning sensation, Finger pulp burning sensation, Fingernail burning sensation, Fingers burning sensation, Fingers cold, Fingers infection, Fingers inflammation, Fingers numb, Fingers redness, Fingers sensitive, Fingers spasm, Fingers swelling, Fingers tingling, Forearm burning sensation, Forearm cold, Forearm infection, Forearm inflammation, Forearm numb, Forearm redness, Forearm sensitive, Forearm spasm, Forearm swelling, Forearm tingling, Foul mouth odour, Genital symptoms, Growth symptoms, Heart damage, Heart disease, Heart rhythm symptoms, Heart symptoms, Heartburn after eating in pregnancy, Heartburn after exercise in pregnancy, Heartburn in pregnancy, Heartburn pain resistant to treatment in pregnancy, Heartburn that worsens if lying down after eating in pregnancy, Heartburn unrelated to eating in pregnancy, Heartburn with acid reflux in pregnancy, Heartburn without reflux in pregnancy, Herniated disk, Hiccups, High lipoprotein level, Hoarse, Humerus burning sensation, Humerus numb, Humerus tingling, Hypoxia in pregnancy, Increased capillary refill time, Increased secretions in the lung, Indigestion in pregnancy, Infant symptoms, Inflammatory symptoms, Irregular rhythm, Kidney symptoms, Knee joint burning sensation, Knee joint cold, Knee joint numb, Knee joint sensitive, Knee joint tingling, Kneecap burning sensation, Kneecap cold, and any combinations thereof.

## XIV. Diagnostic methods

In one aspect, the present disclosure provides methods of identifying, diagnosing, and optionally classifying subjects on these bases, which may include clinical diagnosis, biomarker levels, and other methods known in the art.
In one aspect, the present disclosure provides a pharmaceutical association or composition as defined herein for use in a diagnostic method of a neoplastic disease *(e.g.* a cancer). In one example, such a diagnostic method comprises the detection of a cancer using a method described in US patent application No. 2002/0159986 A1.
Also described herein is a diagnostic method for diagnosing of a neoplastic disease or condition comprising the provision of a pharmaceutical association or composition as defined herein, and the contacting or administration of said pharmaceutical association or composition with a body part of a subject to be diagnosed.
Said method for the diagnosis of cancer in a patient, comprising obtaining a biological sample from a patient and apply fluorescent and/or radiolabeled GFR binding compounds, wherein high localisation of these compounds indicates cancer in the patient.

## XVI. Kits

Also described herein is a variety of kits for conveniently and/or effectively carrying out methods and uses of the present invention. Typically, kits will comprise sufficient amounts and/or numbers of components to allow a user to perform multiple treatments of a subject(s) and/or to perform multiple experiments.
Also described herein are kits for pharmaceutical association production, comprising at least one GFR-binding compound as defined herein, at least one bioactive carrier as defined herein, both provided in an amount effective to produce a pharmaceutical association to convert a neoplastic cell into a non-neoplastic cell and/or treat, prevent or diagnose a neoplastic disease when administered in-vitro, ex-vivo or in-vivo to a neoplastic cell or a subject carrying such a neoplastic cell, and packaging and instructions.
Also described herein are kits for pharmaceutical composition production, comprising a GFR-binding compound as defined herein, a bioactive carrier as defined herein, and a pharmaceutically acceptable excipient, carrier or vehicle, each of them provided in an amount effective to produce a pharmaceutical composition to convert a neoplastic cell into a non-neoplastic cell and/or treat or prevent a neoplastic disease when administered in-vitro, ex-vivo or in-vivo to a neoplastic cell or a subject carrying such a neoplastic cell, and packaging and instructions.
Suitable as solvents for use in kits of the invention include physiologically acceptable solvents, PBS, filtered and deionised water such as Milli-Q® water, alpha-MEM, DMEM and/or IMDM. All physiologically acceptable solvents suitable for implementing embodiments of the present invention are preferably deoxygenated before use.
In one example, said kit further comprises an administration device. In one example, said administration device is a dispensing device such as a syringe.
In one example, said kit comprises a first container containing a (modified) GFR-binding compound as defined herein and a second container containing a bioactive carrier such as a biomaterial.
In one particular example, said kits of the invention may suitably be provided in the form of a sterile packaging.
For example, said kits of the invention comprises more than 2, between 2 and 25, between 2 and 15, or between 2 and 10 of (modified) GFR-binding compound as defined herein, and more than 2, between 2 and 25, between 2 and 15, or between 2 and 10 (modified) GFR-binding compound as defined herein.
In one example, each (modified) GFR-binding compound and each bioactive carrier is conditioned in distinct and

separated compartments, in lyophilised form, in solution or in suspension in a pharmaceutically dermatologically, prophylactically, diagnostically, imaging or cosmetically acceptable excipient, carrier or vehicle.

Also described herein are kit-of-parts comprising a bioactive carrier and a (modified) GFR-binding compound both as defined herein for uses and methods as defined herein.

## XVII. Sequence listing

Examples of (modified) GFR-binding compounds as defined herein are listed in the appended sequence listing which forms an integral part of the present application.

Examples of (modified) cyclic GFR-binding compounds as defined herein are listed in the appended sequence listing which forms an integral part of the present application. (Modified) cyclic GFR-binding compounds of the present disclosure may be represented in non-cyclic, linear, form solely for the purpose of ease of representation but nevertheless remains cyclic compounds wherein the first and the last functional group (e.g. an amino acid) of the linearly represented compound are covalently connected to each other thereby forming a cyclic structure.

Pharmaceutical associations, combinations and compositions as defined in the present disclosure, has been found to lead to multiple and distinct advantages in terms of neoplastic diseases treatment.

As supported by the examples of the present application, pharmaceutical associations, combinations and compositions as defined in the present disclosure provide for a new area of treatment of neoplastic diseases by extracellularly converting or recoding neoplastic cells into functional and healthy cells. The therapy of the invention containing pharmaceutical associations, combinations or compositions as defined herein display advantages over therapies that do not contain it, such as any one of, and preferably a plurality of:

- Enhanced and/or more practical and/or more efficient and/or more cost-effective and/or more adapted to the end-user needs;
- Converting or recoding a neoplastic cell to induce and/or promote and/or improve self-healing and/or self-recovery thereof, particularly in a shorter period of time;
- Converting a neoplastic cell into a non-neoplastic cell and/or protecting a subject from a neoplastic disease, disorder, condition, pathology, such as cancer, or symptoms thereof using non-mutagenic means;
- Converting a neoplastic cell into a non-neoplastic cell and/or protecting a subject from a neoplastic disease, disorder, condition, pathology, such as cancer, or symptoms thereof using extracellular means;
- Restoring a neoplastic cell ability to undergo differentiation, particularly in a shorter period of time;
- Converting and/or recoding a circulating or non-circulating neoplastic cell such as a metastatic or non-metastatic cancer cell, into a non-neoplastic cell, particularly in a shorter period of time;
- Protecting a subject from a neoplastic disease, disorder, condition or pathology such as cancer, and/or any symptoms thereof, particularly in a shorter period of time;
- Providing and/or producing a physiologically functional and/or healthy osteoblast, osteocyte, chondroblast, chondrocyte, from a neoplastic cell, particularly within a shorter period of time;
- Re-establishing, reactivating or restoring the cell adhesion checkpoint of a neoplastic cell;
- Treating a neoplastic cell without substantially inducing cell death;
- Inducing quiescence of a neoplastic cell;
- Dampening, reducing or suppressing cell division/proliferation of a neoplastic cell;
- Activating and/or promoting and/or up-regulating anti-mitogen activity and/or tumor suppressor pathways and/or anti-oncogenic activity in a neoplastic cell;
- Adapted to the treatment of any neoplastic cell;
- Safer for the patient being treated as providing less or no harmful side-effects;
- Offers the possibility for local treatments of neoplastic diseases;
- Relies mainly or only on the activation of physiological cell mechanisms which increases the general safety of the herein defined therapy.

For example, in certain embodiments, pharmaceutical association or combinations, or compositions according to the present disclosure in which PEP1 is SAIS, have been found to lead to unexpectedly fast conversion of a cancerous cell into a healthy and functional cell of the bone lineage.

For example, in certain embodiments, pharmaceutical association or combinations, or compositions according to the present disclosure in which PEP1 is SAIS, have been found to lead to unexpectedly fast conversion of a cancerous cell into a healthy and functional cell of the chondrocytic cell lineage.

For example, in certain embodiments, pharmaceutical association or combinations, or compositions according to the present disclosure in which PEP1 is SAIS, have been found to lead to unexpectedly fast conversion of a cancerous cell into a healthy and functional cell of the adipocyte lineage.

Accordingly, it is possible to achieve the conversion of a neoplastic cell *(e.g.* a cancer cell) into a non-neoplastic cell (*e.g.* a functional and/or healthy cell of the bone, chondrocytic, adipocyte cell lineages) using extracellular, non-mutagenic active principles and is thus useful in the treatment of neoplastic diseases such as cancers.

The present invention provides a recoding therapy for the treatment of neoplastic diseases, conditions or disorders such as cancer and aims at providing a suitable micro-environment to the neoplastic cell containing suitable biological signals so that the neoplastic cell is able to undergo self-healing and become a functional, healthy, non-neoplastic cell.

In addition, it has been found that using the recoding therapy of the invention instead of conventional therapies allows for improved treatment selectivity in such a way that reduced or substantially no adverse side-effect is observed. Without wishing to be bound to any specific theory, this may be explained by considering that the present therapy relies on the activation of physiological cell mechanisms without imposing or forcing the treated cell to undergo *e.g.* apoptosis or cell cycle arrest (which would then most likely unselectively target any cells, healthy and neoplastic) but instead reestablishes the correct cellular microenvironment surrounding this neoplastic cell so that the cell is able to operate self-healing. As a result, a non-neoplastic cell *i.e.* a cell in which the cell cycle and/or cell adhesion checkpoint(s) need not be repaired, brought in contact with such a microenvironment would be less or not be substantially affected.

Remarkably, it has also been found that using the pharmaceutical associations or compositions as defined herein allows for the reduction (in most cases, important reduction) of the time of treatment of a neoplastic cell and by extension of a neoplastic disease in comparison with known technologies.

It is another aim of the present invention to solve the technical problem of providing a therapy for treating neoplastic diseases being completely or at least partially devoid of one or more, preferably a plurality of the disadvantages of known treatments.

Further embodiments and advantages will become apparent to a skilled reader in light of the examples provided below.

## EXAMPLES

In the Examples below, only pharmaceutical associations comprising a GFR binding compound comprising peptides SAIS and LKNYQ i.e. SEQ ID NOs: 1 to 3, 9, 11 to 14, 18, 25, 26, 42, 56 to 58, 77, 78, 87, 96, 98, 197 and 198, form part of the present invention; others are not to be considered as being part of the present invention.

**The following starting materials and reagents were used:**

- Apatite ceramics (also called apatite or ceramic in the present invention) were synthetized as described in Mater Res. 2004; 7(4): 625-630.
- Titanium was obtained from Goodfellow®.
- Hydrogel (poly(acrylamide-co-acrylic acid) gel) was synthetized as described in Langmuir 2011; 27(22):13635-42.
- PEEK was obtained from Goodfellow®.
- PET (Poly(ethylene terephthtalate) was obtained from Goodfellow®.
- Type-I collagen sponge was obtained from Sigma®.
- Hexane was obtained from Sigma®.
- 3-succinimidyl-3-maleimidopropionate (SMP) was obtained from Sigma®.
- DMF was obtained from Sigma®.
- PBS 1X was obtained from Gibco®.
- 3-(ethoxydimethylsilyl)propylamine was obtained from Sigma®.
- Ammonium persulfate was obtained from Biorad®.
- N,N,N',N'-tetramethylethylenediamine was obtained from Aldrich®.
- Acrylamid was obtained from Merck®.
- Acrylic acid was obtained from Merck®.
- N,N-methylene-bis-acrylamide was obtained from Merck®.
- NaOH was obtained from Aldrich®.
- N,N,N',N'-tetramethylethylenediamine was obtained from Aldrich®.
- Dimethylaminopropyl-3-ethylcarbodiimide hydrochloride was obtained from Aldrich®.
- N-hydroxysuccinimide was obtained from Aldrich®.
- 2-(N-morpholino)-ethane sulfonic acid was obtained from Aldrich®.
- MilliQ water: is water characterised in terms of resistivity (typically 18.2 MΩ·cm at 25 °C).
- Low glucose Dulbecco's Modified Eagle Medium (DMEM) was obtained from Invitrogen®
- Minimum Essential Medium Eagle without ascorbic acid (αMEM) was obtained from Invitrogen®.
- All of the cell culture experiments were carried out without any serum in the medium for the first 8 hours of culture.
- Osteosarcoma cells: MG63 cells were obtained from ATCC®. These cells were cultured in DMEM (Eagle's Minimum

Essential Medium) medium supplemented with 10% fetal calf serum (FCS) and 1% penicillin/streptomycin. All cells were used at a low passage number (passage 10), were subconfluently cultured and were seeded at $10^4$cells/$cm^2$ for the purpose of the experiments.

- Chondrosarcoma cells: Hs819.T cells were obtained from ATCC®. These cells were cultured in DMEM (Eagle's Minimum Essential Medium) medium supplemented with 10% fetal calf serum (FCS) and 1% penicillin/streptomycin. All cells were used at a low passage number (passage 10), were subconfluently cultured and were seeded at $10^4$cells/$cm^2$ for the purpose of the experiments.
- Rhabdomyosarcoma cells: A-673 cells were obtained from ATCC®. These cells were cultured in DMEM (Eagle's Minimum Essential Medium) medium supplemented with 10% fetal calf serum (FCS) and 1% penicillin/streptomycin. All cells were used at a low passage number (passage 10), were subconfluently cultured and were seeded at $10^4$cells/$cm^2$ for the purpose of the experiments.
- Adenocarcinoma cells: HCC4006 cells were obtained from ATCC®. These cells were cultured in RPMI-1640 medium (obtained from ATCC®) supplemented with 10% fetal calf serum (FCS) and 1% penicillin/streptomycin. All cells were used at a low passage number (passage 10), were subconfluently cultured and were seeded at $10^4$cells/$cm^2$ for the purpose of the experiments.
- CMFDA is a Cell Tracker Green obtained from Invitrogen®.
- DAPI was obtained from Sigma®.
- Fetal bovine serum (FBS) was obtained from Gibco®.
- Penicillin/streptomycin was obtained from Invitrogen®.
- The AlamarBlue® assay was obtained from Molecular Probes®.
- Anti phospho-p53 antibody was obtained from Santa Cruz Biotechnology®,
- Anti pRB antibody was obtained from Santa Cruz Biotechnology®,
- Monoclonal anti-integrin (extracellular domain) $\alpha$v, $\alpha$5, $\alpha$3, $\alpha$4, $\beta$1, $\beta$3, $\beta$5 and $\beta$9 antibodies were obtained from Santa Cruz Biotechnology®,
- Cell cytoskeletal filamentous actin (F-actin) was visualized by Alexa Fluor® 488 phalloidin Sigma® labeling.
- Monoclonal anti-vinculin antibody (clone hVIN-1, produced in mouse) was obtained from Sigma®.
- Primers for Cyclin D1: 5'-CCGTCCATGCGGAAGATC-3' (Forward) and 5'-GAAGACCTCCTCCTCGCACT-3' (Reverse) were obtained from Invitrogen®.
- Primers for Cyclin D2: 5'-CATCCAACCGTACATGCGCAG-3' (Forward) and 5'-CATGGCCAGAGGAAAGACCTC-3' (Reverse) were obtained from Invitrogen®.
- Primers for Cyclin D3: 5'-TGATTTCCTGGCCTTCATTC -3' (Forward) and 5'-CGGGTACATGGCAAAGGTAT -3' (Reverse) were obtained from Invitrogen®.
- Primers for Paxillin: 5'-AATTCCAGTGCCTCCAACAC -3' (Forward) and 5'-GAGCTCATGACGGTAGGTGA -3' (Reverse) were obtained from Invitrogen®.
- Primers for Vinculin: 5'-GCCAAGCAGTGCACAGATAA -3' (Forward) and 5'-TTCCTTTCTGGTGTGTGAAGC -3' (Reverse) were obtained from Invitrogen®.
- Primers for DMP-1: 5'-AGCATCCTGCTCATGTTCCTTT-3' (Forward) and 5'-GAGCCAAATGACCCTTCCATT-3' (Reverse) were obtained from Invitrogen®.
- Primers for Sclerostin: 5'-CTTAGTTTTCTCAGTCTGTGGTTGAAAT-3' (Forward) and 5'-AGAGTACCCCGAGCC TCC-3' (Reverse) were obtained from Invitrogen®.
- Primers for RANK-L: 5'-CTCAGCCTTTTGCTCATCTCACT-3' (Forward) and 5'-CCAAGAGGACAGACTCACTTT ATGG-3' (Reverse) were obtained from Invitrogen®.
- Primers for MLC-1: 5'-AGAAGGGCTCCATGTCTGACA -3' (Forward) and 5'-AAGATTTCAGGACCCGAGCAG -3' (Reverse) were obtained from Invitrogen®.
- Primers for GATA-4: 5'-AGGCCTCTTGCAATGCGGA-3' (Forward) and 5'-CTGGTGGTGGCGTTGCTGG -3' (Reverse) were obtained from Invitrogen®.
- Primers for $\alpha$-Sarcomeric actin: 5'-GACCACAGCTGAACGTGAGA -3' (Forward) and 5'-CATAGCACGATGGTCG ATTG-3' (Reverse) were obtained from Invitrogen®.
- Primers for Sox9: 5'-GACTTCCGCGACGTGGAC-3' (Forward) and 5'-GTTGGGCGGCAGGTACTG-3' (Reverse) were obtained from Invitrogen®.
- Primers for IBSP: 5'-TGCCTTGAGCCTGCTTCC-3' (Forward) and 5'-GCAAAATTAAAGCAGTCTTCATTTTG-3' (Reverse) were obtained from Invitrogen®.
- Primers for Collagen IV: 5'- CCTGGTCTTGAAAGGTGATAAG -3' (Forward) and 5'-CCCGCTATCCCTTGATCTC -3' (Reverse) were obtained from Invitrogen®.
- Primers for Vimentin: 5'- TGTCCAAATCGATGTGGATGTTTC -3' (Forward) and 5'-TTGTACCATTCTTCTGCCTC CTG-3' (Reverse) were obtained from Invitrogen®.
- Primers for MMP-9: 5'- AATCTCTTCTAGAGACTGGGAAGG AG-3' (Forward) and 5'-AGCTGATTGACTAAAGTA GCTGGA-3' (Reverse) were obtained from Invitrogen®.

- Primers for α-SMA: 5'-CCGACCGAATGCAGAAGGA -3' (Forward) and 5'-ACAGAGTATTTGCGCTCCGAA -3' (Reverse) were obtained from Invitrogen®.
- Primers for GAPDH: 5'- GCAGTACAGCCCCAAAATGG-3' (Forward) and 5'-ACAAAGTCCGGCCTGTATCCAA-3' (Reverse) were obtained from Invitrogen®.
- All of the peptides were synthetized using conventional solution and/or solid phase peptide synthesis methods.
- All of the experiments were performed with a concentration of 400 ng/mL of GFR-binding compound(s) as defined herein.
- All cell culture experiments were performed with cultures on plastic coated with type-I collagen in order to mimic the physiological conditions of the human body.

**The following general methods and procedures were used:**

- X-ray photoelectron spectroscopy:
  For X-ray photoelectron spectroscopy, AVG Scientific ESCALAB photoelectron spectrometer was used for the surface analysis with a non-monochromatized MgK 1253.6 eV source of 100W. The area of the analytical X-ray spot on the sample surface was about 200 $\mu m^2$. A 45° insert angle that corresponds to about 5 nm of analyzed depth was used. A flood gun was used for charge compensation. Acquisition of high resolution spectra was performed at constant pass energy of 20 eV.

- Immunostaining:
  The cells were first fixed for 20 min with 4% paraformaldehyde/PBS at 4°C. After fixation, the cells were permeabilized in PBS containing 1% Triton X-100 for 15 min. Protein immunofluorescent visualization was performed by first treating the cells with 1% (v/v) specific monoclonal antibodies for 1 hour at 37 °C. Then the samples were incubated with Alexa fluor® 568 or 647 (F(ab')2 fragment of IgG(H + L)) during 30 min at room temperature. The cell nuclei were counterstained in 20 ng/mL DAPI for 10 min at room temperature.

- Quantification of positive contact numbers and areas:
  For this type of quantification the freeware image analysis ImageJ® software was used. The raw image was first converted to an 8-bit file, and then the unsharp mask feature was used (settings 1:0.2) to remove the image background (rolling ball radius 10). After smoothing, the resulting image, which appears similar to the original photomicrograph but with minimal background, was then converted to a binary image by setting a threshold. Threshold values were determined empirically by selecting a setting, which gave the most accurate binary image for a subset of randomly selected photomicrographs. The cell area was determined by manual delineation on raw fluorescent images. Total contact area and mean contact area per cell were calculated by "analyse particules" in ImageJ®. A minimum of 50 cells per condition were analyzed.

- Quantitative Real Time Polymerase Chain Reaction (RT-PCR):
  After 24 hours of culture, total RNA was extracted by using the RNeasy total RNA kit (Qiagen®) according to the manufacturer's instructions. Purified total RNA was used as a template in order to make cDNA by a reverse transcription reaction (Gibco Brl®) with random primers (Invitrogen®). The cDNA was then used as a template for a real-time PCR amplification in the presence of SYBR green reagents (Bio-Rad®) by using a thermocycler (iCycler, Biorad®). Data were analyzed with the iCycler IQ™® software and compared by the ΔΔCt method. Briefly, the mean Ct value of the target gene was normalized to its averaged Ct values of the housekeeping gene (GAPDH) to give a ΔCt value, which was then normalized to a control sample to obtain a ΔΔCt value. The results were obtained from two series of experiments performed in triplicate.

- Western Blot:
  After 24 hours of culture, cells were permeabilized (10% SDS, 25 mM NaCl, 10 nM pepstatin and 10 nM leupeptin in distilled water and loading buffer), boiled for 10 minutes and resolved by reducing PAGE (Invitrogen). Proteins were transferred onto nitrocellulose, blocked, and labeled with HRP-conjugated antibodies (Invitrogen). Integrin αv, α5, α3, α4, β1, β3, β5 and β9 were blotted by treating the nitrocellulose with monoclonal anti-integrin (extracellular domain) αv, α5, α3, α4, β1, β3, β5 and β6 antibodies (Santa Cruz Biotechnology). Phosphor-Smad1/5/8 was blotted by treating the nitrocellulose with monoclonal anti-p-Smad1/5/8 (Santa Cruz Biotechnology). For all of the experiments the western blot was performed in triplicate, along with an additional blot for actin and coomassie blue staining to ensure constant protein load among samples.

- Determination of peptide densities by high resolution μ-imager:
  In contrast to scintillation counting, beta-imager performances in the field of sensitivity, linearity and spatial resolution

give a real interest to this system as a new imaging device for solid surface. The detection principle is no longer based on the counting of electron avalanches produced in the gas, but it is based on the analysis of light emitted during the interaction. Beta radioactivity is directly detected by means of a parallel plate avalanche chamber, which is particularly well suited for qualitative and quantitative autoradiography.

Quantitatively, peptide concentration was evaluated by grafting $^{131}$I-peptide alone and by grafting $^{131}$I-peptide in the presence of RGD peptides. For example, polymer samples were placed in a solution of $^{131}$I-peptide diluted in peptide solution ($1\times10^{-3}$ M). Radioactivity of this latter solution was adjusted to 6 μCi/mL. The amount C of radiolabeled $^{131}$I-peptide grafted onto the surface (in nmol/mm$^2$) was calculated according to the following formula:

$$C = A\times C_0/A_0$$

where A is activity of the as-treated samples (in counts per minute/mm$^2$); $C_0$ the peptide concentration in the incubation solution (in g/L); $A_0$ the activity of 1 mL of mother solution (in cpm/L). This latter activity was calibrated by using the b-Vision™ Biospace software to determine the relationship between the μCi and cpm/L units. Such a fitting was performed by measuring the activities of given dilute solutions. By measuring the radioactivity of 10 solutions with β-Imager, A0 was determined to be equal to 1.0 $10^{10}$ cpm/L.

To evaluate the stability of the grafted peptides in water, the $^{131}$I-peptides activity (in cpm/mm$^2$) was calibrated for each analysis. Calibration of the entire probed area was first performed by setting the signal corresponding to the beta activity of 14C standard slides to a constant level. The $^{131}$I-peptides radioactive decay was finally taken into account since its half-life (90 days) is not negligible vs. the experiments duration. Moreover, the high resolution μ-imager provides information about the radiolabeled amino acids distributed onto the surface and proves the homogeneity of the grafting.

- Statistical Analysis:
  In terms of real-time PCR assay, all of the data were expressed as mean ± standard error, and analyzed statistically by the paired Student's t test method.

- General procedures used for the covalent association between GFR-binding compound(s) and bioactive carrier are as follows:

<u>Method 1: Surface preparation and covalent association with hydrogels (such as PLLA)</u>

The preparation of poly(acrylamide-co-acrylic acid) gel substrates was shown as an example in order to illustrate the effect of the GFR-binding compound(s) as defined herein. The synthesis of hydrogels is based on acrylamide and acrylic acid by polymerization in the presence of N,N'-methylene-bis-acrylamide as a cross-linker in an aqueous medium. Practically, 0.3 g of acrylamide (AM, Merck) has been dissolved in 5 mL of PBS 1X (Invitrogen). 30 μL of acrylic acid (AA, Merck), then x g (function as the stiffness) of N,N-methylene-bis-acrylamide was added under stirring. Then, a NaOH solution (1M, Aldrich) was added dropwise to reach a pH 8. The formation of hydrogel proceeded via free radical polymerization. The starting solution was degassed during 10 min by bubbling nitrogen in order to remove the oxygen which acts as an inhibitor for the free radical polymerization process. Nitrogen atmosphere was maintained above the solution and 50 μL (corresponding to 1/100 of the total volume) of Ammonium persulfate 10% (Biorad), (free radical initiator) and then 5 μL (corresponding to 1/1000 of total volume) of N,N,N',N'-tetramethylethylenediamine (catalyst, Aldrich) were added. The polymerizing solution was vortexed gently. Promptly, 0.5 mL of this gel solution was placed before polymerization on a glass slide and another slide was placed on the top of this structure. Reactivity ratios calculated by the Finemann-Ross and Kelen-Tudos methods showed that the copolymers were random with a reactivity ratio of rAM = 3.76 and rAA = 0.28. The materials obtained here have various stiffnesses ranging from 0.1 to 600 kPa.

Chemical functionalization with GFR-binding compound(s) as defined herein of poly(acrylamide-co-acrylic acid) gel substrates was performed via the -COOH (carboxyl) functions of their surface. Hydrogels were immersed in a solution of dimethylaminopropyl-3-ethylcarbodiimide hydrochloride (EDC, 0.2 M) + N-hydroxysuccinimide (NHS, 0.1 M) in 2-(N-morpholino)-ethane sulfonic acid (MES buffer, 0.1 M in MilliQ water) overnight at room temperature without stirring and then rinsed in MilliQ water (50 mL during 30 min). The immobilization of the GFR-binding compound(s) as defined herein was achieved in a solution of peptides/PBS 1X ($C = 10^{-3}$ M) for 18 hours at room temperature by using an orbital shaker. After grafting, the modified hydrogels were rinsed with PBS 1X for one week.

<u>Method 2: Covalent association with solid materials (such as ceramics or titanium)</u>

The material modifications were performed in a controlled Atmosphere (Ar Atmosphere, Glove Box). The strategy for covalent modification of GFR-binding compound(s) as defined herein involved (1) grafting of (3-aminopropyl)-triethoxysilane (concentration of 1×$10^{-2}$M, 4 hours) onto the surface of the biopolymer, (2) substitution of the terminal amine by a hetero-bifunctional cross-linker, a 3-succinimidyl-3-maleimidopropionate (concentration of 1×$10^{-3}$M, 4 hours) in order to (3) reacting of the "outer" maleimide group with the GFR-binding compound(s) as defined herein (concentration of 1×$10^{-3}$M, 18 hours) via a thiol group present in the terminal cysteine. After grafting, the modified materials were rinsed with PBS 1X buffer for 5 days.

Example 3: Covalent association with polymers (such as PET or PEEK)

The polymer used in this study is poly(ethylene terephthalate) (PET). The polymers were first treated in order to create -COOH (carboxyl) functions on the PET surfaces from -OH (hydroxyl) functions. Next, the PET-COOH samples were immersed in a solution of dimethylaminopropyl-3-ethylcarbodiimide hydrochloride (EDC, 0.2 M) + N-hydroxysuccinimide (NHS, 0.1 M) in 2-(N-morpholino)-ethanesulfonic acid (MES) buffer (0.1 M in MilliQ water) and the samples were rinsed in MilliQ water (50 mL for 30 min). The same protocol was used for all the polymers containing -OH (hydroxyl) functions on their extreme surface. Finally, the covalent immobilization of the peptides was achieved by using a solution of peptides/1X PBS (C = $10^{-3}$ M) incubated for 18 hours at room temperature. After grafting, the materials were rinsed in MilliQ water (100 ml) for 1 week.

**Example 1: covalent association between GFR-binding compounds of the present disclosure and titanium**

The bioactive carrier modifications were performed in a controlled atmosphere (Ar Atmosphere, Glove Box). The strategy for covalent association or immobilization of GFR-binding compounds involved (1) the association or grafting of (3-aminopropyl)-triethoxysilane (concentration of 1×$10^{-2}$ M, 4 hours) onto the surface of the bioactive carrier, (2) substitution of the terminal amine by a hetero-bifunctional cross-linker: a 3-succinimidyl-3-maleimidopropionate (concentration of 1×$10^{-3}$ M, 4 hours) in order to (3) react the "outer" maleimide group with a GFR-binding compound (concentration of 1×$10^{-3}$ M, 24 hours) via a thiol group present in the terminal cysteine. After covalent association, the covalent pharmaceutical associations were rinsed with Phosphate Buffered Saline (PBS 1X) buffer for 5 days. PBS is a buffer solution commonly used in the biological research. It is a water-based salt solution containing sodium phosphate, sodium chloride and, in some formulations, potassium chloride and potassium phosphate. The osmolarity and ion concentrations of the solutions match those of the human body (isotonic) and are thus physiological and non-toxic to the cells.

The bioactive carrier surface was characterized by covalently associating fluorescent GFR-binding compounds (herein SEQ ID NO: 1 coupled to fluorescein isothiocyanate (FITC), FIG. 1) and X-ray photoelectron spectroscopy (Table 1). The X-ray photoelectron spectroscopy was performed as described above.

The results presented in Table 1 were obtained with a titanium alloy material (Ti6Al4V metal alloy pellets) as bioactive carrier covalently associated with GFR-binding compounds of SEQ ID NO: 2 to 6.

Table 1

| **Name** | **At. % C1s** | **At. % N1s** |
|---|---|---|
| Titanium | 29,07 | no detection |
| Titanium grafted covalently with SEQ ID NO: 2 | 43,65 | 5,87 |
| Titanium grafted covalently with SEQ ID NO: 3 | 42,02 | 5,47 |
| Titanium grafted covalently with SEQ ID NO: 4 | 39,95 | 5,11 |
| Titanium grafted covalently with SEQ ID NO: 5 | 40,58 | 5,2 |
| Titanium grafted covalently with SEQ ID NO: 6 | 44,01 | 4,89 |

**Example 2: covalent association between GFR-binding compound(s) of the present disclosure and PEEK**

A polyetheretherketone (PEEK) was treated with ethylene diamine ($NH_2$=$NH_2$) to create -$NH_2$ (amine) functions on the PEEK surfaces from ketone (=O) functions. Next, the PEEK-$NH_2$ samples were immersed in a solution of a hetero-bifunctional cross-linker: a 3-succinimidyl-3-maleimidopropionate (concentration of 1×$10^{-3}$ M, 4 hours) in order to react with the "outer" maleimide group with the GFR-binding compound(s) of SEQ ID NO: 7 (concentration of 1×$10^{-3}$ M, 24 hours) via a thiol group present in the terminal cysteine thereof. After covalent association, the pharmaceutical association was rinsed with PBS 1X for 5 days.

The bioactive carrier surface was characterized by covalently associating fluorescent GFR-binding compounds

of SEQ ID NO: 7 and by other methods such as X-ray photoelectron spectroscopy (Table 2). The X-ray photoelectron spectroscopy was performed as described above.

The results presented in Table 2 were obtained by with a PEEK material as a bioactive carrier covalently associated with a GFR-binding compound of SEQ ID NO: 7.

Table 2

| Name | At. % C1s | At. % N1s |
|---|---|---|
| PEEK | 86,56 | no detection |
| PEEK grafted covalently with SEQ ID NO: 7 | 71,61 | 4,98 |

**Example 3: covalent association between GFR-binding compound(s) of the present disclosure and PLLA**

PLLA (Polylactic acid) was immersed in a solution of dimethylaminopropyl)-3-ethylcarbodiimide hydrochloride (EDC, 0.2 M) + N-hydroxysuccinimide (NHS, 0.1 M) in (2-(N-morpholino)-ethanesulfonic acid (MES buffer, 0.1 M in MilliQ water) and then rinsed in MilliQ water (during 30 min). The covalent association was performed in a solution of GFR-binding compounds of SEQ ID NO: 8 (concentration of $1\times10^{-3}$ M, 18 hours, room temperature). After the covalent association, the pharmaceutical association was rinsed with PBS 1X for 5 days.

The bioactive carrier surface was characterized by covalently associating fluorescent GFR-binding compounds of SEQ ID NO: 8 and by using other methods such as X-ray photoelectron spectroscopy (Table 3). The X-ray photoelectron spectroscopy was performed as described above.

The results presented in Table 3 were obtained by using PLLA as bioactive carrier covalently associated with a GFR-binding compound of SEQ ID NO: 8.

Table 3

| Name | At. % C1s | At. % N1s |
|---|---|---|
| PLLA | 69,02 | no detection |
| PLLA grafted covalently with SEQ ID NO: 8 | 72,31 | 5,07 |

**Example 4: Non-covalent association between GFR-binding compound(s) and bioactive carrier(s) of the present disclosure**

Non-covalent associations of the bioactive carriers were performed under atmospheric conditions (air) at room temperature (about 22°C). The non-covalent association was carried out using a mixture of GFR-binding compound of SEQ ID NO: 9 (concentration of $1\times10^{-3}$ M) with the apatite ceramics or a mixture of GFR-binding compound of SEQ ID NO: 10 (concentration of $1\times10^{-3}$ M) with the type-I collagen sponge. The non-covalent pharmaceutical associations were characterized by covalently associating fluorescent GFR-binding compounds of SEQ ID NO: 9 and 10 (covalently bound to fluorescein isothiocyanate (FITC), FIG. 1). The results shown in FIG. 1 were obtained from experiments performed with apatite ceramics and a type-I collagen sponge and demonstrates that the interaction between the GFR-binding compounds and the apatite ceramics or the type-I collagen is stable as no significant release of GFR-binding compounds was observed after 3, 7 or 10 days (FIG. 2a and b).

**Example 5: Osteosarcoma recoding**

Selected GFR-binding compounds (SEQ: ID NO: 11 to 20) were covalently associated with PET using a method described above and contacted with human osteosarcoma cells (obtained from ATCC®). The mixtures tumor cells / pharmaceutical association were then cultured in Dulbecco's Modified Eagle Medium (DMEM, Invitrogen®) supplemented with 10% (v/v) fetal bovine serum (FBS), 1% penicillin/streptomycin and incubated in a humidified atmosphere containing 5% (v/v) $CO_2$ at 37°C. All of the cell culture experiments were performed without any serum in the medium for the first 8 hours of culture. All cells were used at a low passage number (≤ passage 10), were subconfluently cultured and were seeded at 10 000 cells/cm$^2$ for the purpose of the experiments.

The cell phenotype was analyzed after 24 hours of cell culture. The vast majority (> 98%) of the human osteosarcoma cells adhere, spread, and differentiate into osteocyte cells as confirmed by analysis of DMP-1 (Dentin matrix acidic phosphoprotein 1), Sclerostin and RANK-L (receptor activator of nuclear factor kappa B ligand) expression (FIG. 3a). During cell recoding, osteosarcoma cells undergo important changes in their cell morphology such as cell shape

modification, cell body size reduction and increase in cell processes in order to obtain the characteristic osteocyte morphology as shown in FIG. 3b wherein, after 18 and 24h of cell culture, the cells were immunostained with labeled antibodies against actin in order to visualize and monitor the actin cytoskeleton, previously reported to play an important role in the establishment of the osteocyte morphology (FIG. 3b). The cells were also stained with antibodies against vinculin as a cytoplasmic focal cell adhesion marker in order to visualize in detail the cell morphology. The observed morphologies of the recoded cells were compared with the osteosarcoma cell morphology, immunostained using the same procedure. After 24 hours of cell culture the immunofluorescence labeling shows that the cell shape has already undergone some significant modifications and that the dendritic processes have already started (Fig. 3b). These cells presented a characteristic dendritic-like morphology, a total reduction in cell body volume of around 50% and expressed a set of known osteocytic markers. These results demonstrate that GFR-binding compounds associated with bioactive carrier both as defined herein induce the recoding of human osteosarcoma cells into osteocytes in a short period of time (24 hours).

**Example 6: Rhabdosarcoma recoding**

Selected GFR-binding compounds (SEQ: ID NO: 21 to 30) were covalently associated with PET using a method described above and contacted with human rhabdosarcoma cells (obtained from ATCC®). The mixtures tumor cells / pharmaceutical association were then cultured in Dulbecco's Modified Eagle Medium (DMEM, Invitrogen®) supplemented with 10% (v/v) fetal bovine serum (FBS), 1% penicillin/streptomycin and incubated in a humidified atmosphere containing 5% (v/v) $CO_2$ at 37°C.All cell culture experiments were performed without any serum in the medium for the first 8 hours of culture. All cells were used at a low passage number (≤ passage 10), were subconfluently cultured and were seeded at 10 000 cells/$cm^2$ for the purpose of the experiments.

It was observed that these tumor cells were converted into muscle cells (myocyte like-cells) in the presence of pharmaceutical associations of the invention by analyzing the expression of the myogenic biomarkers MLC-1 (myocyte light chain 1), GATA-4 and α-Sarcomeric actin. An increased expression of these genes was observed after 24 hours of culture (FIG. 4). For that, a Quantitative Real Time PCR was performed as described in the Methods section.

**Example 7: Mesenchymal chondrosarcoma recoding**

Selected GFR-binding compounds (SEQ: ID NO: 31 to 40) were covalently associated with PET using a method described above and contacted with human rhabdosarcoma cells (obtained from ATCC®). The mixtures tumor cells / pharmaceutical association were then cultured in Dulbecco's Modified Eagle Medium (DMEM, Invitrogen®) supplemented with 10% (v/v) fetal bovine serum (FBS), 1% penicillin/streptomycin and incubated in a humidified atmosphere containing 5% (v/v) $CO_2$ at 37°C. All cell culture experiments were performed without any serum in the medium for the first 8 hours of culture. All cells were used at a low passage number (≤ passage 10), were subconfluently cultured and were seeded at 10 000 cells/$cm^2$ for the purpose of the experiments.

It was observed that these tumor cells were converted into cartilage cells (chondrocyte like-cells) in the presence of pharmaceutical associations of the invention by analyzing the expression of the chondrocytic biomarkers Sox9, IBSP (Sialoprotein II) and collagen IV. An increased expression of these genes was observed after 24 hours of culture (FIG. 5). For that a Quantitative Real Time PCR was performed as described in the Methods section.

**Example 8: Adenocarcinoma recoding**

Selected GFR-binding compounds (SEQ: ID NO: 41 to 50) were covalently associated with PET using a method described above and contacted with human adenocarcinoma cells (obtained from ATCC®). The mixtures tumor cells / pharmaceutical association were then cultured in RPMI-1640 medium supplemented with 10% (v/v) fetal bovine serum (FBS), 1% penicillin/streptomycin and incubated in a humidified atmosphere containing 5% (v/v) $CO_2$ at 37°C. All cell culture experiments were performed without any serum in the medium for the first 8 hours of culture. All cells were used at a low passage number (≤ passage 10), were subconfluently cultured and were seeded at 10 000 cells/$cm^2$ for the purpose of the experiments.

It was observed that these tumor cells were converted into epithelial cells (fibrocyte like-cells) in the presence of pharmaceutical associations of the invention by analyzing the expression of the fibrocytic biomarkers MMP-9, Vimentin and α-SMA. An increased expression of these genes was observed after 24 hours of culture (FIG. 6). For that a Quantitative Real Time PCR was performed as described in the Methods section.

**Example 9: Recoding of neoplastic cells demonstrated by significant reduction of proliferation markers**

Neoplastic cells (in this example osteosarcoma cells) were cultured on pharmaceutical associations comprising

PET as a bioactive carrier and one of GFR-binding compounds of SEQ ID NO: 11 to 14,24 to 26 and 51 to 63. A rapid (24 hours) decrease of the tumor markers was observed (FIG. 7). The adhesion checkpoint then activates the tumor suppressor proteins p53 and pRB. It was observed that, in tumor cells cultured on pharmaceutical associations of the invention, the expression of p53 decreases as demonstrated by quantification of protein expression level (immunofluorescence staining and quantification using ImageJ as described in the general methods section) (FIG. 7A) and the pRB is dephosphorylated as demonstrated by a quantification of protein phosphorylation level (immunofluorescence staining and subsequent quantification by using the ImageJ software as described in the general methods section) (FIG. 7B).

**Example 10: Adhesion Checkpoint activation**

Neoplastic cells (in this example osteosarcoma cells) were cultured on pharmaceutical associations comprising PET as a bioactive carrier and one of GFR-binding compounds of SEQ ID NO: 12, 19, 30 or 39. The main signaling pathways include: Ras/MAP kinase, FAK/Src kinase and PIP2 which are decreased after 24 hours as shown by western blot quantifications (FIG. 8). This demonstrated by the absence of phosphorylation at Thr202 and Tyr204 on ERK (FIG. 8A). Moreover, no phosphorylation at Thr416 but phosphorylation of Tyr527 was observed on Src kinase (FIG. 8B). Finally, a decrease of 3-phosphoinositide-dependent protein kinase 1 (PDK1) protein expression was also was observed (FIG. 8C).

**Example 11: Decrease of Paxillin and Vinculin gene expression**

Neoplastic cells (in this example osteosarcoma cells) were cultured on pharmaceutical associations comprising PET as a bioactive carrier and one of GFR-binding compounds of SEQ ID NO: 12, 19, 30 or 39. After 24 hours, the Paxillin and Vinculin gene expression decreased as shown by RT-PCR (FIG. 9).

**Example 12: Cyclin D profile**

Neoplastic cells (in this example osteosarcoma cells) were cultured on pharmaceutical associations comprising PET as a bioactive carrier and one of GFR-binding compounds of SEQ ID NO: 12, 19, 30 or 39. The Cyclin D (the arithmetic mean of Cyclin D1, Cyclin D2 and Cyclin D3) gene expression decreased after the first 30 min of cell culture as shown by RT-PCR (FIG. 10). These data indicate the entry of the cells into G0 quiescence phase.

**Example 13: GFR-binding compound density calculation.**

The density of the GFR-binding compound(s) on bioactive carriers is measured using radio-labeled molecules according to the procedure described above in the Methods section and was found to lie between 0.3 and 2.8 pmol/mm$^2$ depending on the size of the GFR-binding compound (SEQ ID No: 13, 20 or 56) (Fig. 11).

**Example 14: Growth factor receptor activation**

The data summarized in Table 4 below demonstrate that the tumor cell recoding action ((in this example: human osteosarcoma cells, MG63, via quantification of pRB) of pharmaceutical associations according to certain embodiments of the present disclosure involves the activation of growth factor receptors as demonstrated by the activation of the Smad1/5/8 signaling pathway (via quantification of western blots of phospho-smad 1/5/8):

Table 4

| SEQ ID N° | AA sequences | p-Smad1/5/8 (%) | pRB (%) |
|---|---|---|---|
| SEQ ID NO: 64 | SIPKASSTPTELSPINMLYF | 37 | 23 |
| SEQ ID NO: 65 | TKPTSTPTKLSPINMLYF | 36 | 10 |
| SEQ ID NO: 66 | YVPKPSSTPTKLSPINMLYF | 38 | 8 |
| SEQ ID NO: 67 | ASAAPSSVPQALSSLSILFF | 38 | 15 |
| SEQ ID NO: 68 | ASASPSSVSQDLSSLSILFF | 40 | 14 |
| SEQ ID NO: 69 | VVPKPSSAPTQLEPISILYL | 39 | 20 |
| SEQ ID NO: 70 | AVPKASSAPTKLEPISILYL | 67 | 21 |
| SEQ ID NO: 71 | KVGKASSVPTKLEPISILYL | 66 | 17 |

(continued)

| SEQ ID N° | AA sequences | p-Smad1/5/8 (%) | pRB (%) |
|---|---|---|---|
| SEQ ID NO: 72 | SSVKSQPSRVHHKPLSMLYV | 47 | 19 |
| SEQ ID NO: 73 | RNVQSRPTQVQLKPLSMLYV | 48 | 16 |
| SEQ ID NO: 74 | KIPKASSVPQELEPLPIVYY | 44 | 9 |
| SEQ ID NO: 75 | GIPEPSSVPQKMEPLPIVYY | 51 | 10 |
| SEQ ID NO: 76 | SIPKASSVPQELEPLPIVYY | 54 | 8 |
| SEQ ID NO: 77 | ASAAPSSVPQALEPLTILYY | 55 | 15 |
| SEQ ID NO: 78 | ASASPSSVSQDLEPLTILYY | 52 | 18 |
| SEQ ID NO: 79 | NDEGLESVPTEEEPLTILYY | 58 | 17 |
| SEQ ID NO: 80 | NDEGLESVPTEESSLSILFF | 27 | 9 |
| SEQ ID NO: 81 | RVPSTSSAPTKTSATSVLYY | 34 | 17 |
| SEQ ID NO: 82 | ASAAPSSAPTALSATSVLYY | 41 | 19 |
| SEQ ID NO: 83 | TVPKPSSAPTQLRSVKVAKV | 35 | 12 |
| SEQ ID NO: 84 | KVGKASSVPQKLEPLPIVYY | 33 | 9 |
| SEQ ID NO: 85 | KASKASSVPQKLEPLPIVYY | 22 | 10 |
| SEQ ID NO: 86 | RNVQSRPVPTQLSPISVLYK | 26 | 24 |
| SEQ ID NO: 87 | KIPKASSVPTELSPISVLYK | 32 | 21 |
| SEQ ID NO: 88 | ASAAPSSVPQALRSVKVAKV | 37 | 18 |
| SEQ ID NO: 89 | VSQDLRSVKVAKV | 36 | 17 |
| SEQ ID NO: 90 | ASASPSSVPQDLRSVKVAKV | 56 | 9 |
| SEQ ID NO: 91 | NDEGLESVPTEERSVKVAKV | 55 | 12 |
| SEQ ID NO: 92 | TQVKMRPVQVRKI | 44 | 8 |
| SEQ ID NO: 93 | KIPKASSTPTELSPINMLYF | 47 | 7 |
| SEQ ID NO: 94 | GIPEPSSTPTKMSPINMLYF | 39 | 9 |
| SEQ ID NO: 95 | VPTGQSAISMLYL | 52 | 17 |
| SEQ ID NO: 96 | NDEGLESVPTEESAISMLYL | 78 | 22 |
| SEQ ID NO: 97 | SSVKSQPSRVHHSPISILFI | 39 | 14 |
| SEQ ID NO: 98 | RNVQSRPTQVQLSPISILFI | 34 | 17 |
| SEQ ID NO: 99 | YVPKPSSAPTKLNAISVLYF | 66 | 21 |
| SEQ ID NO: 100 | HVPKPSSAPTKLEPISILYL | 75 | 19 |

**Example 15: RMSD measurement and recoding activity**

The data summarized in Table 5 below demonstrate that pharmaceutical associations according to certain embodiments of the present disclosure containing GFR-binding compounds as disclosed herein having a RMSD value of 2.45Å or less, induce the particularly efficient recoding (as shown via quantification of pRB) of neoplastic cells (in this example: human osteosarcoma cells, MG63) into healthy cells:

Table 5

| SEQ ID N° | AA sequences | RMSD (Å) | pRB (%) |
|---|---|---|---|
| SEQ ID NO: 64 | SIPKASSTPTELSPINMLYF | 0.73 | 23 |
| SEQ ID NO: 65 | TKPTSTPTKLSPINMLYF | 0.96 | 10 |

(continued)

| SEQ ID N° | AA sequences | RMSD (Å) | pRB (%) |
|---|---|---|---|
| SEQ ID NO: 66 | YVPKPSSTPTKLSPINMLYF | 0.77 | 8 |
| SEQ ID NO: 67 | ASAAPSSVPQALSSLSILFF | 0.81 | 15 |
| SEQ ID NO: 68 | ASASPSSVSQDLSSLSILFF | 0.79 | 14 |
| SEQ ID NO: 69 | VVPKPSSAPTQLEPISILYL | 0.72 | 20 |
| SEQ ID NO: 70 | AVPKASSAPTKLEPISILYL | 0.79 | 21 |
| SEQ ID NO: 71 | KVGKASSVPTKLEPISILYL | 0.79 | 17 |
| SEQ ID NO: 72 | SSVKSQPSRVHHKPLSMLYV | 0.71 | 19 |
| SEQ ID NO: 73 | RNVQSRPTQVQLKPLSMLYV | 0.79 | 16 |
| SEQ ID NO: 74 | KIPKASSVPQELEPLPIVYY | 0.84 | 9 |
| SEQ ID NO: 75 | GIPEPSSVPQKMEPLPIVYY | 0.85 | 10 |
| SEQ ID NO: 76 | SIPKASSVPQELEPLPIVYY | 0.82 | 8 |
| SEQ ID NO: 77 | ASAAPSSVPQALEPLTILYY | 0.87 | 15 |
| SEQ ID NO: 78 | ASASPSSVSQDLEPLTILYY | 0.91 | 18 |
| SEQ ID NO: 79 | NDEGLESVPTEEEPLTILYY | 0.83 | 17 |
| SEQ ID NO: 80 | NDEGLESVPTEESSLSILFF | 0.56 | 9 |
| SEQ ID NO: 81 | RVPSTSSAPTKTSATSVLYY | 0.87 | 17 |
| SEQ ID NO: 82 | ASAAPSSAPTALSATSVLYY | 0.84 | 19 |
| SEQ ID NO: 83 | TVPKPSSAPTQLRSVKVAKV | 1.11 | 12 |
| SEQ ID NO: 84 | KVGKASSVPQKLEPLPIVYY | 1.1 | 9 |
| SEQ ID NO: 85 | KASKASSVPQKLEPLPIVYY | 0.77 | 10 |
| SEQ ID NO: 86 | RNVQSRPVPTQLSPISVLYK | 1.42 | 24 |
| SEQ ID NO: 87 | KIPKASSVPTELSPISVLYK | 1.35 | 21 |
| SEQ ID NO: 88 | ASAAPSSVPQALRSVKVAKV | 1.27 | 18 |
| SEQ ID NO: 89 | VSQDLRSVKVAKV | 0.96 | 17 |
| SEQ ID NO: 90 | ASASPSSVPQDLRSVKVAKV | 1.42 | 9 |
| SEQ ID NO: 91 | NDEGLESVPTEERSVKVAKV | 1.45 | 12 |
| SEQ ID NO: 92 | TQVKMRPVQVRKI | 0.99 | 8 |
| SEQ ID NO: 93 | KIPKASSTPTELSPINMLYF | 1.13 | 7 |
| SEQ ID NO: 94 | GIPEPSSTPTKMSPINMLYF | 1.31 | 9 |
| SEQ ID NO: 95 | VPTGQSAISMLYL | 1.23 | 17 |
| SEQ ID NO: 96 | NDEGLESVPTEESAISMLYL | 0.96 | 22 |
| SEQ ID NO: 97 | SSVKSQPSRVHHSPISILFI | 0.79 | 14 |
| SEQ ID NO: 98 | RNVQSRPTQVQLSPISILFI | 0.79 | 17 |
| SEQ ID NO: 99 | YVPKPSSAPTKLNAISVLYF | 0.71 | 21 |
| SEQ ID NO: 100 | HVPKPSSAPTKLEPISILYL | 0.66 | 19 |

Likewise, using the method detailed in the description, the data summarized in Table 6 below demonstrate that pharmaceutical associations according to certain embodiments of the present disclosure containing GFR-binding peptides as disclosed herein in association with PET as already described herein:

- for SEQ ID NO: 201 to 209, on MG63 cells;

- for SEQ ID NO: 210 to 217, on human rhabdosarcoma cells;
- for SEQ ID NO: 218 to 224, on mesenchymal chondrosarcoma cells; and
- for SEQ ID NO: 225 to 238, on human adenocarcinoma cells;

induce the particularly efficient recoding (as shown via quantification of pRB et P53) of neoplastic cells (in these examples: MG63 cells, human rhabdosarcoma cells, mesenchymal chondrosarcoma cells, and human adenocarcinoma cells, as stated above) into healthy cells. It should also be noted that GFR-binding peptides having a RMSD value of 2.45Å or less, are particularly advantageous and efficient in recoding cells:

Table 6

| SEQ ID N° | AA sequences | RMSD (Å) | P53 (%) | pRB (%) |
|---|---|---|---|---|
| SEQ ID NO: 201 | NDEGLESVPEDLSSLSVLFF | 1,92 | 29 | 12 |
| SEQ ID NO: 202 | RVPSTSSVPTGQSAISTLYL | 1,78 | 25 | 10 |
| SEQ ID NO: 203 | RVPSTSSAPTKMNAISMLYF | 1,5 | 31 | 9 |
| SEQ ID NO: 204 | GIPEPSSAPTELSATSILYY | 1,66 | 33 | 16 |
| SEQ ID NO: 205 | STPPTSSVPTELSPISTLYK | 1,71 | 41 | 20 |
| SEQ ID NO: 206 | GIPEPSSAPVDLKPLSTLYV | 1,53 | 32 | 12 |
| SEQ ID NO: 207 | AASKASSVPQEEEPLPMVYY | 1,54 | 24 | 17 |
| SEQ ID NO: 208 | KIPKASSVSQKMEPLTMLYY | 1,7 | 30 | 12 |
| SEQ ID NO: 209 | KIPKASSVPTGGSN ITVQI M | 1,43 | 45 | 11 |
| SEQ ID NO: 210 | AASKASSSRVELRSVKIAKV | 1,23 | 42 | 22 |
| SEQ ID NO: 211 | KIPKASSTQVRLRPVQIRKI | 1,87 | 42 | 19 |
| SEQ ID NO: 212 | STPPTSSSRVQLSAISMLYL | 1,44 | 27 | 11 |
| SEQ ID NO: 213 | RVPSTSSTQVKMSAISMLYL | 0,97 | 26 | 12 |
| SEQ ID NO: 214 | GIPEPSSVSQEESSLSTLFF | 0,88 | 31 | 18 |
| SEQ ID NO: 215 | KIPKASSSRVRLSSLSTLFF | 1,32 | 30 | 19 |
| SEQ ID NO: 216 | AASKASSTPTDLNAISTLYF | 1,66 | 27 | 19 |
| SEQ ID NO: 217 | RVPSTSSTQVDLNAISVLYF | 1,84 | 24 | 18 |
| SEQ ID NO: 218 | GIPEPSSVSQELSATSMLYY | 1,49 | 28 | 17 |
| SEQ ID NO: 219 | KIPKASSTQVGQSATSILYY | 1,93 | 31 | 14 |
| SEQ ID NO: 220 | STPPTSSAPVGQSPISML YI | 1,83 | 34 | 15 |
| SEQ ID NO: 221 | KIPKASSTPTRLSPISMLFI | 1,45 | 37 | 21 |
| SEQ ID NO: 222 | GIPEPSSAPTRLEPISMLYL | 1,56 | 36 | 8 |
| SEQ ID NO: 223 | KIPKASSVSQQLEPISTLYL | 1,57 | 29 | 9 |
| SEQ ID NO: 224 | STPPTSSVPTEVSPINTLYF | 1,78 | 26 | 13 |
| SEQ ID NO: 225 | AASKASSSRVKMSPINVLYF | 1,75 | 25 | 12 |
| SEQ ID NO: 226 | KIPKASSVPTKMKPLSVLYV | 1,68 | 31 | 16 |
| SEQ ID NO: 227 | RVPSTSSAPTKMKPLSMLYV | 1,48 | 32 | 14 |
| SEQ ID NO: 228 | STPPTSSVSQKMKPLSILYV | 1,46 | 26 | 18 |
| SEQ ID NO: 229 | KIPKASSVPAQLEPLPIVYY | 1,71 | 31 | 23 |
| SEQ ID NO: 230 | GIPEPSSAPVGQEPLPMVYY | 1,74 | 30 | 19 |
| SEQ ID NO: 231 | RVPSTSSVPEKMEPLTMLYY | 1,8 | 22 | 18 |
| SEQ ID NO: 232 | GIPEPSSAPVEEEPLTTLYY | 1,75 | 21 | 13 |
| SEQ ID NO: 233 | STPPTSSTPTKMSNITTQIM | 1,97 | 43 | 23 |

(continued)

| SEQ ID N° | AA sequences | RMSD (Å) | P53 (%) | pRB (%) |
|---|---|---|---|---|
| SEQ ID NO: 234 | RVPSTSSVPAEESNITVQIM | 1,92 | 45 | 22 |
| SEQ ID NO: 235 | AASKASSVPQRLRSVKVAKV | 2,01 | 40 | 26 |
| SEQ ID NO: 236 | KIPKASSTQVRLRSVKTAKV | 2,04 | 37 | 25 |
| SEQ ID NO: 237 | AASKASSVPEKMRPVQTRKI | 2,14 | 42 | 22 |
| SEQ ID NO: 238 | GIPEPSSAPVDMRPVQIRKI | 2,17 | 40 | 23 |

**Example 16: The presence of adhesion components in the extracellular microenvironment inhibits cell conversion**

In order to demonstrate the importance of adhesion proteins inhibition in the recoding process of a neoplastic cell, a PET substrate was covalently functionalized with RGD peptides (the primary integrin recognition site present in many ECM proteins (e.g., fibronectin)) and an exemplary GFR-binding compound according to the present disclosure using methods already described herein.

This modified substrate was then cultured with human osteosarcoma cells from ATCC® in Dulbecco's Modified Eagle Medium (DMEM,Invitrogen®) supplemented with 10% (v/v) fetal bovine serum (FBS), 1% penicillin/streptomycin and incubated in a humidified atmosphere containing 5% (v/v) $CO_2$ at 37°C.All cell culture experiments were performed without any serum in the medium for the first 8 hours of culture. All cells were used at a low passage number (≤ passage 10), were subconfluently cultured and were seeded at 10 000 cells/$cm^2$ for the purpose of the experiments. As shown in FIG. 12A and 12B, the presence of covalently immobilized components favorising the adhesion inhibited the process of recoding of the osteosarcoma cells into osteocytes as characterized by RT-PCR and cell morphology observation.

It was also confirmed that the presence of RGD peptides did not modify the density of covently grafted GFR-binding compounds. Indeed, it was observed that the GFR-binding compound density was stable (around 1.3 pmol/$mm^2$) with or without RGD peptides (FIG. 12C).

It was also shown that the RGD peptides interacted specifically with integrin $\alpha3\beta1$. Indeed, inhibition of $\alpha3\beta1$, $\alpha5\beta3$ and $\alpha v\beta3$ integrins (i.e., the three predominant receptor dimers involved in binding ECM molecules containing RGD motifs for the osteosarcoma cells) by specific antibodies, indicated that cells predominantly engaged $\alpha3\beta1$ integrins to interact with the RGD-modified substrate on the basis of the observed decrease of osteosarcoma cells adhesion in response to anti-∟$\alpha3\beta1$ treatment (Fig. 12D). This was not observed when $\alpha v\beta3$ and $\alpha5\beta3$ were blocked (Fig. 12E). For these antibody inhibition studies, cells were preincubated with 5g/mL anti-$\alpha3\beta1$ (clone JBS5), anti-$\alpha v\beta1$ (clone LM609) and anti-$\alpha5\beta3$.

**Example 17: Integrin engagement inhibits cell conversion**

Using the same setup as used in Example 16 (tumor cell and PET functionalized with RGD peptides and an exemplary GFR-binding compound), silencing of integrin $\alpha3$ and integrin $\beta1$ expression using shRNA was shown to reduce integrin $\alpha3$ and integrin $\beta1$ mRNA levels by 60-70% and 50-60% respectively and integrin $\alpha3$ and integrin $\beta1$ proteins levels by 60-70% after 24h. It was then shown that both integrins $\alpha3$ and $\beta1$ shRNAs modulated osteosarcoma adhesion and spreading on RGD-modified substrates (Fig. 13 A, B).

Without being bound to any specific theory, integrin $\alpha3\beta1$ thus appears to be an important factor in the conservation of osteosarcomas tumourous profile and appears to play an important role in the recoding of osteosarcomas into osteocytes. Herein, $\alpha3\beta1$ integrin engagement appears to be involved in the inhibition of osteocytic recoding but not in osteosarcoma induction. Indeed, it was observed, within the framework of this experiment, that spheroid-like structures of osteosarcomas became progressively predominant (Fig. 13C). These results seems to demonstrate that integrin engagement has the ability to influence the behavior of human osteosarcoma towards spheroid-like structures or towards osteocytes.

**Example 18: In vivo rat study**

A. Osteosarcoma model

The OSRGa rat osteosarcoma orthotopic xenograft model in immunodeficient mice is the model of choice for the present study. This model is indeed characterized by the development of osteosarcoma tumors presenting very high similarities with the human osteosarcoma tumors both with regards to the tumor temporal development as well as its

biological characteristics. Just like the human osteosarcoma, the OSRGa model is characterized by tumor proliferation associated with pulmonary metastasis and excessive bone remodeling. Indeed, OSRGa tumor cells, which multiply in contact with bone, produce bone matrix and induce an increase in mature and immature bone (woven bone) formation as well as a malignant osteolysis. In addition, similar to human osteosarcoma, the OSRGa tumors are comprised of tumor osteoblasts presenting marked cytonuclear abnormalities and high number of mitoses. This model is also highly reproducible and induces local orthotopic tumor formation with a very high incidence.

The OSRGa cell line isolated from osteosarcoma rat tumor is well characterized for both its *in vitro* phenotype and tumor development ability *in vivo.* When injected in contact with the mouse tibia periosteum, these cells induce de local development of osteosarcoma tumor associated with pulmonary metastasis providing a highly reproducible model most for preclinical animal studies.

B. Experimental protocol

B.1. Orthotopic xenograft procedure

After 5 days of adaptation period, 3 to 4 weeks old female immunodeficient mice (Rj:NMRI-nude strain) were subjected to rat osteosarcoma OSRGa cells orthotopic xenograft. For establishing the osteosarcoma model, $2.10^6$ OSRGa cells as a 10μL suspension were injected intramuscularly in contact with the right tibia of the nude mice, after scratching the periosteum. An analgesic administration was performed throughout the injection.

Osteosarcoma tumor development was observed in 100% of the OSRGa orthotopic xenografts of the present study. The tumor growth was assessed twice per week until a mean tumor volume of around 500 $mm^3$ was reached 23 days after OSRGa cell injection. The mice were then randomly assigned to 8 different groups before compound administration.

B.2. Compound administration

24 days after OSRGa cell injection, a $10^{-3}$M 1:1 mixture of GFR-binding peptides (SEQ ID NO: 240 and 239) covalently bound to a ceramic gel was injected inside the tumor. The injected volume of 90 μl was chosen according to the mean tumor volume for an efficient distribution. SEQ ID NO: SEQ ID NO: 240 and 239 were each grafted (via covalent binding of a N-terminal cysteine using a procedure already described herein) on a ceramic gel. The control conditions include a "No administration" condition for which no compound injections were performed in the tumor as well as a non grafted ceramic gel implant (Control 1). Hereinafter the various experimental conditions, the results for which are detailed below:

| Experimental conditions | Code | Type of product | Reference or test product |
|---|---|---|---|
| No administration | No administration | None | Reference |
| Ceramic gel | Control 1 | Gel | Reference |
| Ceramic gel grafted with SEQ ID NO: SEQ ID NO: 240 and 239 | Product | Gel | Test |

Two experimental endpoints were tested in the present study for the 3 experimental conditions: 3 days (1 injection) and 25 days (4 injections) after the first compound administration injection. The experimenters were blinded to group assignment until the completion of the procedure, and again at the time of the results assessment analysis performed with various techniques.

B.3. Histopathological analysis

For all the experimental conditions, the tumors were removed and paraffin embedded after which successive transverse sections were produced. A thorough histopathological analysis has been then performed on these sections in order to assess tissue and cellular structure and therefore tumor progression and tumor cell recoding with the following stainings:

- Hematoxylin-Eosin (HE) staining to qualitatively analyze tissue morphology
- Masson's Trichrome (TM) staining for distinguishing cells from surrounding connective tissue
- Immunohistochemistry assays: E11 antigen, Sclerostin, DMP1, CD44

C. Results

Osteosarcoma cell recoding was assessed by analysis of the cell transformation from tumor cell towards an osteocyte cell. The thorough histopathological analysis was carried out and permitted to determine a percentage of osteosarcoma cell recoding for the 3 experimental conditions and for the 2 endpoints of each condition via various parameters.

Regarding the first endpoint of the study (3 days, 1 injection), the following results have been obtained:

| Experimental conditions | Recoding efficiency(1 injection) |
|---|---|
| No administration | 1,2% |
| Ceramic gel | 1,7% |
| Ceramic gel grafted with SEQ ID NO: SEQ ID NO: 240 and 239 | 32% |

Regarding the second endpoint of the study (25 days, 4 injections), the following results have been obtained:

| Experimental conditions | Recoding efficiency (4 injections) |
|---|---|
| No administration | 0,8% |
| Ceramic gel | 2,6% |
| Ceramic gel grafted with SEQ ID NO: SEQ ID NO: 240 and 239 | 82% |

Almost no tumor osteosarcoma cell recoding towards osteocyte cells was observed for the two control conditions and for both of the experimental endpoints. Tumor cell progression was therefore not decreased. Tumor osteosarcoma cell recoding was observed for the test condition after 1 injection. It was particularly noted that efficiency of the cell recoding process was highly increased after 4 injections of the test condition.

D. Conclusions

It was shown that efficient recoding of osteosarcoma cells in an osteosarcoma animal model presenting all of the characteristics of human osteosarcoma, was possible using representative GFR-binding compounds grafted covalently to a ceramic gel.

The pharmaceutical associations of the present disclosure have been shown to represent new therapeutic solutions for osteosarcoma with the unique ability to induce tumor cell transformation towards osteocytes, a healthy cell type, thereby blocking tumor progression and regenerating the affected tissue.

## Claims

**1.** A pharmaceutical association for use in the treatment, prevention and/or diagnostic of a neoplastic disease, said association comprising at least one growth factor receptor-binding compound, which activates at least one growth factor receptor of a neoplastic cell, and at least one bioactive carrier forming at least one covalent or non-covalent interaction with said at least one growth factor receptor-binding compound;

wherein said association reduces or suppresses, in the neoplastic cell, the gene expression of at least one cyclin D and/or reduces or suppresses the formation of at least one complex formed between said at least one cyclin D and at least one of cyclin dependent-kinase 4 or 6;
wherein said growth factor receptor-binding compound comprises a peptide with four amino acids PEP1;
wherein PEP1 is SAIS;
wherein said growth factor receptor-binding compound is a peptide or a peptidomimetic;
wherein said growth factor receptor-binding compound is a non-cyclic peptide with between 8 and 25 amino acids or a non-cyclic peptidomimetic having a molecular weight comprised between 600 and 4,000 Daltons and comprises between 8 and 25 amino acids;
wherein said growth factor receptor-binding compound is a cyclic peptide with between 10 and 60 amino acids or a cyclic peptidomimetic comprising between 10 and 60 amino acids having a molecular weight comprised

between 1,000 and 7,000 Da;
and wherein said bioactive carrier is a biomaterial.

2. A pharmaceutical association for a use according to claim 1, wherein said growth factor receptor-binding compound comprises a peptide with eight amino acids PEP12; wherein PEP12 is a peptide of general formula PEP1-$AA^{17}$-PEP11; wherein $AA^{17}$ is selected from the group consisting of G, A, V, L, I, P, F, M, W, T and S; wherein PEP11 is a peptide with 3 amino acids of general formula $AA^{18}$-$AA^{19}$-$AA^{20}$; wherein $AA^{18}$ is selected from the group consisting of L, V, Q, A and R; wherein $AA^{19}$ is selected from the group consisting of F, W, H, Y, I and K; wherein $AA^{20}$ is selected from the group consisting of L, F, Y, K, I, V and M.

3. A pharmaceutical association for a use according to claim 2, wherein PEP11 is selected from the group consisting of LYL, LFF, LYF, LYY, LYK, LYI, LFI, LYV, VYY, QIM, AKV and RKI.

4. A pharmaceutical association for a use according to claims 2 or 3, wherein the pair PEP1:PEP11 is selected from the group consisting of SAIS:LYL.

5. A pharmaceutical association for a use according to any one of claims 1 to 4, wherein said growth factor receptor-binding compound comprises at least one bioactive carrier-affinity-containing group, wherein said at least one bioactive carrier-affinity-containing group provides said growth factor receptor-binding compound with the ability to covalently or non-covalently interact with a bioactive carrier; wherein said bioactive carrier-affinity-containing group is a biomaterial affinity-containing group which is adapted for forming at least one covalent bond or non-covalent bond with a biomaterial.

6. A pharmaceutical association for a use according to any one of claims 1 to 5, wherein the RMSD value of the structure coordinates of said peptide or peptidomimetic with respect to PEPREF is 2.45Å (Angstroms) or less, and wherein PEPREF is

| | | | | | | | | |
|---|---|---|---|---|---|---|---|---|
| ATOM | 511 | N | LYS | A | 1 | -14.570 | 46.437 | 27.424 |
| ATOM | 512 | CA | LYS | A | 1 | -13.512 | 45.748 | 28.151 |
| ATOM | 513 | C | LYS | A | 1 | -13.655 | 44.259 | 27.884 |
| ATOM | 514 | O | LYS | A | 1 | -12.769 | 43.463 | 28.197 |
| ATOM | 515 | CB | LYS | A | 1 | -13.605 | 46.029 | 29.652 |
| ATOM | 516 | CG | LYS | A | 1 | -13.640 | 47.509 | 29.991 |
| ATOM | 517 | CD | LYS | A | 1 | -12.615 | 48.297 | 29.183 |
| ATOM | 518 | CE | LYS | A | 1 | -12.625 | 49.768 | 29.575 |
| ATOM | 519 | NZ | LYS | A | 1 | -13.994 | 50.369 | 29.497 |
| ATOM | 520 | N | ILE | A | 2 | -14.792 | 43.890 | 27.309 |
| ATOM | 521 | CA | ILE | A | 2 | -15.051 | 42.499 | 26.967 |
| ATOM | 522 | C | ILE | A | 2 | -14.911 | 42.370 | 25.444 |
| ATOM | 523 | O | ILE | A | 2 | -15.531 | 43.125 | 24.683 |
| ATOM | 524 | CB | ILE | A | 2 | -16.466 | 42.065 | 27.401 |
| ATOM | 525 | CG1 | ILE | A | 2 | -16.630 | 42.238 | 28.915 |
| ATOM | 526 | CG2 | ILE | A | 2 | -16.710 | 40.629 | 26.985 |
| ATOM | 527 | CD1 | ILE | A | 2 | -15.631 | 41.478 | 29.30 |
| ATOM | 528 | N | PRO | A | 3 | -14.085 | 41.411 | 24.989 |
| ATOM | 529 | CA | PRO | A | 3 | -13.789 | 41.109 | 23.588 |
| ATOM | 530 | C | PRO | A | 3 | -14.998 | 40.695 | 22.768 |
| ATOM | 531 | O | PRO | A | 3 | -15.969 | 40.164 | 23.305 |
| ATOM | 532 | CB | PRO | A | 3 | -12.785 | 39.968 | 23.688 |

(continued)

| | | | | | | | | |
|---|---|---|---|---|---|---|---|---|
| ATOM | 533 | CG | PRO | A | 3 | -12.156 | 40.166 | 25.007 |
| ATOM | 534 | CD | PRO | A | 3 | -13.330 | 40.506 | 25.867 |
| ATOM | 535 | N | LYS | A | 4 | -14.937 | 40.937 | 21.463 |
| ATOM | 536 | CA | LYS | A | 4 | -16.023 | 40.529 | 20.590 |
| ATOM | 537 | C | LYS | A | 4 | -15.886 | 39.015 | 20.391 |
| ATOM | 538 | O | LYS | A | 4 | -14.903 | 38.415 | 20.831 |
| ATOM | 539 | CB | LYS | A | 4 | -15.926 | 41.244 | 19.245 |
| ATOM | 540 | CG | LYS | A | 4 | -15.802 | 42.751 | 19.355 |
| ATOM | 541 | CD | LYS | A | 4 | -16.292 | 43.433 | 18.083 |
| ATOM | 542 | CE | LYS | A | 4 | -16.162 | 44.943 | 18.177 |
| ATOM | 543 | NZ | LYS | A | 4 | -16.825 | 45.628 | 17.019 |
| ATOM | 544 | N | ALA | A | 5 | -16.85 | 38.393 | 19.759 |
| ATOM | 545 | CA | ALA | A | 5 | -16.811 | 36.955 | 19.507 |
| ATOM | 546 | C | ALA | A | 5 | -15.772 | 36.771 | 18.416 |
| ATOM | 547 | O | ALA | A | 5 | -15.727 | 37.534 | 17.455 |
| ATOM | 548 | CB | ALA | A | 5 | -18.168 | 36.419 | 19.043 |
| ATOM | 549 | N | CYS | A | 6 | -14.935 | 35.756 | 18.562 |
| ATOM | 550 | CA | CYS | A | 6 | -13.887 | 35.518 | 17.584 |
| ATOM | 551 | C | CYS | A | 6 | -14.347 | 34.765 | 16.338 |
| ATOM | 552 | O | CYS | A | 6 | -15.327 | 34.018 | 16.368 |
| ATOM | 553 | CB | CYS | A | 6 | -12.743 | 34.768 | 18.241 |
| ATOM | 554 | SG | CYS | A | 6 | -11.198 | 34.959 | 17.353 |
| ATOM | 555 | N | CYS | A | 7 | -13.623 | 34.973 | 15.243 |
| ATOM | 556 | CA | CYS | A | 7 | -13.931 | 34.328 | 13.969 |
| ATOM | 557 | C | CYS | A | 7 | -13.091 | 33.071 | 13.798 |
| ATOM | 558 | O | CYS | A | 7 | -11.961 | 33.123 | 13.302 |
| ATOM | 559 | CB | CYS | A | 7 | -13.653 | 35.290 | 12.824 |
| ATOM | 560 | SG | CYS | A | 7 | -13.930 | 34.633 | 11.154 |
| ATOM | 561 | N | VAL | A | 8 | -13.654 | 31.941 | 14.209 |
| ATOM | 562 | CA | VAL | A | 8 | -12.949 | 30.684 | 14.110 |
| ATOM | 563 | C | VAL | A | 8 | -13.653 | 29.733 | 13.157 |
| ATOM | 564 | O | VAL | A | 8 | -14.759 | 30.016 | 12.687 |
| ATOM | 565 | CB | VAL | A | 8 | -12.814 | 30.038 | 15.492 |
| ATOM | 566 | CG1 | VAL | A | 8 | -11.807 | 30.825 | 16.337 |
| ATOM | 567 | CG2 | VAL | A | 8 | -14.161 | 30.006 | 16.170 |
| ATOM | 568 | N | PRO | A | 9 | -13.003 | 28.601 | 12.828 |
| ATOM | 569 | CA | PRO | A | 9 | -13.593 | 27.615 | 11.918 |
| ATOM | 570 | C | PRO | A | 9 | -14.726 | 26.886 | 12.631 |
| ATOM | 571 | O | PRO | A | 9 | -14.581 | 26.476 | 13.780 |
| ATOM | 572 | CB | PRO | A | 9 | -12.423 | 26.676 | 11.601 |

(continued)

| | | | | | | | | |
|---|---|---|---|---|---|---|---|---|
| ATOM | 573 | CG | PRO | A | 9 | -11.204 | 27.487 | 11.925 |
| ATOM | 574 | CD | PRO | A | 9 | -11.620 | 28.226 | 13.163 |
| ATOM | 575 | N | THR | A | 10 | -15.847 | 26.721 | 11.942 |
| ATOM | 576 | CA | THR | A | 10 | -16.999 | 26.060 | 12.527 |
| ATOM | 577 | C | THR | A | 10 | -17.334 | 24.767 | 11.804 |
| ATOM | 578 | O | THR | A | 10 | -18.097 | 23.943 | 12.303 |
| ATOM | 579 | CB | THR | A | 10 | -18.211 | 27.010 | 12.523 |
| ATOM | 580 | OG1 | THR | A | 10 | -18.491 | 27.445 | 11.185 |
| ATOM | 581 | CG2 | THR | A | 10 | -17.902 | 28.230 | 13.375 |
| ATOM | 582 | N | GLU | A | 11 | -16.750 | 24.586 | 10.627 |
| ATOM | 583 | CA | GLU | A | 11 | -16.980 | 23.377 | 9.848 |
| ATOM | 584 | C | GLU | A | 11 | -15.643 | 22.935 | 9.246 |
| ATOM | 585 | O | GLU | A | 11 | -15.029 | 23.666 | 8.464 |
| ATOM | 586 | CB | GLU | A | 11 | -17.981 | 23.624 | 8.715 |
| ATOM | 587 | CG | GLU | A | 11 | -19.421 | 23.807 | 9.163 |
| ATOM | 588 | CD | GLU | A | 11 | -19.686 | 25.166 | 9.770 |
| ATOM | 589 | OE1 | GLU | A | 11 | -19.478 | 26.175 | 9.073 |
| ATOM | 590 | OE2 | GLU | A | 11 | -20.111 | 25.227 | 10.939 |
| ATOM | 591 | N | LEU | A | 12 | -15.183 | 21.749 | 9.622 |
| ATOM | 592 | CA | LEU | A | 12 | -13.923 | 21.254 | 9.104 |
| ATOM | 593 | C | LEU | A | 12 | -14.062 | 19.912 | 8.386 |
| ATOM | 594 | O | LEU | A | 12 | -15.136 | 19.299 | 8.359 |
| ATOM | 595 | CB | LEU | A | 12 | -12.893 | 21.144 | 10.230 |
| ATOM | 596 | CG | LEU | A | 12 | -12.660 | 22.422 | 11.054 |
| ATOM | 597 | CD1 | LEU | A | 12 | -13.475 | 22.350 | 12.337 |
| ATOM | 598 | CD2 | LEU | A | 12 | -11.181 | 22.586 | 11.399 |
| ATOM | 599 | N | SER | A | 13 | -12.971 | 19.476 | 7.771 |
| ATOM | 600 | CA | SER | A | 13 | -12.964 | 18.218 | 7.046 |
| ATOM | 601 | C | SER | A | 13 | -11.568 | 17.628 | 7.164 |
| ATOM | 602 | O | SER | A | 13 | -10.613 | 18.320 | 7.550 |
| ATOM | 603 | CB | SER | A | 13 | -13.346 | 18.435 | 5.578 |
| ATOM | 604 | OG | SER | A | 13 | -12.404 | 19.261 | 4.923 |
| ATOM | 605 | N | ALA | A | 14 | -11.449 | 16.352 | 6.818 |
| ATOM | 606 | CA | ALA | A | 14 | -10.179 | 15.665 | 6.949 |
| ATOM | 607 | C | ALA | A | 14 | -9.421 | 15.471 | 5.652 |
| ATOM | 608 | O | ALA | A | 14 | -9.941 | 15.720 | 4.563 |
| ATOM | 609 | CB | ALA | A | 14 | -10.413 | 14.306 | 7.626 |
| ATOM | 610 | N | ILE | A | 15 | -8.171 | 15.046 | 5.783 |
| ATOM | 611 | CA | ILE | A | 15 | -7.343 | 14.746 | 4.623 |
| ATOM | 612 | C | ILE | A | 15 | -6.475 | 13.559 | 5.004 |

(continued)

| | | | | | | | | |
|---|---|---|---|---|---|---|---|---|
| ATOM | 613 | O | ILE | A | 15 | -6.212 | 13.316 | 6.183 |
| ATOM | 614 | CB | ILE | A | 15 | -6.401 | 15.916 | 4.183 |
| ATOM | 615 | CG1 | ILE | A | 15 | -5.284 | 16.106 | 5.200 |
| ATOM | 616 | CG2 | ILE | A | 15 | -7.188 | 17.211 | 3.982 |
| ATOM | 617 | CD1 | ILE | A | 15 | -4.173 | 16.973 | 4.696 |
| ATOM | 618 | N | SER | A | 16 | -6.045 | 12.806 | 3.999 |
| ATOM | 619 | CA | SER | A | 16 | -5.187 | 11.662 | 4.242 |
| ATOM | 620 | C | SER | A | 16 | -3.740 | 12.089 | 4.217 |
| ATOM | 621 | O | SER | A | 16 | -3.360 | 13.020 | 3.508 |
| ATOM | 622 | CB | SER | A | 16 | -5.416 | 10.584 | 3.185 |
| ATOM | 623 | OG | SER | A | 16 | -6.667 | 9.971 | 3.401 |
| ATOM | 624 | N | MET | A | 17 | -2.933 | 11.409 | 5.012 |
| ATOM | 625 | CA | MET | A | 17 | -1.518 | 11.700 | 5.047 |
| ATOM | 626 | C | MET | A | 17 | -0.778 | 10.414 | 5.244 |
| ATOM | 627 | O | MET | A | 17 | -1.137 | 9.594 | 6.078 |
| ATOM | 628 | CB | MET | A | 17 | -1.170 | 12.694 | 6.164 |
| ATOM | 629 | CG | MET | A | 17 | -1.848 | 14.042 | 5.974 |
| ATOM | 630 | SD | MET | A | 17 | -1.017 | 15.431 | 6.760 |
| ATOM | 631 | CE | MET | A | 17 | -0.799 | 14.823 | 8.475 |
| ATOM | 632 | N | LEU | A | 18 | 0.238 | 10.231 | 4.426 |
| ATOM | 633 | CA | LEU | A | 18 | 1.077 | 9.065 | 4.508 |
| ATOM | 634 | C | LEU | A | 18 | 2.289 | 9.610 | 5.264 |
| ATOM | 635 | O | LEU | A | 18 | 2.939 | 10.565 | 4.818 |
| ATOM | 636 | CB | LEU | A | 18 | 1.461 | 8.608 | 3.100 |
| ATOM | 637 | CG | LEU | A | 18 | 2.324 | 7.355 | 2.955 |
| ATOM | 638 | CD1 | LEU | A | 18 | 1.553 | 6.145 | 3.445 |
| ATOM | 639 | CD2 | LEU | A | 18 | 2.723 | 7.190 | 1.492 |
| ATOM | 640 | N | TYR | A | 19 | 2.581 | 9.029 | 6.418 |
| ATOM | 641 | CA | TYR | A | 19 | 3.706 | 9.501 | 7.196 |
| ATOM | 642 | C | TYR | A | 19 | 4.434 | 8.333 | 7.835 |
| ATOM | 643 | O | TYR | A | 19 | 4.081 | 7.186 | 7.603 |
| ATOM | 644 | CB | TYR | A | 19 | 3.222 | 10.458 | 8.281 |
| ATOM | 645 | CG | TYR | A | 19 | 2.386 | 9.782 | 9.346 |
| ATOM | 646 | CD1 | TYR | A | 19 | 1.029 | 9.527 | 9.147 |
| ATOM | 647 | CD2 | TYR | A | 19 | 2.961 | 9.379 | 10.550 |
| ATOM | 648 | CE1 | TYR | A | 19 | 0.273 | 8.894 | 10.128 |
| ATOM | 649 | CE2 | TYR | A | 19 | 2.218 | 8.745 | 11.526 |
| ATOM | 650 | CZ | TYR | A | 19 | 0.877 | 8.508 | 11.317 |
| ATOM | 651 | OH | TYR | A | 19 | 0.134 | 7.922 | 12.318 |
| ATOM | 652 | N | LEU | A | 20 | 5.439 | 8.651 | 8.650 |

(continued)

| | | | | | | | | |
|---|---|---|---|---|---|---|---|---|
| ATOM | 653 | CA | LEU | A | 20 | 6.255 | 7.661 | 9.347 |
| ATOM | 654 | C | LEU | A | 20 | 6.210 | 7.946 | 10.847 |
| ATOM | 655 | O | LEU | A | 20 | 6.685 | 8.992 | 11.288 |
| ATOM | 656 | CB | LEU | A | 20 | 7.701 | 7.763 | 8.871 |
| ATOM | 657 | CG | LEU | A | 20 | 7.901 | 7.850 | 7.359 |
| ATOM | 658 | CD1 | LEU | A | 20 | 9.300 | 8.379 | 7.039 |
| ATOM | 659 | CD2 | LEU | A | 20 | 7.669 | 6.482 | 6.748 |

**7.** A pharmaceutical association for a use according to any one of claims 1 to 6, wherein said growth factor receptor-binding compound comprises a peptide selected from the group consisting of SEQ ID NOs: 3, 8, 9, 10, 12, 13, 14, 15, 16, 17, 18, 19, 21, 46, 58, 59, 62, 95, 96, 202, 212, 213, 239, 240, 241, 242, 243, 244, 245, 246, 247, 248, 249, 250, 251, 252, 253, 254, 255, 256, 257, 258, 628, 629, 1133, 1135, 1484, 1489, 1491, 1492, 1501, 1505, 1506, 1508, 1509, 1511, 1514, 1516, 1517, 1518, 1533, 1534, 1535, 1539, 1540, 1543, 1548, 1549, 1554, 1558, 1562, 1568, 1570, 1574, 1578, 1581, 1591, 1594, 1597, 1598, 1600, 1601, 1602, 1605, 1607, 1609, 1613, 1615, 1616, 1617, 1618, 1623, 1624, 1626, 1627, 1640, 1652, 1654, 1655, 1660, 1665, 1666, 1667, 1670, 1676, 1677, 1678, 1679, 1682, 1684, 1686, 1691, 1692, 1699, 1700, 1706, 1708, 1709, 1711, 1736, 1738, 1740, 1756, 1760, 1762, 1764, 1786, 1790, 1796, 1800, 2047, 2064, 2071, 2085, 2162, 2184, 2193, 2211, 2230, 2242, 2267, 4745, 4746, 4747, 4748, 4749, 4750, 4751, 4752, 4753, 4754, 4755, 4756, 4757, 4758, 4759, 4760, 4761, 4762, 4763, 4764, 4765, 4766, 4767, 4768, 4769, 4770, 4771, 4772, 4773, 4774, 4775, 4776, 4777, 4778, 4779, 4780, 4781, 4782, 4783, 4784, 4785, 4786, 4787, 4788, 4789, 4790, 4791, 4792, 4793, 4794, 4795, 4796, 4797, 4798, 4799, 4800, 4801, 4802, 4803, 4804, 4805, 4808, 4811, 4812, 4814, 4818, 4820, 4821, 4822, 4824, 4842, 4845, 4848, 4849, 4850, 4901, 4902, 4903, 4904, 4905, 4906, 4907, 4908, 4909, 4910, 4911, 4912, 4913, 4914, 4915, 4916, 4917, 4918, 5204, 5205, 5234, 5249, 5250, 5279, 5294, 5295, 5324, 5339, 5340, 5341, 5342, 5343, 5344, 5345, 5346, 5347, 5348, 5349, 5350, 5351, 5352, 5353, 5354, 5355, 5356, 7069, 7073, 7074, 7081, 7084, 7087, 7088, 7089, 7099, 7100, 7101, 7102, 7106, 7107, 7110, 7111, 7112, 7114, 7117, 7118, 7120, 7121, 7123, 7126, 7134, 7135, 7138, 7140, 7144, 7145, 7152, 7154, 7155, 7160, 7169, 7170, 7172, 7175, 7179, 7181, 7187, 7191, 7202, 7203, 7205, 7206, 7208, 7209, 7210, 7213, 7215, 7216, 7217, 7220, 7223, 7224, 7226, 7229, 7232, 7239, 7244, 7252, 7254, 7262, 7269, 7270, 7271, 7272, 7274, 7280, 7281, 7282, 7284, 7289, 7290, 7295, 7296, 7298, 7303, 7308, 7310, 7316, 7321, 7323, 7325, 7327, 7338, 7350, 7353, 7367, 7371, 7373, 7380, 7383, 7385, 7387, 7394, 7401, 7407, 7409, 7410, 7425, 7429, 7431, 7443, 7446, 7447, 7449, 7450, 7460, 7461, 7462, 7463, 7471, 7473, 7474, 7480, 7485, 7486, 7490, 7492, 7499, 7503, 7505, 7508, 7509, 7520, 7527, 7531, 7545, 7554, 7567, 7572, 7577, 7580, 7581, 7586, 7590, 7593, 7595, 7599, 7616, 7619, 7621, 7624, 7625, 7627, 7630, 7632, 7633, 7636, 7637, 7644, 7645, 7647, 7652, 7653, 7657, 7658, 7666, 7668, 7687, 7688, 7697, 7701, 7719, 7725, 7740, 7744, 8207, 8219, 8227, 8259, 8293, 8306, 8354, 8374, 8377, 8380, 8412, 8422, 8433, 8451, 8542, 8568, 8578, 8586, 8597, 8622, and 13455 to 13562.

**8.** A pharmaceutical association for a use according to any one of claims 1 to 6, wherein said growth factor receptor-binding compound is selected from the group consisting of SEQ ID NOs: as defined in claim 7.

**9.** A pharmaceutical association for a use according to any one of claims 1 to 8, wherein said biomaterial is selected from the group consisting of metals and alloys, ceramics, polymeric biomaterials, and biocomposite materials.

**10.** A pharmaceutical association for a use according to any one of claims 1 to 9, wherein said biomaterial is selected from the group consisting of (a) a biopolymer such as (a1) collagen, (a2) fibrin; (b) a synthetic polymer such as (b1) ultra-high molecular weight polyethylene (UHMWPE), (b2) polyurethane (PE), (b3) polyurethane (PU), (b4) polytetrafuoroethylene (PTFE), (b5) polyacetal (PA), (b6) polymethylmethacrylate (PMMA), (b7) polyethylene terepthalate (PET), (b8) silicone rubber (SR), (b9) polyetheretherketone (PEEK), (b10) poly(lactic acid) (PLA), (b11) polysulfone (PS), (b12) PLLA, (b13) PLGA or (b14) PLDA; (c) metals and metal oxides such as (c1) gold and gold alloys, (c2) silver and silver alloys, (c3) platinum and platinum alloys, (c4) tantalum, (c5) Ti6Al4V, (c6) 316L stainless steel, (c7) Co-Cr Alloys, (c8) titanium alloys such as such as $\alpha$-type, $\beta$-type, $\alpha$+$\beta$-type Ti alloy, Ti-Nb alloys such as Ti29Nb13Ta4.6Zr or Ti35Nb4Sn); (d) metallic glasses; (e) amorphous alloys such as Zr-based alloys; (f) porous metals; (g) gel or solid ceramics such as (g1) alumina, (g2) zirconia, (g3) carbon, (g4) titania, (g5) bioglass, or (g6) hydroxyapatite (HA); (h) composites such as (h1) silica/SR, (h2) CF/UHMWPE, (h3) CF/PTFE, (h4) HA/PE, (h5) CF/epoxy, (h6) CF/PEEK, (h7) CF/C or (h8) $Al_2O_3$/PTFE; (i) hydrogels such as (i1) polyisocyanopeptide hydrogels such as oligo(ethylene)glycol polyisocyanopeptides, (i2) polysaccharides such as alginates, chitosans, chitins, guar gums, pectins, gellan gums,

heparins, carrageenans, hyaluronans, starches, agars, xanthan gums, methylcellulose, carboxymethylcellulose, hydroxypropyl methyl cellulose, (i3) polyglycols such as polyethyleneglycol or polypropyleneglycol, (i4) polyvinylpyrrolidone, (i5) poly(vinylalcohol), (i6) polyacrylic acids, (i7) glycerophosphates, (i8) 2-acrylamido-2-methylpropanesulfonic acid, (i9) polyphosphazenes; (j) demineralized bone matrix; and any combinations thereof.

**11.** A pharmaceutical association for a use according to any one of claims 1 to 10, wherein said biomaterial is selected from the group consisting of a solid ceramic component, in particular a granulated ceramic powder or ceramic scaffolds; a gel ceramic component; collagen, in particular collagen types I, II, III and XI; and a biodegradable hydrogel.

**12.** A medical composition comprising at least one pharmaceutical association according to any one of claims 1 to 11 and at least one medically acceptable excipient, carrier or vehicle for use in the treatment, prevention and/or diagnostic of a neoplastic disease.

**13.** A method for converting a neoplastic cell into a non-neoplastic cell, in-vitro or ex-vivo, said method comprising administering to a neoplastic cell an effective amount of a pharmaceutical association or composition as defined in any one of claims 1 to 12.

## Patentansprüche

**1.** Pharmazeutische Assoziation zur Verwendung bei der Behandlung, Vorbeugung und/oder Diagnose einer neoplastischen Erkrankung, wobei die Assoziation mindestens eine Wachstumsfaktor-Rezeptor-bindende Verbindung, die mindestens einen Wachstumsfaktor-Rezeptor einer neoplastischen Zelle aktiviert, und mindestens einen bioaktiven Träger umfasst, der mindestens eine kovalente oder nicht-kovalente Wechselwirkung mit der mindestens einen Wachstumsfaktor-Rezeptor-bindenden Verbindung bildet;

wobei die Assoziation in der neoplastischen Zelle die Genexpression von mindestens einem Cyclin D reduziert oder unterdrückt und/oder die Bildung von mindestens einem Komplex, der zwischen dem mindestens einen Cyclin D und mindestens einer der Cyclin-abhängigen Kinasen 4 oder 6 gebildet wird, reduziert oder unterdrückt;
wobei die den Wachstumsfaktorrezeptor bindende Verbindung ein Peptid mit vier Aminosäuren PEP1 umfasst;
wobei PEP1 SAIS ist;
wobei die den Wachstumsfaktorrezeptor bindende Verbindung ein Peptid oder ein Peptidomimetikum ist;
wobei die Wachstumsfaktor-Rezeptor-bindende Verbindung ein nicht-zyklisches Peptid mit zwischen 8 und 25 Aminosäuren oder ein nicht-zyklisches Peptidomimetikum mit einem Molekulargewicht zwischen 600 und 4.000 Dalton ist und zwischen 8 und 25 Aminosäuren umfasst;
wobei die Wachstumsfaktor-Rezeptor-bindende Verbindung ein zyklisches Peptid mit zwischen 10 und 60 Aminosäuren oder ein zyklisches Peptidomimetikum ist, das zwischen 10 und 60 Aminosäuren umfasst und ein Molekulargewicht zwischen 1.000 und 7.000 Da aufweist;
und wobei der bioaktive Träger ein Biomaterial ist.

**2.** Pharmazeutische Assoziation für eine Verwendung nach Anspruch 1, wobei die Wachstumsfaktor-Rezeptor-bindende Verbindung ein Peptid mit acht Aminosäuren PEP12 umfasst; wobei PEP12 ein Peptid der allgemeinen Formel PEP1-$AA^{17}$-PEP11 ist; wobei $AA^{17}$ ausgewählt ist aus der Gruppe bestehend aus G, A, V, L, I, P, F, M, W, T und S; wobei PEP11 ein Peptid mit 3 Aminosäuren der allgemeinen Formel $AA^{18}$-$AA^{19}$-$AA^{20}$ ist; wobei $AA^{18}$ ausgewählt ist aus der Gruppe bestehend aus L, V, Q, A und R; wobei $AA^{19}$ ausgewählt ist aus der Gruppe bestehend aus F, W, H, Y, I und K; wobei $AA^{20}$ ausgewählt ist aus der Gruppe bestehend aus L, F, Y, K, I, V und M.

**3.** Pharmazeutische Assoziation für eine Verwendung nach Anspruch 2, wobei PEP11 ausgewählt ist aus der Gruppe bestehend aus LYL, LFF, LYF, LYY, LYK, LYI, LFI, LYV, VYY, QIM, AKV und RKI.

**4.** Pharmazeutische Assoziation für eine Verwendung nach Anspruch 2 oder 3, wobei das Paar PEP1:PEP11 ausgewählt ist aus der Gruppe bestehend aus SAIS:LYL.

**5.** Pharmazeutische Assoziation für eine Verwendung nach einem der Ansprüche 1 bis 4, wobei die Wachstumsfaktor-Rezeptor-bindende Verbindung mindestens eine bioaktive Träger-Affinitäts-enthaltende Gruppe umfasst, wobei die mindestens eine bioaktive Träger-Affinitäts-enthaltende Gruppe die Wachstumsfaktor-Rezeptor-bindende Verbindung mit der Fähigkeit ausstattet, kovalent oder nicht-kovalent mit einem bioaktiven Träger zu interagieren; wobei die

bioaktive Träger-Affinitäts-enthaltende Gruppe eine Biomaterial-Affinitäts-enthaltende Gruppe ist, die zur Bildung mindestens einer kovalenten Bindung oder nicht-kovalenten Bindung mit einem Biomaterial angepasst ist.

**6.** Pharmazeutische Assoziation für eine Verwendung nach einem der Ansprüche 1 bis 5, wobei der RMSD-Wert der Strukturkoordinaten des Peptids oder Peptidomimetikums in Bezug auf PEPREF 2,45 Å (Angström) oder weniger beträgt, und wobei PEPREF ist

| | | | | | | | | |
|---|---|---|---|---|---|---|---|---|
| ATOM | 511 | N | LYS | A | 1 | -14.570 | 46.437 | 27.424 |
| ATOM | 512 | CA | LYS | A | 1 | -13.512 | 45.748 | 28.151 |
| ATOM | 513 | C | LYS | A | 1 | -13.655 | 44.259 | 27.884 |
| ATOM | 514 | O | LYS | A | 1 | -12.769 | 43.463 | 28.197 |
| ATOM | 515 | CB | LYS | A | 1 | -13.605 | 46.029 | 29.652 |
| ATOM | 516 | CG | LYS | A | 1 | -13.640 | 47.509 | 29.991 |
| ATOM | 517 | CD | LYS | A | 1 | -12.615 | 48.297 | 29.183 |
| ATOM | 518 | CE | LYS | A | 1 | -12.625 | 49.768 | 29.575 |
| ATOM | 519 | NZ | LYS | A | 1 | -13.994 | 50.369 | 29.497 |
| ATOM | 520 | N | ILE | A | 2 | -14.792 | 43.890 | 27.309 |
| ATOM | 521 | CA | ILE | A | 2 | -15.051 | 42.499 | 26.967 |
| ATOM | 522 | C | ILE | A | 2 | -14.911 | 42.370 | 25.444 |
| ATOM | 523 | O | ILE | A | 2 | -15.531 | 43.125 | 24.683 |
| ATOM | 524 | CB | ILE | A | 2 | -16.466 | 42.065 | 27.401 |
| ATOM | 525 | CG1 | ILE | A | 2 | -16.630 | 42.238 | 28.915 |
| ATOM | 526 | CG2 | ILE | A | 2 | -16.710 | 40.629 | 26.985 |
| ATOM | 527 | CD1 | ILE | A | 2 | -15.631 | 41.478 | 29.30 |
| ATOM | 528 | N | PRO | A | 3 | -14.085 | 41.411 | 24.989 |
| ATOM | 529 | CA | PRO | A | 3 | -13.789 | 41.109 | 23.588 |
| ATOM | 530 | C | PRO | A | 3 | -14.998 | 40.695 | 22.768 |
| ATOM | 531 | O | PRO | A | 3 | -15.969 | 40.164 | 23.305 |
| ATOM | 532 | CB | PRO | A | 3 | -12.785 | 39.968 | 23.688 |
| ATOM | 533 | CG | PRO | A | 3 | -12.156 | 40.166 | 25.007 |
| ATOM | 534 | CD | PRO | A | 3 | -13.330 | 40.506 | 25.867 |
| ATOM | 535 | N | LYS | A | 4 | -14.937 | 40.937 | 21.463 |
| ATOM | 536 | CA | LYS | A | 4 | -16.023 | 40.529 | 20.590 |
| ATOM | 537 | C | LYS | A | 4 | -15.886 | 39.015 | 20.391 |
| ATOM | 538 | O | LYS | A | 4 | -14.903 | 38.415 | 20.831 |
| ATOM | 539 | CB | LYS | A | 4 | -15.926 | 41.244 | 19.245 |
| ATOM | 540 | CG | LYS | A | 4 | -15.802 | 42.751 | 19.355 |
| ATOM | 541 | CD | LYS | A | 4 | -16.292 | 43.433 | 18.083 |
| ATOM | 542 | CE | LYS | A | 4 | -16.162 | 44.943 | 18.177 |
| ATOM | 543 | NZ | LYS | A | 4 | -16.825 | 45.628 | 17.019 |
| ATOM | 544 | N | ALA | A | 5 | -16.85 | 38.393 | 19.759 |
| ATOM | 545 | CA | ALA | A | 5 | -16.811 | 36.955 | 19.507 |
| ATOM | 546 | C | ALA | A | 5 | -15.772 | 36.771 | 18.416 |

(continued)

| | | | | | | | | |
|---|---|---|---|---|---|---|---|---|
| ATOM | 547 | O | ALA | A | 5 | -15.727 | 37.534 | 17.455 |
| ATOM | 548 | CB | ALA | A | 5 | -18.168 | 36.419 | 19.043 |
| ATOM | 549 | N | CYS | A | 6 | -14.935 | 35.756 | 18.562 |
| ATOM | 550 | CA | CYS | A | 6 | -13.887 | 35.518 | 17.584 |
| ATOM | 551 | C | CYS | A | 6 | -14.347 | 34.765 | 16.338 |
| ATOM | 552 | O | CYS | A | 6 | -15.327 | 34.018 | 16.368 |
| ATOM | 553 | CB | CYS | A | 6 | -12.743 | 34.768 | 18.241 |
| ATOM | 554 | SG | CYS | A | 6 | -11.198 | 34.959 | 17.353 |
| ATOM | 555 | N | CYS | A | 7 | -13.623 | 34.973 | 15.243 |
| ATOM | 556 | CA | CYS | A | 7 | -13.931 | 34.328 | 13.969 |
| ATOM | 557 | C | CYS | A | 7 | -13.091 | 33.071 | 13.798 |
| ATOM | 558 | O | CYS | A | 7 | -11.961 | 33.123 | 13.302 |
| ATOM | 559 | CB | CYS | A | 7 | -13.653 | 35.290 | 12.824 |
| ATOM | 560 | SG | CYS | A | 7 | -13.930 | 34.633 | 11.154 |
| ATOM | 561 | N | VAL | A | 8 | -13.654 | 31.941 | 14.209 |
| ATOM | 562 | CA | VAL | A | 8 | -12.949 | 30.684 | 14.110 |
| ATOM | 563 | C | VAL | A | 8 | -13.653 | 29.733 | 13.157 |
| ATOM | 564 | O | VAL | A | 8 | -14.759 | 30.016 | 12.687 |
| ATOM | 565 | CB | VAL | A | 8 | -12.814 | 30.038 | 15.492 |
| ATOM | 566 | CG1 | VAL | A | 8 | -11.807 | 30.825 | 16.337 |
| ATOM | 567 | CG2 | VAL | A | 8 | -14.161 | 30.006 | 16.170 |
| ATOM | 568 | N | PRO | A | 9 | -13.003 | 28.601 | 12.828 |
| ATOM | 569 | CA | PRO | A | 9 | -13.593 | 27.615 | 11.918 |
| ATOM | 570 | C | PRO | A | 9 | -14.726 | 26.886 | 12.631 |
| ATOM | 571 | O | PRO | A | 9 | -14.581 | 26.476 | 13.780 |
| ATOM | 572 | CB | PRO | A | 9 | -12.423 | 26.676 | 11.601 |
| ATOM | 573 | CG | PRO | A | 9 | -11.204 | 27.487 | 11.925 |
| ATOM | 574 | CD | PRO | A | 9 | -11.620 | 28.226 | 13.163 |
| ATOM | 575 | N | THR | A | 10 | -15.847 | 26.721 | 11.942 |
| ATOM | 576 | CA | THR | A | 10 | -16.999 | 26.060 | 12.527 |
| ATOM | 577 | C | THR | A | 10 | -17.334 | 24.767 | 11.804 |
| ATOM | 578 | O | THR | A | 10 | -18.097 | 23.943 | 12.303 |
| ATOM | 579 | CB | THR | A | 10 | -18.211 | 27.010 | 12.523 |
| ATOM | 580 | OG1 | THR | A | 10 | -18.491 | 27.445 | 11.185 |
| ATOM | 581 | CG2 | THR | A | 10 | -17.902 | 28.230 | 13.375 |
| ATOM | 582 | N | GLU | A | 11 | -16.750 | 24.586 | 10.627 |
| ATOM | 583 | CA | GLU | A | 11 | -16.980 | 23.377 | 9.848 |
| ATOM | 584 | C | GLU | A | 11 | -15.643 | 22.935 | 9.246 |
| ATOM | 585 | O | GLU | A | 11 | -15.029 | 23.666 | 8.464 |
| ATOM | 586 | CB | GLU | A | 11 | -17.981 | 23.624 | 8.715 |

(continued)

| | | | | | | | | |
|---|---|---|---|---|---|---|---|---|
| ATOM | 587 | CG | GLU | A | 11 | -19.421 | 23.807 | 9.163 |
| ATOM | 588 | CD | GLU | A | 11 | -19.686 | 25.166 | 9.770 |
| ATOM | 589 | OE1 | GLU | A | 11 | -19.478 | 26.175 | 9.073 |
| ATOM | 590 | OE2 | GLU | A | 11 | -20.111 | 25.227 | 10.939 |
| ATOM | 591 | N | LEU | A | 12 | -15.183 | 21.749 | 9.622 |
| ATOM | 592 | CA | LEU | A | 12 | -13.923 | 21.254 | 9.104 |
| ATOM | 593 | C | LEU | A | 12 | -14.062 | 19.912 | 8.386 |
| ATOM | 594 | O | LEU | A | 12 | -15.136 | 19.299 | 8.359 |
| ATOM | 595 | CB | LEU | A | 12 | -12.893 | 21.144 | 10.230 |
| ATOM | 596 | CG | LEU | A | 12 | -12.660 | 22.422 | 11.054 |
| ATOM | 597 | CD1 | LEU | A | 12 | -13.475 | 22.350 | 12.337 |
| ATOM | 598 | CD2 | LEU | A | 12 | -11.181 | 22.586 | 11.399 |
| ATOM | 599 | N | SER | A | 13 | -12.971 | 19.476 | 7.771 |
| ATOM | 600 | CA | SER | A | 13 | -12.964 | 18.218 | 7.046 |
| ATOM | 601 | C | SER | A | 13 | -11.568 | 17.628 | 7.164 |
| ATOM | 602 | O | SER | A | 13 | -10.613 | 18.320 | 7.550 |
| ATOM | 603 | CB | SER | A | 13 | -13.346 | 18.435 | 5.578 |
| ATOM | 604 | OG | SER | A | 13 | -12.404 | 19.261 | 4.923 |
| ATOM | 605 | N | ALA | A | 13 | -11.449 | 16.352 | 6.818 |
| ATOM | 606 | CA | ALA | A | 13 | -10.179 | 15.665 | 6.949 |
| ATOM | 607 | C | ALA | A | 13 | -9.421 | 15.471 | 5.652 |
| ATOM | 608 | O | ALA | A | 13 | -9.941 | 15.720 | 4.563 |
| ATOM | 609 | CB | ALA | A | 13 | -10.413 | 14.306 | 7.626 |
| ATOM | 610 | N | ILE | A | 14 | -8.171 | 15.046 | 5.783 |
| ATOM | 611 | CA | ILE | A | 14 | -7.343 | 14.746 | 4.623 |
| ATOM | 612 | C | ILE | A | 14 | -6.475 | 13.559 | 5.004 |
| ATOM | 613 | O | ILE | A | 14 | -6.212 | 13.316 | 6.183 |
| ATOM | 614 | CB | ILE | A | 14 | -6.401 | 15.916 | 4.183 |
| ATOM | 615 | CG1 | ILE | A | 14 | -5.284 | 16.106 | 5.200 |
| ATOM | 616 | CG2 | ILE | A | 14 | -7.188 | 17.211 | 3.982 |
| ATOM | 617 | CD1 | ILE | A | 14 | -4.173 | 16.973 | 4.696 |
| ATOM | 618 | N | SER | A | 15 | -6.045 | 12.806 | 3.999 |
| ATOM | 619 | CA | SER | A | 15 | -5.187 | 11.662 | 4.242 |
| ATOM | 620 | C | SER | A | 15 | -3.740 | 12.089 | 4.217 |
| ATOM | 621 | O | SER | A | 15 | -3.360 | 13.020 | 3.508 |
| ATOM | 622 | CB | SER | A | 15 | -5.416 | 10.584 | 3.185 |
| ATOM | 623 | OG | SER | A | 15 | -6.667 | 9.971 | 3.401 |
| ATOM | 624 | N | MET | A | 16 | -2.933 | 11.409 | 5.012 |
| ATOM | 625 | CA | MET | A | 16 | -1.518 | 11.700 | 5.047 |
| ATOM | 626 | C | MET | A | 16 | -0.778 | 10.414 | 5.244 |

(continued)

| | | | | | | | | |
|---|---|---|---|---|---|---|---|---|
| ATOM | 627 | O | MET | A | 16 | -1.137 | 9.594 | 6.078 |
| ATOM | 628 | CB | MET | A | 16 | -1.170 | 12.694 | 6.164 |
| ATOM | 629 | CG | MET | A | 16 | -1.848 | 14.042 | 5.974 |
| ATOM | 630 | SD | MET | A | 16 | -1.017 | 15.431 | 6.760 |
| ATOM | 631 | CE | MET | A | 16 | -0.799 | 14.823 | 8.475 |
| ATOM | 632 | N | LEU | A | 17 | 0.238 | 10.231 | 4.426 |
| ATOM | 633 | CA | LEU | A | 17 | 1.077 | 9.065 | 4.508 |
| ATOM | 634 | C | LEU | A | 17 | 2.289 | 9.610 | 5.264 |
| ATOM | 635 | O | LEU | A | 17 | 2.939 | 10.565 | 4.818 |
| ATOM | 636 | CB | LEU | A | 17 | 1.461 | 8.608 | 3.100 |
| ATOM | 637 | CG | LEU | A | 17 | 2.324 | 7.355 | 2.955 |
| ATOM | 638 | CD1 | LEU | A | 17 | 1.553 | 6.145 | 3.445 |
| ATOM | 639 | CD2 | LEU | A | 17 | 2.723 | 7.190 | 1.492 |
| ATOM | 640 | N | TYR | A | 18 | 2.581 | 9.029 | 6.418 |
| ATOM | 641 | CA | TYR | A | 18 | 3.706 | 9.501 | 7.196 |
| ATOM | 642 | C | TYR | A | 18 | 4.434 | 8.333 | 7.835 |
| ATOM | 643 | O | TYR | A | 18 | 4.081 | 7.186 | 7.603 |
| ATOM | 644 | CB | TYR | A | 18 | 3.222 | 10.458 | 8.281 |
| ATOM | 645 | CG | TYR | A | 18 | 2.386 | 9.782 | 9.346 |
| ATOM | 646 | CD1 | TYR | A | 18 | 1.029 | 9.527 | 9.147 |
| ATOM | 647 | CD2 | TYR | A | 18 | 2.961 | 9.379 | 10.550 |
| ATOM | 648 | CE1 | TYR | A | 18 | 0.273 | 8.894 | 10.128 |
| ATOM | 649 | CE2 | TYR | A | 18 | 2.218 | 8.745 | 11.526 |
| ATOM | 650 | CZ | TYR | A | 18 | 0.877 | 8.508 | 11.317 |
| ATOM | 651 | OH | TYR | A | 18 | 0.134 | 7.922 | 12.318 |
| ATOM | 652 | N | LEU | A | 19 | 5.439 | 8.651 | 8.650 |
| ATOM | 653 | CA | LEU | A | 19 | 6.255 | 7.661 | 9.347 |
| ATOM | 654 | C | LEU | A | 19 | 6.210 | 7.946 | 10.847 |
| ATOM | 655 | O | LEU | A | 19 | 6.685 | 8.992 | 11.288 |
| ATOM | 656 | CB | LEU | A | 19 | 7.701 | 7.763 | 8.871 |
| ATOM | 657 | CG | LEU | A | 19 | 7.901 | 7.850 | 7.359 |
| ATOM | 658 | CD1 | LEU | A | 19 | 9.300 | 8.379 | 7.039 |
| ATOM | 659 | CD2 | LEU | A | 19 | 7.669 | 6.482 | 6.748 |

**7.** Pharmazeutische Assoziation für eine Verwendung nach einem der Ansprüche 1 bis 6, wobei die den Wachstumsfaktorrezeptor bindende Verbindung ein Peptid umfasst, das aus der Gruppe ausgewählt ist, die aus SEQ ID NOs: 3, 8, 9, 10, 12, 13, 14, 15, 16, 17, 18, 19, 21, 46, 58, 59, 62, 95, 96, 202, 212, 213, 239, 240, 241, 242, 243, 244, 245, 246, 247, 248, 249, 250, 251, 252, 253, 254, 255, 256, 257, 258, 628, 629, 1133, 1135, 1484, 1489, 1491, 1492, 1501, 1505, 1506, 1508, 1509, 1511, 1514, 1516, 1517, 1518, 1533, 1534, 1535, 1539, 1540, 1543, 1548, 1549, 1554, 1558, 1562, 1568, 1570, 1574, 1578, 1581, 1591, 1594, 1597, 1598, 1600, 1601, 1602, 1605, 1607, 1609, 1613, 1615, 1616, 1617, 1618, 1623, 1624, 1626, 1627, 1640, 1652, 1654, 1655, 1660, 1665, 1666, 1667, 1670, 1676, 1677, 1678, 1679, 1682, 1684, 1686, 1691, 1692, 1699, 1700, 1706, 1708, 1709, 1711, 1736, 1738, 1740, 1756, 1760, 1762, 1764, 1786, 1790, 1796, 1800, 2047, 2064, 2071, 2085, 2162, 2184, 2193, 2211, 2230, 2242, 2267, 4745, 4746, 4747, 4748, 4749, 4750,

4751, 4752, 4753, 4754, 4755, 4756, 4757, 4758, 4759, 4760, 4761, 4762, 4763, 4764, 4765, 4766, 4767, 4768, 4769, 4770, 4771, 4772, 4773, 4774, 4775, 4776, 4777, 4778, 4779, 4780, 4781, 4782, 4783, 4784, 4785, 4786, 4787, 4788, 4789, 4790, 4791, 4792, 4793, 4794, 4795, 4796, 4797, 4798, 4799, 4800, 4801, 4802, 4803, 4804, 4805, 4808, 4811, 4812, 4814, 4818, 4820, 4821, 4822, 4824, 4842, 4845, 4848, 4849, 4850, 4901, 4902, 4903, 4904, 4905, 4906, 4907, 4908, 4909, 4910, 4911, 4912, 4913, 4914, 4915, 4916, 4917, 4918, 5204, 5205, 5234, 5249, 5250, 5279, 5294, 5295, 5324, 5339, 5340, 5341, 5342, 5343, 5344, 5345, 5346, 5347, 5348, 5349, 5350, 5351, 5352, 5353, 5354, 5355, 5356, 7069, 7073, 7074, 7081, 7084, 7087, 7088, 7089, 7099, 7100, 7101, 7102, 7106, 7107, 7110, 7111, 7112, 7114, 7117, 7118, 7120, 7121, 7123, 7126, 7134, 7135, 7138, 7140, 7144, 7145, 7152, 7154, 7155, 7160, 7169, 7170, 7172, 7175, 7179, 7181, 7187, 7191, 7202, 7203, 7205, 7206, 7208, 7209, 7210, 7213, 7215, 7216, 7217, 7220, 7223, 7224, 7226, 7229, 7232, 7239, 7244, 7252, 7254, 7262, 7269, 7270, 7271, 7272, 7274, 7280, 7281, 7282, 7284, 7289, 7290, 7295, 7296, 7298, 7303, 7308, 7310, 7316, 7321, 7323, 7325, 7327, 7338, 7350, 7353, 7367, 7371, 7373, 7380, 7383, 7385, 7387, 7394, 7401, 7407, 7409, 7410, 7425, 7429, 7431, 7443, 7446, 7447, 7449, 7450, 7460, 7461, 7462, 7463, 7471, 7473, 7474, 7480, 7485, 7486, 7490, 7492, 7499, 7503, 7505, 7508, 7509, 7520, 7527, 7531, 7545, 7554, 7567, 7572, 7577, 7580, 7581, 7586, 7590, 7593, 7595, 7599, 7616, 7619, 7621, 7624, 7625, 7627, 7630, 7632, 7633, 7636, 7637, 7644, 7645, 7647, 7652, 7653, 7657, 7658, 7666, 7668, 7687, 7688, 7697, 7701, 7719, 7725, 7740, 7744, 8207, 8219, 8227, 8259, 8293, 8306, 8354, 8374, 8377, 8380, 8412, 8422, 8433, 8451, 8542, 8568, 8578, 8586, 8597, 8622, und 13455 bis 13562.

**8.** Pharmazeutische Assoziation für eine Verwendung nach einem der Ansprüche 1 bis 6, wobei die Wachstumsfaktor-Rezeptor-bindende Verbindung ausgewählt ist aus der Gruppe bestehend aus SEQ ID NOs: wie in Anspruch 7 definiert.

**9.** Pharmazeutische Assoziation für eine Verwendung nach einem der Ansprüche 1 bis 8, wobei das Biomaterial ausgewählt ist aus der Gruppe bestehend aus Metallen und Legierungen, Keramiken, polymeren Biomaterialien und Biokompositmaterialien.

**10.** Pharmazeutische Assoziation für eine Verwendung nach einem der Ansprüche 1 bis 9, wobei das Biomaterial ausgewählt ist aus der Gruppe bestehend aus (a) einem Biopolymer wie (a1) Kollagen, (a2) Fibrin; (b) einem synthetischen Polymer wie (b1) Polyethylen mit ultrahohem Molekulargewicht (UHMWPE), (b2) Polyurethan (PE), (b3) Polyurethan (PU), (b4) Polytetrafluorethylen (PTFE), (b5) Polyacetal (PA), (b6) Polymethylmethacrylat (PMMA), (b7) Polyethylenterepthalat (PET), (b8) Silikonkautschuk (SR), (b9) Polyetheretherketon (PEEK), (b10) Poly(milchsäure) (PLA), (b11) Polysulfon (PS), (b12) PLLA, (b13) PLGA oder (b14) PLDA; (c) Metalle und Metalloxide wie (c1) Gold und Goldlegierungen, (c2) Silber und Silberlegierungen, (c3) Platin und Platinlegierungen, (c4) Tantal, (c5) Ti6Al4V, (c6) Edelstahl 316L, (c7) Co-Cr-Legierungen, (c8) Titanlegierungen wie $\alpha$, $\beta$ , $\alpha+\beta$, Ti-Legierung, Ti-Nb-Legierungen wie Ti29Nb13Ta4.6Zr oder Ti35Nb4Sn; d) metallische Gläser; e) amorphe Legierungen wie Legierungen auf Zr-Basis; f) poröse Metalle; g) Gel oder feste Keramiken wie (g1) Aluminiumoxid, (g2) Zirkoniumoxid, (g3) Kohlenstoff, (g4) Titanoxid, (g5) Bioglas oder (g6) Hydroxylapatit (HA); (h) Verbundstoffe wie (h1) Siliziumdioxid/SR, (h2) CF/UHMWPE, (h3) CF/PTFE, (h4) HA/PE, (h5) CF/Epoxid, (h6) CF/PEEK, (h7) CF/C oder (h8) $Al_2O_3$ /PTFE; (i) Hydrogele wie (i1) Polyisocyanpeptid-Hydrogele wie Oligo(ethylen)glykol-Polyisocyanpeptide, (i2) Polysaccharide wie Alginate, Chitosane, Chitine, Guar-Gummis, Pektine, Gellan-Gummis, Heparine, Carrageenane, Hyaluronane, Stärken, Agar, Xanthan-Gummis, Methylcellulose, Carboxymethylcellulose, Hydroxypropylmethylcellulose, (i3) Polyglykole wie Polyethylenglykol oder Polypropylenglykol, (i4) Polyvinylpyrrolidon, (i5) Poly(vinylalkohol), (i6) Polyacrylsäuren, (i7) Glycerophosphate, (i8) 2-Acrylamido-2-methylpropansulfonsäure, (i9) Polyphosphazene; (j) demineralisierte Knochenmatrix; und jegliche Kombinationen davon.

**11.** Pharmazeutische Assoziation für eine Verwendung nach einem der Ansprüche 1 bis 10, wobei das Biomaterial ausgewählt ist aus der Gruppe bestehend aus einer festen keramischen Komponente, insbesondere einem granulierten keramischen Pulver oder keramischen Gerüsten; einer keramischen Gelkomponente; Kollagen, insbesondere Kollagen der Typen I, II, III und XI; und einem biologisch abbaubaren Hydrogel.

**12.** Medizinische Zusammensetzung, umfassend mindestens eine pharmazeutische Assoziation nach einem der Ansprüche 1 bis 11 und mindestens einen medizinisch akzeptablen Hilfsstoff, Träger oder Vehikel zur Verwendung bei der Behandlung, Vorbeugung und/oder Diagnose einer neoplastischen Erkrankung.

**13.** Verfahren zur Umwandlung einer neoplastischen Zelle in eine nicht-neoplastische Zelle, in vitro oder ex vivo, wobei das Verfahren die Verabreichung einer wirksamen Menge einer pharmazeutischen Assoziation oder Zusammensetzung, wie in einem der Ansprüche 1 bis 12 definiert, an eine neoplastische Zelle umfasst.

## Revendications

1. Association pharmaceutique destinée à être utilisée dans le traitement, la prévention et/ou le diagnostic d'une maladie néoplasique, ladite association comprenant au moins un composé se liant à un récepteur de facteur de croissance, qui active au moins un récepteur de facteur de croissance d'une cellule néoplasique, et au moins un support bioactif formant au moins une interaction covalente ou non covalente avec ledit au moins un composé se liant au récepteur de facteur de croissance ;

    dans laquelle ladite association réduit ou supprime, dans la cellule néoplasique, l'expression du gène d'au moins une cycline D et/ou réduit ou supprime la formation d'au moins un complexe formé entre ladite au moins une cycline D et au moins une cycline-kinase dépendante 4 ou 6 ;
    dans lequel ledit composé se liant à un récepteur de facteur de croissance comprend un peptide ayant quatre acides aminés PEP1 ;
    où PEP1 est SAIS ;
    dans lequel ledit composé se liant à un récepteur du facteur de croissance est un peptide ou un peptidomimétique ;
    dans lequel ledit composé se liant à un récepteur de facteur de croissance est un peptide non cyclique ayant entre 8 à 25 acides aminés ou un peptidomimétique non cyclique ayant un poids moléculaire compris entre 600 et 4 000 Daltons et comprenant entre 8 et 25 acides aminés ;
    dans lequel ledit composé se liant à un récepteur de facteur de croissance est un peptide cyclique ayant entre 10 et 60 acides aminés ou un peptidomimétique cyclique comprenant entre 10 et 60 acides aminés ayant un poids moléculaire compris entre 1 000 et 7 000 Da ;
    et dans lequel ledit support bioactif est un biomatériau.

2. Association pharmaceutique pour une utilisation selon la revendication 1, dans laquelle ledit composé se liant à un récepteur de facteur de croissance comprend un peptide ayant huit acides aminés PEP12 ; dans lequel PEP12 est un peptide de formule générale PEP1-$AA^{17}$-PEP11 ; dans lequel $AA^{17}$ est choisi dans le groupe constitué de G, A, V, L, I, P, F, M, W, T et S ; dans lequel PEP11 est un peptide ayant 3 acides aminés de formule générale $AA^{18}$-$AA^{19}$ -$AA^{20}$ ; dans lequel $AA^{18}$ est choisi dans le groupe constitué de L, V, Q, A et R ; dans lequel $AA^{19}$ est choisi dans le groupe constitué de F, W, H, Y, I et K; dans lequel $AA^{20}$ est choisi dans le groupe constitué par L, F, Y, K, I, V et M.

3. Association pharmaceutique pour une utilisation selon la revendication 2, dans laquelle PEP11 est choisi dans le groupe constitué de LYL, LFF, LYF, LYY, LYK, LYI, LFI, LYV, VYY, QIM, AKV et RKI.

4. Association pharmaceutique pour une utilisation selon les revendications 2 ou 3, dans laquelle la paire PEP1:PEP11 est choisie dans le groupe constitué par SAIS:LYL.

5. Association pharmaceutique pour une utilisation selon l'une quelconque des revendications 1 à 4, dans laquelle ledit composé se liant à un récepteur de facteur de croissance comprend au moins un groupe ayant une affinité pour un support bioactif, dans lequel ledit au moins un groupe ayant une affinité pour un support bioactif confère au composé se liant à un récepteur de facteur de croissance la capacité d'interagir de manière covalente ou non covalente avec un support bioactif ; dans lequel ledit groupe ayant une affinité pour un support bioactif est un groupe ayant une affinité avec un biomatériau qui est adapté pour former au moins une liaison covalente ou une liaison non covalente avec un biomatériau.

6. Association pharmaceutique pour une utilisation selon l'une quelconque des revendications 1 à 5, dans laquelle la valeur du RMSD des coordonnées tridimensionnelles de la structure dudit peptide ou peptidomimétique par rapport à PEPREF est de 2.45 Å (Angstroms) ou moins, et où PEPREF est

| | | | | | | | | |
|---|---|---|---|---|---|---|---|---|
| ATOM | 511 | N | LYS | A | 1 | -14.570 | 46.437 | 27.424 |
| ATOM | 512 | CA | LYS | A | 1 | -13.512 | 45.748 | 28.151 |
| ATOM | 513 | C | LYS | A | 1 | -13.655 | 44.259 | 27.884 |
| ATOM | 514 | O | LYS | A | 1 | -12.769 | 43.463 | 28.197 |
| ATOM | 515 | CB | LYS | A | 1 | -13.605 | 46.029 | 29.652 |
| ATOM | 516 | CG | LYS | A | 1 | -13.640 | 47.509 | 29.991 |

(continued)

| | | | | | | | | |
|---|---|---|---|---|---|---|---|---|
| ATOM | 517 | CD | LYS | A | 1 | -12.615 | 48.297 | 29.183 |
| ATOM | 518 | CE | LYS | A | 1 | -12.625 | 49.768 | 29.575 |
| ATOM | 519 | NZ | LYS | A | 1 | -13.994 | 50.369 | 29.497 |
| ATOM | 520 | N | ILE | A | 2 | -14.792 | 43.890 | 27.309 |
| ATOM | 521 | CA | ILE | A | 2 | -15.051 | 42.499 | 26.967 |
| ATOM | 522 | C | ILE | A | 2 | -14.911 | 42.370 | 25.444 |
| ATOM | 523 | O | ILE | A | 2 | -15.531 | 43.125 | 24.683 |
| ATOM | 524 | CB | ILE | A | 2 | -16.466 | 42.065 | 27.401 |
| ATOM | 525 | CG1 | ILE | A | 2 | -16.630 | 42.238 | 28.915 |
| ATOM | 526 | CG2 | ILE | A | 2 | -16.710 | 40.629 | 26.985 |
| ATOM | 527 | CD1 | ILE | A | 2 | -15.631 | 41.478 | 29.30 |
| ATOM | 528 | N | PRO | A | 3 | -14.085 | 41.411 | 24.989 |
| ATOM | 529 | CA | PRO | A | 3 | -13.789 | 41.109 | 23.588 |
| ATOM | 530 | C | PRO | A | 3 | -14.998 | 40.695 | 22.768 |
| ATOM | 531 | O | PRO | A | 3 | -15.969 | 40.164 | 23.305 |
| ATOM | 532 | CB | PRO | A | 3 | -12.785 | 39.968 | 23.688 |
| ATOM | 533 | CG | PRO | A | 3 | -12.156 | 40.166 | 25.007 |
| ATOM | 534 | CD | PRO | A | 3 | -13.330 | 40.506 | 25.867 |
| ATOM | 535 | N | LYS | A | 4 | -14.937 | 40.937 | 21.463 |
| ATOM | 536 | CA | LYS | A | 4 | -16.023 | 40.529 | 20.590 |
| ATOM | 537 | C | LYS | A | 4 | -15.886 | 39.015 | 20.391 |
| ATOM | 538 | O | LYS | A | 4 | -14.903 | 38.415 | 20.831 |
| ATOM | 539 | CB | LYS | A | 4 | -15.926 | 41.244 | 19.245 |
| ATOM | 540 | CG | LYS | A | 4 | -15.802 | 42.751 | 19.355 |
| ATOM | 541 | CD | LYS | A | 4 | -16.292 | 43.433 | 18.083 |
| ATOM | 542 | CE | LYS | A | 4 | -16.162 | 44.943 | 18.177 |
| ATOM | 543 | NZ | LYS | A | 4 | -16.825 | 45.628 | 17.019 |
| ATOM | 544 | N | ALA | A | 5 | -16.85 | 38.393 | 19.759 |
| ATOM | 545 | CA | ALA | A | 5 | -16.811 | 36.955 | 19.507 |
| ATOM | 546 | C | ALA | A | 5 | -15.772 | 36.771 | 18.416 |
| ATOM | 547 | O | ALA | A | 5 | -15.727 | 37.534 | 17.455 |
| ATOM | 548 | CB | ALA | A | 5 | -18.168 | 36.419 | 19.043 |
| ATOM | 549 | N | CYS | A | 6 | -14.935 | 35.756 | 18.562 |
| ATOM | 550 | CA | CYS | A | 6 | -13.887 | 35.518 | 17.584 |
| ATOM | 551 | C | CYS | A | 6 | -14.347 | 34.765 | 16.338 |
| ATOM | 552 | O | CYS | A | 6 | -15.327 | 34.018 | 16.368 |
| ATOM | 553 | CB | CYS | A | 6 | -12.743 | 34.768 | 18.241 |
| ATOM | 554 | SG | CYS | A | 6 | -11.198 | 34.959 | 17.353 |
| ATOM | 555 | N | CYS | A | 7 | -13.623 | 34.973 | 15.243 |
| ATOM | 556 | CA | CYS | A | 7 | -13.931 | 34.328 | 13.969 |

(continued)

| | | | | | | | | |
|---|---|---|---|---|---|---|---|---|
| ATOM | 557 | C | CYS | A | 7 | -13.091 | 33.071 | 13.798 |
| ATOM | 558 | O | CYS | A | 7 | -11.961 | 33.123 | 13.302 |
| ATOM | 559 | CB | CYS | A | 7 | -13.653 | 35.290 | 12.824 |
| ATOM | 560 | SG | CYS | A | 7 | -13.930 | 34.633 | 11.154 |
| ATOM | 561 | N | VAL | A | 8 | -13.654 | 31.941 | 14.209 |
| ATOM | 562 | CA | VAL | A | 8 | -12.949 | 30.684 | 14.110 |
| ATOM | 563 | C | VAL | A | 8 | -13.653 | 29.733 | 13.157 |
| ATOM | 564 | O | VAL | A | 8 | -14.759 | 30.016 | 12.687 |
| ATOM | 565 | CB | VAL | A | 8 | -12.814 | 30.038 | 15.492 |
| ATOM | 566 | CG1 | VAL | A | 8 | -11.807 | 30.825 | 16.337 |
| ATOM | 567 | CG2 | VAL | A | 8 | -14.161 | 30.006 | 16.170 |
| ATOM | 568 | N | PRO | A | 9 | -13.003 | 28.601 | 12.828 |
| ATOM | 569 | CA | PRO | A | 9 | -13.593 | 27.615 | 11.918 |
| ATOM | 570 | C | PRO | A | 9 | -14.726 | 26.886 | 12.631 |
| ATOM | 571 | O | PRO | A | 9 | -14.581 | 26.476 | 13.780 |
| ATOM | 572 | CB | PRO | A | 9 | -12.423 | 26.676 | 11.601 |
| ATOM | 573 | CG | PRO | A | 9 | -11.204 | 27.487 | 11.925 |
| ATOM | 574 | CD | PRO | A | 9 | -11.620 | 28.226 | 13.163 |
| ATOM | 575 | N | THR | A | 10 | -15.847 | 26.721 | 11.942 |
| ATOM | 576 | CA | THR | A | 10 | -16.999 | 26.060 | 12.527 |
| ATOM | 577 | C | THR | A | 10 | -17.334 | 24.767 | 11.804 |
| ATOM | 578 | O | THR | A | 10 | -18.097 | 23.943 | 12.303 |
| ATOM | 579 | CB | THR | A | 10 | -18.211 | 27.010 | 12.523 |
| ATOM | 580 | OG1 | THR | A | 10 | -18.491 | 27.445 | 11.185 |
| ATOM | 581 | CG2 | THR | A | 10 | -17.902 | 28.230 | 13.375 |
| ATOM | 582 | N | GLU | A | 11 | -16.750 | 24.586 | 10.627 |
| ATOM | 583 | CA | GLU | A | 11 | -16.980 | 23.377 | 9.848 |
| ATOM | 584 | C | GLU | A | 11 | -15.643 | 22.935 | 9.246 |
| ATOM | 585 | O | GLU | A | 11 | -15.029 | 23.666 | 8.464 |
| ATOM | 586 | CB | GLU | A | 11 | -17.981 | 23.624 | 8.715 |
| ATOM | 587 | CG | GLU | A | 11 | -19.421 | 23.807 | 9.163 |
| ATOM | 588 | CD | GLU | A | 11 | -19.686 | 25.166 | 9.770 |
| ATOM | 589 | OE1 | GLU | A | 11 | -19.478 | 26.175 | 9.073 |
| ATOM | 590 | OE2 | GLU | A | 11 | -20.111 | 25.227 | 10.939 |
| ATOM | 591 | N | LEU | A | 12 | -15.183 | 21.749 | 9.622 |
| ATOM | 592 | CA | LEU | A | 12 | -13.923 | 21.254 | 9.104 |
| ATOM | 593 | C | LEU | A | 12 | -14.062 | 19.912 | 8.386 |
| ATOM | 594 | O | LEU | A | 12 | -15.136 | 19.299 | 8.359 |
| ATOM | 595 | CB | LEU | A | 12 | -12.893 | 21.144 | 10.230 |
| ATOM | 596 | CG | LEU | A | 12 | -12.660 | 22.422 | 11.054 |

(continued)

| | | | | | | | | |
|---|---|---|---|---|---|---|---|---|
| ATOM | 597 | CD1 | LEU | A | 12 | -13.475 | 22.350 | 12.337 |
| ATOM | 598 | CD2 | LEU | A | 12 | -11.181 | 22.586 | 11.399 |
| ATOM | 599 | N | SER | A | 13 | -12.971 | 19.476 | 7.771 |
| ATOM | 600 | CA | SER | A | 13 | -12.964 | 18.218 | 7.046 |
| ATOM | 601 | C | SER | A | 13 | -11.568 | 17.628 | 7.164 |
| ATOM | 602 | O | SER | A | 13 | -10.613 | 18.320 | 7.550 |
| ATOM | 603 | CB | SER | A | 13 | -13.346 | 18.435 | 5.578 |
| ATOM | 604 | OG | SER | A | 13 | -12.404 | 19.261 | 4.923 |
| ATOM | 605 | N | ALA | A | 13 | -11.449 | 16.352 | 6.818 |
| ATOM | 606 | CA | ALA | A | 13 | -10.179 | 15.665 | 6.949 |
| ATOM | 607 | C | ALA | A | 13 | -9.421 | 15.471 | 5.652 |
| ATOM | 608 | O | ALA | A | 13 | -9.941 | 15.720 | 4.563 |
| ATOM | 609 | CB | ALA | A | 13 | -10.413 | 14.306 | 7.626 |
| ATOM | 610 | N | ILE | A | 14 | -8.171 | 15.046 | 5.783 |
| ATOM | 611 | CA | ILE | A | 14 | -7.343 | 14.746 | 4.623 |
| ATOM | 612 | C | ILE | A | 14 | -6.475 | 13.559 | 5.004 |
| ATOM | 613 | O | ILE | A | 14 | -6.212 | 13.316 | 6.183 |
| ATOM | 614 | CB | ILE | A | 14 | -6.401 | 15.916 | 4.183 |
| ATOM | 615 | CG1 | ILE | A | 14 | -5.284 | 16.106 | 5.200 |
| ATOM | 616 | CG2 | ILE | A | 14 | -7.188 | 17.211 | 3.982 |
| ATOM | 617 | CD1 | ILE | A | 14 | -4.173 | 16.973 | 4.696 |
| ATOM | 618 | N | SER | A | 15 | -6.045 | 12.806 | 3.999 |
| ATOM | 619 | CA | SER | A | 15 | -5.187 | 11.662 | 4.242 |
| ATOM | 620 | C | SER | A | 15 | -3.740 | 12.089 | 4.217 |
| ATOM | 621 | O | SER | A | 15 | -3.360 | 13.020 | 3.508 |
| ATOM | 622 | CB | SER | A | 15 | -5.416 | 10.584 | 3.185 |
| ATOM | 623 | OG | SER | A | 15 | -6.667 | 9.971 | 3.401 |
| ATOM | 624 | N | MET | A | 16 | -2.933 | 11.409 | 5.012 |
| ATOM | 625 | CA | MET | A | 16 | -1.518 | 11.700 | 5.047 |
| ATOM | 626 | C | MET | A | 16 | -0.778 | 10.414 | 5.244 |
| ATOM | 627 | O | MET | A | 16 | -1.137 | 9.594 | 6.078 |
| ATOM | 628 | CB | MET | A | 16 | -1.170 | 12.694 | 6.164 |
| ATOM | 629 | CG | MET | A | 16 | -1.848 | 14.042 | 5.974 |
| ATOM | 630 | SD | MET | A | 16 | -1.017 | 15.431 | 6.760 |
| ATOM | 631 | CE | MET | A | 16 | -0.799 | 14.823 | 8.475 |
| ATOM | 632 | N | LEU | A | 17 | 0.238 | 10.231 | 4.426 |
| ATOM | 633 | CA | LEU | A | 17 | 1.077 | 9.065 | 4.508 |
| ATOM | 634 | C | LEU | A | 17 | 2.289 | 9.610 | 5.264 |
| ATOM | 635 | O | LEU | A | 17 | 2.939 | 10.565 | 4.818 |
| ATOM | 636 | CB | LEU | A | 17 | 1.461 | 8.608 | 3.100 |

(continued)

| | | | | | | | | |
|---|---|---|---|---|---|---|---|---|
| ATOM | 637 | CG | LEU | A | 17 | 2.324 | 7.355 | 2.955 |
| ATOM | 638 | CD1 | LEU | A | 17 | 1.553 | 6.145 | 3.445 |
| ATOM | 639 | CD2 | LEU | A | 17 | 2.723 | 7.190 | 1.492 |
| ATOM | 640 | N | TYR | A | 18 | 2.581 | 9.029 | 6.418 |
| ATOM | 641 | CA | TYR | A | 18 | 3.706 | 9.501 | 7.196 |
| ATOM | 642 | C | TYR | A | 18 | 4.434 | 8.333 | 7.835 |
| ATOM | 643 | O | TYR | A | 18 | 4.081 | 7.186 | 7.603 |
| ATOM | 644 | CB | TYR | A | 18 | 3.222 | 10.458 | 8.281 |
| ATOM | 645 | CG | TYR | A | 18 | 2.386 | 9.782 | 9.346 |
| ATOM | 646 | CD1 | TYR | A | 18 | 1.029 | 9.527 | 9.147 |
| ATOM | 647 | CD2 | TYR | A | 18 | 2.961 | 9.379 | 10.550 |
| ATOM | 648 | CE1 | TYR | A | 18 | 0.273 | 8.894 | 10.128 |
| ATOM | 649 | CE2 | TYR | A | 18 | 2.218 | 8.745 | 11.526 |
| ATOM | 650 | CZ | TYR | A | 18 | 0.877 | 8.508 | 11.317 |
| ATOM | 651 | OH | TYR | A | 18 | 0.134 | 7.922 | 12.318 |
| ATOM | 652 | N | LEU | A | 19 | 5.439 | 8.651 | 8.650 |
| ATOM | 653 | CA | LEU | A | 19 | 6.255 | 7.661 | 9.347 |
| ATOM | 654 | C | LEU | A | 19 | 6.210 | 7.946 | 10.847 |
| ATOM | 655 | O | LEU | A | 19 | 6.685 | 8.992 | 11.288 |
| ATOM | 656 | CB | LEU | A | 19 | 7.701 | 7.763 | 8.871 |
| ATOM | 657 | CG | LEU | A | 19 | 7.901 | 7.850 | 7.359 |
| ATOM | 658 | CD1 | LEU | A | 19 | 9.300 | 8.379 | 7.039 |
| ATOM | 659 | CD2 | LEU | A | 19 | 7.669 | 6.482 | 6.748 |

7. Association pharmaceutique pour une utilisation selon l'une quelconque des revendications 1 à 6, dans laquelle ledit composé se liant à un récepteur de facteur de croissance comprend un peptide choisi dans le groupe constitué de SEQ ID NOs : 3, 8, 9, 10, 12, 13, 14, 15, 16, 17, 18, 19, 21, 46, 58, 59, 62, 95, 96, 202, 212, 213, 239, 240, 241, 242, 243, 244, 245, 246, 247, 248, 249, 250, 251, 252, 253, 254, 255, 256, 257, 258, 628, 629, 1133, 1135, 1484, 1489, 1491, 1492, 1501, 1505, 1506, 1508, 1509, 1511, 1514, 1516, 1517, 1518, 1533, 1534, 1535, 1539, 1540, 1543, 1548, 1549, 1554, 1558, 1562, 1568, 1570, 1574, 1578, 1581, 1591, 1594, 1597, 1598, 1600, 1601, 1602, 1605, 1607, 1609, 1613, 1615, 1616, 1617, 1618, 1623, 1624, 1626, 1627, 1640, 1652, 1654, 1655, 1660, 1665, 1666, 1667, 1670, 1676, 1677, 1678, 1679, 1682, 1684, 1686, 1691, 1692, 1699, 1700, 1706, 1708, 1709, 1711, 1736, 1738, 1740, 1756, 1760, 1762, 1764, 1786, 1790, 1796, 1800, 2047, 2064, 2071, 2085, 2162, 2184, 2193, 2211, 2230, 2242, 2267, 4745, 4746, 4747, 4748, 4749, 4750, 4751, 4752, 4753, 4754, 4755, 4756, 4757, 4758, 4759, 4760, 4761, 4762, 4763, 4764, 4765, 4766, 4767, 4768, 4769, 4770, 4771, 4772, 4773, 4774, 4775, 4776, 4777, 4778, 4779, 4780, 4781, 4782, 4783, 4784, 4785, 4786, 4787, 4788, 4789, 4790, 4791, 4792, 4793, 4794, 4795, 4796, 4797, 4798, 4799, 4800, 4801, 4802, 4803, 4804, 4805, 4808, 4811, 4812, 4814, 4818, 4820, 4821, 4822, 4824, 4842, 4845, 4848, 4849, 4850, 4901, 4902, 4903, 4904, 4905, 4906, 4907, 4908, 4909, 4910, 4911, 4912, 4913, 4914, 4915, 4916, 4917, 4918, 5204, 5205, 5234, 5249, 5250, 5279, 5294, 5295, 5324, 5339, 5340, 5341, 5342, 5343, 5344, 5345, 5346, 5347, 5348, 5349, 5350, 5351, 5352, 5353, 5354, 5355, 5356, 7069, 7073, 7074, 7081, 7084, 7087, 7088, 7089, 7099, 7100, 7101, 7102, 7106, 7107, 7110, 7111, 7112, 7114, 7117, 7118, 7120, 7121, 7123, 7126, 7134, 7135, 7138, 7140, 7144, 7145, 7152, 7154, 7155, 7160, 7169, 7170, 7172, 7175, 7179, 7181, 7187, 7191, 7202, 7203, 7205, 7206, 7208, 7209, 7210, 7213, 7215, 7216, 7217, 7220, 7223, 7224, 7226, 7229, 7232, 7239, 7244, 7252, 7254, 7262, 7269, 7270, 7271, 7272, 7274, 7280, 7281, 7282, 7284, 7289, 7290, 7295, 7296, 7298, 7303, 7308, 7310, 7316, 7321, 7323, 7325, 7327, 7338, 7350, 7353, 7367, 7371, 7373, 7380, 7383, 7385, 7387, 7394, 7401, 7407, 7409, 7410, 7425, 7429, 7431, 7443, 7446, 7447, 7449, 7450, 7460, 7461, 7462, 7463, 7471, 7473, 7474, 7480, 7485, 7486, 7490, 7492, 7499, 7503, 7505, 7508, 7509, 7520, 7527, 7531, 7545, 7554, 7567, 7572, 7577, 7580, 7581, 7586, 7590, 7593, 7595, 7599, 7616, 7619, 7621, 7624, 7625, 7627, 7630, 7632,

7633, 7636, 7637, 7644, 7645, 7647, 7652, 7653, 7657, 7658, 7666, 7668, 7687, 7688, 7697, 7701, 7719, 7725, 7740, 7744, 8207, 8219, 8227, 8259, 8293, 8306, 8354, 8374, 8377, 8380, 8412, 8422, 8433, 8451, 8542, 8568, 8578, 8586, 8597, 8622, and 13455 to 13562.

**8.** Association pharmaceutique pour une utilisation selon l'une quelconque des revendications 1 à 6, dans laquelle le composé se liant au récepteur de facteur de croissance est choisi dans le groupe constitué des SEQ ID NOs tels que définis dans la revendication 7.

**9.** Association pharmaceutique pour une utilisation selon l'une quelconque des revendications 1 à 8, dans laquelle ledit biomatériau est choisi dans le groupe constitué par les métaux et les alliages, les céramiques, les biomatériaux polymères et les matériaux biocomposites.

**10.** Association pharmaceutique pour une utilisation selon l'une quelconque des revendications 1 à 9, dans laquelle ledit biomatériau est choisi dans le groupe consistant en (a) un biopolymère tel que (a1) le collagène, (a2) la fibrine ; (b) un polymère synthétique tel que (b1) le polyéthylène à poids moléculaire ultra-élevé (UHMWPE), (b2) le polyuréthane (PE), (b3) le polyuréthane (PU), (b4) le polytétrafuoroéthylène (PTFE), (b5) le polyacétal (PA), (b6) polyméthacrylate de méthyle (PMMA), (b7) polyéthylène térépthalate (PET), (b8) caoutchouc de silicone (SR), (b9) polyétheréthercétone (PEEK), (b10) poly(acide lactique) (PLA), (b11) polysulfone (PS), (b12) PLLA, (b13) PLGA ou (b14) PLDA ; (c) métaux et oxydes métalliques tels que (c1) or et alliages d'or, (c2) argent et alliages d'argent, (c3) platine et alliages de platine, (c4) tantale, (c5) Ti6Al4V, (c6) acier inoxydable 316L, (c7) alliages de Co-Cr, (c8) alliages de titane tels que les alliages de Ti de type $\alpha$, de type $\beta$, de type $\alpha+\beta$, alliages de Ti-Nb tels que Ti29Nb13Ta4.6Zr ou Ti35Nb4Sn ; (d) verres métalliques ; (e) alliages amorphes tels que les alliages à base de Zr ; (f) métaux poreux ; (g) céramiques gélifiées ou solides telles que (g1) alumine, (g2) zircone, (g3) carbone, (g4) titane, (g5) bioverre, ou (g6) hydroxyapatite (HA) ; (h) composites tels que(h1) silice/SR, (h2) CF/UHMWPE, (h3) CF/PTFE, (h4) HA/PE, (h5) CF/époxy, (h6) CF/PEEK, (h7) CF/C ou (h8) $Al_2O_3$/PTFE ; (i) hydrogels tels que (i1) hydrogels de polyisocyanopeptide tels que les polyisocyanopeptides d'oligo(éthylène)glycol, (i2) polysaccharides tels que les alginates, les chitosanes, les chitines, les gommes de guar, les pectines, les gommes de gellane, les héparines, les carraghénanes, les hyaluronanes, les amidons, les agars, les gommes de xanthane, méthylcellulose, carboxyméthylcellulose, hydroxypropylméthylcellulose, (i3) polyglycols tels que le polyéthylèneglycol ou le polypropylèneglycol, (i4) polyvinylpyrrolidone, (i5) poly(vinylalcool), (i6) acides polyacryliques, (i7) glycérophosphates, (i8) acide 2-acrylamido-2-méthylpropanesulfonique, (i9) polyphosphazènes ; (j) matrice osseuse déminéralisée ; et l'une quelconque de leurs combinaisons.

**11.** Association pharmaceutique pour une utilisation selon l'une quelconque des revendications 1 à 10, dans laquelle ledit biomatériau est choisi dans le groupe constitué d'un composant céramique solide, en particulier un poudre céramique granulée ou une matrice céramique ; un composant céramique gélifié ; du collagène, en particulier du collagène de type I, II, **III** et XI ; et un hydrogel biodégradable.

**12.** Composition médicale comprenant au moins une association pharmaceutique selon l'une quelconque des revendications 1 à **11** et au moins un excipient, un support ou un véhicule médicalement acceptable pour son utilisation dans le traitement, la prévention et/ou le diagnostic d'une maladie néoplasique.

**13.** Méthode de conversion d'une cellule néoplasique en une cellule non néoplasique, in vitro ou ex vivo, ladite méthode comprenant l'administration à une cellule néoplasique d'une quantité efficace d'une association ou composition pharmaceutique telle que définie dans l'une quelconque des revendications 1 à 12.

FIGURE 1

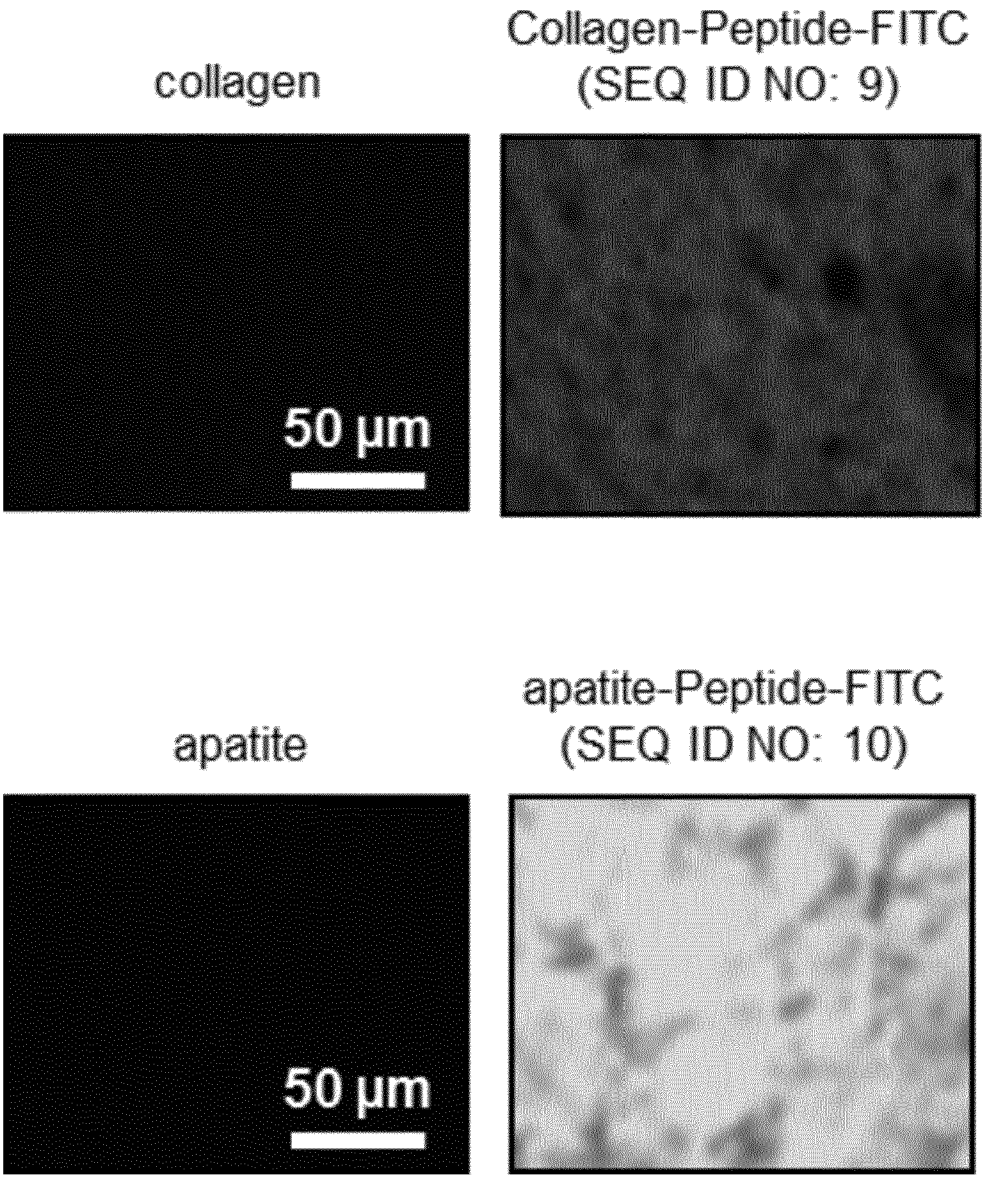
collagen
Collagen-Peptide-FITC
(SEQ ID NO: 9)
50 µm
apatite
apatite-Peptide-FITC
(SEQ ID NO: 10)
50 µm

FIGURE 2

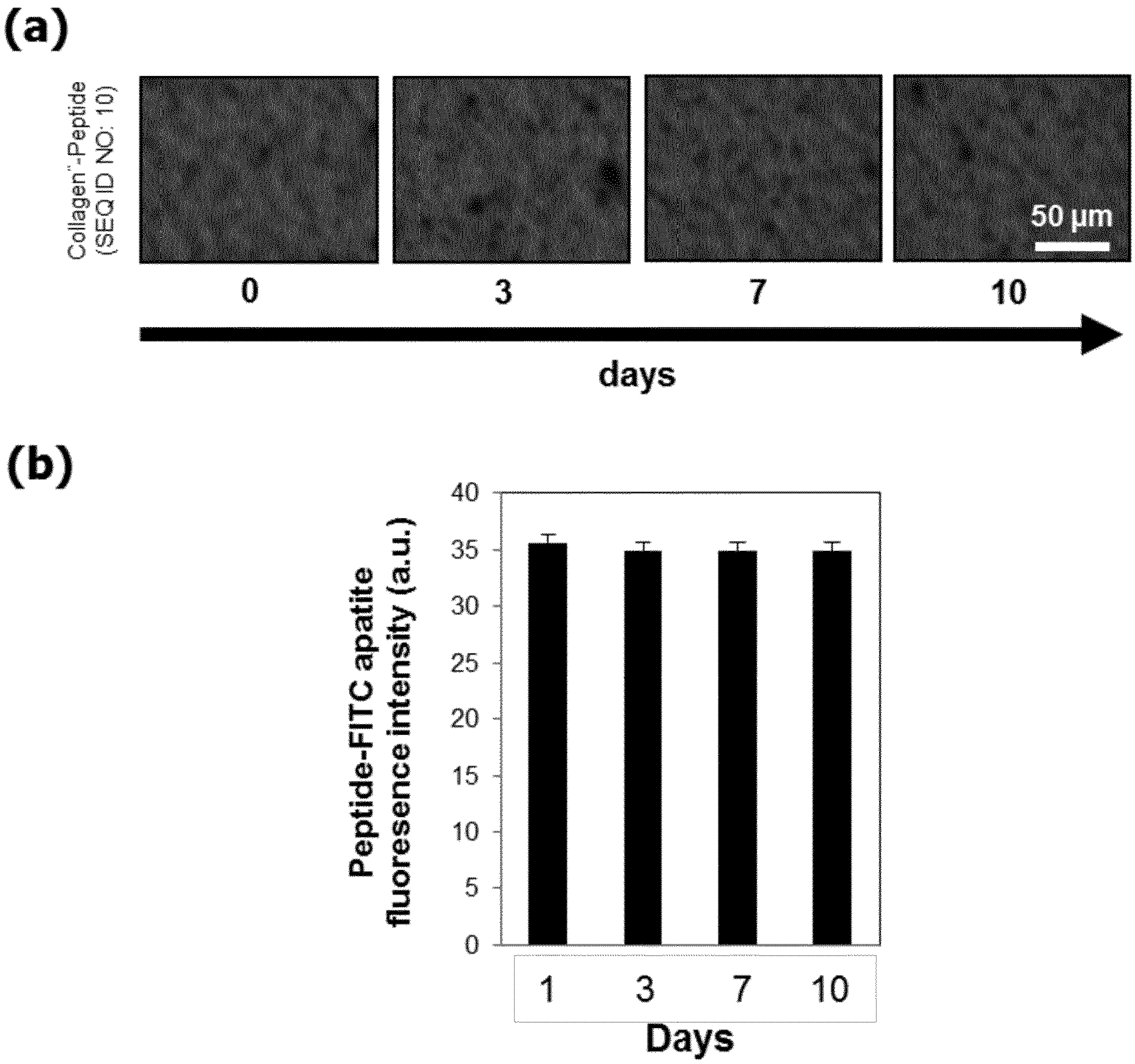
(a)
Collagen⁻-Peptide
(SEQ ID NO: 10)
50 µm
0
3
7
10
days
(b)
Peptide-FITC apatite fluoresence intensity (a.u.)
40
35
30
25
20
15
10
5
0
1
3
7
10
Days

FIGURE 3

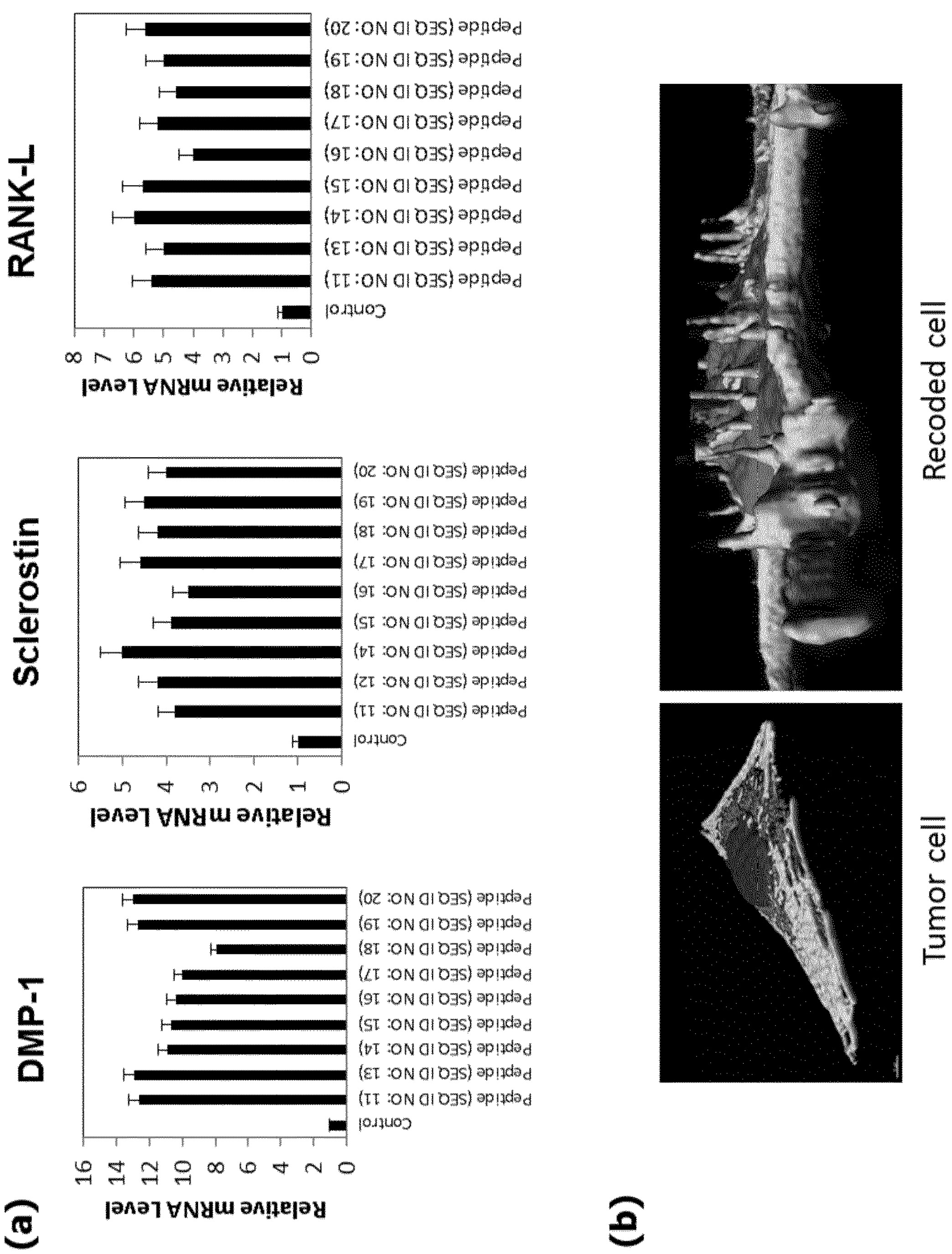
(a)
DMP-1
Sclerostin
RANK-L
Relative mRNA Level
Control
Peptide (SEQ ID NO: 11)
Peptide (SEQ ID NO: 12)
Peptide (SEQ ID NO: 13)
Peptide (SEQ ID NO: 14)
Peptide (SEQ ID NO: 15)
Peptide (SEQ ID NO: 16)
Peptide (SEQ ID NO: 17)
Peptide (SEQ ID NO: 18)
Peptide (SEQ ID NO: 19)
Peptide (SEQ ID NO: 20)
(b)
Tumor cell
Recoded cell

FIGURE 4

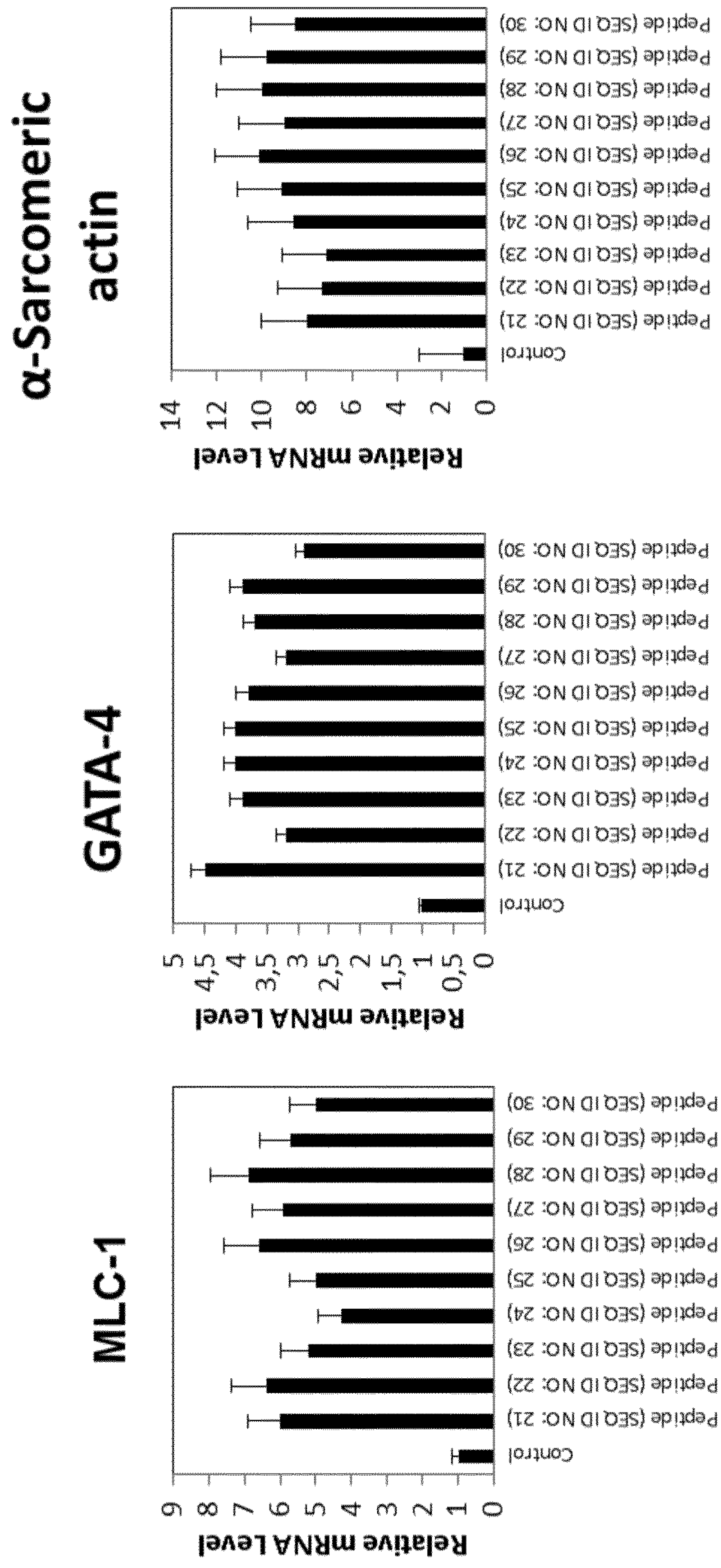

α-Sarcomeric actin
GATA-4
MLC-1
Relative mRNA Level
Control
Peptide (SEQ ID NO: 21)
Peptide (SEQ ID NO: 22)
Peptide (SEQ ID NO: 23)
Peptide (SEQ ID NO: 24)
Peptide (SEQ ID NO: 25)
Peptide (SEQ ID NO: 26)
Peptide (SEQ ID NO: 27)
Peptide (SEQ ID NO: 28)
Peptide (SEQ ID NO: 29)
Peptide (SEQ ID NO: 30)

FIGURE 5

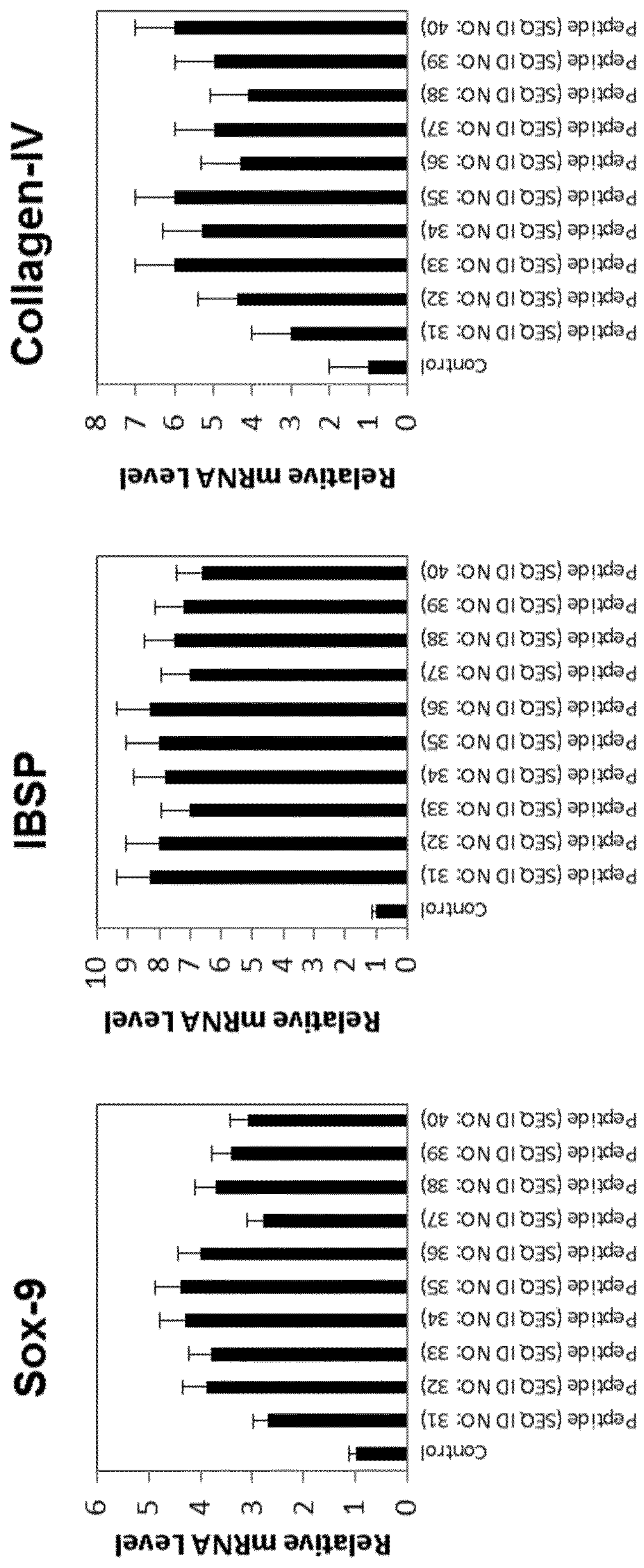
Collagen-IV
Relative mRNA Level
0
1
2
3
4
5
6
7
8
Control
Peptide (SEQ ID NO: 31)
Peptide (SEQ ID NO: 32)
Peptide (SEQ ID NO: 33)
Peptide (SEQ ID NO: 34)
Peptide (SEQ ID NO: 35)
Peptide (SEQ ID NO: 36)
Peptide (SEQ ID NO: 37)
Peptide (SEQ ID NO: 38)
Peptide (SEQ ID NO: 39)
Peptide (SEQ ID NO: 40)
IBSP
Relative mRNA Level
0
1
2
3
4
5
6
7
8
9
10
Control
Peptide (SEQ ID NO: 31)
Peptide (SEQ ID NO: 32)
Peptide (SEQ ID NO: 33)
Peptide (SEQ ID NO: 34)
Peptide (SEQ ID NO: 35)
Peptide (SEQ ID NO: 36)
Peptide (SEQ ID NO: 37)
Peptide (SEQ ID NO: 38)
Peptide (SEQ ID NO: 39)
Peptide (SEQ ID NO: 40)
Sox-9
Relative mRNA Level
0
1
2
3
4
5
6
Control
Peptide (SEQ ID NO: 31)
Peptide (SEQ ID NO: 32)
Peptide (SEQ ID NO: 33)
Peptide (SEQ ID NO: 34)
Peptide (SEQ ID NO: 35)
Peptide (SEQ ID NO: 36)
Peptide (SEQ ID NO: 37)
Peptide (SEQ ID NO: 38)
Peptide (SEQ ID NO: 39)
Peptide (SEQ ID NO: 40)

FIGURE 6

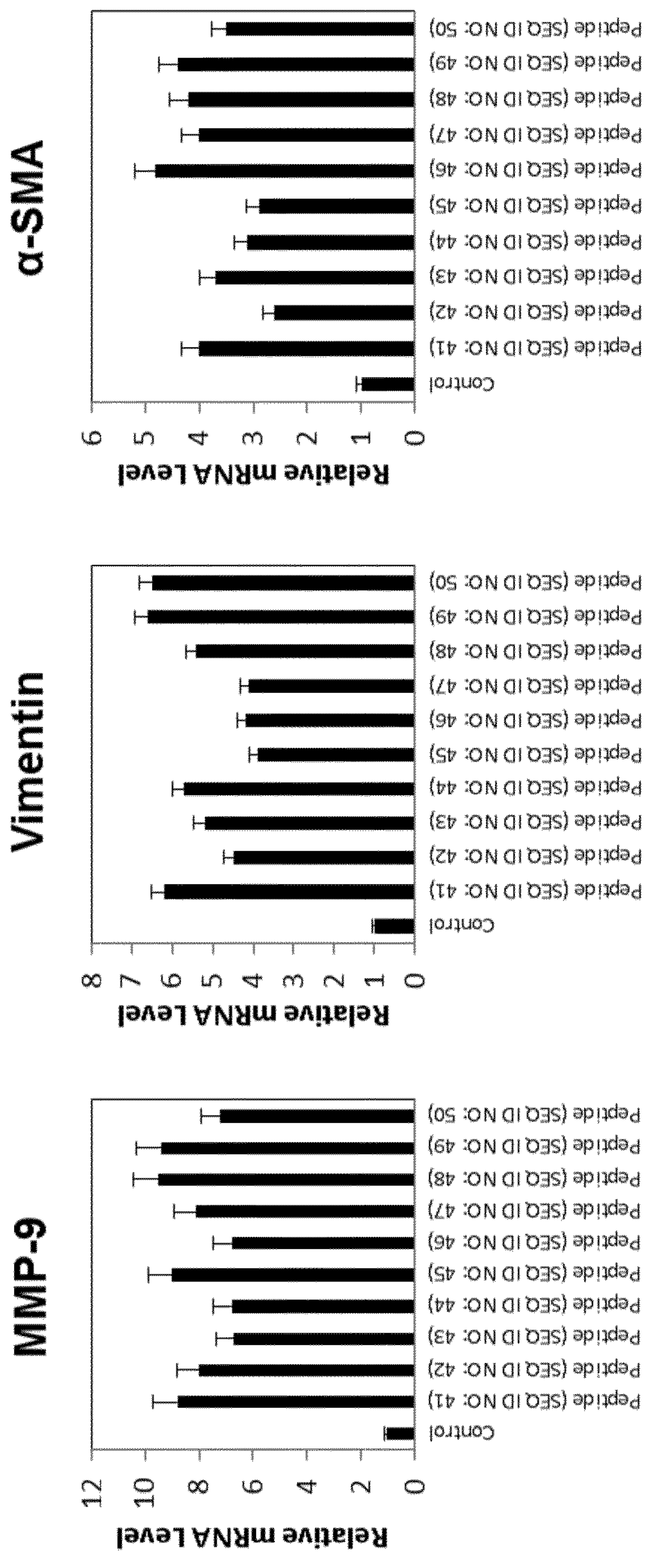
α-SMA
Vimentin
MMP-9
Relative mRNA Level
Control
Peptide (SEQ ID NO: 41)
Peptide (SEQ ID NO: 42)
Peptide (SEQ ID NO: 43)
Peptide (SEQ ID NO: 44)
Peptide (SEQ ID NO: 45)
Peptide (SEQ ID NO: 46)
Peptide (SEQ ID NO: 47)
Peptide (SEQ ID NO: 48)
Peptide (SEQ ID NO: 49)
Peptide (SEQ ID NO: 50)

FIGURE 7

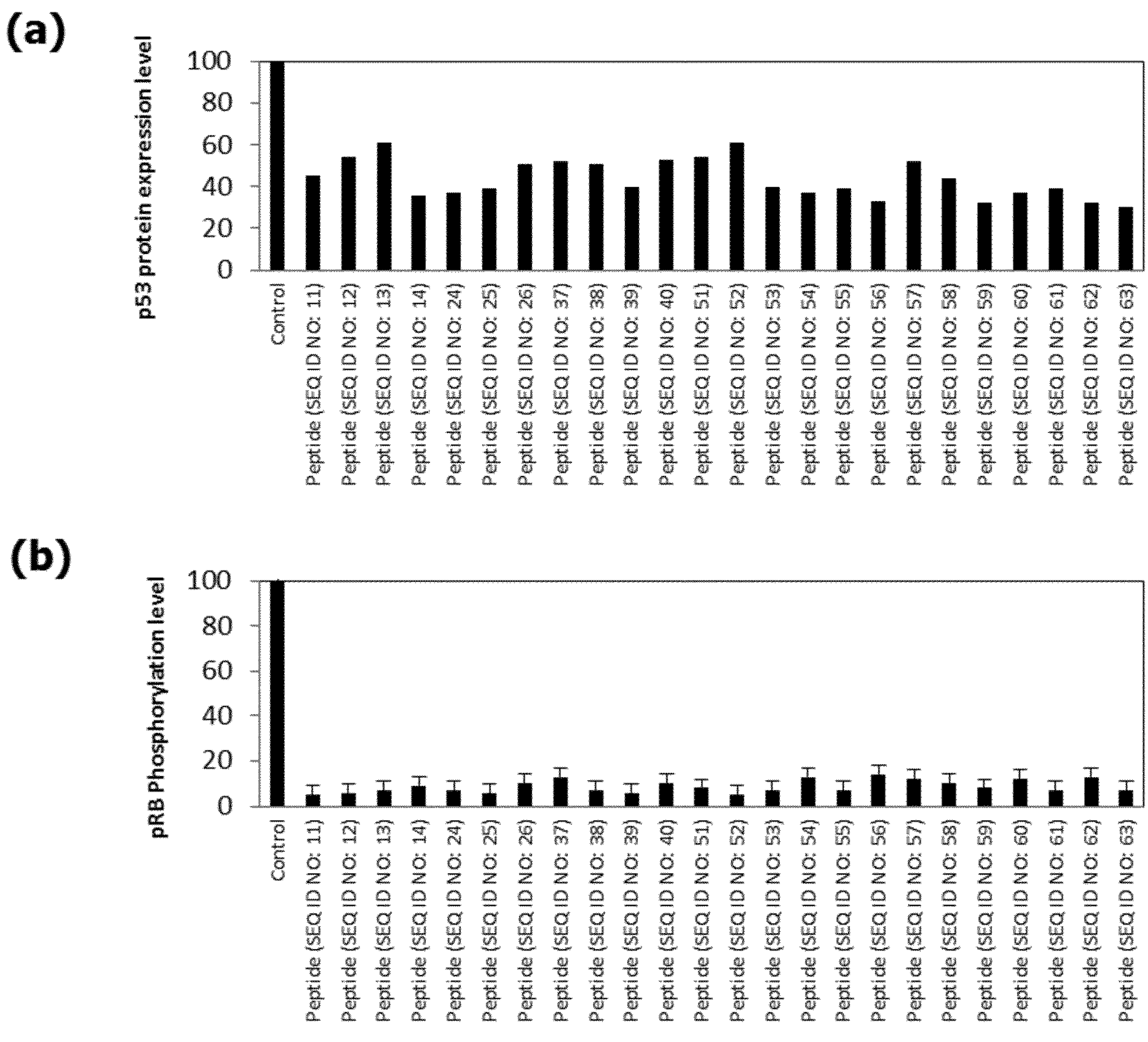
(a)
p53 protein expression level
100
80
60
40
20
0
Control
Peptide (SEQ ID NO: 11)
Peptide (SEQ ID NO: 12)
Peptide (SEQ ID NO: 13)
Peptide (SEQ ID NO: 14)
Peptide (SEQ ID NO: 24)
Peptide (SEQ ID NO: 25)
Peptide (SEQ ID NO: 26)
Peptide (SEQ ID NO: 37)
Peptide (SEQ ID NO: 38)
Peptide (SEQ ID NO: 39)
Peptide (SEQ ID NO: 40)
Peptide (SEQ ID NO: 51)
Peptide (SEQ ID NO: 52)
Peptide (SEQ ID NO: 53)
Peptide (SEQ ID NO: 54)
Peptide (SEQ ID NO: 55)
Peptide (SEQ ID NO: 56)
Peptide (SEQ ID NO: 57)
Peptide (SEQ ID NO: 58)
Peptide (SEQ ID NO: 59)
Peptide (SEQ ID NO: 60)
Peptide (SEQ ID NO: 61)
Peptide (SEQ ID NO: 62)
Peptide (SEQ ID NO: 63)
(b)
pRB Phosphorylation level
100
80
60
40
20
0
Control
Peptide (SEQ ID NO: 11)
Peptide (SEQ ID NO: 12)
Peptide (SEQ ID NO: 13)
Peptide (SEQ ID NO: 14)
Peptide (SEQ ID NO: 24)
Peptide (SEQ ID NO: 25)
Peptide (SEQ ID NO: 26)
Peptide (SEQ ID NO: 37)
Peptide (SEQ ID NO: 38)
Peptide (SEQ ID NO: 39)
Peptide (SEQ ID NO: 40)
Peptide (SEQ ID NO: 51)
Peptide (SEQ ID NO: 52)
Peptide (SEQ ID NO: 53)
Peptide (SEQ ID NO: 54)
Peptide (SEQ ID NO: 55)
Peptide (SEQ ID NO: 56)
Peptide (SEQ ID NO: 57)
Peptide (SEQ ID NO: 58)
Peptide (SEQ ID NO: 59)
Peptide (SEQ ID NO: 60)
Peptide (SEQ ID NO: 61)
Peptide (SEQ ID NO: 62)
Peptide (SEQ ID NO: 63)

FIGURE 8

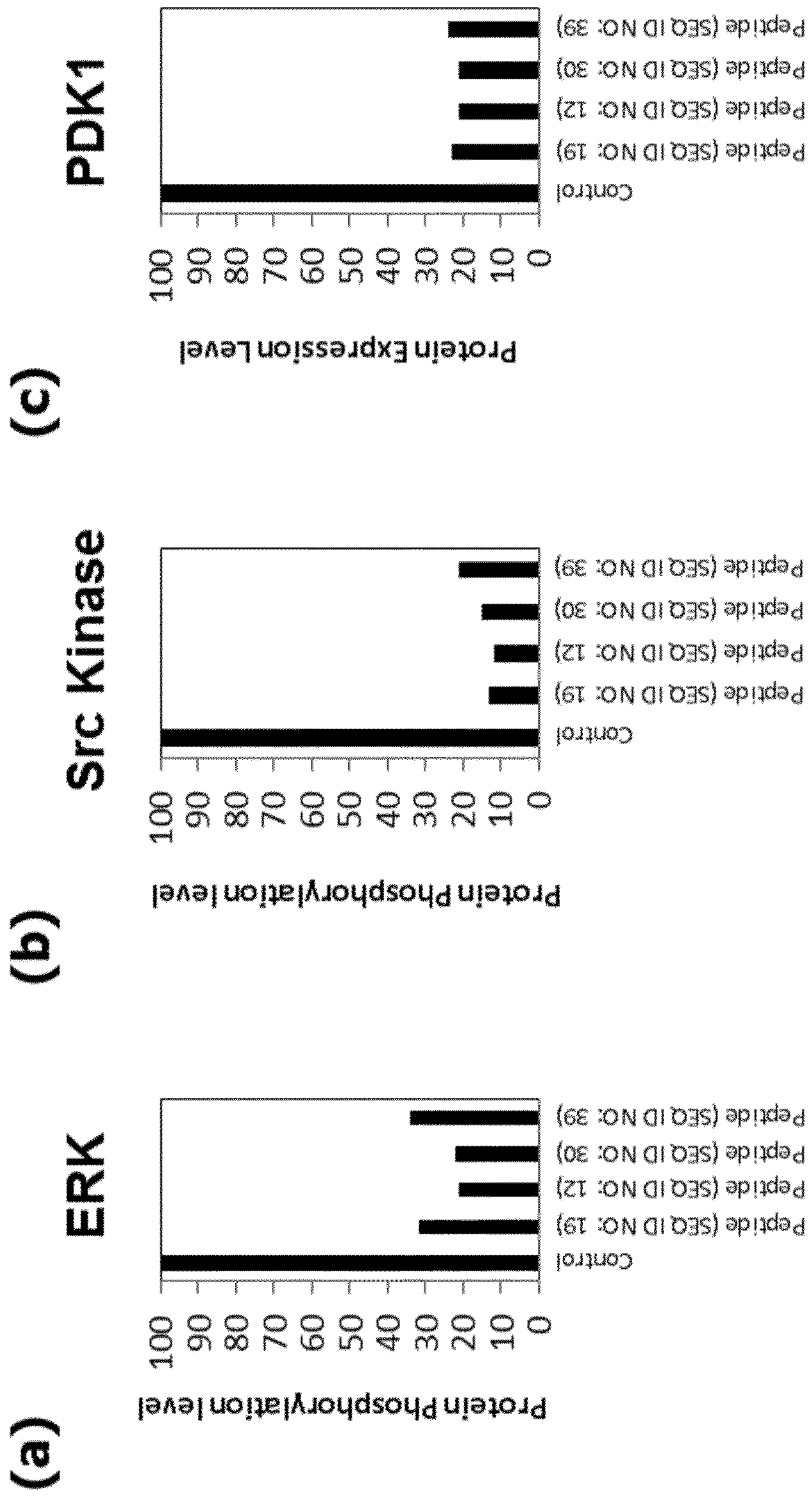
(a)
ERK
Protein Phosphorylation level
0
10
20
30
40
50
60
70
80
90
100
Control
Peptide (SEQ ID NO: 19)
Peptide (SEQ ID NO: 12)
Peptide (SEQ ID NO: 30)
Peptide (SEQ ID NO: 39)
(b)
Src Kinase
Protein Phosphorylation level
0
10
20
30
40
50
60
70
80
90
100
Control
Peptide (SEQ ID NO: 19)
Peptide (SEQ ID NO: 12)
Peptide (SEQ ID NO: 30)
Peptide (SEQ ID NO: 39)
(c)
PDK1
Protein Expression Level
0
10
20
30
40
50
60
70
80
90
100
Control
Peptide (SEQ ID NO: 19)
Peptide (SEQ ID NO: 12)
Peptide (SEQ ID NO: 30)
Peptide (SEQ ID NO: 39)

FIGURE 9

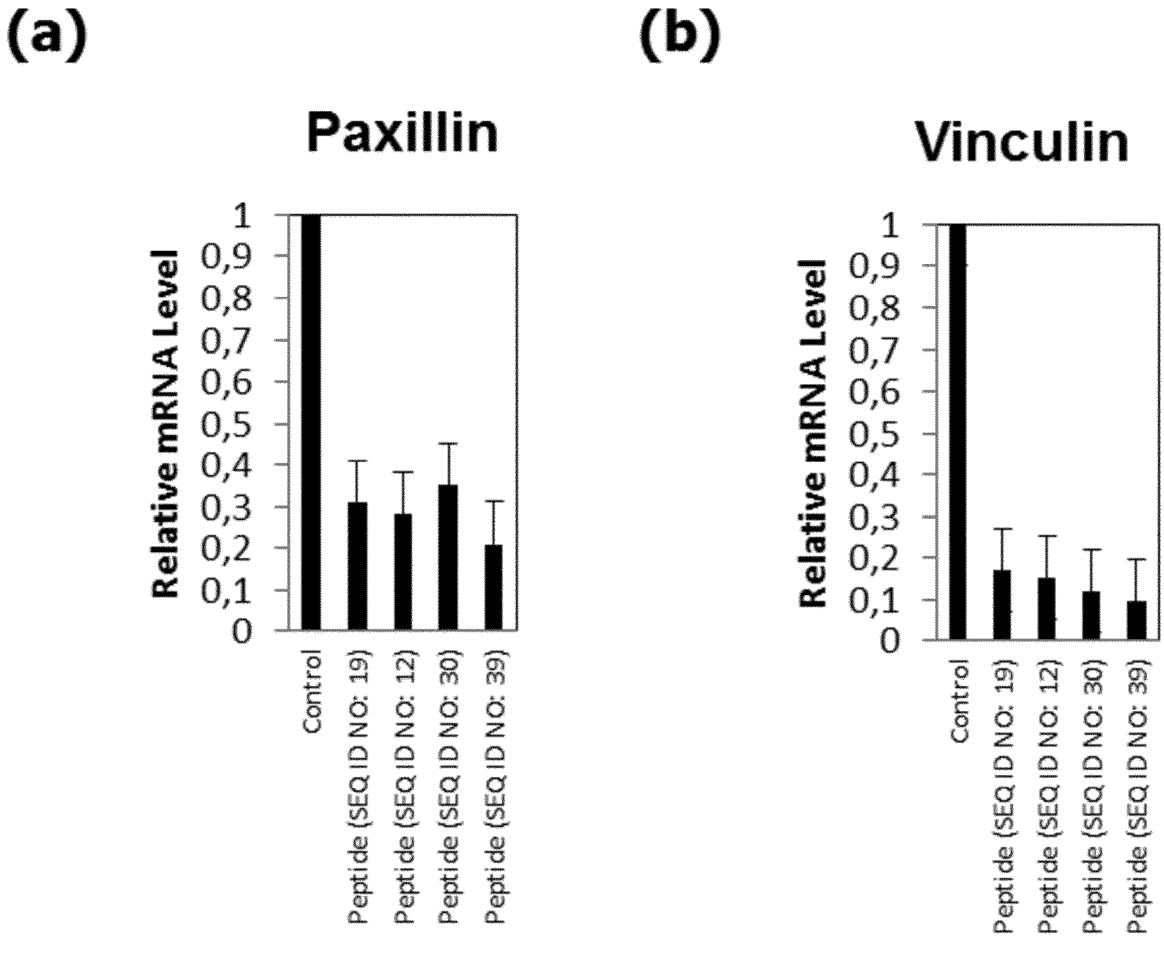
(a)
Paxillin
Relative mRNA Level
1
0,9
0,8
0,7
0,6
0,5
0,4
0,3
0,2
0,1
0
Control
Peptide (SEQ ID NO: 19)
Peptide (SEQ ID NO: 12)
Peptide (SEQ ID NO: 30)
Peptide (SEQ ID NO: 39)
(b)
Vinculin
Relative mRNA Level
1
0,9
0,8
0,7
0,6
0,5
0,4
0,3
0,2
0,1
0
Control
Peptide (SEQ ID NO: 19)
Peptide (SEQ ID NO: 12)
Peptide (SEQ ID NO: 30)
Peptide (SEQ ID NO: 39)

FIGURE 10

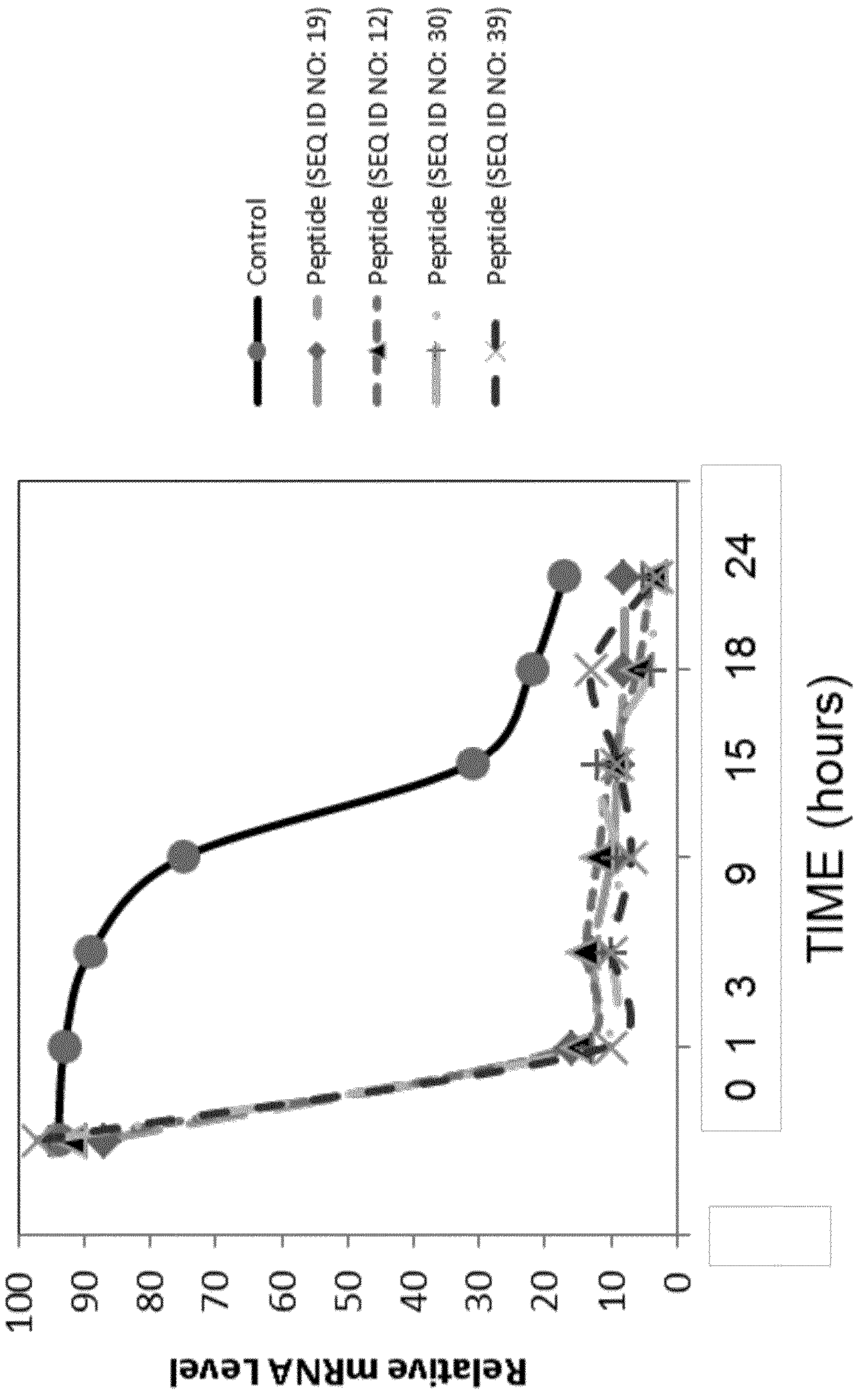
Control
Peptide (SEQ ID NO: 19)
Peptide (SEQ ID NO: 12)
Peptide (SEQ ID NO: 30)
Peptide (SEQ ID NO: 39)
Relative mRNA Level
100
90
80
70
60
50
40
30
20
10
0
0 1 3 9 15 18 24
TIME (hours)

FIGURE 11

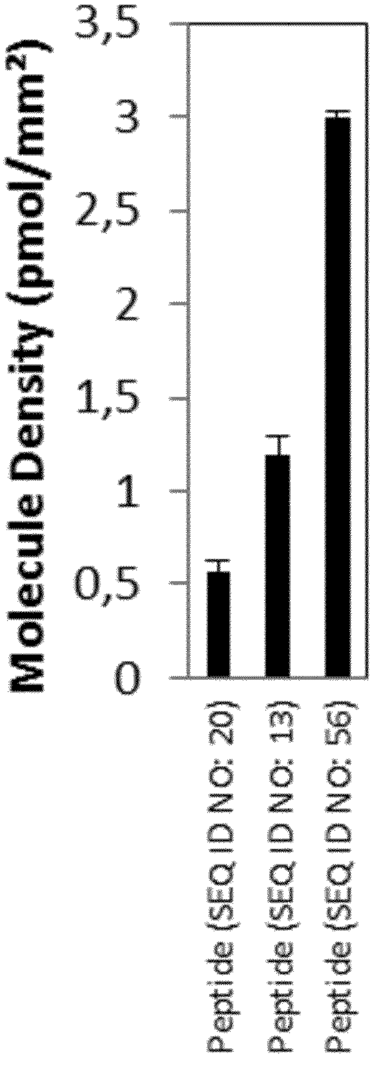
Molecule Density (pmol/mm²)
3,5
3
2,5
2
1,5
1
0,5
0
Peptide (SEQ ID NO: 20)
Peptide (SEQ ID NO: 13)
Peptide (SEQ ID NO: 56)

FIGURE 12

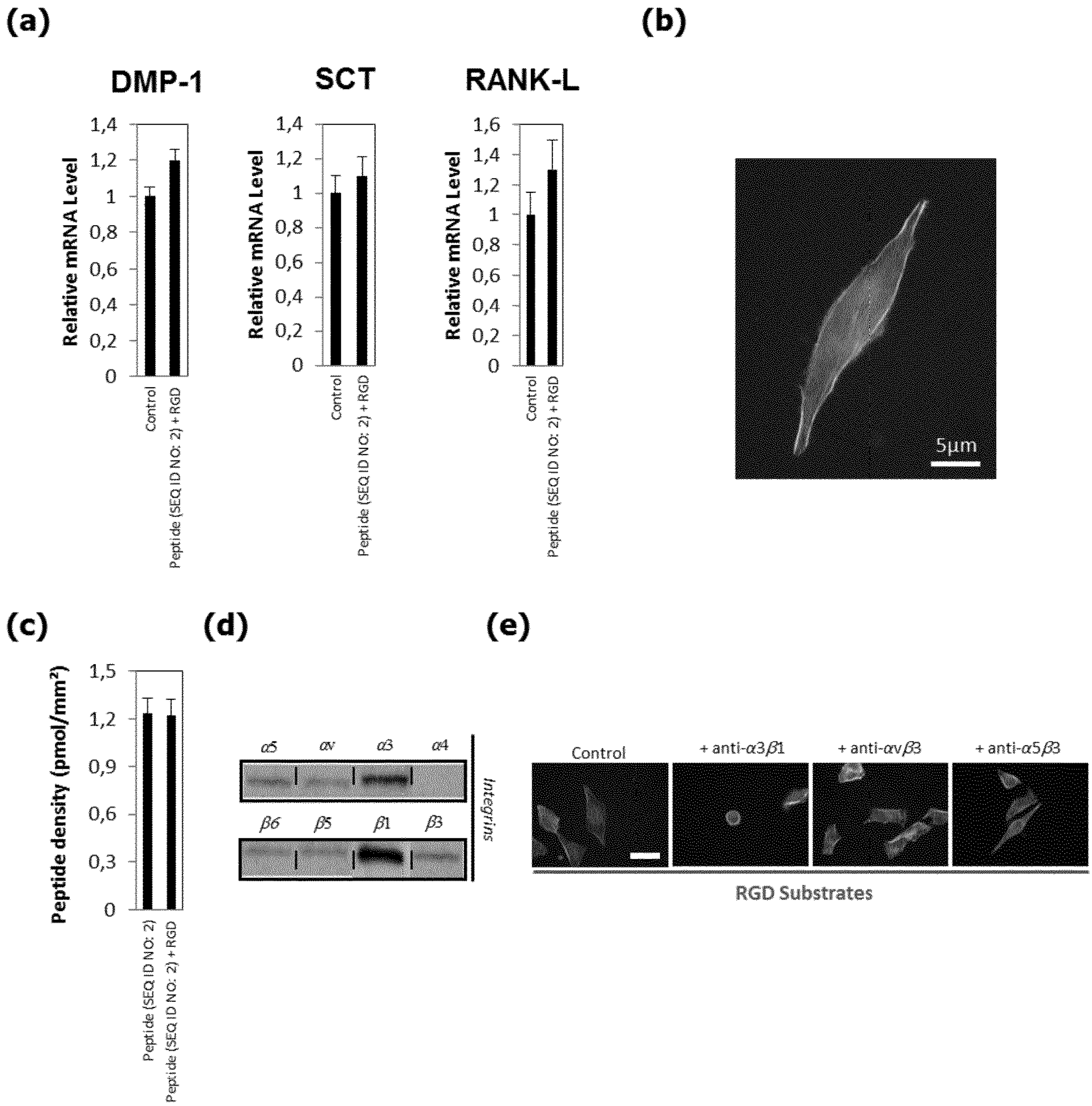
(a)
(b)
DMP-1
SCT
RANK-L
Relative mRNA Level
Control
Peptide (SEQ ID NO: 2) + RGD
5µm
(c)
(d)
(e)
Peptide density (pmol/mm²)
Peptide (SEQ ID NO: 2)
Peptide (SEQ ID NO: 2) + RGD
α5
αv
α3
α4
β6
β5
β1
β3
Integrins
Control
+ anti-α3β1
+ anti-αvβ3
+ anti-α5β3
RGD Substrates

FIGURE 13

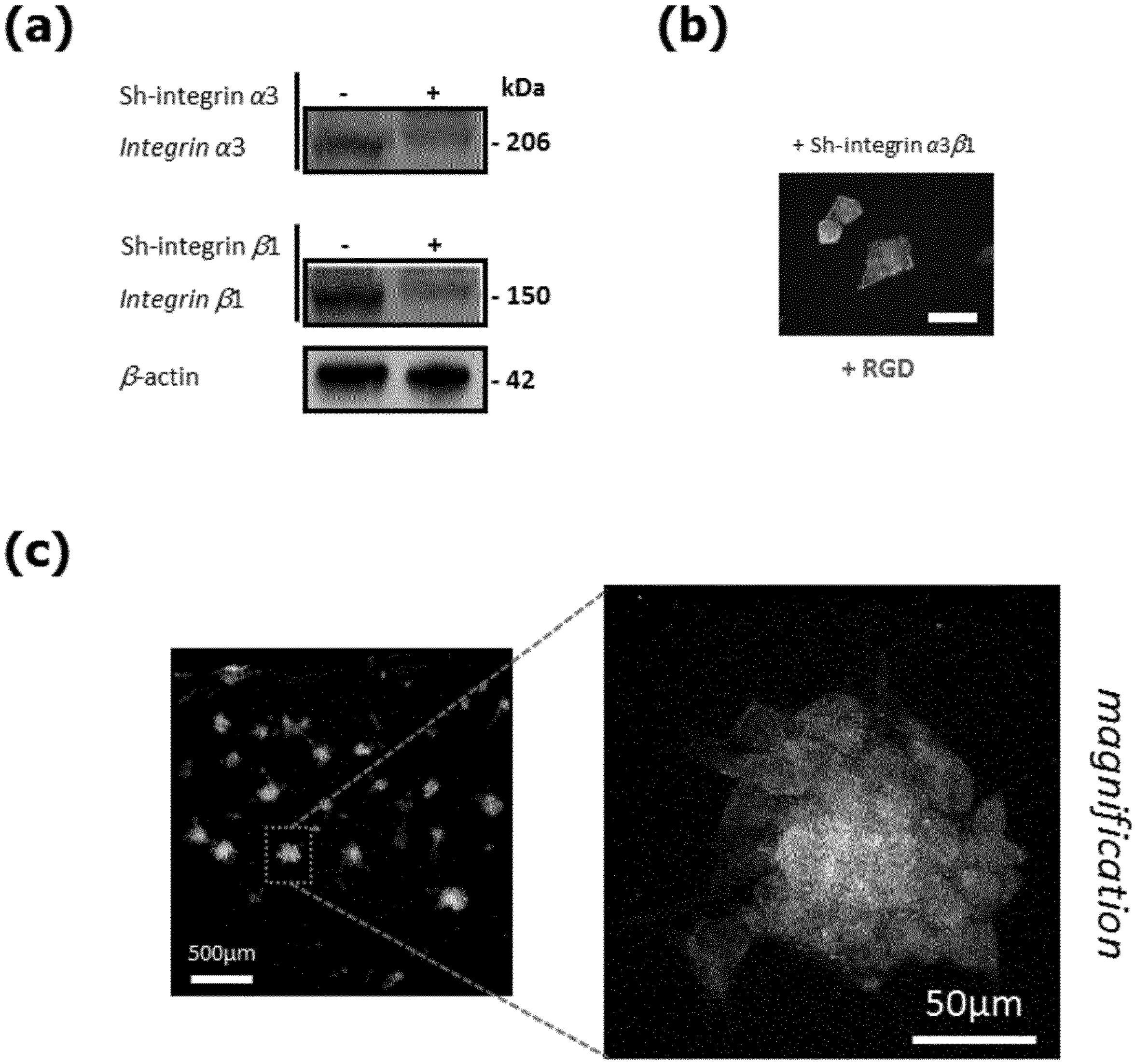
(a)
Sh-integrin α3
-
+
kDa
Integrin α3
- 206
Sh-integrin β1
-
+
Integrin β1
- 150
β-actin
- 42
(b)
+ Sh-integrin α3β1
+ RGD
(c)
500μm
50μm
magnification

FIGURE 14

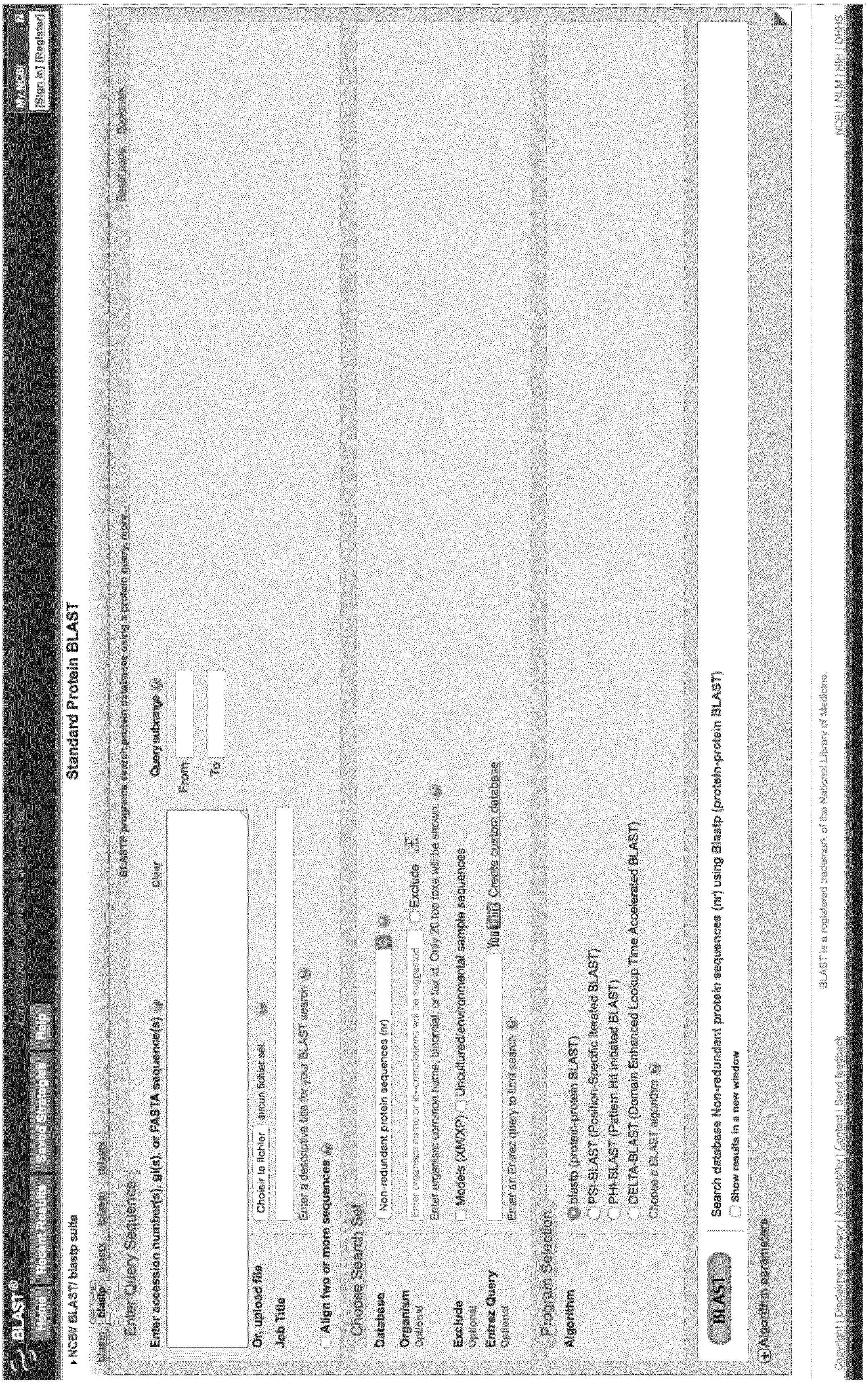
BLAST®
Basic Local Alignment Search Tool
Home
Recent Results
Saved Strategies
Help
My NCBI
[Sign In] [Register]
NCBI/ BLAST/ blastp suite
Standard Protein BLAST
blastn
blastp
blastx
tblastn
tblastx
Reset page
Bookmark
BLASTP programs search protein databases using a protein query. more...
Enter Query Sequence
Enter accession number(s), gi(s), or FASTA sequence(s)
Clear
Query subrange
From
To
Or, upload file
Choisir le fichier
aucun fichier sél.
Job Title
Enter a descriptive title for your BLAST search
Align two or more sequences
Choose Search Set
Database
Non-redundant protein sequences (nr)
Organism
Optional
Enter organism name or id--completions will be suggested
Exclude
Enter organism common name, binomial, or tax id. Only 20 top taxa will be shown.
Exclude
Optional
Models (XM/XP)
Uncultured/environmental sample sequences
Entrez Query
Optional
Create custom database
Enter an Entrez query to limit search
Program Selection
Algorithm
blastp (protein-protein BLAST)
PSI-BLAST (Position-Specific Iterated BLAST)
PHI-BLAST (Pattern Hit Initiated BLAST)
DELTA-BLAST (Domain Enhanced Lookup Time Accelerated BLAST)
Choose a BLAST algorithm
BLAST
Search database Non-redundant protein sequences (nr) using Blastp (protein-protein BLAST)
Show results in a new window
Algorithm parameters
BLAST is a registered trademark of the National Library of Medicine.
Copyright | Disclaimer | Privacy | Accessibility | Contact | Send feedback
NCBI | NLM | NIH | DHHS

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007010394 A2
- US 2004023322 A1
- US 2003185792 A1
- WO 9118098 A1
- US 6475516 B
- US 20080268015 A1
- US 6818620 B2
- US 20020159986 A1


### Non-patent literature cited in the description


- **ZOUANI et al.** *Biomaterials*, vol. 31 (32), 8245-8253
- **GEIGER et al.** *Advanced Drug Delivery Reviews*, vol. 55 (12), 1613-1629
- **IDE et al.** *Cancer Research*, vol. 57 (22), 5022-5027
- **LIAO et al.** *Tumor Biology*, vol. 36 (4), 2773-2778
- **PARK et al.** *Anti-Cancer Drugs*, vol. 24 (3), 278-290
- **GREENE**. Protective Groups in Organic Synthesis. Wiley, 2007
- **HOJO H.** Recent progress in the chemical synthesis of proteins. *Curr Opin Struct Biol*, 2014, vol. 26C, 16-23
- **SARANYA CHANDRUDU et al.** Chemical Methods for Peptide and Protein Production. *Molecules*, 2013, vol. 18, 4373-4388
- *Scientific World Journal*, 2013, vol. 2013, 732340
- *Curr Opin Chem Biol.*, 1998, vol. 2 (6), 733-42
- *J Pept Sci.*, 1999, vol. 5 (5), 203-20
- *J Pept Sci.*, 2008, vol. 14 (1), 2-43
- *Protein Sci.*, 2003, vol. 12 (9), 2001-14
- *J. Computat. Chem.*, 2005, vol. 26, 1668-1688
- **QIANG WANG**. SCWRL and MolIDE: computer programs for side-chain conformation prediction and homology modeling. *Nature Protocols*, 2008, vol. 3 (12)
- **HESS B** ; **KUTZNER C** ; **VAN DER SPOEL D** ; **LINDAHL E**. GROMACS 4: Algorithms for Highly Efficient, Load-Balanced, and Scalable Molecular Simulation. *J Chem Theory Comput*, 2008, vol. 4, 2
- **MARK A. LEMMON** ; **JOSEPH SCHLESSINGER**. Cell Signaling by Receptor Tyrosine Kinases. *Cell*, 2010, vol. 141 (7), 1117-1134
- **LIVAK, K. J.** ; **T. D. SCHMITTGEN**. Analysis of relative gene expression data using real-time quantitative PCR and the 2(-Delta Delta C(T)) Method. *Methods*, 2001, vol. 25 (4), 402-408
- **RYAN et al.** *Biomaterials*, 2006, vol. 27, 2651
- **LOPEZ-HEREDIA et al.** *Biomaterials*, 2008, vol. 29, 2608
- **RYAN et al.** *Biomaterials*, 2008, vol. 29, 3625
- **LI et al.** *Biomaterials*, 2007, vol. 28, 2810
- **HOLLANDER et al.** *Biomaterials*, 2006, vol. 27, 955
- **VAN BUUL et al.** *Chem. Sci.*, 2013, vol. 4, 2357-2363
- **NITESH et al.** *International Journal of Emerging Technology and Advanced Engineering*, 2012, vol. 2 (4), ISSN 2250-2459
- **GONG JP et al.** Double-network hydrogels with extremely high mechanical strength. *Adv Mater*, 2003, vol. 15 (14), 1155e8
- L-Asparaginase: A Promising Enzyme for Treatment of Acute Lymphoblastic Leukiemia. *People's Journal of Scientific Research*, January 2012, vol. 5, 1
- **H. FREICHEL et al.** *Macromol. Rapid Commun*, 2011, vol. 32, 616-621
- **V. POURCELLE et al.** *Biomacromol.*, 2009, vol. 10, 966-974
- Remington's Pharmaceutical Sciences. Mack Publishing Company, 1985, 1418
- Pharmaceutical Salts: Properties, Selection, and Use. Wiley-VCH, 2008
- Prodrugs as Novel Delivery Systems. **T. HIGUCHI** ; **W. STELLA**. ACS Symposium Series, and Bioreversible Carriers in Drug Design. Pergamon Press, 1987, vol. 14
- Remington's Pharmaceutical Sciences. Mack Publishing Company, 1995
- The CTFA Cosmetic Ingredient Handbook. 1992
- **WHITE AC** ; **LOWRY WE**. Refining the role for adult stem cells as cancer cells of origin. *Trends Cell Biol.*, 18 September 2014, vol. 14, S0962-8924
- **TANNISHTHA REYA et al.** review article Stem cells, cancer, and cancer stem cells. *Nature*, 01 November 2001, vol. 414, 105-111
- *Mater Res.*, 2004, vol. 7 (4), 625-630

- *Langmuir*, 2011, vol. 27 (22), 13635-42